# EUROPÄISCHE PATENTSCHRIFT

(11) **EP 3 126 353 B1**
(45) Veröffentlichungstag und Bekanntmachung des Hinweises auf die Patenterteilung: **15.08.2018**
(21) Anmeldenummer: 15713883.5
(22) Anmeldetag: 01.04.2015
(51) Int. Cl.: C07D 403/04, C07D 405/04, C07D 409/04, C07D 413/04, C07D 417/04, A01N 43/56, A01N 43/653, A01N 43/74, A01P 5/00, A01P 7/00

(54) **NEUE 3-[(PYRAZOL-5-YL)-HETEROARYL]-BENZAMID DERIVATE UND IHRE VERWENDUNG ALS SCHÄDLINGSBEKÄMPFUNGSMITTEL**
NEW 3-[(PYRAZOL-5-YL)-HETEROARYL]-BENZAMIDE DERIVATIVES AND THEIR USE AS PESTICIDES
NOUVEAUX DÉRIVÉS DE 3-[(PYRAZOL-5-YL)-HÉTÉROARYL]-BENZAMIDE ET LEUR UTILISATION EN TANT QUE PESTICIDES

(30) Priorität: 02.04.2014 EP 14163157
(43) Veröffentlichungstag der Anmeldung: 08.02.2017
(73) Patentinhaber: Bayer CropScience Aktiengesellschaft, 40789 Monheim am Rhein (DE)
(72) Erfinder: MAUE, Michael, 40764 Langenfeld (DE); DECOR, Anne, 40764 Langenfeld (DE); BRETSCHNEIDER, Thomas, 53797 Lohmar (DE); HAHN, Julia Johanna, 40597 Düsseldorf (DE); HALLENBACH, Werner, 40789 Monheim (DE); FISCHER, Reiner, 40789 Monheim (DE); SCHWARZ, Hans-Georg, 46282 Dorsten (DE); GÖRGENS, Ulrich, 40882 Ratingen (DE); ILG, Kerstin, 50670 Köln (DE); RAMING, Klaus, 51375 Leverkusen (DE); KÖBBERLING, Johannes, 41466 Neuss (DE); HÜBSCH, Walter, 42113 Wuppertal (DE); TURBERG, Andreas, 42781 Haan (DE)
(74) Vertreter: BIP Patents
(86) Internationale Anmeldenummer: PCT/EP2015/057155
(87) Internationale Veröffentlichungsnummer: WO 2015/150442

(56) Entgegenhaltungen:
- WO-A1-2012/107434
- WO-A2-2010/051926

## Beschreibung

Die vorliegende Anmeldung betrifft neue Halogen-substituierte Verbindungen, Verfahren zu ihrer Herstellung und ihre Verwendung zur Bekämpfung von tierischen Schädlingen, vor allem von Arthropoden und insbesondere von Insekten, Spinnentieren und Nematoden.

Es ist bekannt das ähnliche Verbindungen als FAAH Inhibitoren [WO2009-152025], LXR Modulatoren [WO2007-002559] und ATP Bindungskassetten Transporter Modulatoren [WO2004-080972] WO2012107434 offenbart substituierte Triazole als Insektizide. Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften. Aufgabe der vorliegenden Erfindung war es, Verbindungen bereitzustellen, durch die das Spektrum der Schädlingsbekämpfungsmittel unter verschiedenen Aspekten verbreitert und/oder ihre Aktivität verbessert wird.

Es wurde nun überraschenderweise gefunden, dass bestimmte Halogen-substituierte Verbindungen, sowie deren *N*-Oxide und Salze biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

Die erfindungsgemäßen Halogen-substituierten Verbindungen sind durch die allgemeine Formel (I) 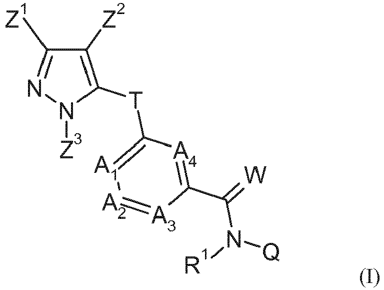 definiert, in denen
- R¹: für Wasserstoff, gegebenenfalls substituiertes Alkyl, Alkenyl, Alkinyl, Cycloalkyl, CycloalkylalkylAlkylcarbonyl, Alkoxycarbonyl, Arylalkyl, Heteroarylalkyl,
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, gegebenenfalls substituiertes Alkyl, Cycloalkyl, Alkoxy, N-Alkoxy-imino-alkyl, Alkylsulfanyl, Alkylsulfinyl, Alkylsulfonyl, *N*-Alkylamino oder *N*,*N*-Dialkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
- W: für Sauerstoff oder Schwefel steht;
- Q: für Wasserstoff, Formyl, Hydroxy, Amino oder eine der gegebenenfalls substituierten Gruppierungen Alkyl, Alkyloxy, Alkenyl, Alkinyl, Cycloalkyl, Heterocycloalkyl, Cycloalkylalkyl, Heterocycloalkylalkyl, Arylalkyl, Heteroarylalkyl oder für eine Gruppierung *N*-Alkylamino, *N*-Alkylcarbonylamino, *N,N*-Dialkylamino steht; oder
- Q: für einen gegebenenfalls einfach bis mehrfach mit V substituierten, einfach bis mehrfach ungesättigten 5- bis 6-gliedrigen Carbozyklus steht, der gegebenenfalls durch Heteroatome unterbrochen sein kann, wobei
- V: für Halogen, Cyano, Nitro, gegebenenfalls substituiertes Alkyl, Alkenyl, Alkinyl, Cycloalkyl, Alkoxy, *N*-Alkoxyiminoalkyl, Alkylsulfanyl, Alkylsulfinyl, Alkylsulfonyl, *N,N*-Dialkylamino steht
- T: für einen der nachfolgend aufgeführten 5 gliedrigen Heteroaromaten T1-T35 steht, wobei die Bindung zur Pyrazolkopfgruppe mit einem Sternchen gekennzeichnet ist, 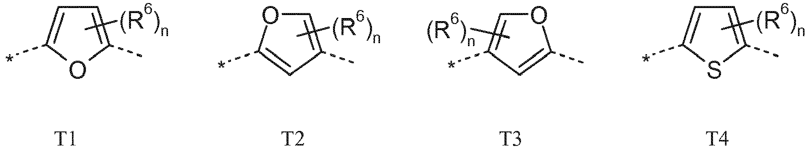 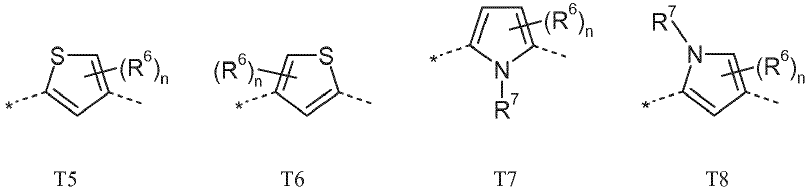 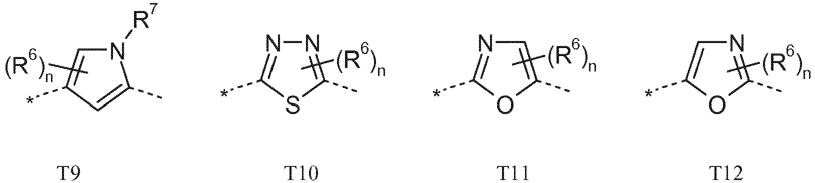 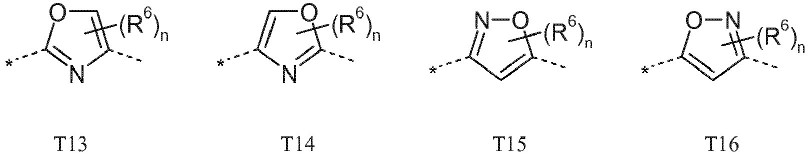 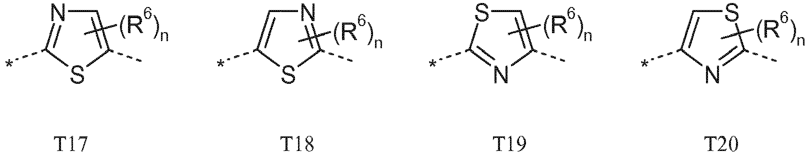 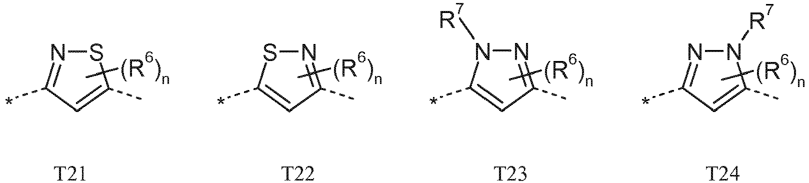 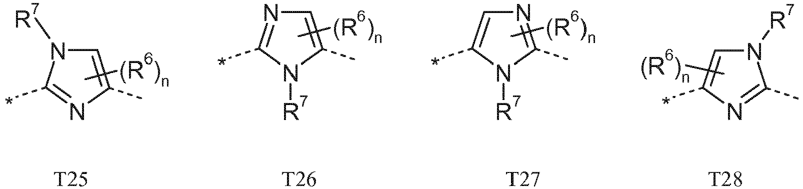 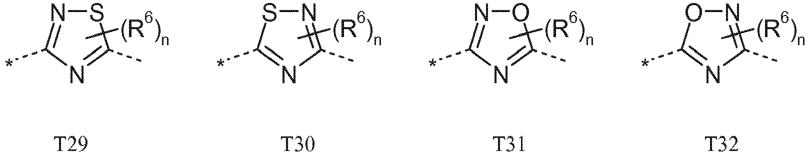 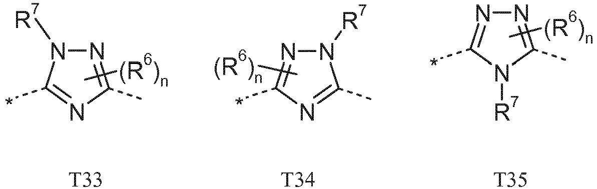
wobei
- R⁶: unabhängig voneinander für Halogen, Cyano, Nitro, Amino oder ein gegebenenfalls substituiertes Alkyl, Alkyloxy, Alkylcarbonyl, Alkylsulfanyl, Alkylsulfinyl, Alkylsulfonyl, und
- n: für die Werte 0-2 stehen;
- R⁷: für Wasserstoff, oder ein gegebenenfalls substituiertes Alkyl oder Cycloalkyl, in welchem ggf. eine Methylengruppe durch ein Heteroatom substituiert ist, steht;
- Z¹: für ein gegebenenfalls substituiertes Halogenalkyl oder Halogencycloalkyl, und
- Z²: für Wasserstoff, Halogen, Cyano, Nitro, Amino oder ein gegebenenfalls substituiertes Alkyl, Alkylcarbonyl, Alkylsulfanyl, Alkylsulfinyl, Alkylsulfonyl, und
- Z³: für Wasserstoff oder ein gegebenenfalls substituiertes Alkyl, Cycloalkyl, Heterocycloalkyl, Alkenyl, Alkinyl stehen;, wobei
für den Fall, dass T gleich T23 oder T24 ist, einer der Reste Z¹, Z² oder Z³ mit mindestens 3 Halogenatomen substituiert ist.

Bevorzugt sind Verbindungen der Formel (I) 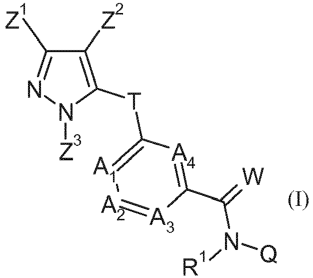 definiert, in denen
- R¹: für Wasserstoff, gegebenenfalls substituiertes C₁-C₆-Alkyl, C₃-C₆-Alkenyl, C₃-C₆-Alkinyl, C₃-C₇-Cycloalkyl, C₃-C₇-Cycloalkyl-C₁-C₃-alkyl, C₁-C₆-Alkylcarbonyl, C₁-C₆-Alkoxycarbonyl, Aryl(C₁-C₃)-alkyl, Heteroaryl(C₁-C₃)-alkyl,
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆-alkylamino, stehen, oder wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
- W: für Sauerstoff oder Schwefel steht;
- Q: für Wasserstoff, Formyl, Hydroxy, Amino oder eine der gegebenenfalls substituierten Gruppierungen C₁-C₆-Alkyl, C₃-C₆-Alkenyl, C₃-C₆-Alkinyl, C₃-C₆-Cycloalkyl, C₁-C₅-Heterocycloalkyl, C₁-C₄-Alkoxy, C₃-C₆-Cycloalkyl-C₁-C₆-alkyl, Aryl-(C₁-C₃)-alkyl, Heteroaryl-(C₁-C₃)-alkyl oder für eine Gruppierung *N*-C₁-C₄-Alkylamino, *N*-C₁-C₄-Alkylcarbonylamino, *N,N-*Di-C₁-C₄-alkylamino steht; oder
- Q: für einen gegebenenfalls einfach bis mehrfach mit V substituierten, einfach bis dreifach ungesättigten 5 bis 6-gliedrigen Carbozyklus steht, oder für einen gegebenenfalls mehrfach mit V substituierten, einfach bis dreifach ungesättigten 5- bzw. 6-gliedrigen, heterozyklischen Ring steht, wobei
- V: unabhängig voneinander für Halogen, Cyano, Nitro, gegebenenfalls substituiertes C₁-C₆-Alkyl, C₁-C₄-Alkenyl, C₁-C₄-Alkinyl, C₃-C₆-Cycloalkyl, C₁-C₆-Alkoxy, *N*-C₁-C₆₋Alkoxy-imino-C₁-C₃-alkyl, C₁-C₆-Alkylsulfanyl, C₁-C₆-_{A}lkylsulfinyl, C₁-C₆-Alkylsulfonyl, *N,N*-Di-(C₁-C₆-alkyl)amino steht;
- T: für einen der nachfolgend aufgeführten 5 gliedrigen Heteroaromaten T1-T35 steht, wobei die Bindung zur Pyrazolkopfgruppe mit einem Sternchen gekennzeichnet ist, 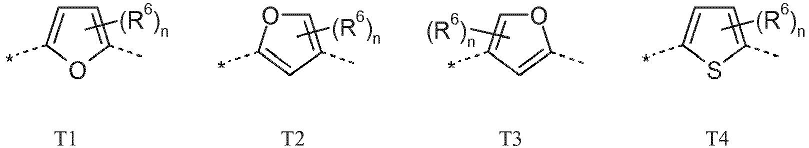 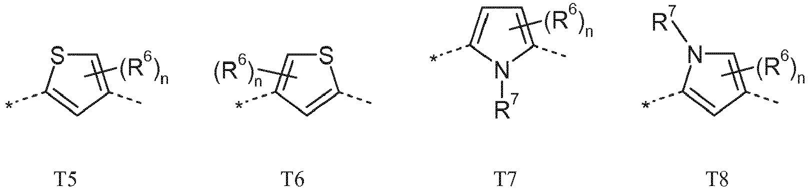 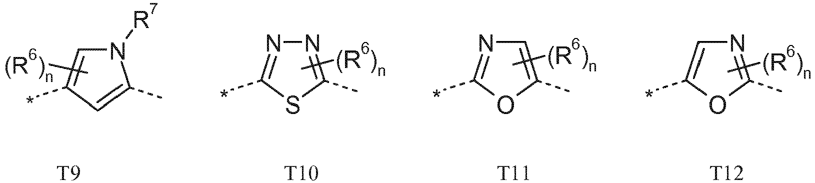 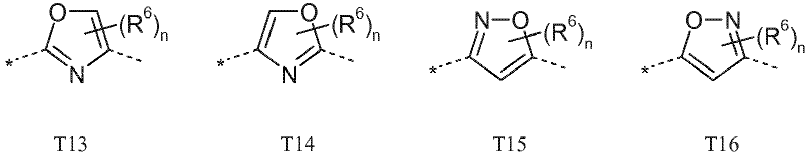 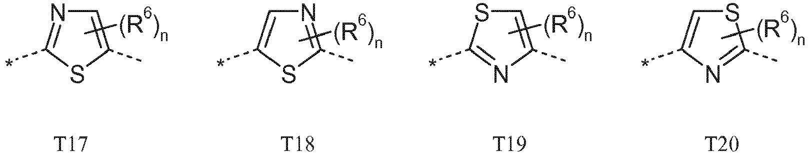 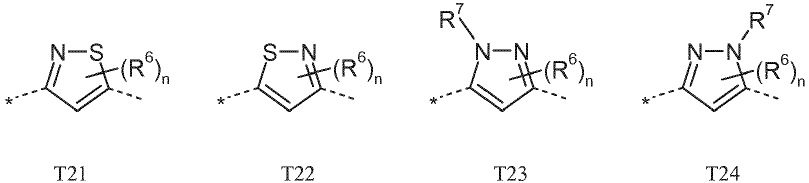 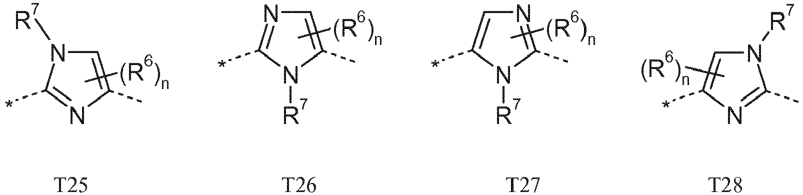 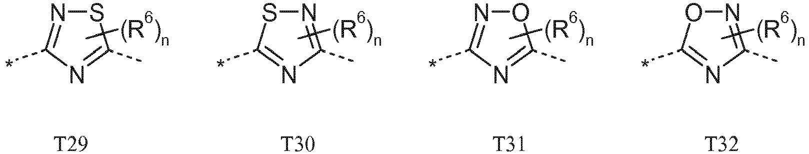 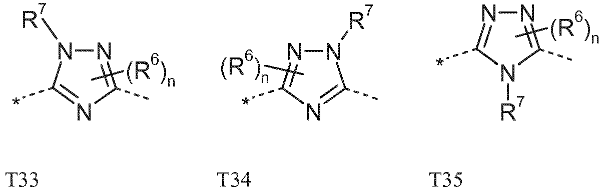
wobei
- R⁶: unabhängig voneinander für Halogen, Cyano, Nitro, Amino oder ein gegebenenfalls mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-_{A}lkylsulfonyl, und
- n: für die Werte 0-1 stehen;
- R⁷: für Wasserstoff, oder ein gegebenenfalls substituiertes C₁-C₆-Alkyl oder C₃-C₆-Cycloalkyl, in welchem ggf. eine Methylengruppe durch Heteroatome substituiert sein kann, steht;
- Z¹: für ein gegebenenfalls substituiertes C₁-C₆-Halogenalkyl, C₃-C₆-Halogencycloalkyl, und
- Z²: für Wasserstoff Halogen, Cyano, Nitro, Amino oder ein gegebenenfalls substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
- Z³: für Wasserstoff oder ein gegebenenfalls substituiertes C₁-C₆-Alkyl, C₃-C₆-Cycloalkyl, C₃-C₅-Heterocycloalkyl, C₃-C₆-Alkenyl, C₃-C₆-Alkinyl, stehen,
wobei für den Fall, dass T gleich T23 oder T24 ist, einer der Reste Z¹, Z² oder Z³ mit mindestens 3 Halogenatomen substituiert ist.

Besonders bevorzugt sind Verbindungen der Formel (I) 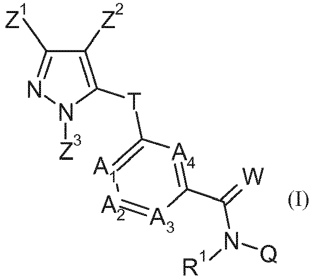 definiert, in denen
- R¹: für Wasserstoff, gegebenenfalls ein- bis fünffach unabhängig voneinander mit Fluor, Chlor, Cyano, Alkoxy und Alkoxycarbonyl substituiertes C₁-C₆-Alkyl, C₃-C₆-Alkenyl, C₃-C₆-Alkinyl, C₃-C₇-Cycloalkyl, C₃-C₇-Cycloalkyl-C₁-C₃-alkyl,C₁-C₆-Alkylcarbonyl, C₁-C₆-Alkoxycarbonyl, Aryl(C₁-C₃)-alkyl, Heteroaryl(C₁-C₃)-alkyl,
die chemischen Gruppierungen
- A₁: für CR² oder Stickstoff,
- A₂: für CR³ oder Stickstoff,
- A₃: für CR⁴ oder Stickstoff, und
- A₄: für CR³ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Fluor, Chlor, Brom, Iod, Cyano, Nitro, gegebenenfalls durch einfach bis fünffach mit Fluor, Chlor, Cyano oder C₁-C₄-Alkoxy substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N-*Di-C₁-C₆-alkylamino stehen;
- W: für Sauerstoff oder Schwefel steht;
- Q: für Wasserstoff, Hydroxy, Formyl, Amino oder eine der gegebenenfalls unabhängig voneinander ein- oder mehrfach mit Hydoxy, Nitro, Amino, Fluor, Chlor, Brom, Iod, C₁-C₄-Alkoxy, Cyano, Hydroxycarbonyl. C₁-C₄-Alkoxycarbonyl, Carbamoyl, Thiocarbamoyl, C₁-C₄-Alkylcarbamoyl, C₃-C₆-Cycloalkylcarbamoyl, Phenyl substituierten Gruppierungen C₁-C₆-Alkyl, C₃-C₆-Alkenyl, C₃-C₆-Alkinyl, C₃-C₆-Cycloalkyl, C₂-C₆-Heterocycloalkyl, C₁-C₄-Alkoxy, C₁-C₆-Alkyl-C₃-C₆-cycloalkyl, C₃-C₆-Cycloalkyl-C₁-C₆-alkyl, C₁-C₆-Hydroxyalkyl, Aryl(C₁-C₃)-alkyl, Heteroaryl(C₁-C₃)-alkyl, *N*-C₁-C₄-Alkylamino, *N*-C₁-C₄-Alkylcarbonylamino oder *N,N*-Di-C₁-C₄-alkylamino steht; oder
- Q: für ein mit 0, 1,2 oder 3 Substituenten V substituiertes Aryl oder für ein mit 0, 1,2 oder 3 Substituenten V substituierten 5 bzw. 6 gliedrigen Heteroaromaten steht, wobei
- V: unabhängig voneinander für Fluor, Chlor, Brom, Iod, Cyano, Nitro, gegebenenfalls substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N*-Di-(C₁-C₆-alkyl)amino steht;
- T: für einen der nachfolgend aufgeführten 5 gliedrigen Heteroaromaten T1-T35 steht, wobei die Bindung zur Pyrazolkopfgruppe mit einem Sternchen gekennzeichnet ist, 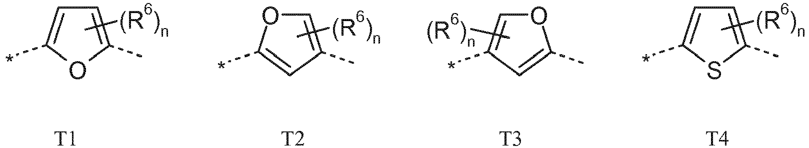 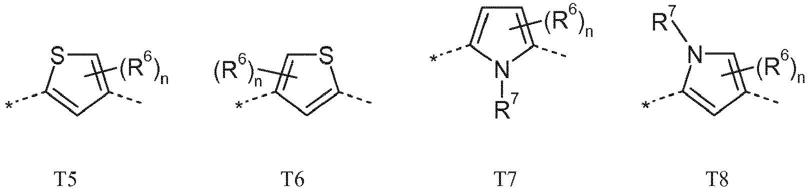 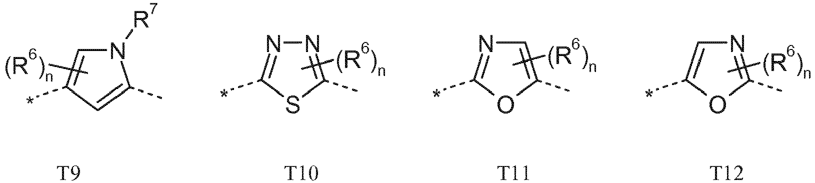 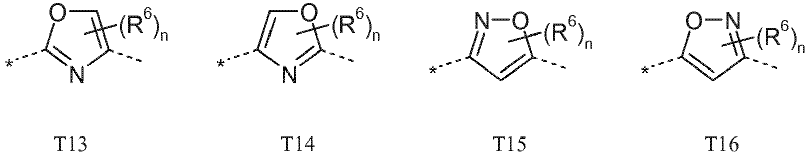 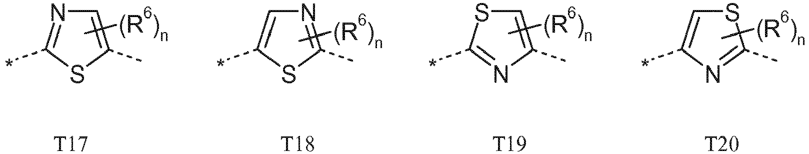 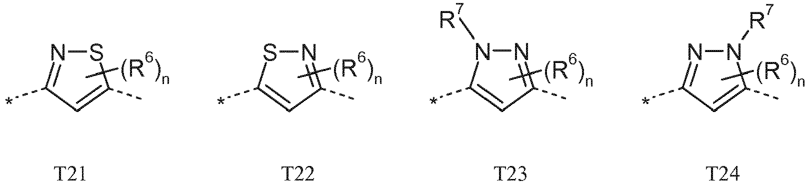 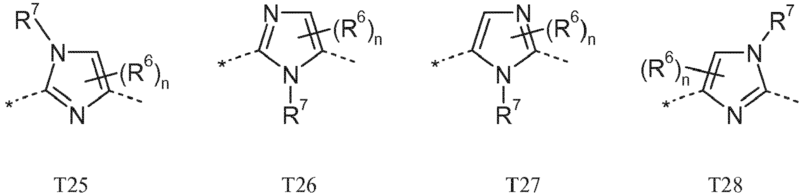 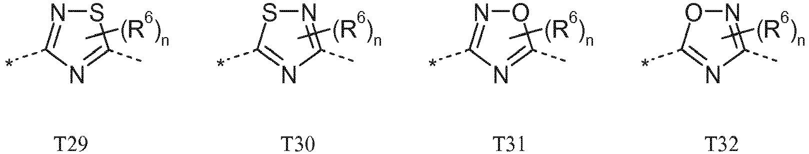 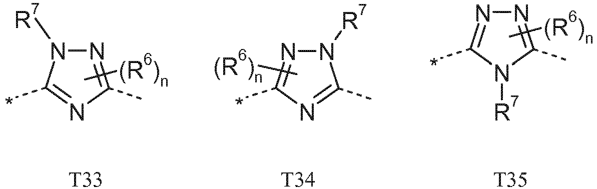
wobei
- R⁶: unabhängig voneinander für Fluor, Chlor, Brom, Iod, Cyano, Nitro, Amino oder ein gegebenenfalls einfach bis fünffach mit Fluor oder Chlor substituiertes C₁-C₆-Alkyl, C₁-C₆-Alkoxy, C₁-C₆-Alkylcarbonyl, C₁-C₆-Alkylsulfanyl, C₁-C₆-_{A}lkylsulfinyl, C₁-C₆-Alkylsulfonyl, und
- n: für die Werte 0-1 stehen;
- R⁷: für Wasserstoff, oder ein gegebenenfalls einfach bis fünffach mit Fluor, Chlor, Cyano oder C₁-C₄-Alkoxy substituiertes C₁-C₆-Alkyl oder C₃-C₆-Cycloalkyl, in welchem ggf. eine Methylengruppe durch Heteroatome substituiert sein kann, steht; Z¹ für ein gegebenenfalls substituiertes C₁-C₆-Haloalkyl, C₃-C₆-Halocycloalkyl, und
- Z²: für Wasserstoff, Halogen, Cyano, Nitro, Amino oder ein gegebenenfalls einfach bis fünffach substituiertes C₁-C₆-Alkyl, C₁-C₆-Alkylcarbonyl, C₁-C₆-Alkylsulfanyl, C₁-C₆-_{A}lkylsulfinyl, C₁-C₆-Alkylsulfonyl, und
- Z³: für Wasserstoff oder ein gegebenenfalls einfach bis fünffach mit Fluor, Chlor, Cyano oder C₁-C₄-Alkoxy substituiertes C₁-C₆-Alkyl, C₃-C₆-Cycloalkyl, C₁-C₄-Alkenyl, C₁-C₄-Alkinyl, stehen;
wobei für den Fall, dass T gleich T23 oder T24 ist, einer der Reste Z¹, Z² oder Z³ mit mindestens 3 Halogenatomen substituiert ist.

Ganz besonders bevorzugt sind Verbindungen der Formel (I) 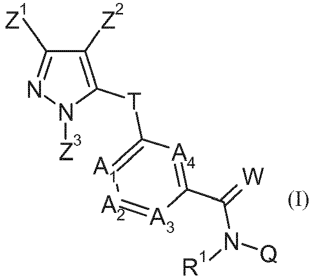 definiert, in denen
- R¹: für Wasserstoff, Methyl, Ethyl, n-Propyl, Isopropyl, n-Butyl, Isobutyl, s-Butyl, t-Butyl, 2,2,-Difluorethyl, 2,2,2-Trifluorethyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht;
die chemischen Gruppierungen
- A₁: für CR² oder Stickstoff,
- A₂: für CR³ oder Stickstoff,
- A₃: für CR⁴ oder Stickstoff, und
- A₄: für CR³ oder Stickstoff stehen,
wobei aber nicht mehr als drei der chemischen Gruppierungen A₁ bis A₄ gleichzeitig für Stickstoff stehen;
R² und R⁵ unabhängig voneinander für Wasserstoff, Methyl, Fluor und Chlor stehen und
R³ und R⁴ unabhängig voneinander für Wasserstoff, Fluor, Chlor, Brom, Iod, Cyano, Nitro, Methyl, Ethyl, Cyclopropyl, Fluormethyl, Difluormethyl, Chlordifluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl stehen
- W: für Sauerstoff oder Schwefel steht;
- Q: für für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxyethyl, 2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Methyl-cyclopropyl, 1-Trifluormethyl-cyclopropyl, 1-Carbamoyl-cyclopropyl, 1-Thiocarbamoyl-cyclopropyl, 1-Methoxycarbonyl-cyclopropyl, 1-(*N-*Methylcarbamoyl)cyclopropyl, 1-(*N*-Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 4-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N-*Dimethylamino, *N,N*-Diethylamino steht; oder
- Q: für ein mit 0, 1, 2 oder 3 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino steht;
- T: für einen der nachfolgend aufgeführten 5 gliedrigen Heteroaromaten T1-T35 steht, wobei die Bindung zur Pyrazolkopfgruppe mit einem Sternchen gekennzeichnet ist, 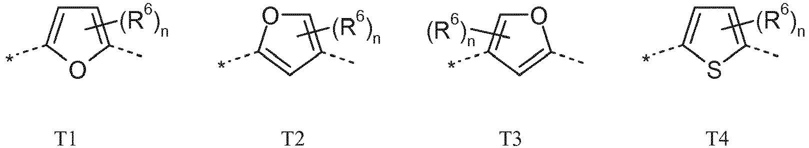 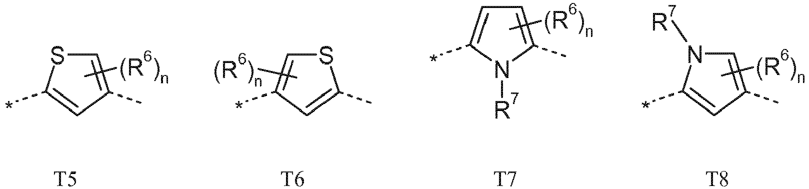 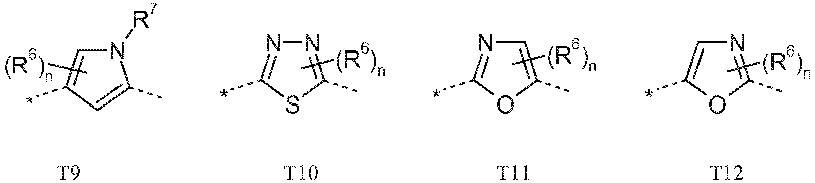 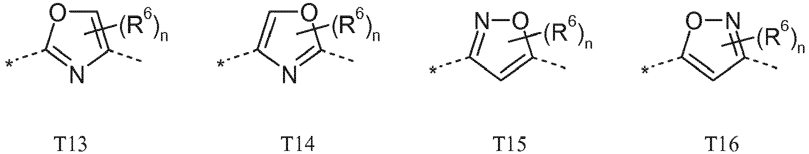 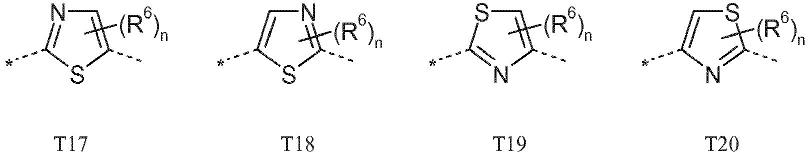 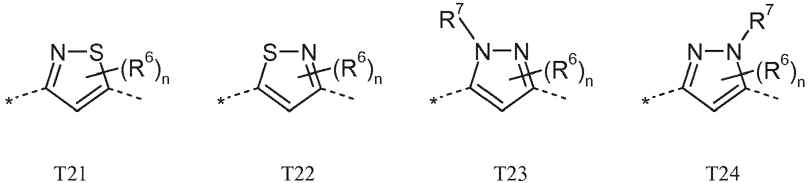 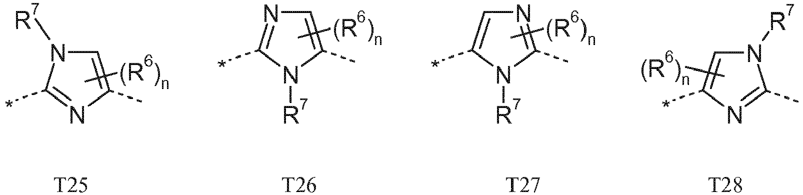 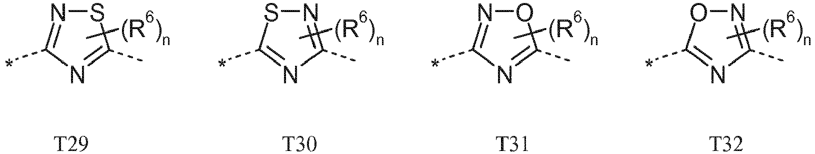 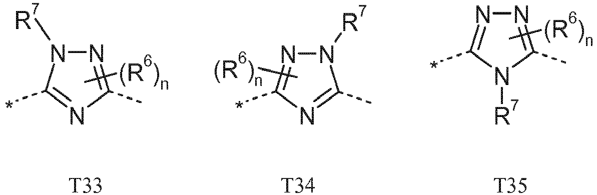
wobei
- R⁶: unabhängig voneinander für Fluor, Chlor, Brom, Iod, Cyano, Nitro, Amino, Methyl, Ethyl, n-Propyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl, und
- n: für die Werte 0-1 stehen;
- R⁷: für Wasserstoff, Methyl, Ethyl, n-Propyl, Isopropyl, n-Butyl, Isobutyl, s-Butyl, t-Butyl, Ethenyl, 1-Propenyl, 2-Propenyl, 1-Propinyl, 1-Butinyl, Difluormethyl, Trifluormethyl, 1-Fluorethyl, 1-Fluor-1-methylethyl, 2-Fluorethyl, 2,2-Difluorethyl, 2,2,2-Trifluorethyl, Cyclopropyl, Cyclobutyl, Cyclopentyl, Cyclohexyl stehen;
- Z¹: für Difluormethyl, Trichlormethyl, Chlordifluormethyl, Dichlorfluormethyl, Trifluormethyl, Bromdichlormethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Heptafluor-n-propyl, Heptafluor-isopropyl, Nonafluor-n-butyl, 1-Chlorcyclopropyl, 1-Fluorcyclopropyl, 1-Bromcyclopropyl, 1-Trifluormehtyl-cyclopropyl, und 2,2-Difluor-1-methyl-cyclopropyl, und
- Z²: für Wasserstoff, Fluor, Chlor, Brom, Iod, Cyano, Nitro, Amino, Methyl, Ethyl, n-Propyl, 1-Methylethyl, 1,1-Dimethylethyl, Difluormethyl, Trichlormethyl, Chlordifluormethyl, Dichlorfluormethyl, Trifluormethyl, Bromdichlormethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Methylsulfanyl, Methylsulfinyl, Methylsulfonyl, Ethylsulfanyl, Ethylsulfinyl, Ethylsulfonyl, Trifluormethylsulfanyl, Trifluormethylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl und
- Z³: für Wasserstoff, Methyl, Ethyl, n-Propyl, Isopropyl, n-Butyl, Isobutyl, s-Butyl, t-Butyl, Prop-2-enyl, Prop-2-inyl, But-3-inyl, Difluormethyl, Trifluormethyl, 1-Fluorethyl, 1-Fluor-1-methylethyl, 2-Fluorethyl, 2,2-Difluorethyl, 2,2,2-Trifluorethyl, stehen; wobei für den Fall, dass T gleich T23 oder T24 ist, einer der Reste Z¹, Z² oder Z³ mit mindestens 3 Halogenatomen substituiert ist.

Insbesondere bevorzugt sind Verbindungen der allgemeinen Formel (I), in denen
- Z¹: für Trifluormethyl, 1-Chlor-cyclopropyl, 1-Fluor-cyclopropyl oder Pentafluorethyl steht,
- Z²: für Trifluormethyl, Nitro, Methylsulfanyl, Methylsulfinyl, Methylsulfonyl, Fluor, Chlor, Brom, Cyano oder Iod steht,
- Z³: für Methyl, Ethyl, n-Propyl oder Wasserstoff steht,
- R¹: für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht,
- A¹ und A⁴: für CH stehen und A2 für CH oder N steht
- A₃: für CR⁴ und
- R⁴: für Fluor, Chlor, Brom, Iod oder Methyl steht
- T: für einen der nachfolgend aufgeführten 5 gliedrigen Heteroaromaten T1-T35 steht, wobei die Bindung zur Pyrazolkopfgruppe mit einem Sternchen gekennzeichnet ist, 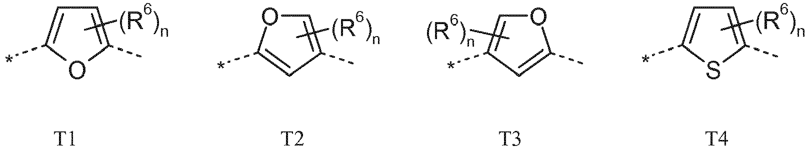 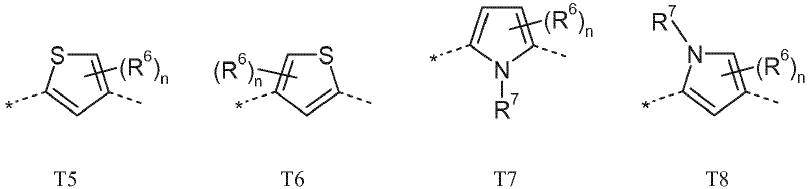 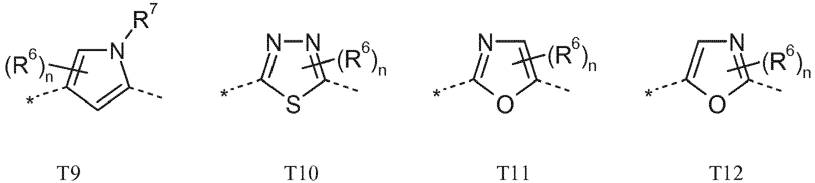 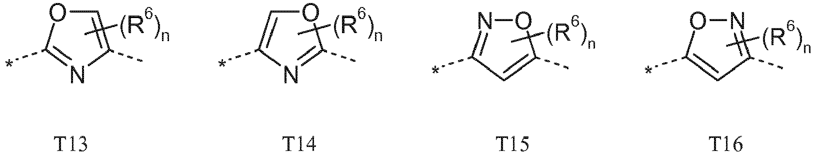 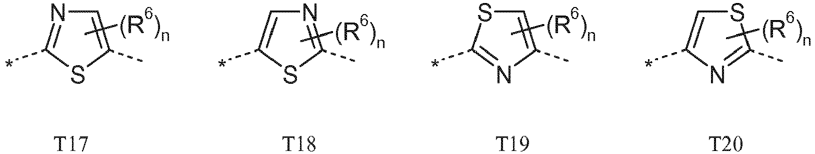 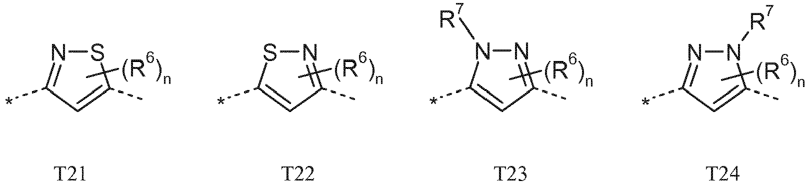 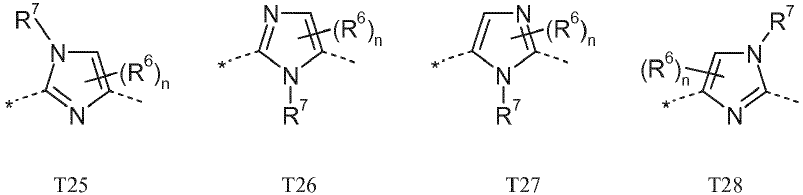 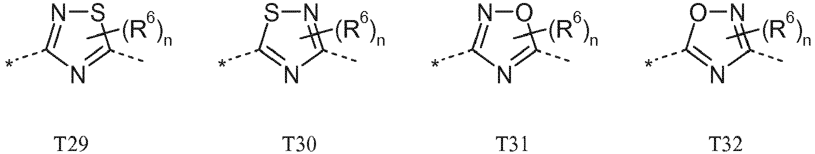 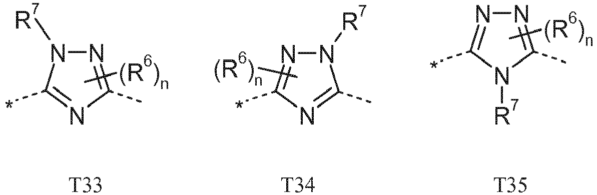
wobei
- R⁶: für Wasserstoff, Methyl, Ethyl, 2-Methylethyl, 2,2-Dimethylethyl, Fluor, Chlor, Brom, Iod, Nitro, Trifluormethyl, Amino, und
- n: für die Werte 0-1 steht;;
- W: für Sauerstoff steht und
- Q: für für Wasserstoff, Methyl, Ethyl, n-Propyl, 1-Methylethyl, 1,1-Dimethylethyl, n-Butyl, 1-Methylpropyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Methyl-cyclopropyl, 1-Trifluormethyl-cyclopropyl, 1-Carbamoyl-cyclopropyl, 1-Thiocarbamoyl-cyclopropyl, 1-Methoxycarbonyl-cyclopropyl, 1-(*N-*Methylcarbamoyl)cyclopropyl, 1-(*N*-Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 4-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, (6-Chlor-pyridin-3-yl)methyl, 5-Fluorpyridin-2-ylmethyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N-*Dimethylamino, *N,N*-Diethylamino steht; oder
- Q: für ein mit 0, 1, 2 oder 3 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino steht;
wobei für den Fall, dass T gleich T23 oder T24 ist, einer der Reste Z¹, Z² oder Z³ mit mindestens 3 Halogenatomen substituiert ist.

Insbesondere bevorzugt sind ferner die Verbindungen, die jeweils durch eine der allgemeinen Formeln (Iaa) - (Ibi) definiert sind, in denen die Reste A₁-A₄, n, W, Q, R¹ und Z¹-Z³ die oben jeweils beschriebenen allgemeinen, bevorzugten oder besonders bevorzugten Bedeutungen haben. 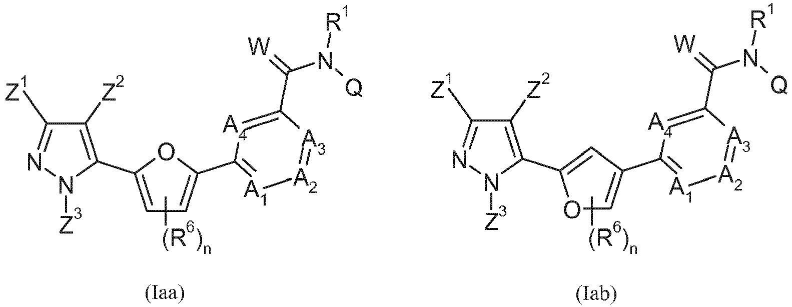 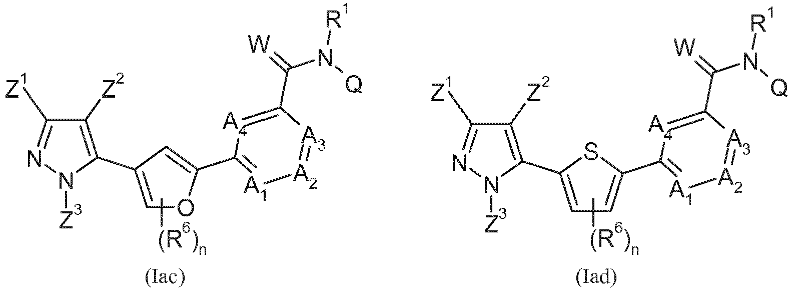 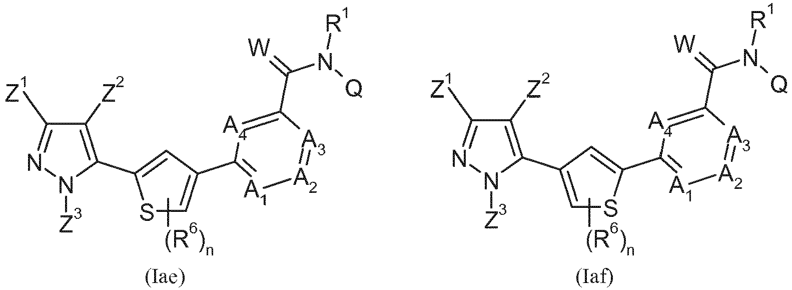 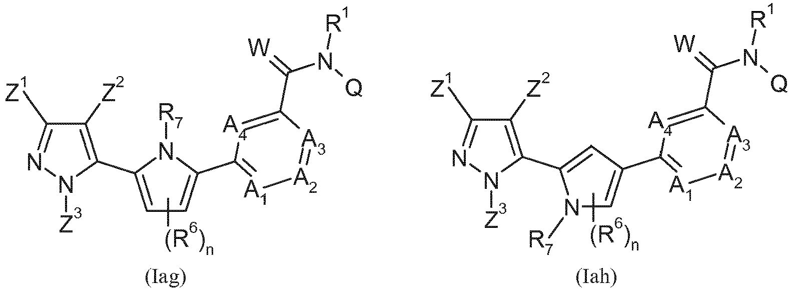 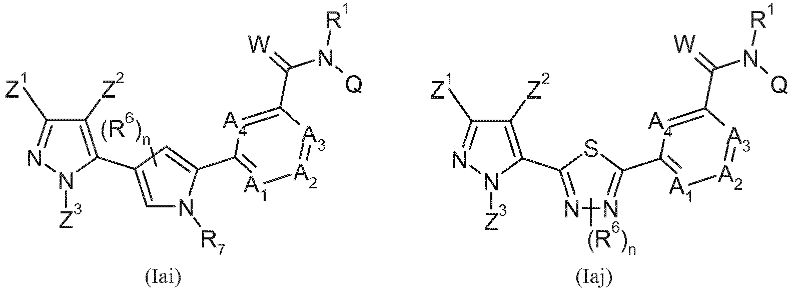 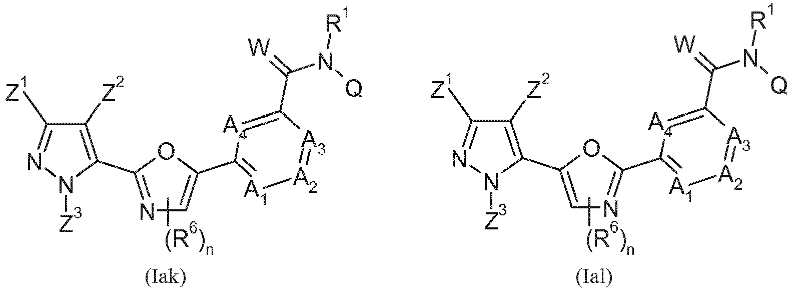 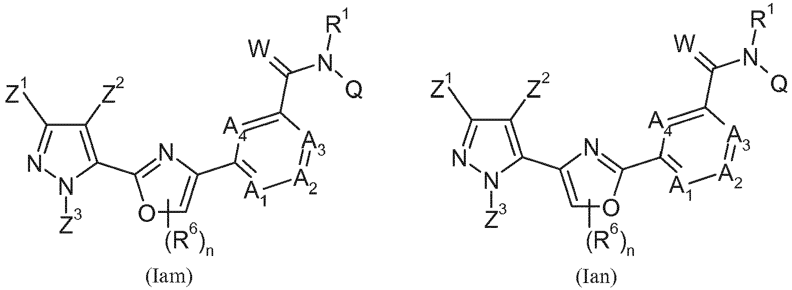 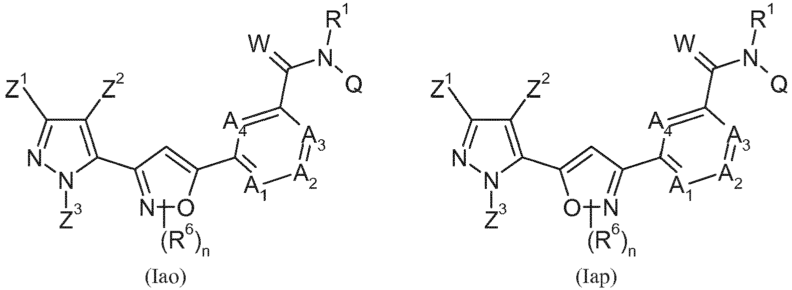 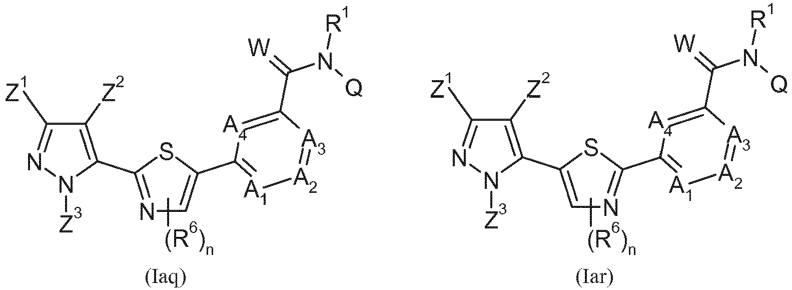 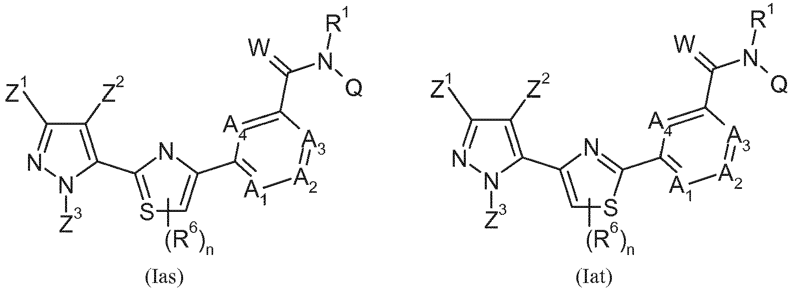 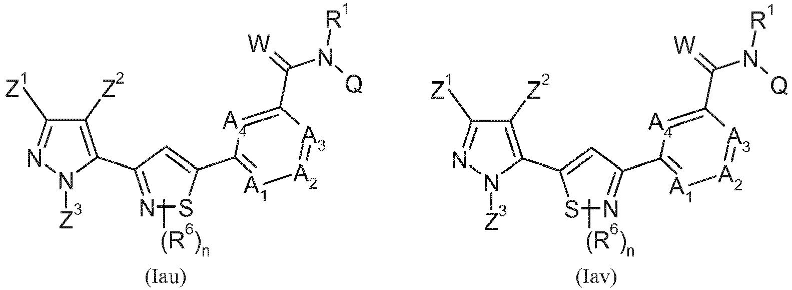 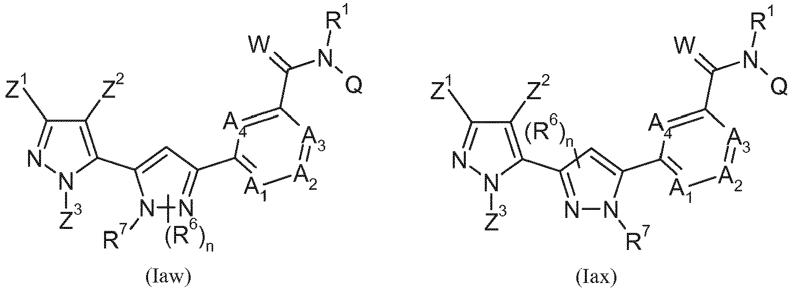 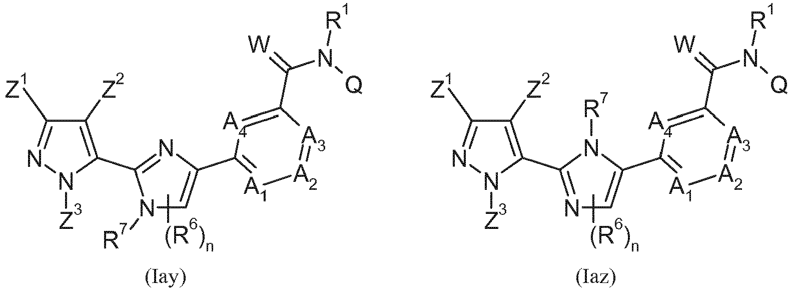 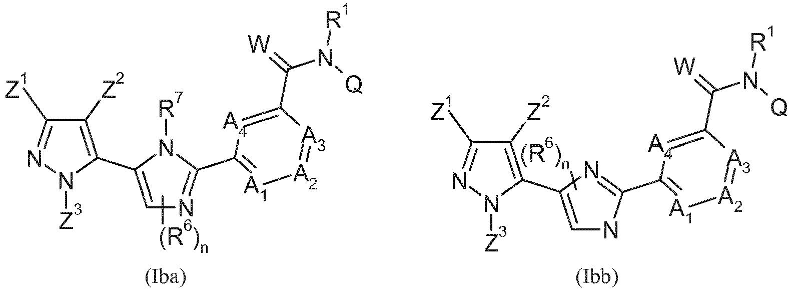 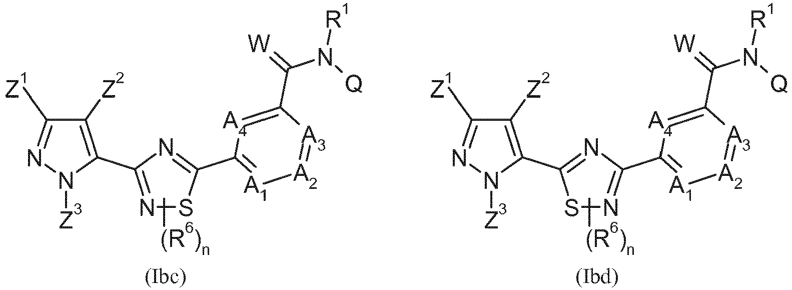 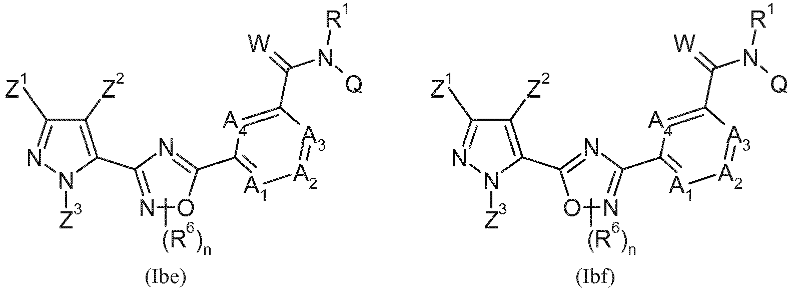 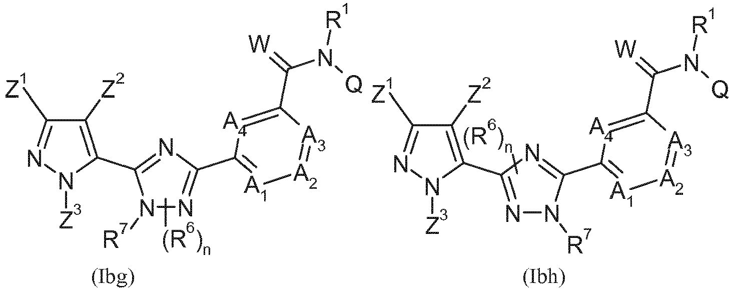 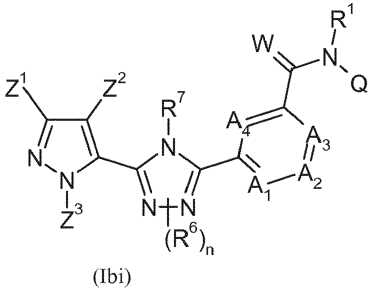 wobei für den Fall die allgemeinen Formeln (Iaw) und (Iax) gilt, dass einer der Reste Z¹, Z² oder Z³ mit mindestens 3 Halogenatomen substituiert ist.

Insbesondere bevorzugt sind Verbindungen der Formeln (Iap).

Weiterhin insbesondere bevorzugt sind Verbindungen der Formel (Ibg).

Weiterhin insbesondere bevorzugt sind Verbindungen der Formel (Iac).

Weiterhin insbesondere bevorzugt sind Verbindungen der Formel (Iaq).

Weiterhin insbesondere bevorzugt sind Verbindungen der Formel (Ibd).

Weiterhin insbesondere bevorzugt sind Verbindungen der Formel (Ibe).

Weiterhin insbesondere bevorzugt sind Verbindungen der Formel (Ibf).

Weiterhin insbesondere bevorzugt sind Verbindungen der Formel (Ibh).

Weiterhin insbesondere bevorzugt sind Verbindungen der Formel (Ibi).

Weiterhin insbesondere bevorzugt sind Verbindungen der Formel (Iaf).

Weiterhin insbesondere bevorzugt sind Verbindungen der Formel (Iaa).

Weiterhin bevorzugt sind Verbindungen der Formeln (Iap), (Ibg), (Iac), (Iaq), (Ibd), (Ibe), (Ibf), (Ibh), (Ibi), (Iaf) und (Iaa) worin W für Sauerstoff steht.

Weiterhin bevorzugt sind Verbindungen der Formeln (Iap), (Ibg), (Iac), (Iaq), (Ibd), (Ibe), (Ibf), (Ibh), (Ibi), (Iaf) und (Iaa) worin W für Sauerstoff steht und R¹ für Wasserstoff steht.

Weiterhin bevorzugt sind Verbindungen der Formeln (Iap), (Ibg), (Iac), (Iaq), (Ibd), (Ibe), (Ibf), (Ibh), (Ibi), (Iaf) und (Iaa) worin, wenn vorhanden, R⁶, R^{6a}, R^{6b}, R⁷ für Wasserstoff oder C₁-C₄-Alkyl, bevorzugt für Wasserstoff, stehen.

Ganz besonders bevorzugt sind Verbindungen der allgemeinen Formel (Iaa) - (Ibi), in denen Z¹ für CF₂CF₃ steht, Z² für CF₃, Z³ für CH₃, die Reste R¹ und R⁶ für Wasserstoff stehen, A¹ und A⁴ für CH stehen und A² für CH oder N steht, A₃ für C-Cl, W für Sauerstoff und R¹ für Wasserstoff steht und Q für Wasserstoff, halogeniertem C₁-C₄-Alkyl wie Trifluoroethyl (z. B. CH₂CF₃), gegebenenfalls mit Cyano oder halogeniertem C₁-C₄-Alkyl substituiertem Cyclopropyl wie Cyclopropyl, 1-(cyano)cyclopropyl, 1-(trifluoromethyl)cyclopropyl, gegebenenfalls mit 1, 2 oder 3 Substituenten (bevorzugt mit einem Substituenten) unabhängig voneinander ausgewählt aus C₁-C₄-Alkyl, C₁-C₄-Alkoxy, Cl, F, I substituiertem Phenyl wie z. B. Phenyl oder Benzyl oder 4-fluorphenyl.

Ganz besonders bevorzugt sind Verbindungen der allgemeinen Formel (Iap), (Ibg), (Iac), (Iaq), (Ibd), (Ibe), (Ibf), (Ibh), (Ibi), (Iaf) und (Iaa), in denen Z¹ für CF₂CF₃ steht, Z² für CF₃, Z³ für CH₃, die Reste R¹ und R⁶ für Wasserstoff stehen, A¹ und A⁴ für CH stehen und A² für CH oder N steht, A₃ für C-Cl oder C-H, W für Sauerstoff und R¹ für Wasserstoff steht und Q für Wasserstoff, halogeniertem C₁-C₄-Alkyl wie Trifluoroethyl (z. B. CH₂CF₃), gegebenenfalls mit Cyano oder halogeniertem C₁-C₄-Alkyl substituiertem Cyclopropyl wie Cyclopropyl, 1-(cyano)cyclopropyl, 1-(trifluoromethyl)cyclopropyl, gegebenenfalls mit 1, 2 oder 3 Substituenten (bevorzugt mit einem Substituenten) unabhängig voneinander ausgewählt aus C₁-C₄-Alkyl, C₁-C₄-Alkoxy, Cl, F, I substituiertem Phenyl wie z. B. Phenyl oder Benzyl oder 4-fluorphenyl.

Insbesondere bevorzugt sind auch Verbindungen der allgemeinen Formel (Iap), in denen Q ausgewählt ist aus: halogeniertem C₁-C₄-Alkyl wie Trifluoroethyl (z. B. CH₂CF₃), gegebenenfalls mit Cyano oder halogeniertem C₁-C₄-Alkyl substituiertem Cyclopropyl wie 1-(cyano)cyclopropyl oder 1-(trifluoromethyl)cyclopropyl, Thiethan-3-yl, 2-oxo-2-(2,2,2-trifluoroethylamino)ethyl.

Insbesondere bevorzugt sind auch Verbindungen der allgemeinen Formel (Iap), in denen Z¹ für CF₂CF₃ steht, Z² für CF₃, Z³ für CH₃, die Reste R¹ und R⁶ für Wasserstoff stehen, A¹ und A⁴ für CH stehen und A² für CH steht, A₃ für C-Cl, W für Sauerstoff und R¹ für Wasserstoff steht und Q ausgewählt ist aus: halogeniertem C₁-C₄-Alkyl wie Trifluoroethyl (z. B. CH₂CF₃), gegebenenfalls mit Cyano oder halogeniertem C₁-C₄-Alkyl substituiertem Cyclopropyl wie 1-(cyano)cyclopropyl oder 1-(trifluoromethyl)cyclopropyl, Thiethan-3-yl, 2-oxo-2-(2,2,2-trifluoroethylamino)ethyl.

Insbesondere bevorzugt sind auch Verbindungen der allgemeinen Formel (Ibg), in denen Q ausgewählt ist aus: halogeniertem C₁-C₄-Alkyl wie Trifluoroethyl (z. B. CH₂CF₃), gegebenenfalls mit Cyano oder halogeniertem C₁-C₄-Alkyl substituiertem Cyclopropyl wie Cyclopropyl, gegebenenfalls mit 1, 2 oder 3 Substituenten (bevorzugt mit einem Substituenten) unabhängig voneinander ausgewählt aus C₁-C₄-Alkyl, C₁-C₄-Alkoxy, Cl, F, I substituiertem Phenyl wie z. B. Phenyl oder Benzyl.

Insbesondere bevorzugt sind auch Verbindungen der allgemeinen Formel (Ibg), in denen Z¹ für CF₂CF₃ steht, Z² für CF₃, Z³ für CH₃, die Reste R¹ und R⁷ für Wasserstoff oder C₁-C₄-Alkyl stehen, A¹ und A⁴ für CH stehen und A² für CH steht, A³ für C-Cl, W für Sauerstoff und R¹ für Wasserstoff steht und Q ausgewählt ist aus: halogeniertem C₁-C₄-Alkyl wie Trifluoroethyl (z. B. CH₂CF₃), gegebenenfalls mit Cyano oder halogeniertem C₁-C₄-Alkyl substituiertem Cyclopropyl wie Cyclopropyl, gegebenenfalls mit 1, 2 oder 3 Substituenten (bevorzugt mit einem Substituenten) unabhängig voneinander ausgewählt aus C₁-C₄-Alkyl, C₁-C₄-Alkoxy, Cl, F, I substituiertem Phenyl wie z. B. Phenyl oder Benzyl.

Insbesondere bevorzugt sind auch Verbindungen der allgemeinen Formel (Ibh), in denen Q ausgewählt ist aus: halogeniertem C₁-C₄-Alkyl wie Trifluoroethyl (z. B. CH₂CF₃), gegebenenfalls mit Cyano oder halogeniertem C₁-C₄-Alkyl substituiertem Cyclopropyl wie Cyclopropyl, gegebenenfalls mit 1, 2 oder 3 Substituenten (bevorzugt mit einem Substituenten) unabhängig voneinander ausgewählt aus C₁-C₄-Alkyl, C₁-C₄-Alkoxy, Cl, F, I substituiertem Phenyl wie z. B. Benzyl.

Insbesondere bevorzugt sind auch Verbindungen der allgemeinen Formel (Ibh), in denen Z¹ für CF₂CF₃ steht, Z² für CF₃, Z³ für CH₃, die Reste R¹ und R⁷ für Wasserstoff stehen, A¹ und A⁴ für CH stehen und A² für CH steht, A³ für C-Cl, W für Sauerstoff und R¹ für Wasserstoff steht und Q ausgewählt ist aus: halogeniertem C₁-C₄-Alkyl wie Trifluoroethyl (z. B. CH₂CF₃), gegebenenfalls mit Cyano oder halogeniertem C₁-C₄-Alkyl substituiertem Cyclopropyl wie Cyclopropyl, gegebenenfalls mit 1, 2 oder 3 Substituenten (bevorzugt mit einem Substituenten) unabhängig voneinander ausgewählt aus C₁-C₄-Alkyl, C₁-C₄-Alkoxy, Cl, F, I substituiertem Phenyl wie z. B. Benzyl.

In einer besonders bevorzugten Ausführungsform steht Q in Verbindungen der Formeln (Iap), (Ibg), (Iac), (Iaq), (Ibd), (Ibe), (Ibf), (Ibh), (Ibi), (Iaf) und (Iaa) für 1-Cyano-cyclopropyl oder Cyclopropyl.

Erfindungsgemäß steht "Alkyl" - in Alleinstellung oder als Bestandteil einer chemischen Gruppe - für geradkettige oder verzweigte Kohlenstoffwasserstoffe, vorzugsweise mit 1 bis 6 Kohlenstoffatomen, wie beispielsweise Methyl, Ethyl, n-Propyl, Isopropyl, n-Butyl, Isobutyl, s-Butyl, t-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt für Alkyle mit 1 bis 4 Kohlenstoffatomen, wie unter anderem Methyl, Ethyl, n-Propyl, Isopropyl, n-Butyl, Isobutyl, s-Butyl oder t-Butyl. Die erfindungsgemäßen Alkyle können mit einem oder mehreren, gleichen oder verschiedenen Resten substituiert sein.

Erfindungsgemäß steht "Alkenyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt für Alkenyle mit 2 bis 4 Kohlenstoffatomen, wie unter anderem 2-Propenyl, 2-Butenyl oder 1-Methyl-2-propenyl. Die erfindungsgemäßen Alkenyle können mit einem oder mehreren, gleichen oder verschiedenen Resten substituiert sein.

Erfindungsgemäß steht "Alkinyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt für Alkinyle mit 2 bis 4 Kohlenstoffatomen wie unter anderem Ethinyl, 2-Propinyl oder 2-Butinyl-2-propenyl. Die erfindungsgemäßen Alkinyle können mit einem oder mehreren, gleichen oder verschiedenen Resten substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt für Cycloalkyle mit 3, 4, 5, 6 oder 7 Kohlenstoffatomen, wie unter anderem Cyclopropyl oder Cyclobutyl. Die erfindungsgemäßen Cycloalkyle können mit einem oder mehreren, gleichen oder verschiedenen Resten substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, wie beispielsweise Ethylcyclopropyl, Isopropylcyclobutyl, 3-Methylcyclopentyl und 4-Methyl-cyclohexyl. Ferner bevorzugt für Alkylcycloalkyle mit 4, 5 oder 7 Kohlenstoffatomen wie unter anderen Ethylcyclopropyl oder 4-Methyl-cyclohexyl. Die erfindungsgemäßen Alkylcycloalkyle können mit einem oder mehreren, gleichen oder verschiedenen Resten substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, wie beispielsweise Cyclopropylmethyl, Cyclobutylmethyl, Cyclopentylmethyl, Cyclohexylmethyl und Cyclopentylethyl. Ferner bevorzugt für Cycloalkylalkyle mit 4, 5 oder 7 Kohlenstoffatomen wie unter anderen Cyclopropylmethyl oder Cyclobutylmethyl. Die erfindungsgemäßen Cycloalkylalkyle können mit einem oder mehreren, gleichen oder verschiedenen Resten substituiert sein.

Erfindungsgemäß steht "Halogen" für Fluor, Chlor, Brom oder Iod, insbesondere für Fluor, Chlor oder Brom.

Die erfindungsgemäßen mit Halogen substituierten chemischen Gruppen, wie beispielsweise Halogenalkyl, Halogencycloalkyl, Halogenalkyloxy, HalogenAlkylsulfanyl, Halogenalkylsulfinyl oder Halogenalkylsulfonyl sind einfach oder mehrfach bis zur maximal möglichen Substituentenzahl mit Halogen substituiert. Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor.

Erfindungsgemäß steht "Halogencycloalkyl" für mono-, bi- oder tricyclisches Halogencycloalkyl, vorzugsweise mit 3 bis 10 Kohlenstoffatomen, wie unter anderen 1-Fluor-cyclopropyl, 2-Fluor-cyclopropyl oder 1-Fluor-cyclobutyl. Ferner bevorzugt für Halogencycloalkyl mit 3, 5 oder 7 Kohlenstoffatomen. Die erfindungsgemäßen Halogencycloalkyle können mit einem oder mehreren, gleichen oder verschiedenen Resten substituiert sein.

Erfindungsgemäß steht "Halogenalkyl" "Halogenalkenyl" oder "Halogenalkinyl" für mit Halogen substituierte Alkyle, Alkenyle oder Alkinyle mit vorzugsweise 1 bis 9 gleichen oder verschiedenen Halogenatomen, wie beispielsweise Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃. Entsprechendes gilt für Haloalkenyl und andere durch Halogen substituierten Reste. Haloalkoxy ist z.B. OCF₃, OCHF₂, OCH₂F, OCF₂CF₃, OCH₂CF₃ und OCH₂CH₂Cl;
Weitere Beispiele für Halogenalkyle sind Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl und Pentafluor-t-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl.

Erfindungsgemäß steht "Hydroxyalkyl" für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, s-Butanol und t-Butanol. Ferner bevorzugt für Hydroxyalkylgruppen mit 1 bis 4 Kohlenstoffatomen. Die erfindungsgemäßen Hydroxyalkylgruppen können mit einem oder mehreren, gleichen oder verschiedenen Resten substituiert sein

Erfindungsgemäß steht "Alkoxy" für geradkettiges oder verzweigtes O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, s-Butoxy und t-Butoxy. Ferner bevorzugt für Alkoxygruppen mit 1 bis 4 Kohlenstoffatomen. Die erfindungsgemäßen Alkoxygruppen können mit einem oder mehreren, gleichen oder verschiedenen Resten substituiert sein.

Erfindungsgemäß steht "Halogenalkoxy" für mit Halogen substituiertes geradkettiges oder verzweigtes O-Alkyl, vorzugsweise mit 1 bis 6 Kohlenstoffatomen, wie unter anderem Difluormethoxy, Trifluormethoxy, 2,2-Difluorethoxy, 1,1,2,2-Tetrafluorethoxy, 2,2,2-Trifluorethoxy und 2-Chlor-1,1,2-trifluorethoxy. Ferner bevorzugt für Halogenalkoxygruppen mit 1 bis 4 Kohlenstoffatomen. Die erfindungsgemäßen Halogenalkoxygruppen können mit einem oder mehreren, gleichen oder verschiedenen Resten substituiert sein.

Erfindungsgemäß steht "Alkylsulfanyl" für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, s-Butylthio und t-Butylthio. Ferner bevorzugt für Alkylsulfanylgruppen mit 1 bis 4 Kohlenstoffatomen. Die erfindungsgemäßen Alkylsulfanylgruppen können mit einem oder mehreren, gleichen oder verschiedenen Resten substituiert sein.

Beispiele für Halogenalkylsulfanylalkyle, d.h. mit Halogen substituierte Alkylsulfanylgruppen, sind unter anderem Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio.

Erfindungsgemäß steht "Alkylsulfinyl" für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, s-Butylsulfinyl und t-Butylsulfinyl. Ferner bevorzugt für Alkylsulfinylgruppen mit 1 bis 4 Kohlenstoffatomen. Die erfindungsgemäßen Alkylsulfinylgruppen können mit einem oder mehreren, gleichen oder verschiedenen Resten substituiert sein.

Beispiele für Halogenalkylsulfinylgrupen, d.h. mit Halogen substituierte Alkylsulfinylgruppen, sind unter anderem Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl.

Erfindungsgemäß steht "Alkylsulfonyl" für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, s-Butylsulfonyl und t-Butylsulfonyl. Ferner bevorzugt für Alkylsulfonylgruppen mit 1 bis 4 Kohlenstoffatomen. Die erfindungsgemäßen Alkylsulfonylgruppen können mit einem oder mehreren, gleichen oder verschiedenen Resten substituiert sein.

Beispiele für Halogenalkylsulfonylgrupen, d.h. mit Halogen substituierte Alkylsulfonylgruppen sind unter anderem Difluormethylsulfonyl, Trifluormethylsulfonyl, Trichlormethylsulfonyl, Chlordifluormethylsulfonyl, 1 -Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl.

Erfindungsgemäß steht "Alkylcarbonyl" für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, s-Butylcarbonyl und t-Butylcarbonyl. Ferner bevorzugt für Alkylcarbonyle mit 1 bis 4 Kohlenstoffatomen. Die erfindungsgemäßen Alkylcarbonyle können mit einem oder mehreren, gleichen oder verschiedenen Resten substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, wie beispielsweise Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptyl, Bycyclo[2.2.2]octylcarbonyl und Adamantylcarbonyl. Ferner bevorzugt für Cycloalkylcarbonyl mit 3, 5 oder 7 Kohlenstoffatomen im Cycloalkylteil. Die erfindungsgemäßen Cycloalkylcarbonylgruppen können mit einem oder mehreren, gleichen oder verschiedenen Resten substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, s-Butoxycarbonyl und t-Butoxycarbonyl. Die erfindungsgemäßen Alkoxycarbonylgruppen können mit einem oder mehreren, gleichen oder verschiedenen Resten substituiert sein.

Erfindungsgemäß steht "Alkylaminocarbonyl" für geradkettiges oder verzweigtes Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Proylaminocarbonyl, Isopropylaminocarbonyl, s-Butylaminocarbonyl und t-Butylaminocarbonyl. Die erfindungsgemäßen Alkylaminocarbonylgruppen können mit einem oder mehreren, gleichen oder verschiedenen Resten substituiert sein.

Erfindungsgemäß steht "*N,N*-Dialkylamino-carbonyl" für geradkettiges oder verzweigtes *N,N-*Dialkylaminocarbonyl mit vorzugsweise 1 bis 6 Kohlenstoffatomen oder 1 bis 4 Kohlenstoffatomen im Alkylteil, wie beispielsweise *N,N*-Dimethylamino-carbonyl, N.N-Diethylamino-carbonyl, *N,N*-Di(n-propylamino)-carbonyl, *N,N*-Di-(isopropylamino)-carbonyl und *N,N*-Di-(s-butylamino)-carbonyl. Die erfindungsgemäßen *N,N*-Dialkylamino-carbonylgruppen können mit einem oder mehreren, gleichen oder verschiedenen Resten substituiert sein.

Erfindungsgemäß steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten substituiert sein.

Beispiele substitutierter Aryle stellen die Arylalkyle dar, die gleichfalls mit einem oder mehreren, gleichen oder verschiedenen Resten im Alkyl- und/oder Arylteil substituiert sein können. Beispiele solcher Arylalkyle sind unter anderem Benzyl und 1-Phenylethyl.

Erfindungsgemäß steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder mit einem Substituenten Z substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein.

Substituierte Gruppen, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylsulfanyl, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und *N,N*-Dialkylamino-carbonyl, substituiertes Amino, wie Acylamino, Mono- und *N,N*-Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylsulfanylalkyl, Alkoxy-alkyl, gegebenfalls substituiertes Mono- und *N,N*-Dialkyl-aminoalkyl und Hydroxyalkyl bedeutet.

Im Begriff "substituierte Gruppen" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und *N,N*-Dialkenylamino-carbonyl, Mono- und Dialkinylaminocarbonyl, Mono- und *N,N-*Dialkenylamino, Mono- und *N,N*-Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe sowie einer substituierten Iminogruppe.

Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

### Bevorzugte Substituenten für die Substituentenebenen sind beispielsweise

Amino, Hydroxy, Halogen, Nitro, Cyano, Isocyano, Mercapto, Isothiocyanato, Carboxy, Carboxamid, SF₅, Aminosulfonyl, Alkyl, Cycloalkyl, Alkenyl, Cycloalkenyl, Alkinyl, *N*-Mono-alkyl-amino, *N,N-*Dialkylamino, *N*-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylsulfanyl, Cycloalkylsulfanyl, Alkenylthio, Cycloalkenylthio, Alkinylthio, Alkylsulfenyl und Alkylsulfinyl, wobei beide Enantiomere der Alkylsulfinylgruppe umfasst sind, Alkylsulfonyl, *N*-Mono-alkyl-aminosulfonyl, *N,N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N,N*-Dialkyl-amino-carbonyl, N-Alkanoylamino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt Alkoxyalkyl, Alkylsulfanylalkyl, Alkylsulfanylalkoxy, Alkoxyalkoxy, Phenethyl, Benzyloxy, Halogenalkyl, Halogencycloalkyl, Halogenalkoxy, Halogenalkylsulfanyl, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylsulfanyl, Halogenalkoxyalkanoyl, Halogenalkoxyalkyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N*-substituiert sind; vorzugsweise *N*-Mono- und *N,N*-Dialkylamino, (z.B. Methylamino, Ethylamino, *N,N*-Dimethylamino, *N,N-*Diethylamino, *N,N*-Di-n-propylamino, *N,N*-Diisopropylamino oder *N,N*-Dibutylamino), *N*-Mono- oder *N,N*-Dialkoxyalkylaminogruppen (z.B. *N*-Methoxymethylamino, *N*-Methoxyethylamino, *N,N*-Di-(methoxymethyl)-amino oder *N,N*-Di-(methoxyethyl)-amino), *N*-Mono- und *N,N*-Diarylamino, wie gegebenenfalls substituierte Aniline, Acylamino, *N,N*-diacylamino, *N*-Alkyl-*N*-arylamino, *N*-Alkyl-*N-*acylamino sowie gesättigte *N*-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Erfindungsgemäß umfasst der Begriff "cyclische Aminogruppen" heteroaromatische oder aliphatische Ringsysteme mit einem oder mehreren Stickstoffatomen. Die Heterocyclen sind gesättigt oder ungesättigt, bestehen aus einem oder mehreren, gegebenenfalls kondensierten Ringsystemen und beinhalten gegebenenfalls weitere Heteroatome, wie beispielsweise ein oder zwei Stickstoff-, Sauerstoff- und/oder Schwefelatome. Ferner umfasst der Begriff auch solche Gruppen, die einen Spiroring oder verbrücktes Ringsystem aufweisen. Die Anzahl der Atome, die die cyclische Aminogruppe bilden, ist beliebig und kann z.B. im Falle eines Einringsystems aus 3 bis 8 Ringatomen und im Falle eines Zweiringsystems aus 7 bis 11 Atomen bestehen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihvdro-pyrimidin-1-yl, Perhydropyrimidin-1-yl, 1,4-Diazacycloheptan-1-yl, genannt; beispielhaft für cyclische Aminogruppen mit gesättigten und ungesättigten monocyclischen Gruppen mit einem oder zwei Sauerstoffatomen und einem bis drei Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mit gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Alkoxy-(C₁-C₄)alkoxy , (C₁-C₄)Alkoxy-(C₁-C₄)alkyl, (C₁-C₄)Halogenalkyl, (C₁-C₄)Halogenalkoxy, (C₁-C₄)Alkylsulfanyl, (C₁-C₄)Halogenalkylsulfanyl, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy , (C₁-C₄)Alkoxy-(C₁-C₄)alkyl, (C₁-C₄)Halogenalkyl und (C₁-C₄)Halogenalkoxy substituiert ist, insbesondere durch einen oder zwei (C₁-C₄)Alkylreste substituiert ist,

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy , (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Beispiele für Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl,, Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäß geeignete Salze der erfindungsgemäßen Verbindungen, beispielsweise Salze mit Basen oder Säureadditionssalze, sind alle üblichen nicht toxischen Salze, vorzugsweise landwirtschaftlich und/oder physiologisch annehmbare Salze. Beispielsweise Salze mit Basen oder Säureadditionssalze. Bevorzugt werden Salze mit anorganischen Basen, wie beispielsweise Alkalimetallsalze (z.B. Natrium-, Kalium- oder Cäsiumsalze), Erdalkalimetallsalze (z.B. Calzium- oder Magnesiumsalze), Ammoniumsalze oder Salze mit organischen Basen, insbesondere mit organischen Aminen, wie beispielsweise Triethylammonium-, Dicyclohexylammonium-, *N,N'*-Dibenzylethylen-diammonium-, Pyridinium-, Picolinium- oder Ethanolammoniumsalze, Salze mit anorganischen Säuren (z.B. Hydrochloride, Hydrobromide, Dihydrosulfate, Trihydrosulfate, oder Phosphate), Salze mit organischen Carbonsäuren oder organischen Sulfosäuren (z.b. Formiate, Acetate, Trifluoracetate, Maleate, Tartrate, Methansulfonate, Benzolsulfonate oder 4-Toluolsulfonate). Bekannterweise können t-Amine, wie beispielsweise manche der erfindungsgemäßen Verbindungen, *N*-Oxide bilden, welche ebenfalls erfindungsgemäße Salze darstellen.

Die erfindungsgemäßen Verbindungen können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die erfindungsgemäßen Verbindungen können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

### Verwendung

Die Erfindung betrifft auch nicht-therapeutische Verfahren zur Bekämpfung von tierischen Schädlingen, bei dem man erfindungsgemäße Verbindungen der allgemeinen Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt.

Die erfindungsgemäßen Verbindungen der Formel (I) eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., Aceria kuko, Aceria sheldoni, Aculops spp., Aculus spp., Amblyomma spp., Amphitetranychus viennensis, Argas spp., Boophilus spp., Brevipalpus spp., Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., Epitrimerus pyri, Eutetranychus spp., Eriophyes spp., Glycyphagus domesticus, Halotydeus destructor, Hemitarsonemus spp., Hyalomma spp., Ixodes spp., Latrodectus spp., Loxosceles spp., Metatetranychus spp., Neutrombicula autumnalis, Nuphersa spp., Oligonychus spp., Ornithodorus spp., Ornithonyssus spp., Panonychus spp., Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., Tetranychus spp., Trombicula alfreddugesi, Vaejovis spp., Vasates lycopersici.;
aus der Klasse der Chilopoda z.B. Geophilus spp., Scutigera spp.;
aus der Ordnung oder der Klasse der Collembola z.B. Onychiurus armatus;
aus der Klasse der Diplopoda z.B. Blaniulus guttulatus;
aus der Klasse der Insecta, z.B. aus der Ordnung der Blattodea z.B. Blattella asahinai, Blattella germanica, Blatta orientalis, Leucophaea maderae, Panchlora spp., Parcoblatta spp., Periplaneta spp., Supella longipalpa;
aus der Ordnung der Coleoptera z.B. Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Baris caerulescens, Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptolestes ferrugineus, Cryptorhynchus lapathi, Cylindrocopturus spp., Dermestes spp., Diabrotica spp., Dichocrocis spp., Dicladispa armigera, Diloboderus spp., Epilachna spp., Epitrix spp., Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., Lachnosterna consanguinea, Lasioderma serricorne, Latheticus oryzae, Lathridius spp., Lema spp., Leptinotarsa decemlineata, Leucoptera spp., Lissorhoptrus oryzophilus, Lixus spp., Luperomorpha xanthodera, Luperodes spp., Lyctus spp., Megascelis spp., Melanotus spp., Meligethes aeneus, Melolontha spp., Migdolus spp., Monochamus spp., Naupactus xanthographus, Necrobia spp., Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllophaga helleri, Phyllotreta spp., Popillia japonica, Premnotrypes spp., Prostephanus truncatus, Psylliodes spp., Ptinus spp., Rhizobius ventralis, Rhizopertha dominica, Sitophilus spp., Sitophilus oryzae, Sphenophorus spp., Stegobium paniceum, Sternechus spp., Symphyletes spp., Tanymecus spp., Tenebrio molitor, Tenebrioides mauretanicus, Tribolium spp., Trogoderma spp., Tychius spp., Xylotrechus spp., Zabrus spp.;
aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Calliphora vicina, Ceratitis capitata, Chironomus spp., Chrysomyia spp., Chrysops spp., Chrysozona pluvialis, Cochliomyia spp., Contarinia spp., Cordylobia anthropophaga, Cricotopus sylvestris, Culex spp., Culicoides spp., Culiseta spp., Cuterebra spp., Dacus oleae, Dasyneura spp., Delia spp., Dermatobia hominis, Drosophila spp., z.B. Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., Lucilia spp., Lutzomyia spp., Mansonia spp., Musca spp., Oestrus spp.,
Oscinella frit, Paratanytarsus spp., Paralauterborniella subcincta, Pegomyia spp., Phlebotomus spp., Phorbia spp., Phormia spp., Piophila casei, Prodiplosis spp., Psila rosae, Rhagoletis spp., Sarcophaga spp., Simulium spp., Stomoxys spp., Tabanus spp., Tetanops spp., Tipula spp.;
aus der Ordnung der Heteroptera z.B. Anasa tristis, Antestiopsis spp., Boisea spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Homoptera z.B. Acizzia acaciaebaileyanae, Acizzia dodonaeae, Acizzia uncatoides, Acrida turrita, Acyrthosipon spp., Acrogonia spp., Aeneolamia spp., Agonoscena spp., Aleyrodes proletella, Aleurolobus barodensis, Aleurothrixus floccosus, Allocaridara malayensis, Amrasca spp., Anuraphis cardui, Aonidiella spp., Aphanostigma piri, Aphis spp., Arboridia apicalis, Arytainilla spp., Aspidiella spp., Aspidiotus spp., Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., Cryptomyzus ribis, Cryptoneossa spp., Ctenarytaina spp., Dalbulus spp., Dialeurodes citri, Diaphorina citri, Diaspis spp., Drosicha spp., Dysaphis spp., Dysmicoccus spp., Empoasca spp., Eriosoma spp., Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Macrosteles facifrons, Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nettigoniclla spectra, Nilaparvata lugens, Oncometopia spp., Orthezia praelonga, Oxya chinensis, Pachypsylla spp., Parabemisia myricae, Paratrioza spp., Parlatoria spp., Pemphigus spp., Peregrinus maidis, Phenacoccus spp., z.B. Phenacoccus madeirensis, Phloeomyzus passerinii, Phorodon humuli, Phylloxera spp., Pinnaspis aspidistrae, Planococcus spp., Prosopidopsylla flava, Protopulvinaria pyriformis, Pseudaulacaspis pentagona, Pseudococcus spp., z.B. Pseudococcus viburni, Psyllopsis spp., Psylla spp., Pteromalus spp., Pyrilla spp., Quadraspidiotus spp., Quesada gigas, Rastrococcus spp., Rhopalosiphum spp., Saissetia spp., Scaphoideus titanus, Schizaphis graminum, Selenaspidus articulatus, Sogata spp., Sogatella furcifera, Sogatodes spp., Stictocephala festina, Siphoninus phillyreae, Tenalaphara malayensis,
Tetragonocephela spp., Tinocallis caryaefoliae, Tomaspis spp., Toxoptera spp., Trialeurodes vaporariorum, Trioza spp., Typhlocyba spp., Unaspis spp., Viteus vitifolii, Zygina spp.;
aus der Ordnung der Hymenoptera z.B. Acromyrmex spp., Athalia spp., Atta spp., Diprion spp., Hoplocampa spp., Lasius spp., Monomorium pharaonis, Sirex spp., Solenopsis invicta, Tapinoma spp., Urocerus spp., Vespa spp., Xeris spp.;
aus der Ordnung der Isopoda z.B. Armadillidium vulgare, Oniscus asellus, Porcellio scaber;
aus der Ordnung der Isoptera z.B. Coptotermes spp., Cornitermes cumulans, Cryptotermes spp., Incisitermes spp., Microtermes obesi, Odontotermes spp., Reticulitermes spp.;
aus der Ordnung der Lepidoptera z.B. Achroia grisella, Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamstra brassicae, Melanitis leda, Mocis spp., Monopis obviella, Mythimna separata, Nemapogon cloacellus, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudaletia unipuncta, Pseudoplusia includens, Pyrausta nubilalis, Rachiplusia nu, Schoenobius spp., Scirpophaga spp., Scirpophaga innotata, Scotia segetum, Sesamia spp., Sesamia inferens, Sparganothis spp., Spodoptera spp., Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., Tryporyza incertulas, Tuta absoluta, Virachola spp.;
aus der Ordnung der Orthoptera oder Saltatoria z.B. Acheta domesticus, Dichroplus spp., Gryllotalpa spp., Hieroglyphus spp., Locusta spp., Melanoplus spp., Schistocerca gregaria;
aus der Ordnung der Phthiraptera z.B. Damalinia spp., Haematopinus spp., Linognathus spp., Pediculus spp., Phylloera vastatrix, Phtirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., Pulex irritans, Tunga penetrans, Xenopsylla cheopsis;
aus der Ordnung der Thysanoptera z.B. Anaphothrips obscurus, Baliothrips biformis, Drepanothrips reuteri, Enneothrips flavens, Frankliniella spp., Heliothrips spp., Hercinothrips femoralis, Rhipiphorothrips cruentatus, Scirtothrips spp., Taeniothrips cardamomi, Thrips spp.;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp.;
Schädlinge aus dem Stamm der Mollusca, insbesondere aus der Klasse der Bivalvia, z.B. Dreissena spp., sowie aus der Klasse der Gastropoda z.B. Arion spp., Biomphalaria spp., Bulinus spp., Deroceras spp., Galba spp., Lymnaea spp., Oncomelania spp., Pomacea spp., Succinea spp.;
Tierparasiten aus den Stämmen der Plathelminthes und Nematoda, z.B. Ancylostoma duodenale, Ancylostoma ceylanicum, Ancylostoma braziliensis, Ancylostoma spp., Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti;
Pflanzenschädlinge aus dem Stamm der Nematoda, d.h. pflanzenparasitäre Nematoden, insbesondere Aphelenchoides spp., Bursaphelenchus spp., Ditylenchus spp., Globodera spp., Heterodera spp., Longidorus spp., Meloidogyne spp., Pratylenchus spp., Radopholus spp., Trichodorus spp., Tylenchulus spp., Xiphinema spp., Helicotylenchus spp., Tylenchorhynchus spp., Scutellonema spp., Paratrichodorus spp., Meloinema spp., Paraphelenchus spp., Aglenchus spp., Belonolaimus spp., Nacobbus spp., Rotylenchulus spp., Rotylenchus spp., Neotylenchus spp., Paraphelenchus spp., Dolichodorus spp., Hoplolaimus spp., Punctodera spp., Criconemella spp., Quinisulcius spp., Hemicycliophora spp., Anguina spp., Subanguina spp., Hemicriconemoides spp., Psilenchus spp.,
Pseudohalenchus spp., Criconemoides spp., Cacopaurus spp.

Weiterhin lässt sich aus dem Unterreich der Protozoa die Ordnung der Coccidia z.B. Eimeria spp. bekämpfen.

### Formulierungen

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder
mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natür-liche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(IR)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise
   Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide,

Weitere Wirkstoffe mit unbekanntem Wirkmechanismus, wie beispielsweise Afidopyropen, Azadirachtin, Benclothiaz, Benzoximate, Bifenazate, Bromopropylate, Chinomethionat, Cryolite,

Dicofol, Diflovidazin, Fluensulfone, Flometoquin, Flufenerim, Flufenoxystrobin, Flufiprole, Fluopyram, Flupyradifurone, Fufenozide, Heptafluthiin, Imidaclothiz, Iprodione, Meperfluthrin, Paichongding, Pyflubumide, Pyrifluquinazon, Pyriminostrobin, Tetramethylfluthrin und Iodmethan; desweiteren Präparate auf Basis vonBacillus firmus (1-1582, BioNeem, Votivo), sowie folgende bekannte wirksame Verbindungen:3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) und 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-metloxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{5-[3-Chlor-5-(trifluormethyl)phenyl]-5-(trifluormethyl)-4,5-dihydro-1,2-oxazol-3-yl}-N-{2-oxo-2-[(2,2,2-trifluorethyl)amino]ethyl}-1-naphthamid (bekannt aus WO2009/002809), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 3-Chlor-N-(2-cyanpropan-2-yl)-N-[4-(1,1,1,2,3,3,3-heptafluorpropan-2-yl)-2-methylphenyl]phthalamid (bekannt aus WO2012/034472), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-

pyrido[1,2-a]pyrimidin-1-ium-2-olat (bekannt aus WO2009/099929), (5S,8R)-1-[(6-Chlorpyridin-3-yl)methyl]-9-nitro-2,3,5,6,7,8-hexahydro-1H-5,8-epoxyimidazo[1,2-a]azepin (bekannt aus WO2010/069266), (2E)-1-[(6-Chlorpyridin-3-yl)methyl]-N'-nitro-2-pentylidenhydrazincarboximidamid (bekannt aus WO2010/060231), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969).

### Fungizide

(1) Inhibitoren der Ergosterol-Biosynthese, wie beispielsweise (1.1) Aldimorph (1704-28-5), (1.2) Azaconazol (60207-31-0), (1.3) Bitertanol (55179-31-2), (1.4) Bromuconazol (116255-48-2), (1.5) Cyproconazol (113096-99-4), (1.6) Diclobutrazol (75736-33-3), (1.7) Difenoconazol (119446-68-3), (1.8) Diniconazol (83657-24-3), (1.9) Diniconazol-M (83657-18-5), (1.10) Dodemorph (1593-77-7), (1.11) Dodemorph Acetat (31717-87-0), (1.12) Epoxiconazol (106325-08-0), (1.13) Etaconazol (60207-93-4), (1.14) Fenarimol (60168-88-9), (1.15) Fenbuconazol (114369-43-6), (1.16) Fenhexamid (126833-17-8), (1.17) Fenpropidin (67306-00-7), (1.18) Fenpropimorph (67306-03-0), (1.19) Fluquinconazol (136426-54-5), (1.20) Flurprimidol (56425-91-3), (1.21) Flusilazol (85509-19-9), (1.22) Flutriafol (76674-21-0), (1.23) Furconazol (112839-33-5), (1.24) Furconazol-Cis (112839-32-4), (1.25) Hexaconazol (79983-71-4), (1.26) Imazalil (60534-80-7), (1.27) Imazalil Sulfat (58594-72-2), (1.28) Imibenconazol (86598-92-7), (1.29) Ipconazol (125225-28-7), (1.30) Metconazol (125116-23-6), (1.31) Myclobutanil (88671-89-0), (1.32) Naftifin (65472-88-0), (1.33) Nuarimol (63284-71-9), (1.34) Oxpoconazol (174212-12-5), (1.35) Paclobutrazol (76738-62-0), (1.36) Pefurazoat (101903-30-4), (1.37) Penconazol (66246-88-6), (1.38) Piperalin (3478-94-2), (1.39) Prochloraz (67747-09-5), (1.40) Propiconazol (60207-90-1), (1.41) Prothioconazol (178928-70-6), (1.42) Pyributicarb (88678-67-5), (1.43) Pyrifenox (88283-41-4), (1.44) Quinconazol (103970-75-8), (1.45) Simeconazol (149508-90-7), (1.46) Spiroxamin (118134-30-8), (1.47) Tebuconazol (107534-96-3), (1.48) Terbinafin (91161-71-6), (1.49) Tetraconazol (112281-77-3), (1.50) Triadimefon (43121-43-3), (1.51) Triadimenol (89482-17-7), (1.52) Tridemorph (81412-43-3), (1.53) Triflumizol (68694-11-1), (1.54) Triforin (26644-46-2), (1.55) Triticonazol (131983-72-7), (1.56) Uniconazol (83657-22-1), (1.57) Uniconazol-p (83657-17-4), (1.58) Viniconazol (77174-66-4), (1.59) Voriconazol (137234-62-9), (1.60) 1-(4-Chlorphenyl)-2-(1H-1,2,4-triazol-1-yl)cycloheptanol (129586-32-9), (1.61) Methyl-1-(2,2-dimethyl-2,3-dihydro-1H-inden-1-yl)-1H-imidazol-5-carboxylat (110323-95-0), (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat (111226-71-2).
(2) Inhibitoren der Respiration (Atmungsketten-Inhibitoren), wie beispielsweise (2.1) Bixafen (581809-46-3), (2.2) Boscalid (188425-85-6), (2.3) Carboxin (5234-68-4), (2.4) Diflumetorim (130339-07-0), (2.5) Fenfuram (24691-80-3), (2.6) Fluopyram (658066-35-4), (2.7) Flutolanil (66332-96-5), (2.8) Fluxapyroxad (907204-31-3), (2.9) Furametpyr (123572-88-3), (2.10) Furmecyclox (60568-05-0), (2.11) Isopyrazam Mischung des syn-epimeren Razemates 1RS,4SR,9RS und des anti-epimeren Razemates 1RS,4SR,9SR (881685-58-1), (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil (55814-41-0), (2.19) Oxycarboxin (5259-88-1), (2.20) Penflufen (494793-67-8), (2.21) Penthiopyrad (183675-82-3), (2.22) Sedaxane (874967-67-6), (2.23) Thifluzamid (130000-40-7), (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid (1092400-95-7), (2.28) 5,8-Difluor-N-[2-(2-fluor-4-{[4-(trifluormethyl)pyridin-2-yl]oxy}phenyl)ethyl]quinazolin-4-amin (1210070-84-0) (bekannt aus WO2010025451), (2.29) N-[9-(Dichlormethylen)-1,2,3,4-tetrahydro-1,4-methanonaphthalen-5-yl]-3-(difluormethyl)-1-methyl-1H-pyrazol-4-carboxamid, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise (3.1) Ametoctradin (865318-97-4), (3.2) Amisulbrom (348635-87-0), (3.3) Azoxystrobin (131860-33-8), (3.4) Cyazofamid (120116-88-3), (3.5) Coumethoxystrobin (850881-30-0), (3.6) Coumoxystrobin (850881-70-8), (3.5) Dimoxystrobin (141600-52-4), (3.6) Enestroburin (238410-11-2) (bekannt aus WO 2004/058723), (3.9) Famoxadon (131807-57-3) (bekannt aus WO 2004/058723), (3.10) Fenamidon (161326-34-7) (bekannt aus WO 2004/058723), (3.11) Fenoxystrobin (918162-02-4), (3.12) Fluoxastrobin (361377-29-9) (bekannt aus WO 2004/058723), (3.13) Kresoxim-Methyl (143390-89-0) (bekannt aus WO 2004/058723), (3.14) Metominostrobin (133408-50-1) (bekannt aus WO 2004/058723), (3.15) Orysastrobin (189892-69-1) (bekannt aus WO 2004/058723), (3.16) Picoxystrobin (117428-22-5) (bekannt aus WO 2004/058723), (3.17) Pyraclostrobin (175013-18-0) (bekannt aus WO 2004/058723), (3.18) Pyrametostrobin (915410-70-7) (bekannt aus WO 2004/058723), (3.19) Pyraoxystrobin (862588-11-2) (bekannt aus WO 2004/058723), (3.20) Pyribencarb (799247-52-2) (bekannt aus WO 2004/058723), (3.21) Triclopyricarb (902760-40-1), (3.22) Trifloxystrobin (141517-21-7) (bekannt aus WO 2004/058723), (3.23) (2E)-2-(2-{[6-(3-Chlor-2-methylphenoxy)-5-fluorpyrimidin-4-yl]oxy}phenyl)-2-(methoxyimino)-N-methylethanamid (bekannt aus WO 2004/058723), (3.24) (2E)-2-(Methoxyimino)-N-methyl-2-(2-{[({(1E)-1-[3-(trifluormethyl)phenyl]ethyliden}amino)oxy]methyl}phenyl)ethanamid (bekannt aus WO 2004/058723), (3.25) (2E)-2-(Methoxyimino)-N-methyl-2-{2-[(E)-({1-[3-(trifluormethyl)phenyl]ethoxy}imino)methyl]phenyl}ethanamid (158169-73-4), (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid (326896-28-0), (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid (119899-14-8), (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl [(4-methoxyphenyl)imino]methyl} sulfanyl)methyl]phenyl} -3-methoxyprop-2-enoat (149601-03-6), (3.31) N-(3-Ethyl-3,5,5-trimethylcyclohexyl)-3-(formylamino)-2-hydroxybenzamid (226551-21-9), (3.32) 2-{2-[(2,5-Dimethylphenoxy)methyl]phenyl}-2-methoxy-N-methylacetamid (173662-97-0) und (3.33) (2R)-2-{2-[(2,5-Dimethylphenoxy)methyl]phenyl}-2-methoxy-N-methylacetamid (394657-24-0).
(4) Inhibitoren der Mitose und Zellteilung, wie beispielsweise (4.1) Benomyl (17804-35-2), (4.2) Carbendazim (10605-21-7), (4.3) Chlorfenazol (3574-96-7), (4.4) Diethofencarb (87130-20-9), (4.5) Ethaboxam (162650-77-3), (4.6) Fluopicolid (239110-15-7), (4.7) Fuberidazol (3878-19-1), (4.8) Pencycuron (66063-05-6), (4.9) Thiabendazol (148-79-8), (4.10) Thiophanat-Methyl (23564-05-8), (4.11) Thiophanat (23564-06-9), (4.12) Zoxamid (156052-68-5), (4.13) 5-Chlor-7-(4-methylpiperidin-1-yl)-6-(2,4,6-trifluorphenyl)[1,2,4]triazolo[1,5-a]pyrimidin (214706-53-3) und (4.14) 3-Chlor-5-(6-chlorpyridin-3-yl)-6-methyl-4-(2,4,6-trifluorphenyl)pyridazin (1002756-87-7).
(5) Verbindungen mit Multisite-Aktivität, wie beispielsweise (5.1) Bordeauxmischung (8011-63-0), (5.2) Captafol (2425-06-1), (5.3) Captan (133-06-2) (bekannt aus WO 02/12172), (5.4) Chlorothalonil (1897-45-6), (5.5) Kupferzubereitungen wie Kupferhydroxid (20427-59-2), (5.6) Kupfernaphthenat (1338-02-9), (5.7) Kupferoxid (1317-39-1), (5.8) Kupferoxychlorid (1332-40-7), (5.9) Kupfersulfat (7758-98-7), (5.10) Dichlofluanid (1085-98-9), (5.11) Dithianon (3347-22-6), (5.12) Dodine (2439-10-3), (5.13) Dodine freie Base, (5.14) Ferbam (14484-64-1), (5.15) Fluorofolpet (719-96-0), (5.16) Folpet (133-07-3), (5.17) Guazatin (108173-90-6), (5.18) Guazatinacetat, (5.19) Iminoctadin (13516-27-3), (5.20) Iminoctadinalbesilat (169202-06-6), (5.21) Iminoctadintriacetat (57520-17-9), (5.22) Mankupfer (53988-93-5), (5.23) Mancozeb (8018-01-7), (5.24) Maneb (12427-38-2), (5.25) Metiram (9006-42-2), (5.26) Zinkmetiram (9006-42-2), (5.27) Kupfer-Oxin (10380-28-6), (5.28) Propamidin (104-32-5), (5.29) Propineb (12071-83-9), (5.30) Schwefel und Schwefelzubereitungen wie beispielsweise Calciumpolysulfid (7704-34-9), (5.31) Thiram (137-26-8), (5.32) Tolylfluanid (731-27-1), (5.33) Zineb (12122-67-7) und (5.34) Ziram (137-30-4).
(6) Resistenzinduktoren, wie beispielsweise (6.1) Acibenzolar-S-Methyl (135158-54-2), (6.2) Isotianil (224049-04-1), (6.3) Probenazol (27605-76-1) und (6.4) Tiadinil (223580-51-6).
(7) Inhibitoren der Aminosäure- und Protein-Biosynthese, wie beispielsweise (7.1) Andoprim (23951-85-1), (7.2) Blasticidin-S (2079-00-7), (7.3) Cyprodinil (121552-61-2), (7.4) Kasugamycin (6980-18-3), (7.5) Kasugamycin Hydrochlorid Hydrat (19408-46-9), (7.6) Mepanipyrim (110235-47-7), (7.7) Pyrimethanil (53112-28-0) und (7.8) 3-(5-Fluor-3,3,4,4-tetramethyl-3,4-dihydroisoquinolin-1-yl)quinolin (861647-32-7) (bekannt aus WO2005070917).
(8) Inhibitoren der ATP Produktion, wie beispielsweise (8.1) Fentin Acetat (900-95-8), (8.2) Fentin Chlorid (639-58-7), (8.3) Fentin Hydroxid (76-87-9) und (8.4) Silthiofam (175217-20-6).
(9) Inhibitoren der Zellwandsynthese, wie beispielsweise (9.1) Benthiavalicarb (177406-68-7), (9.2) Dimethomorph (110488-70-5), (9.3) Flumorph (211867-47-9), (9.4) Iprovalicarb (140923-17-7), (9.5) Mandipropamid (374726-62-2), (9.6) Polyoxins (11113-80-7), (9.7) Polyoxorim (22976-86-9), (9.8) Validamycin A (37248-47-8) und (9.9) Valifenalat (283159-94-4; 283159-90-0).
(10) Inhibitoren der Lipid- und Membran-Synthese, wie beispielsweise (10.1) Biphenyl (92-52-4), (10.2) Chloroneb (2675-77-6), (10.3) Dicloran (99-30-9), (10.4) Edifenphos (17109-49-8), (10.5) Etridiazol (2593-15-9), (10.6) Iodocarb (55406-53-6), (10.7) Iprobenfos (26087-47-8), (10.8) Isoprothiolan (50512-35-1), (10.9) Propamocarb (25606-41-1), (10.10) Propamocarb Hydrochlorid (25606-41-1), (10.11) Prothiocarb (19622-08-3), (10.12) Pyrazophos (13457-18-6), (10.13) Quintozen (82-68-8), (10.14) Tecnazene (117-18-0) und (10.15) Tolclofos-Methyl (57018-04-9).
(11) Inhibitoren der Melanin-Biosynthese, wie beispielsweise (11.1) Carpropamid (104030-54-8), (11.2) Diclocymet (139920-32-4), (11.3) Fenoxanil (115852-48-7), (11.4) Fthalid (27355-22-2), (11.5) Pyroquilon (57369-32-1), (11.6) Tricyclazol (41814-78-2) und (11.7) 2,2,2-Trifluorethyl {3-methyl-1-[(4-methylbenzoyl)amino]butan-2-yl}carbamat (851524-22-6) (bekannt aus WO2005042474).
(12) Inhibitoren der Nukleinsäuresynthese, wie beispielsweise (12.1) Benalaxyl (71626-11-4), (12.2) Benalaxyl-M (Kiralaxyl) (98243-83-5), (12.3) Bupirimat (41483-43-6), (12.4) Clozylacon (67932-85-8), (12.5) Dimethirimol (5221-53-4), (12.6) Ethirimol (23947-60-6), (12.7) Furalaxyl (57646-30-7), (12.8) Hymexazol (10004-44-1), (12.9) Metalaxyl (57837-19-1), (12.10) Metalaxyl-M (Mefenoxam) (70630-17-0), (12.11) Ofurace (58810-48-3), (12.12) Oxadixyl (77732-09-3) und (12.13) Oxolinsäure (14698-29-4).
(13) Inhibitoren der Signaltransduktion, wie beispielsweise (13.1) Chlozolinat (84332-86-5), (13.2) Fenpiclonil (74738-17-3), (13.3) Fludioxonil (131341-86-1), (13.4) Iprodion (36734-19-7), (13.5) Procymidon (32809-16-8), (13.6) Quinoxyfen (124495-18-7) und (13.7) Vinclozolin (50471-44-8).
(14) Entkoppler, wie beispielsweise (14.1) Binapacryl (485-31-4), (14.2) Dinocap (131-72-6), (14.3) Ferimzon (89269-64-7), (14.4) Fluazinam (79622-59-6) und (14.5) Meptyldinocap (131-72-6).
(15) Weitere Verbindungen, wie beispielsweise (15.1) Benthiazol (21564-17-0), (15.2) Bethoxazin (163269-30-5), (15.3) Capsimycin (70694-08-5), (15.4) Carvon (99-49-0), (15.5) Chinomethionat (2439-01-2), (15.6) Pyriofenon (Chlazafenon) (688046-61-9), (15.7) Cufraneb (11096-18-7), (15.8) Cyflufenamid (180409-60-3), (15.9) Cymoxanil (57966-95-7), (15.10) Cyprosulfamide (221667-31-8), (15.11) Dazomet (533-74-4), (15.12) Debacarb (62732-91-6), (15.13) Dichlorophen (97-23-4), (15.14) Diclomezin (62865-36-5), (15.15) Difenzoquat (49866-87-7), (15.16) Difenzoquat Methylsulphat (43222-48-6), (15.17) Diphenylamin (122-39-4), (15.18) Ecomat, (15.19) Fenpyrazamin (473798-59-3), (15.20) Flumetover (154025-04-4), (15.21) Fluoromid (41205-21-4), (15.22) Flusulfamid (106917-52-6), (15.23) Flutianil (304900-25-2), (15.24) Fosetyl-Aluminium (39148-24-8), (15.25) Fosetyl-Calcium, (15.26) Fosetyl-Natrium (39148-16-8), (15.27) Hexachlorbenzol (118-74-1), (15.28) Irumamycin (81604-73-1), (15.29) Methasulfocarb (66952-49-6), (15.30) Methylisothiocyanat (556-61-6), (15.31) Metrafenon (220899-03-6), (15.32) Mildiomycin (67527-71-3), (15.33) Natamycin (7681-93-8), (15.34) Nickel Dimethyldithiocarbamat (15521-65-0), (15.35) Nitrothal-Isopropyl (10552-74-6), (15.36) Octhilinone (26530-20-1), (15.37) Oxamocarb (917242-12-7), (15.38) Oxyfenthiin (34407-87-9), (15.39) Pentachlorphenol und dessen Salze (87-86-5), (15.40) Phenothrin, (15.41) Phosphorsäure und deren Salze (13598-36-2), (15.42) Propamocarb-Fosetylat, (15.43) Propanosin-Natrium (88498-02-6), (15.44) Proquinazid (189278-12-4), (15.45) Pyrimorph (868390-90-3), (15.45e) (2E)-3-(4-Tert-butylphenyl)-3-(2-chlorpyridin-4-yl)-1-(morpholin-4-yl)prop-2-en-1-on (1231776-28-5), (15.45z) (2Z)-3-(4-Tert-butylphenyl)-3-(2-chlorpyridin-4-yl)-1-(morpholin-4-yl)prop-2-en-1-on (1231776-29-6), (15.46) Pyrrolnitrin (1018-71-9) (bekannt aus EP-A 1 559 320), (15.47) Tebufloquin (376645-78-2), (15.48) Tecloftalam (76280-91-6), (15.49) Tolnifanid (304911-98-6), (15.50) Triazoxid (72459-58-6), (15.51) Trichlamid (70193-21-4), (15.52) Zarilamid (84527-51-5), (15.53) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat (517875-34-2) (bekannt aus WO2003035617), (15.54) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-79-6), (15.55) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003319-80-9), (15.56) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon (1003318-67-9), (15.57) 1-(4-Methoxyphenoxy)-3,3-dimethylbutan-2-yl-1H-imidazol-1-carboxylat (111227-17-9), (15.58) 2,3,5,6-Tetrachlor-4-(methylsulfonyl)pyridin (13108-52-6), (15.59) 2,3-Dibutyl-6-chlorthieno[2,3-d]pyrimidin-4(3H)-on (221451-58-7), (15.60) 2,6-Dimethyl-1H,5H-[1,4]dithiino[2,3-c:5,6-c']dipyrrol-1,3,5,7(2H,6H)-tetron, (15.61) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon (1003316-53-7), (15.62) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon (1003316-54-8), (15.63) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon (1003316-51-5), (15.64) 2-Butoxy-6-iod-3-propyl-4H-chromen-4-on, (15.65) 2-Chlor-5-[2-chlor-1-(2,6-difluor-4-methoxyphenyl)-4-methyl-1H-imidazol-5-yl]pyridin, (15.66) 2-Phenylphenol und dessen Salze (90-43-7), (15.67) 3-(4,4,5-Trifluor-3,3-dimethyl-3,4-dihydroisoquinolin-1-yl)quinolin (861647-85-0) (bekannt aus WO2005070917), (15.68) 3,4,5-Trichlorpyridin-2,6-dicarbonitril (17824-85-0), (15.69) 3-[5-(4-Chlorphenyl)-2,3-dimethyl-1,2-oxazolidin-3-yl]pyridin, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazol-2-thiol, (15.73) 5-Chlor-N'-phenyl-N'-(prop-2-in-1-yl)thiophen-2-sulfonohydrazid (134-31-6), (15.74) 5-Fluor-2-[(4-fluorbenzyl)oxy]pyrimidin-4-amin (1174376-11-4) (bekannt aus WO2009094442), (15.75) 5-Fluor-2-[(4-methylbenzyl)oxy]pyrimidin-4-amin (1174376-25-0) (bekannt aus WO2009094442), (15.76) 5-Methyl-6-octyl[1,2,4]triazolo[1,5-a]pyrimidin-7-amin, (15.77) Ethyl-(2Z)-3-amino-2-cyan-3-phenylprop-2-enoat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-in-1-yloxy)phenyl]propanamid, (15.80) N-[(4-Chlorphenyl)(cyan)methyl]-3-[3-methoxy-4-(prop-2-in-1-yloxy)phenyl]propanamid, (15.81) N-[(5-Brom-3-chlorpyridin-2-yl)methyl]-2,4-dichlorpyridin-3-carboxamid, (15.82) N-[1-(5-Brom-3-chlorpyridin-2-yl)ethyl]-2,4-dichlorpyridin-3-carboxamid, (15.83) N-[1-(5-Brom-3-chlorpyridin-2-yl)ethyl]-2-fluor-4-iodpyridin-3-carboxamid, (15.84) N-{(E)-[(Cyclopropylmethoxy)imino][6-(difluormethoxy)-2,3-difluorphenyl]methyl}-2-phenylacetamid (221201-92-9), (15.85) N-{(Z)-[(Cyclopropylmethoxy)imino][6-(difluormethoxy)-2,3-difluorphenyl]methyl}-2-phenylacetamid (221201-92-9), (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid (922514-49-6), (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid (922514-07-6), (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid (922514-48-5), (15.90) Pentyl-{6-[({[(1-methyl-1H-tetrazol-5-yl)(phenyl)methyliden]amino}oxy)methyl]pyridin-2-yl}carbamat, (15.91) Phenazin-1-carbonsäure, (15.92) Chinolin-8-ol (134-31-6), (15.93) Chinolin-8-olsulfat(2:1) (134-31-6) und (15.94) Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise (16.1) 1-Methyl-3-(trifluormethyl)-N-[2'-(trifluormethyl)biphenyl-2-yl]-1H-pyrazol-4-carboxamid, (16.2) N-(4'-Chlorbiphenyl-2-yl)-3-(difluormethyl)-1-methyl-1H-pyrazol-4-carboxamid, (16.3) N-(2',4'-Dichlorbiphenyl-2-yl)-3-(difluormethyl)-1-methyl-1H-pyrazol-4-carboxamid, (16.4) 3-(Difluormethyl)-1-methyl-N-[4'-(trifluormethyl)biphenyl-2-yl]-1H-pyrazol-4-carboxamid, (16.5) N-(2',5'-Difluorbiphenyl-2-yl)-1-methyl-3-(trifluormethyl)-1H-pyrazol-4-carboxamid, (16.6) 3-(Difluormethyl)-1-methyl-N-[4'-(prop-1-in-1-yl)biphenyl-2-yl]-1H-pyrazol-4-carboxamid, (16.7) 5-Fluor-1,3-dimethyl-N-[4'-(prop-1-in-1-yl)biphenyl-2-yl]-1H-pyrazol-4-carboxamid, (16.8) 2-Chlor-N-[4'-(prop-1-in-1-yl)biphenyl-2-yl]pyridin-3-carboxamid, (16.9) 3-(Difluormethyl)-N-[4'-(3,3-dimethylbut-1-in-1-yl)biphenyl-2-yl]-1-methyl-1H-pyrazol-4-carboxamid, (16.10) N-[4'-(3,3-Dimethylbut-1-in-1-yl)biphenyl-2-yl]-5-fluor-1,3-dimethyl-1H-pyrazol-4-carboxamid, (16.11) 3-(Difluormethyl)-N-(4'-ethinylbiphenyl-2-yl)-1-methyl-1H-pyrazol-4-carboxamid, (16.12) N-(4'-Ethinylbiphenyl-2-yl)-5-fluor-1,3-dimethyl-1H-pyrazol-4-carboxamid, (16.13) 2-Chlor-N-(4'-ethinylbiphenyl-2-yl)pyridin-3-carboxamid, (16.14) 2-Chlor-N-[4'-(3,3-dimethylbut-1-in-1-yl)biphenyl-2-yl]pyridin-3-carboxamid (bekannt aus EP-A 1 559 320), (16.15) 4-(Difluormethyl)-2-methyl-N-[4'-(trifluormethyl)biphenyl-2-yl]-1,3-thiazol-5-carboxamid, (16.16) 5-Fluor-N-[4'-(3-hydroxy-3-methylbut-1-in-1-yl)biphenyl-2-yl]-1,3-dimethyl-1H-pyrazol-4-carboxamid, (16.17) 2-Chlor-N-[4'-(3-hydroxy-3-methylbut-1-in-1-yl)biphenyl-2-yl]pyridin-3-carboxamid, (16.18) 3-(Difluormethyl)-N-[4'-(3-methoxy-3-methylbut-1-in-1-yl)biphenyl-2-yl]-1-methyl-1H-pyrazol-4-carboxamid, (16.19) 5-Fluor-N-[4'-(3-methoxy-3-methylbut-1-in-1-yl)biphenyl-2-yl]-1,3-dimethyl-1H-pyrazol-4-carboxamid, (16.20) 2-Chlor-N-[4'-(3-methoxy-3-methylbut-1-in-1-yl)biphenyl-2-yl]pyridin-3-carboxamid, (16.21) (5-Brom-2-methoxy-4-methylpyridin-3-yl)(2,3,4-trimethoxy-6-methylphenyl)methanon, (16.22) N-[2-(4-{[3-(4-Chlorphenyl)prop-2-in-1-yl]oxy}-3-methoxyphenyl)ethyl]-N2-(methylsulfonyl)valinamid (220706-93-4), (16.23) 4-Oxo-4-[(2-phenylethyl)amino]butansäure und (16.24) But-3-yn-1-yl {6-[({[(Z)-(1-methyl-1H-tetrazol-5-yl)(phenyl)methylen]amino}oxy)methyl]pyridin-2-yl}carbamat.

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

### Planzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflan-zensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

### Pflanzenschutz - Behandlungsarten

Die Behandlung der Pflanzen und Pflanzenteile mit den erfindungsgemäßen Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Eine bevorzugte direkte Behandlung der Pflanzen ist die Blattapplikation, d.h. erfindungsgemäße Verbindungen der Formel (I) werden auf das Blattwerk aufgebracht, wobei die Behandlungsfrequenz und die Aufwandmenge auf den Befallsdruck des jeweiligen Schädlings abgestimmt sein kann.

Bei systemisch wirksamen Verbindungen gelangen die erfindungsgemäßen Verbindungen der Formel (I) auch über das Wurzelwerk in die Pflanzen. Die Behandlung der Pflanzen erfolgt dann durch Einwirkung der erfindungsgemäßen Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der erfindungsgemäßen Verbindungen der Formel (I) getränkt, oder durch die Bodenapplikation, d.h. die erfindungsgemäßen Verbindungen der Formel (I) werden in fester Form, (z.B. in Form eines Granulats) in den Standort der Pflanzen eingebracht. Bei Wasserreiskulturen kann das auch durch Zudosieren der Erfindung in einer festen Anwendungsform (z.B. als Granulat) in ein überflutetes Reisfeld sein.

### Saatgutbehandlung

Die Bekämpfung von Tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch Tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer erfindungsgemäßen Verbindungen der Formel (I) behandelt wird. Das erfindungsgemäße Verfahren zum Schutz von Saatgut und keimenden Pflanzen vor dem Befall von Schädlingen umfasst ein Verfahren, in dem das Saatgut gleichzeitig in einem Vorgang oder sequentiell mit einem Wirkstoff der Formel (I) und Mischungspartner behandelt wird. Es umfasst auch ein Verfahren, in dem das Saatgut zu unterschiedlichen Zeiten mit einem Wirkstoff der Formel (I) und Mischungspartner behandelt wird.

Die Erfindung bezieht sich ebenfalls auf die Verwendung der erfindungsgemäßen Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor Tierischen Schädlingen mit einer erfindungsgemäßen Verbindung der Formel (I) behandelt wurde. Die Erfindung bezieht sich auch auf Saatgut, welches zur gleichen Zeit mit einem Wirkstoff der Formel (I) und Mischungspartner behandelt wurde. Die Erfindung bezieht sich weiterhin auf Saatgut, welches zu unterschiedlichen Zeiten mit einem Wirkstoff der Formel (I) und Mischungspartner behandelt wurde. Bei Saatgut, welches zu unterschiedlichen Zeiten mit einem Wirkstoff der Formel (I) und Mischungs-partner behandelt wurde, können die einzelnen Wirkstoffe des erfindungsgemäßen Mittels in unterschiedlichen Schichten auf dem Saatgut enthalten sein. Dabei können die Schichten, die einen Wirkstoff der Formel (I) und Mischungspartner enthalten, gegebenenfalls durch eine Zwischenschicht getrennt sein. Die Erfindung bezieht sich auch auf Saatgut, bei dem ein Wirkstoff der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit der erfindungsgemäßen Verbindung der Formel (I) einem Filmcoating - Verfahren unterzogen wird, um Staubabrieb am Saatgut zu vermeiden.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit der erfindungsgemäßen Verbindung der Formel (I) Keimung und Auflauf des behandelten Saatguts gefördert werden können.

Ebenso ist es als vorteilhaft anzusehen, dass erfindungsgemäße Verbindungen der Formel (I) insbesondere auch bei transgenem Saatgut eingesetzt werden können.

Zu nennen ist auch, dass erfindungsgemäße Verbindungen der Formel (I) in Kombination mit Mitteln der Signaltechnologie eingesetzt werden können, wodurch beispielhaft eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einem erfindungsgemäßen Verbindungen der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die erfindungsgemäße Verbindung der Formel (I) allein oder in einer geeigneten Formulierung auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristyrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der erfindungsgemäßen Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

(Chirurgische, therapeutische und diagnostische Behandlung des menschlichen oder tierischen Körpers sind nicht Teil des erfindungsgemässen Gegenstandes). Darüber hinaus können die erfindungsgemäßen Wirkstoffe / Wirkstoffkombinationen zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Offenbarung schließt somit auch ein Verfahren zur Bekämpfung von Schädlingen ein.

Auf dem Gebiet der Tiergesundheit, d.h. dem Gebiet der Tiermedizin, sind die erfindungsgemäßen Wirkstoffe gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die erfindungsgemäßen Verbindungen, die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z.B. in der Aquakultur; oder gegebenenfalls auch Insekten wie Bienen.

Zu den Haustieren zählen zum Beispiel Säugetiere wie Hamster, Meerschweinchen, Ratten, Mäuse, Chinchillas, Frettchen oder insbesondere Hunde, Katzen; Stubenvögel; Reptilien; Amphibien oder Aquariumfische.

Gemäß einer bevorzugten Ausführungsform werden die erfindungsgemäßen Verbindungen an Säugetiere verabreicht.

Gemäß einer weiteren bevorzugten Ausführungsform werden die erfindungsgemäßen Verbindungen an Vögel, nämlich Stubenvögel oder insbesondere Geflügel, verabreicht.

Durch Verwendung der erfindungsgemäßen Wirkstoffe für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Wirkstoffe wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass der Wirkstoff den jeweiligen Parasiten abtöten, sein Wachstum verhindern oder seine Vermehrung verhindern kann.

Zu Beispielen für Arthropoden zählen, jedoch ohne Einschränkung:
aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.; aus der Ordnung Heteropterida,
zum Beispiel Cimex spp., Triatoma spp., Rhodnius spp., Panstrongylus spp.; sowie Lästlinge und Hygieneschädlinge aus der Ordnung Blattarida.

Weiterhin sind unter den Arthropoden die folgenden Akari beispielhaft, jedoch ohne Einschränkung, zu nennen:
aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Stemostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Zu Beispielen für parasitäre Protozoen zählen, jedoch ohne Einschränkung:
Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis.

Sarcomastigophora (Rhizopoda), wie Entamoebidae, zum Beispiel Entamoeba histolytica, Hartmanellidae, zum Beispiel Acanthamoeba sp., Harmanella sp.

Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii,

Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec.

Zu Beispielen für pathogene Endoparasiten, bei denen es sich um Helminthen handelt, zählen Plattwürmer (z.B. Monogenea, Cestodes und Trematodes), Rundwürmer, Acanthocephala und Pentastoma. Zu weiteren Helminthen zählen, jedoch ohne Einschränkung:
Monogenea: z.B.: Gyrodactylus spp., Dactylogyrus spp., Polystoma spp.

Cestodes: aus der Ordnung Pseudophyllidea zum Beispiel: Diphyllobothrium spp., Spirometra spp., Schistocephalus spp., Ligula spp., Bothridium spp., Diplogonoporus spp.

Aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.

Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.

Ründwürmer: Trichinellida zum Beispiel: Trichuris spp., Capillaria spp., Trichomosoides spp., Trichinella spp.

Aus der Ordnung Tylenchida zum Beispiel: Micronema spp., Strongyloides spp.

Aus der Ordnung Rhabditina zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.

Aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp.

Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.,

Aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der erfindungsgemäßen Wirkstoffe nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf erfindungsgemäße Verbindungen für die Verwendung als Arzneimittel.

Ein weiterer Aspekt bezieht sich auf erfindungsgemäße Verbindungen für die Verwendung als Antiendoparasitikum, insbesondere ein Helminthizid oder ein Mittel gegen Protozoen. Zum Beispiel eignen sich erfindungsgemäße Verbindungen für die Verwendung als Antiendoparasitikum, insbesondere ein Helminthizid oder Mittel gegen Protozoen, z.B. in der Tierzucht, in der Tierhaltung, in Ställen und auf
dem Hygienesektor.

Ein weiterer Aspekt wiederum betrifft erfindungsgemäße Verbindungen für die Verwendung als Antiektoparasitikum, insbesondere ein Arthropodizid wie ein Insektizid oder ein Akarizid. Zum Beispiel eignen sich erfindungsgemäße Verbindungen für die Verwendung als Antiektoparasitikum, insbesondere ein Arthropodizid wie ein Insektizid oder Akarizid, zum Beispiel in der Tierhaltung, in der Tierzucht, in Ställen, auf dem Hygienesektor.

### Vektorkontrolle

Die erfindungsgemäßen Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhaut-entzündung (FSME), Krim-Kongo-Fieber, Fleckfieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der vorliegenden Erfindung sind zur Verwendung in der Prävention von Krankheiten bzw. vor Krankheitserregern, die durch Vektoren übertragen werden, geeignet. Somit ist ein weiter Aspekt der vorliegenden Erfindung die Verwendung von erfindungsgemäßen Verbindungen zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die erfindungsgemäßen Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer erfindungsgemäßen Ausführungsform enthalten die erfindungsgemäßen Zusammensetzungen oder Mittel noch mindestens ein weiteres Insektizid und/oder mindestens ein Fungizid.

In einer weiteren Ausführungsform ist diese erfindungsgemäße Zusammensetzung eine anwendungsfertige (ready-to-use) Zusammensetzung, d.h., sie kann ohne weitere Änderungen auf das entsprechende Material aufgebracht werden. Als weitere Insektizide oder als Fungizide kommen die oben genannten in Frage.

Überraschenderweise wurde auch gefunden, dass die erfindungsgemäßen Wirkstoffe und Zusammensetzungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die erfindungsgemäßen Wirkstoffe und Zusammensetzungen allein oder in Kombinationen mit anderen Wirkstoffen als Antifouling-Mittel eingesetzt werden.

### Bekämpfung von tierischen Schädlingen auf dem Hygienesektor

Die erfindungsgemäßen Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Wirkstoffe oder Zusammensetzungen allein oder in Kombination mit anderen Wirk- und/oder Hilfsstoffen verwendet. Bevorzugt werden sie in Haushaltsinsektizid-Produkten verwendet. Die erfindungsgemäßen Wirkstoffe sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Verbindungen können nach üblichen, dem Fachmann bekannten Methoden hergestellt werden.

Im Reaktionsschema 1 ist das allgemeine Darstellungsverfahren A für die erfindungsgemäßen Verbindungen **(Iap)** abgebildet.

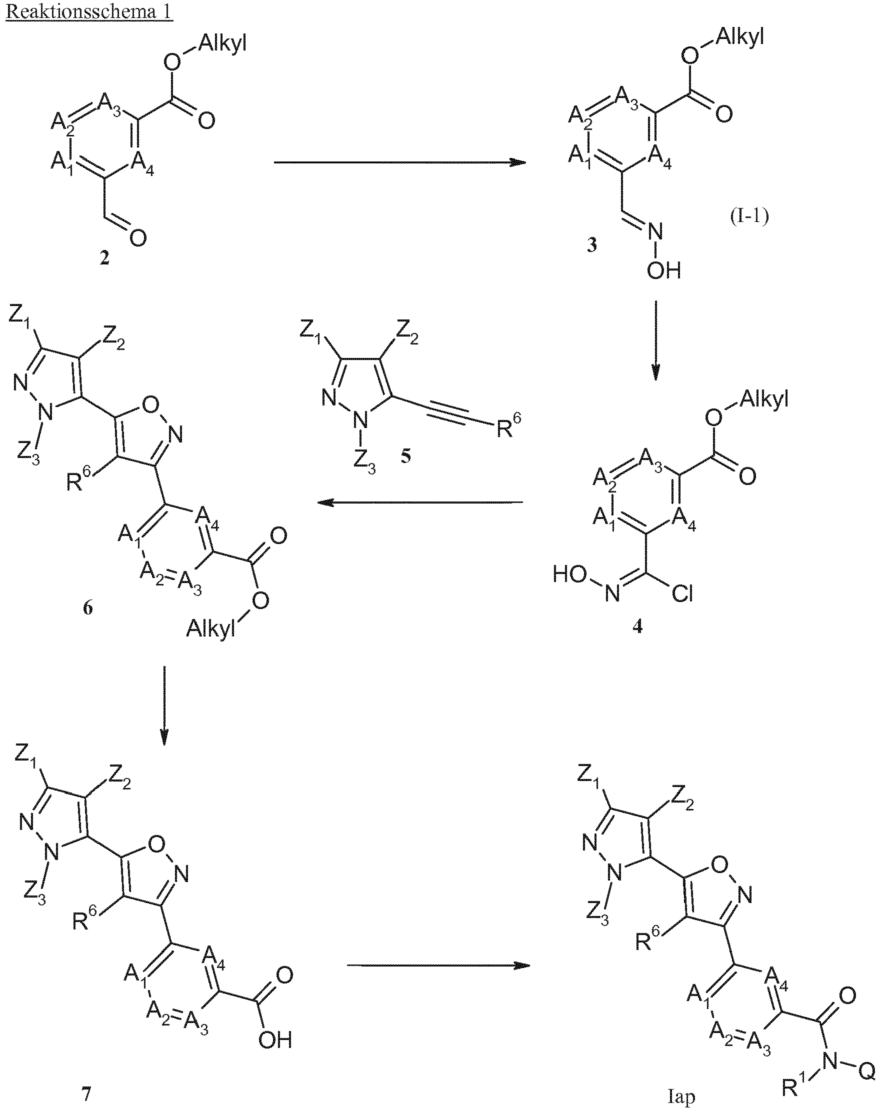

Die Reste A₁-A₄, Q, R⁶, R¹ und Z¹-Z³ haben die oben beschriebenen Bedeutungen.

### Stufe 1 des Darstellungsverfahrens der erfindungsgemäßen Verbindungen (Iap):

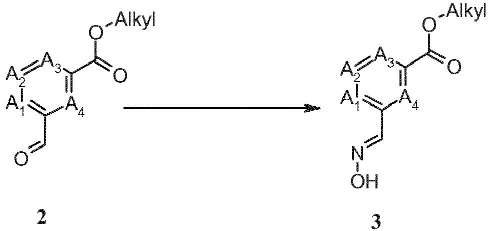

Erfindungsgemäße Verbindungen der allgemeinen Struktur **3** können in Analogie zu literaturbekannten Methoden aus den Ausgangsmaterialien der Struktur **2** hergestellt werden. Die Reste A1-A4 und Alkyl haben die oben beschriebenen Bedeutungen. Ausgangsverbindungen der Struktur **2** sind bekannt oder können nach bekannten Methoden hergestellt werden. Als Beispiele seien 3-Carbomethoxybenzaldehyd, 3-Carbomethoxy-4-chlor-benzaldehyd, 3-Carbomethoxy-4-brom-benzaldehyd,3-Carbomethoxy-4-fluor-benzaldehyd, 3-Carbomethoxy-4-chlor-5-fluor-benzaldehyd bzw. die entsprechenden Ethylester genannt. Sie können z.B. nach den in WO2010011584, Seiten 19-20; Journal of Organic Chemistry, 76 (2011) 1062 - 1071; WO2012114268, Seite 137; Journal of the American Chemical Society, 108 (1986) Seite 452-461 beschriebenen Methoden hergestellt werden.

Die Verbindungen der Strukturformel **3** sind teilweise bekannt, z.B. 3-Carbomethoxy-benzaldoxim, WO2004072050, Seite 14, teilweise jedoch noch unbekannt z.B. 3-Carbomethoxy-4-chlor-benzaldoxim, 3-Carbomethoxy-4-fluor-benzaldoxim, 3-Carbomethoxy-4-chlor-5-fluor-benzaldoxim, 3-Carbomethoxy-4-brom-benzaloxim. Die Herstellung der noch unbekannten Verbindungen **3** kann in Analogie zu den bekannten Verfahren zur Herstellung von Oximen aus Aldehyden erfolgen (H. Metzger in Houben-Weyl, Band X/4, Seite 55 ff, Georg Thieme Verlag Stuttgart 1968). Die Verbindungen der Strukturformel **3** können als reine Stereoisomere, aber auch als Mischungen der Konfigurationsisomere vorliegen.

### Stufe 2 des Darstellungsverfahrens der erfindungsgemäßen Verbindungen (Iap):

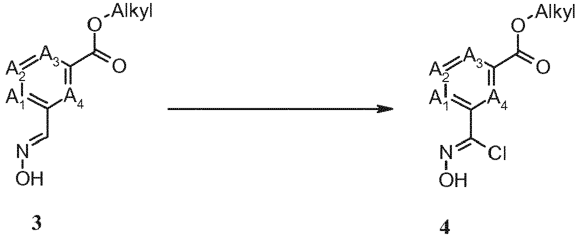

Erfindungsgemäße Verbindungen der allgemeinen Struktur **4** werden hergestellt indem man die Oxime der Struktur (X2) mit halogenierenden Mitteln umsetzt.

Die Reste A1-A4, Alkyl und Z1-Z3 haben die oben beschriebenen Bedeutungen.

Typische Verbindungen der Struktur **4** sind z.B. Carbomethoxy-4-chlor-N-hydroxybenzimidoylchlorid, 3-Carbomethoxy-4-fluor- N-hydroxybenzimidoylchlorid, 3-Carbomethoxy-4-chlor-5-fluor- N-hydroxybenzimidoylchlorid, 3 -Carbomethoxy-4-brom- N-hydroxybenzimidoylchlorid.

Geeignete halogenierende Verbindungen sind dem Fachmann bekannt wie z.B. Chlor, Brom, Iod, N-Chlorsuccinimid, N-Bromsuccinimid, N-Iodsuccinimid, 1,3-Dichlor-5-5-dimethylhydantoin, 1,3-Dibrom-5-5-dimethylhydantoin, Benzyltrimethylammonium-tetrachloroiodat und Natriumhypochlorit. Bevorzugt werden chlorierende Reagenzien eingesetzt.

Zur Durchführung der Reaktion können geeignete Lösungsmittel verwendet werden.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmop holin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril). Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphor- säuretriamid, Formamid, N-Methyl-formamid, N,N-Dimethyl-formamid, N,N-Dipropyl-formamid, N,N-Dibutyl-formamid, N-Methyl-pyrrolidin, N-Methyl-caprolactam, 1,3-Dimethyl-3 ,4,5,6- tetrahydro-2(1H)-pyrimidin, Octylpyrrolidon, Octylcaprolactam, 1,3-Dimethyl-2-imidazolindion, N-Formyl-piperidin, N,N'-1,4-Diformyl-piperazin) und Ketone (z.B. Aceton, Acetophenon, Methylethylketon, Methylbutylketon).

Als bevorzugte-Verdünnungsmittel kann jedes-Lösungsmittel verwendet-werden, das die Reaktion nicht beeinträchtigt, wie zum Beispiel Wasser. In Frage kommen aromatische Kohlenwasserstoffe wie Benzol, Toluol, Xylol oder Chlorbenzol; halogenierte Kohlenwasserstoffe wie Dichlormethan, Chloroform, 1,2-Dichlorethan oder Tetrachlorkohlenwasserstoff, offenkettige oder zyklische Ether wie Diethylether, Dioxan, Tetrahydrofuran oder 1 ,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid, N-Methyl-pyrrolidinon; Nitrile wie Acetonitril oder Propionitril; und andere inerte Lösungsmittel wie 1,3-Dimethyl-2- imidazolidinon; die Lösungsmittel können allein oder in Kombination von 2 oder mehr eingesetzt werden.

Die Reaktion kann in einem weiten Temperaturbereich ausgeführt werden. Gewöhnlich wird sie in einem Temperaturbereich von -78 bis 200 °C, bevorzugt bei Temperaturen zwischen -10 bis 150 °C durchgeführt. Die Reaktion kann unter erhöhtem als auch vermindertem Druck ausgeführt werden. Bevorzugt wird sie aber unter Normaldruck durchgeführt. Die Reaktionszeiten liegen zwischen 0,1 bis 72 Stunden, bevorzugt 1 bis 24 Stunden.

Zur Durchführung der Reaktion werden 1 bis 3 Mol, bevorzugt 1 bis 1,5 Mol an Halogenierungsmittel pro Mol der Verbindung der Struktur **3** in einem Lösungsmittel wie z.B. Dimethylformamid (DMF) umgesetzt

### Stufe 3 des Darstellungsverfahrens der erfindungsgemäßen Verbindungen (Iap):

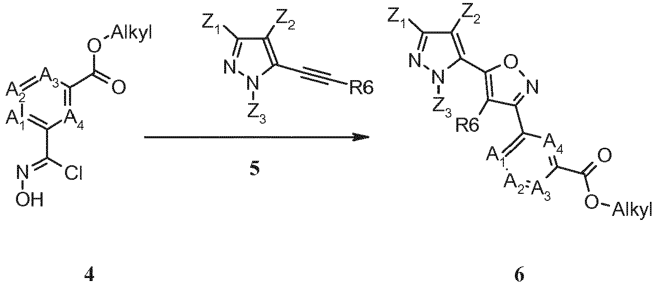

Erfindungsgemäße Verbindungen der allgemeinen Struktur **6** werden hergestellt indem man die Hydroxamsäurechloride der Struktur **4** mit Acetylenen der Struktur **5** umsetzt.

Die Reste A1-A4, R6, Alkyl und Z1-Z3 haben die oben beschriebenen Bedeutungen.

Typische Verbindungen der Struktur **4** sind z.B. Carbomethoxy-4-chlor-N-hydroxy-benzimidoylchlorid, 3-Carbomethoxy-4-fluor- N-hydroxy-benzimidoylchlorid, 3-Carbomethoxy-4-chlor-5-fluor- N-hydroxy-benzimidoylchlorid, 3-Carbomethoxy-4-brom-N-hydroxy-benzimidoylchlorid.

Zur Durchführung der Reaktion können geeignete Lösungsmittel verwendet werden.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmoϕholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril). Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 700C bis 1900C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphor- säuretriamid, Formamid, N-Methyl-formamid, N,N-Dimethyl-formamid, N,N-Dipropyl-formamid, N,N-Dibutyl-formamid, N-Methyl-pyrrolidin, N-Methyl-caprolactam, 1,3-Dimethyl-3 ,4,5,6- tetrahydro-2(1H)-pyrimidin, Octylpyrrolidon, Octylcaprolactam, 1,3-Dimethyl-2-imidazolindion, N-Formyl-piperidin, N,N'-1,4-Diformyl-piperazin) und Ketone (z.B. Aceton, Acetophenon, Methylethylketon, Methylbutylketon).

### Bevorzugte Lösungsmittel

Als bevorzugte-Verdünnungsmittel kann jedes-Lösungsmittel verwendet-werden, das die Reaktion nicht beeinträchtigt, wie zum Beispiel Wasser. In Frage kommen aromatische Kohlenwasserstoffe wie Benzol, Toluol, Xylol oder Chlorbenzol; halogenierte Kohlenwasserstoffe wie Dichlormethan, Chloroform, 1,2-Dichlorethan oder Tetrachlorkohlenwasserstoff, offenkettige oder zyklische Ether wie Diethylether, Dioxan, Tetrahydrofuran oder 1 ,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid, N-Methyl-pyrrolidinon; Nitrile wie Acetonitril oder Propionitril; und andere inerte Lösungsmittel wie 1,3-Dimethyl-2- imidazolidinon; die Lösungsmittel können allein oder in Kombination von 2 oder mehr eingesetzt werden.

Bei den Umsetzungen der Verbindungen der Struktur 4 mit den Acetylenen der Struktur 5 können Basen zugesetzt werden. Als Beispiele sind zu nennen Erdalkali- oder Alkalimetallverbindungen (z.B. Hydroxide, Hydride, Oxide und Carbonate des Lithiums, Natriums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetra-butyl-guanidin (CyTBG), Cyclohexyltetramethylguanidin (CyTMG), N,N,N,N-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methyl-piperidin, N-Methyl-imidazol, N-Methyl-pyrazol, N-Methyl-morpholin, N-Methylhexamethylendiamin, Pyridin, 4-Pyrrolidinopyridin. 4-Dimethyl-amino-pyridin, Chinolin, α-Picolin, β-Picolin, Isochinolin, Pyrimidin, Acridin, N,N,N',N'-Tetra-methylendiamin, N,N,N',N'-Tetraethylendiamin, Chinoxalin, N-Propyl-diisopropylamin, N-Ethyl-diisopropylamin, N,N'-Dimethylcyclohexylamin, 2,6-Lutidin, 2,4-Lutidin oder Triethyldiamin). Des Weiteren kann auch Silber(I)cyanid als Base und Aktivator eingesetzt werden [Journal of Organic Chemistry. 1992, 57, 4394-4400; Journal of Medicina Chemistry 1992, 35, 3905-3918; Journal of Organic Chemistry 2003, 68, 1843-1851]

### Bevorzugte Basen

Als bevorzugtes basisches Reaktionshilfsmittel kann eine organische Base wie Triethylamin, Ethyl-diisopropylamin, Tri-n-butylamin, Pyridin und 4-Dimethylamino-pyridin verwendet werden; Des Weiteren können beispielsweise folgende Basen eingesetzt werden: Alkalimetallhydroxide wie z.B Natriumhydroxid und Kaliumhydroxid; Carbonate wie Natriumhydrogencarbonat und Kaliumcarbonat; Phosphate wie Dikalium-hydrogenphosphat und Tri-natriumphosphat.

Die Reaktion kann in einem weiten Temperaturbereich ausgeführt werden. Gewöhnlich wird sie in einem Temperaturbereich von -78 bis 200 °C, bevorzugt bei Temperaturen zwischen -10 bis 150 °C durchgeführt. Die Reaktion kann unter erhöhtem als auch vermindertem Druck ausgeführt werden. Bevorzugt wird sie aber unter Normaldruck durchgeführt. Die Reaktionszeiten liegen zwischen 0,1 bis 72 Stunden, bevorzugt 1 bis 24 Stunden.

Zur Durchführung der Reaktion werden z.B. 1-2 Moläquivalente der Verbindungen der Struktur 5 und 1 Moläquivalent bis zu einem leichten Überschuss an Base pro Mol der Verbindung der Struktur 4 in einem Lösungsmittel wie z.B. Dimethylformamid (DMF) umgesetzt

Die Stufen 2 und 3 zur Herstellung der Verbindungen der Struktur **6** können in einzelnen Schritten oder auch als Eintopf-Reaktion durchgeführt werden.

Die letzten Stufen zur Herstellung der erfindungsgemäßen Verbindungen (Iap) sind die Hydrolyse des Carbonsäureesters **6** und Amidierung der Carbonsäure **7** [WO2010-051926; WO2010-133312].

Im Reaktionsschema **2** ist das allgemeine Darstellungsverfahren B für die erfindungsgemäßen Verbindungen **(Ibg-Ibi)** abgebildet.

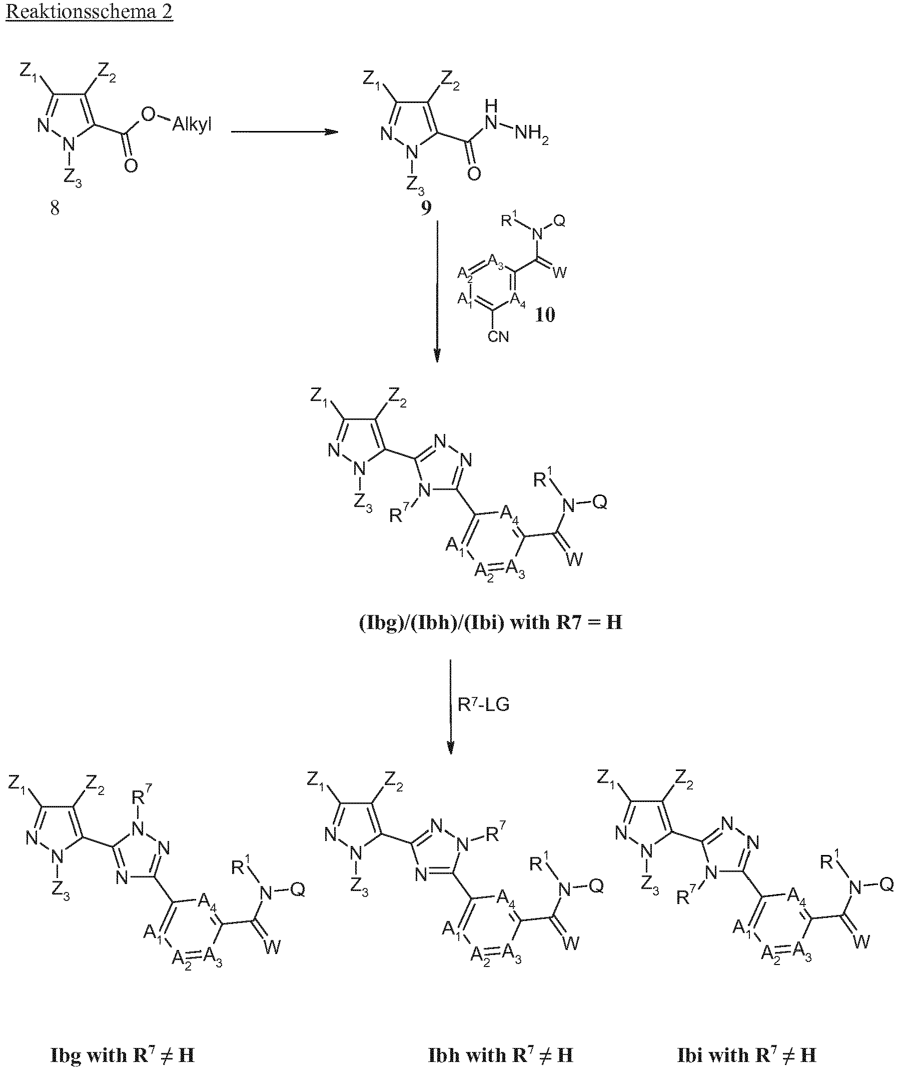

Die Reste A₁-A₄, Q, W, R⁷, R⁶, R¹ und Z¹-Z³ haben die oben beschriebenen Bedeutungen. "Alkyl" steht für einen Alkylrest wie z.B. Methyl oder Ethyl. "LG" steht für eine Abgangsgruppe, z.B. Chlor, Brom oder Iod.

### Stufe 1 des Darstellungsverfahrens der erfindungsgemäßen Verbindungen (Ibg)/(Ibh)/(Ibi):

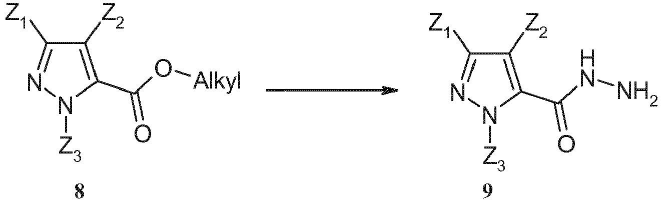

Die Reste Z¹-Z³ haben die oben beschriebenen Bedeutungen. "Alkyl" steht für einen Alkylrest wie z.B. Methyl oder Ethyl.

Verbindungen der allgemeinen Struktur **9** können in Analogie zu literaturbekannten Darstellungsverfahren [WO2011-121137; WO2010-0071196] aus Ausgangsverbindungen der allgemeinen Struktur 8 hergestellt werden.

### Stufe 2 des Darstellungsverfahrens der erfindungsgemäßen Verbindungen (Ibg)/(Ibh)/(Ibi):

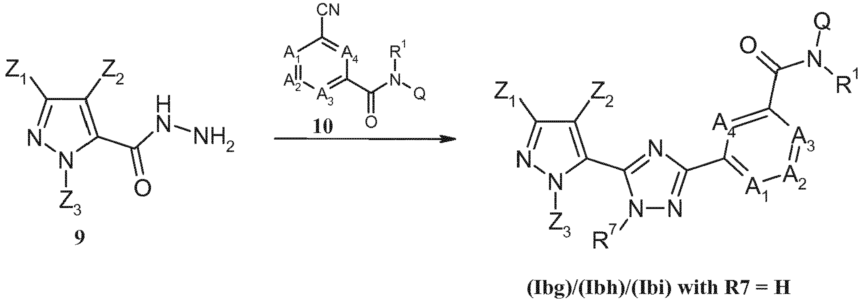

Die Reste A₁-A₄, Q, W, R¹ und Z¹-Z³ haben die oben beschriebenen Bedeutungen. R⁷ steht für Wasserstoff.

Verbindungen der allgemeinen Struktur **(Ibg)/(Ibh)/(Ibi)** mit R⁷ = H können in Analogie zu literaturbekommanten Darstellungsverfahren [WO2010-025558;WO2004-052280; Tetrahedron Letters 2005, 46, 3429-3432] aus den Verbindungen der allgemeinen Strukturen **9** und **10** dargestellt werden.

### Stufe 3 des Darstellungsverfahrens der erfindungsgemäßen Verbindungen (Ibg)/(Ibh)/(Ibi):

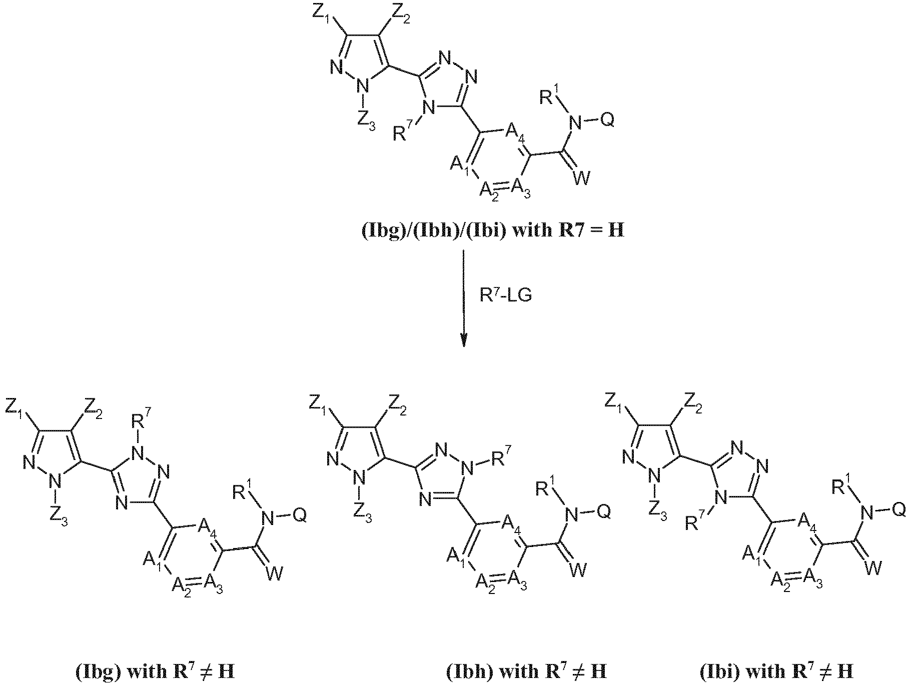

Verbindungen der allgemeinen Struktur **(Ibg/Ibh/Ibi)** mit R⁷ ≠ H können in Analogie zu literaturbekommanten Darstellungsverfahren [WO2007-071900; WO21012137089] aus den Verbindungen der allgemeinen Strukturen **(Ibg/Ibh/Ibi)** mit R⁷ = H dargestellt werden.

Im Reaktionsschema 3 ist das allgemeine Darstellungsverfahren C für die erfindungsgemäßen Verbindungen **(Iaa, Iac und Iaf)** abgebildet.

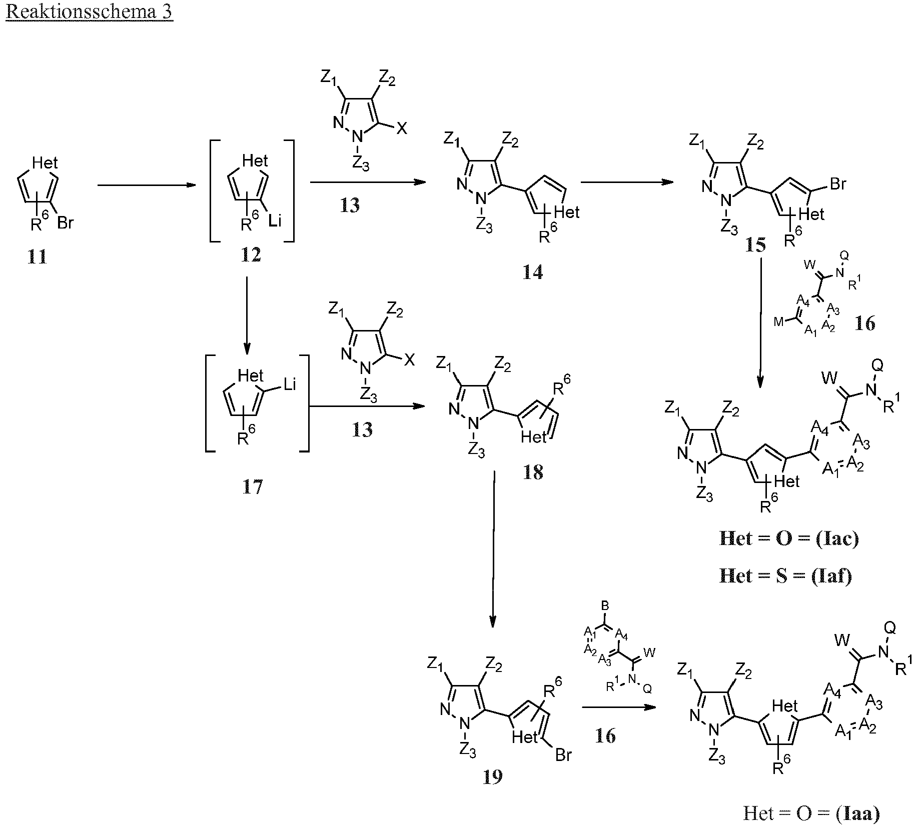

Die Reste A₁-A₄, Q, W, R⁶, R¹ und Z¹-Z³ haben die oben beschriebenen Bedeutungen. "Het" steht für ein Sauerstoff- bzw. Schwefelatom. "X" steht für eine Abgangsgruppe, z.B. Chlor, oder Fluor. "M" steht für eine Boronsäure, einen Boronsäureester oder ein Trifluorboronat.

### 1. Stufe des Darstellungsverfahren von (Iac) & (Iaf)

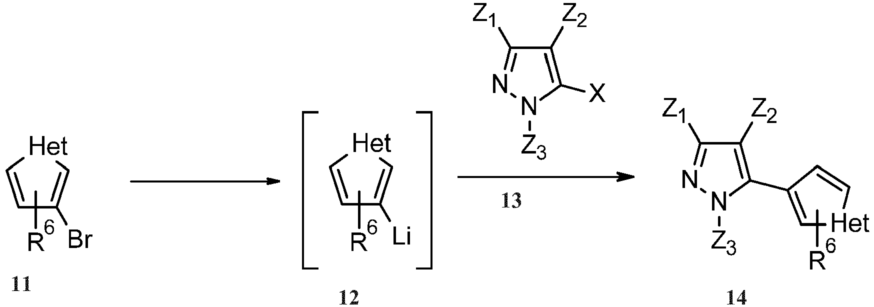

Die Reste R⁶ und Z¹-Z³ haben die oben beschriebenen Bedeutungen. "Het" steht für ein Sauerstoff- bzw. Schwefelatom. "X" steht für eine Abgangsgruppe, z.B. Chlor, oder Fluor.

Verbindungen der allgemeinen Struktur **14** können durch die Umsetzung Verbindungen der allgemeinen Struktur **13** und in situ hergestellten Lithiumorganylverbindungen der allgemeinen Struktur **12** dagestellt werden, Die Darstellung von Lithuimorganylverbindungen der allgemeinen Struktur **12** aus den korrespondierenden Bromverbindungen und deren Umsetzung in nucleophilen Substitutionsreaktionen ist literaturbekannt [Tetrahedron 1988, 44 (1), 81-90].

### 2. Stufe des Darstellungsverfahren von Iac & Iaf

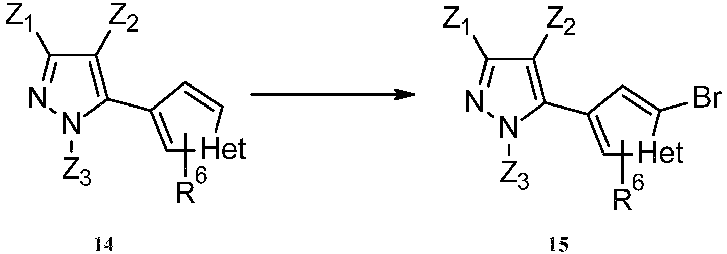

Die Reste R⁶ und Z¹-Z³ haben die oben beschriebenen Bedeutungen. "Het" steht für ein Sauerstoff- bzw. Schwefelatom.

Verbindungen der allgemeinen Struktur **15** können in Analogie zu literaturbekannten Verfahren durch Bromierung von Verbindungen der allgemeinenen Struktur **14** dargestellt werden [z.B. Organic Letters 2001, 13, 2129-2131].

### 3. Stufe des Darstellungsverfahren von Iac & Iaf

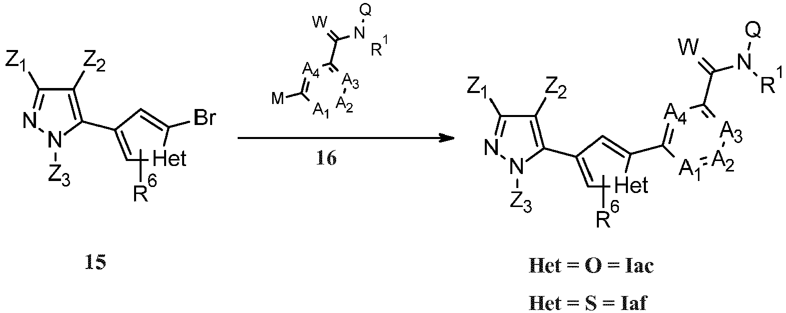

Die Reste A₁-A₄, Q, W, R⁶, R¹ und Z¹-Z³ haben die oben beschriebenen Bedeutungen. "Het" steht für ein Sauerstoff- bzw. Schwefelatom. "M" steht für eine Boronsäure, einen Boronsäureester oder ein Trifluorboronat.

Erfindungsgemäße Verbindungen der allgemeinen Struktur **Iac** und **Iaf** können mittels einer Palladiumkatalysierten Suzuki Reaktion in Analogie zu literaturbekannten Verfahren aus den Verbindungen der allgemeinen Struktur **15** und **16** hergestellt werden [WO2005-040110; WO2009-089508].

### 1. Stufe des Darstellungsverfahren von den erfingdungsgemäßen Verbindungen der allgemeinen Struktur Iaa

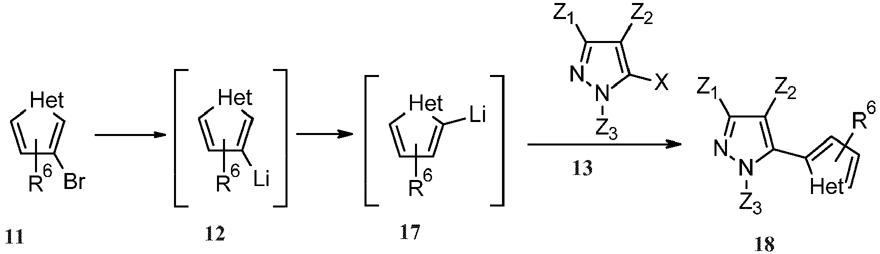

Die Reste R⁶ und Z¹-Z³ haben die oben beschriebenen Bedeutungen. "Het" steht für ein Sauerstoff- bzw. Schwefelatom. "X" steht für eine Abgangsgruppe, z.B. Chlor, oder Fluor.

Verbindungen der allgemeinen Struktur **18** können durch die Umsetzung Verbindungen der allgemeinen Struktur **13** und in situ hergestellten Lithiumorganylverbindungen der allgemeinen Struktur **17** dagestellt werden, Die Lithuimorganylverbindungen der allgemeinen Struktur **17** entstehen durch Isomerisierung aus den Lithiumorganylverbindungen der allgemeinen Struktur **12.** Die Lithiumorganylverbindungen der allgemeinen Struktur **12** können aus den korrespondierenden Bromverbindungen dargestellten werden.

Die Herstellung von solcherLithiumorganylverbindungen und deren Umsetzung in nucleophilen Substitutionsreaktionen ist literaturbekannt [z.B. Tetrahedron 1988, 44 (1), 81-90].

Alle weiteren Umsetzungen zur Darstellung der erfindungsgemäßen Verbindungen der allgemeinen Struktur **(Iaa)** erfolgen in Analogie zu den den oben beschriebenen Verfahren zur Darstellungen von **(Iac)** und **(Iaf).**

Im Reaktionsschema 4 ist das allgemeine Darstellungsverfahren D für die erfindungsgemäßen Verbindungen **(Iaj)** abgebildet.

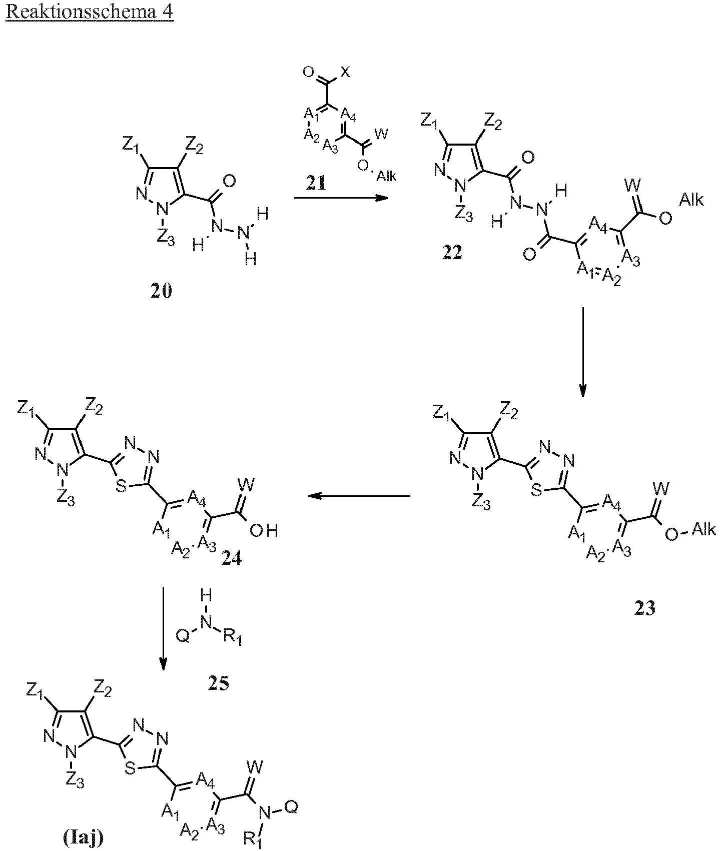

Die Reste A₁-A₄, Q, W, R¹ und Z¹-Z³ haben die oben beschriebenen Bedeutungen. "X" steht für eine Abgangsgruppe, z.B. Chlor. "Alk" steht für eine Alkylgruppe, z.B. Methyl oder Ethyl.

### 1. Stufe des Darstellungsverfahren D zur Herstellung der erfindungsgemäßen Verbindungen der allgemeinen Struktur (Iaj)

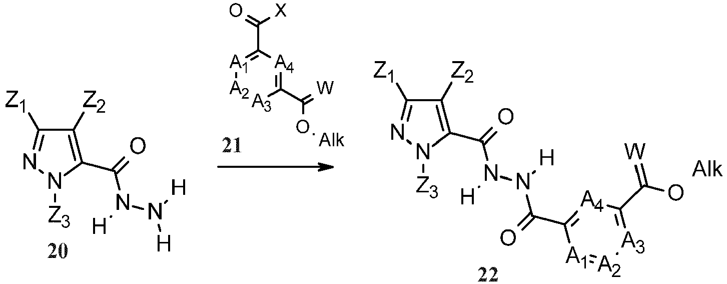

Die Reste A₁-A₄, W und Z¹-Z³ haben die oben beschriebenen Bedeutungen. "X" steht für eine Abgangsgruppe, z.B. Chlor. "Alk" steht für eine Alkylgruppe, z.B. Methyl oder Ethyl.

Verbindungen der allgemeinen Struktur **22** können in Analogie zu literaturbekannten Verfahren durch die Umsetzung von Verbindungen mit der allgemeinen Struktur **20** mit Verbindungen der allgemeinen Struktur 21 hergestellt werden [US2013-13953965].

### 2. Stufe des Darstellungsverfahren D zur Herstellung der erfindungsgemäßen Verbindungen der allgemeinen Struktur (Iaj)

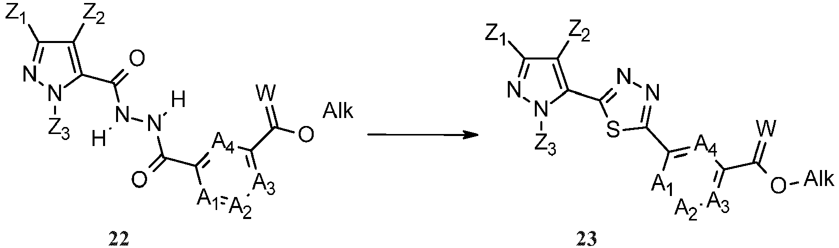

Die Reste A₁-A₄, W und Z¹-Z³ haben die oben beschriebenen Bedeutungen. "Alk" steht für eine Alkylgruppe, z.B. Methyl oder Ethyl.

Verbindungen der allgemeinen Struktur **22** können in Analogie zu literaturbekannten Verfahren durch die Umsetzung von der Verbindung der allgemeinen Struktur **23** mit Schwelfelungsreagentien wie z.B. Lawesson's Reagenz hergestellt werden [WO2013-149121].

Die letzten Stufen zur Herstellung der erfindungsgemäßen Verbindungen **(Iaj),** Hydrolyse des Carbonsäureesters **23** und Amidierung der Carbonsäure **24** mit Aminen der allgemeinen Struktur **25** können in Analogie zu literaturbekannten Verfahren durchgeführt werden [WO2010-051926; WO2010-133312]

Im Reaktionsschema **5** ist das allgemeine Darstellungsverfahren E für die erfindungsgemäßen Verbindungen **(Iaq)** abgebildet.

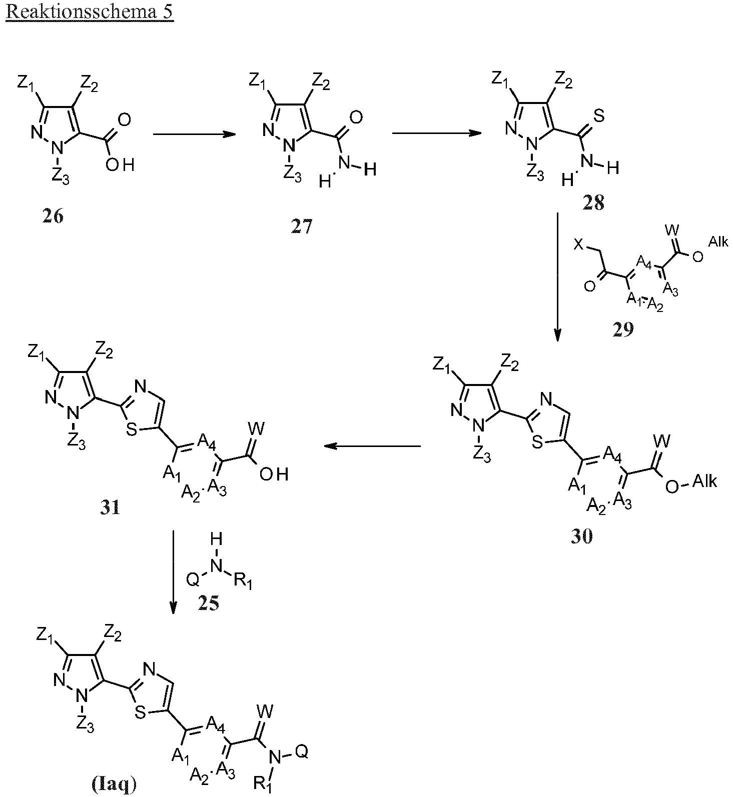

Die Reste A₁-A₄, Q, W, R¹ und Z¹-Z³ haben die oben beschriebenen Bedeutungen. "X" steht für eine Abgangsgruppe, z.B. Chlor oder Brom. "Alk" steht für eine Alkylgruppe, z.B. Methyl oder Ethyl.

Die 1. und die 2. Stufe des Darstellungsverfahren E zur Herstellung der erfindungsgemäßen Verbindungen der allgemeinen Struktur **(Iaq)** erfolgen nach literaturbekannten Vorschriften [Amidierung; WO2009-071706, Thiierung der CarbonylfunktionWO2013-018695]

### 3. Stufe des Darstellungsverfahren E zur Herstellung der erfindungsgemäßen Verbindungen der allgemeinen Struktur (Iaq)

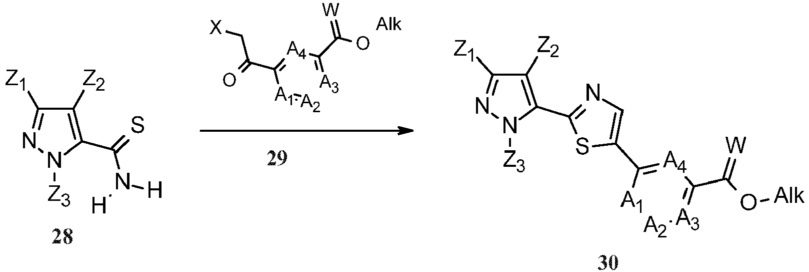

Die Reste A₁-A₄, W und Z¹-Z³ haben die oben beschriebenen Bedeutungen. "X" steht für eine Abgangsgruppe, z.B. Chlor oder Brom. "Alk" steht für eine Alkylgruppe, z.B. Methyl oder Ethyl.

Verbindungen der allgemeinen Struktur **30** können in Analogie zu literaturbekannten Verfahren durch die Umsetzung von der Verbindung der allgemeinen Struktur **28** mit alpha-Haloketonen der allgemeinen Struktur **29** hergestellt werden [WO2013-062027].

Die letzten Stufen zur Herstellung der erfindungsgemäßen Verbindungen (**Iaq**), Hydrolyse des Carbonsäureesters **30** und Amidierung der Carbonsäure **31** mit Aminen der allgemeinen Struktur **25** können in Analogie zu literaturbekannten Verfahren durchgeführt werden [WO2010-051926; WO2010-133312]

Im Reaktionsschema 6 ist das allgemeine Darstellungsverfahren F für die erfindungsgemäßen Verbindungen **(Iaw)** und **(Iax)** abgebildet.

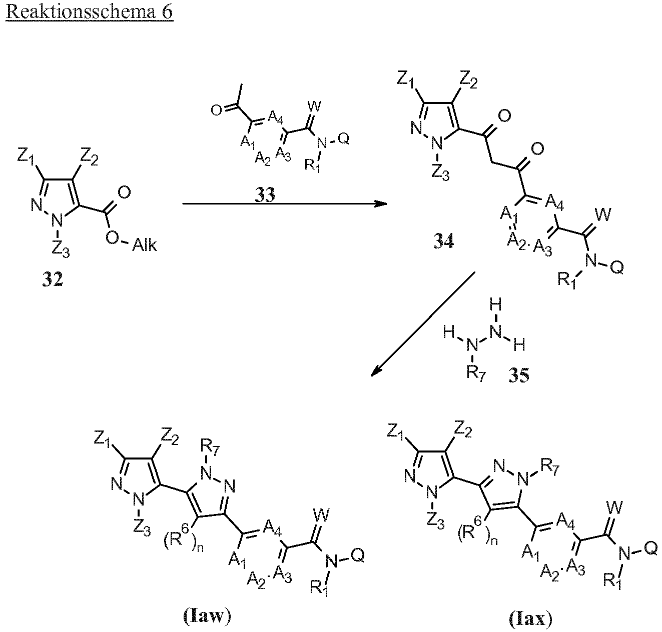

Die Reste A₁-A₄, Q, W, R⁶ R⁷ und Z¹-Z³ haben die oben beschriebenen Bedeutungen, wobei n =0 ist. R¹ steht für die oben beschriebenen Reste mit Ausnahme von Wasserstoff. "Alk" steht für eine Alkylgruppe, z.B. Methyl oder Ethyl.

### 1. Stufe des Darstellungsverfahren F zur Herstellung der erfindungsgemäßen Verbindungen der allgemeinen Strukturen (Iaw) und (Iax).

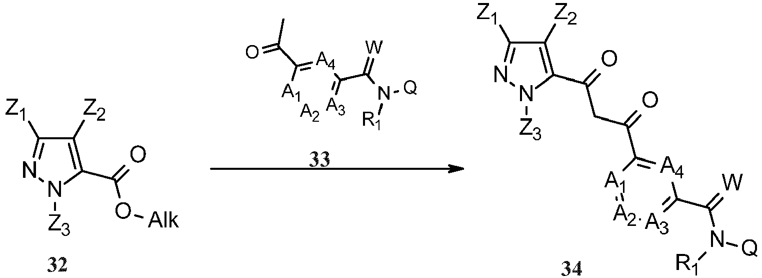

Die Reste A₁-A₄, W und Z¹-Z³ haben die oben beschriebenen Bedeutungen. R¹ steht für die oben benschriebenen Reste mit Ausnahme von Wasserstoff. "Alk" steht für eine Alkylgruppe, z.B. Methyl oder Ethyl.

Verbindungen der allgemeinen Struktur **34** können in Analogie zu literaturbekannten Verfahren durch die Umsetzung von Verbindungen mit der allgemeinen Struktur **32** mit Verbindungen der allgemeinen Struktur **33** hergestellt werden [US2012-13684606].

### 2. Stufe des Darstellungsverfahren F zur Herstellung der erfindungsgemäßen Verbindungen der allgemeinen Strukturen (Iaw) und (Iax).

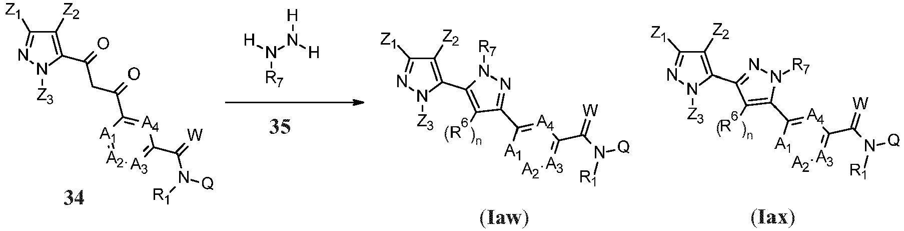

Die Reste A₁-A₄, W, R⁶, R⁷ und Z¹-Z³ haben die oben beschriebenen Bedeutungen, wobei n = 0 ist. R¹ steht für die oben benschriebenen Reste mit Ausnahme von Wasserstoff.

Verbindungen der allgemeinen Struktur **(Iaw)** und (**Iax**) können in Analogie zu literaturbekannten Verfahren durch die Umsetzung von Verbindungen mit der allgemeinen Struktur **34** mit Hydrazinen der allgemeinen Struktur **35** hergestellt werden [WO 2012052412].

Im Reaktionsschema 7 ist das allgemeine Darstellungsverfahren G für die erfindungsgemäßen Verbindungen (**Ibd**) abgebildet.

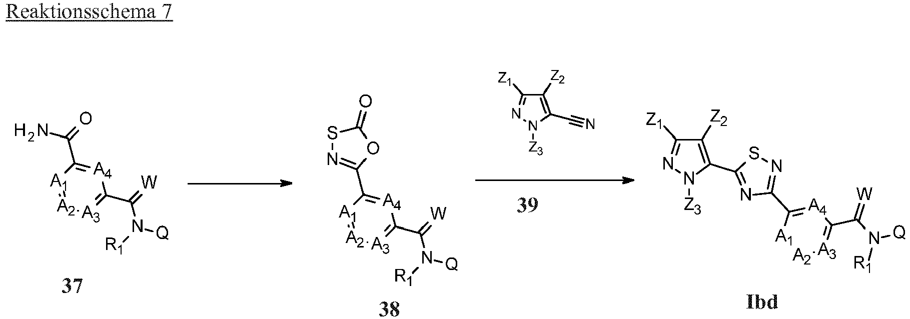

Die Reste A₁-A₄, Q, W, R¹ und Z¹-Z³ haben die oben beschriebenen Bedeutungen.

### 1. Stufe des Darstellungsverfahren G zur Herstellung der erfindungsgemäßen Verbindungen der allgemeinen Struktur (Ibd).

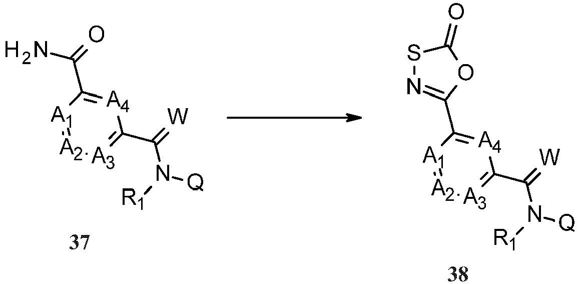

Die Reste A₁-A₄, R¹, Q und W haben die oben beschriebenen Bedeutungen.

Verbindungen der allgemeinen Struktur **38** können in Analogie zu literaturbekannten Verfahren durch die Umsetzung von Verbindungen mit der allgemeinen Struktur **37** mit Chlorcarbonylsulfenylchlorid hergestellt werden [WO2009-023721].

### 2. Stufe des Darstellungsverfahren G zur Herstellung der erfindungsgemäßen Verbindungen der allgemeinen Struktur (Ibd).

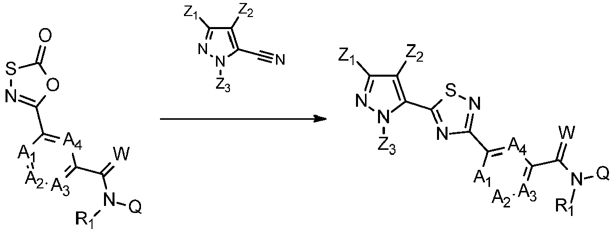

Die Reste A₁-A₄, Q, W, R¹ und Z¹-Z³ haben die oben beschriebenen Bedeutungen.

Verbindungen der allgemeinen Struktur (**Ibd**) können in Analogie zu literaturbekannten Verfahren durch die Umsetzung von Verbindungen mit der allgemeinen Struktur **38** mit Cyanoverbindungen der allgemeinen Strukur **39** hergestellt werden [WO2009-023721].

Im Reaktionsschema 8 ist das allgemeine Darstellungsverfahren H für die erfindungsgemäßen Verbindungen **(Ibe)** abgebildet.

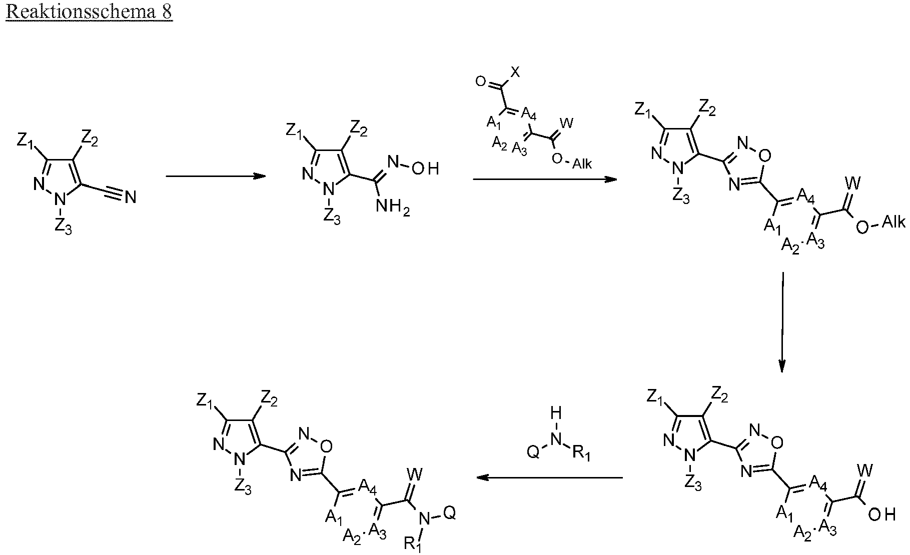

Die Reste A₁-A₄, Q, W, R¹ und Z¹-Z³ haben die oben beschriebenen Bedeutungen. "X" steht für eine Abgangsgruppe, z.B. Chlor oder Brom. "Alk" steht für eine Alkylgruppe, z.B. Methyl oder Ethyl.

### 1. Stufe des Darstellungsverfahren H zur Herstellung der erfindungsgemäßen Verbindungen der allgemeinen Struktur (Ibe).

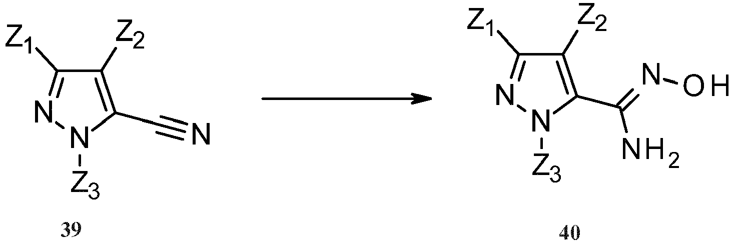

Die Reste Z¹-Z³ haben die oben beschriebenen Bedeutungen.

Verbindungen der allgemeinen Struktur **40** können in Analogie zu literaturbekannten Verfahren durch die Umsetzung von Verbindungen mit der allgemeinen Struktur **39** mit *N*-Hydroxylamin, bzw. dessen Salzen hergestellt werden [WO2014-008257].

### 2. Stufe des Darstellungsverfahren H zur Herstellung der erfindungsgemäßen Verbindungen der allgemeinen Struktur (Ibe).

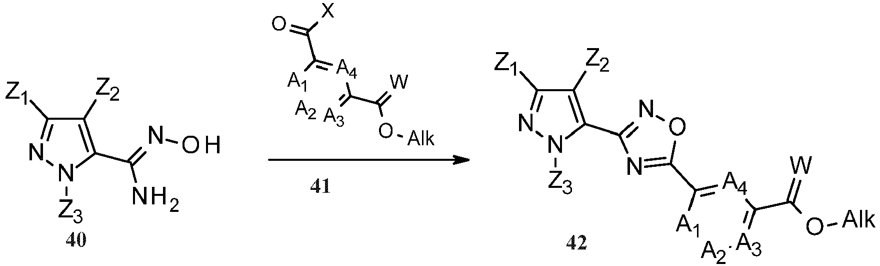

Die Reste A₁-A₄, Q, W, R¹ und Z¹-Z³ haben die oben beschriebenen Bedeutungen. "X" steht für eine Abgangsgruppe, z.B. Chlor oder Brom. "Alk" steht für eine Alkylgruppe, z.B. Methyl oder Ethyl.

Verbindungen der allgemeinen Struktur **42** können in Analogie zu literaturbekannten Verfahren durch die Umsetzung von Verbindungen mit der allgemeinen Struktur **40** mit Verbindungen der allgemeinen Struktur **41** hergestellt werden [WO2012-035023].

Die letzten Stufen zur Herstellung der erfindungsgemäßen Verbindungen **(Ibe),** Hydrolyse des Carbonsäureesters **42** und Amidierung der Carbonsäure **43** mit Aminen der allgemeinen Struktur **25** können in Analogie zu literaturbekannten Verfahren durchgeführt werden [WO2010-051926; WO2010-133312]

Im Reaktionsschema 9 ist das allgemeine Darstellungsverfahren I für die erfindungsgemäßen Verbindungen (**Ibf**) abgebildet.

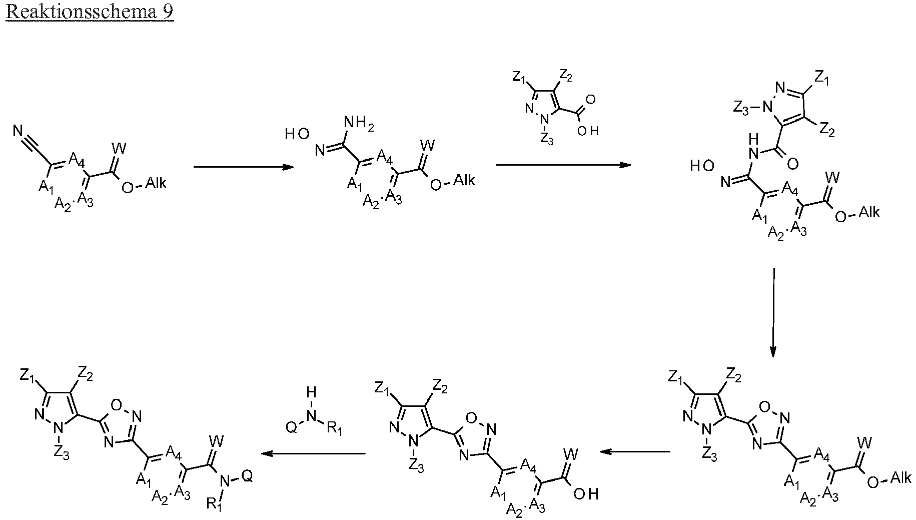

Die Reste A₁-A₄, Q, W, R¹ und Z¹-Z³ haben die oben beschriebenen Bedeutungen. "Alk" steht für eine Alkylgruppe, z.B. Methyl oder Ethyl.

Die 1. Stufe des Darstellungsverfahren I zur Herstellung von Verbindungen der allgemeinen Struktur **(45)** aus Verbindungen der allgemeinen Struktur **44** erfolgt in Analogie zu literaturbekannten Vorschriften [WO2014-008257].

### 2. Stufe des Darstellungsverfahren I zur Herstellung der erfindungsgemäßen Verbindungen der allgemeinen Struktur (Ibf).

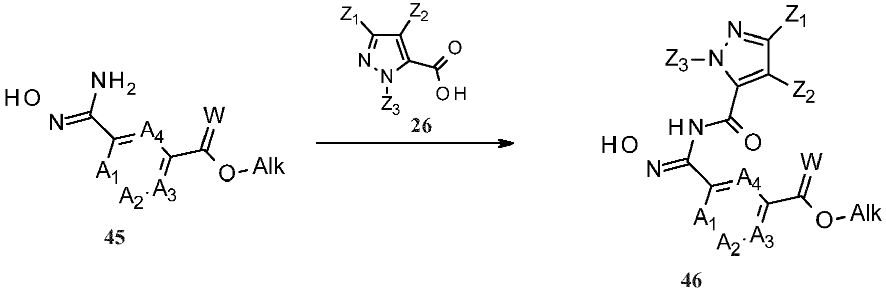

Die Reste A₁-A₄, Q, W, R¹ und Z¹-Z³ haben die oben beschriebenen Bedeutungen. "Alk" steht für eine Alkylgruppe, z.B. Methyl oder Ethyl.

Verbindungen der allgemeinen Struktur **46** können in Analogie zu literaturbekannten Verfahren durch die Umsetzung von Verbindungen mit der allgemeinen Struktur **45** mit Verbindungen der allgemeinen Struktur **26** hergestellt werden [WO2008-137816].

### 3. Stufe des Darstellungsverfahren I zur Herstellung der erfindungsgemäßen Verbindungen der allgemeinen Struktur (Ibf).

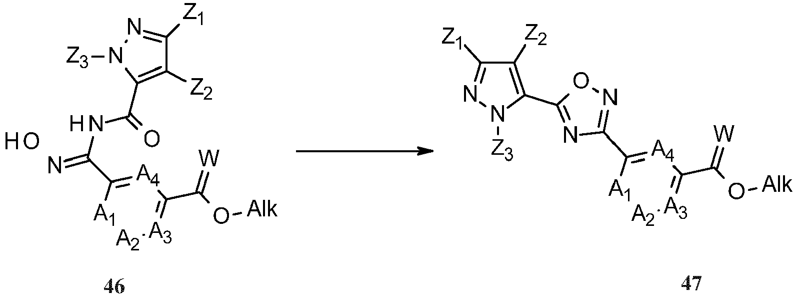

Die Reste A₁-A₄, Q, W, R¹ und Z¹-Z³ haben die oben beschriebenen Bedeutungen. "Alk" steht für eine Alkylgruppe, z.B. Methyl oder Ethyl.

Verbindungen der allgemeinen Struktur **47** können in Analogie zu literaturbekannten Verfahren aus Verbindungen mit der allgemeinen Struktur **46** hergestellt werden [WO2008-137816].

Die letzten Stufen zur Herstellung der erfindungsgemäßen Verbindungen (**Ibf**), Hydrolyse des Carbonsäureesters **47** und Amidierung der Carbonsäure **48** mit Aminen der allgemeinen Struktur **25** können in Analogie zu literaturbekannten Verfahren durchgeführt werden [WO2010-051926; WO2010-133312]

Im Reaktionsschema 10 ist das allgemeine Darstellungsverfahren J für die erfindungsgemäßen Verbindungen (**Iat**) abgebildet.

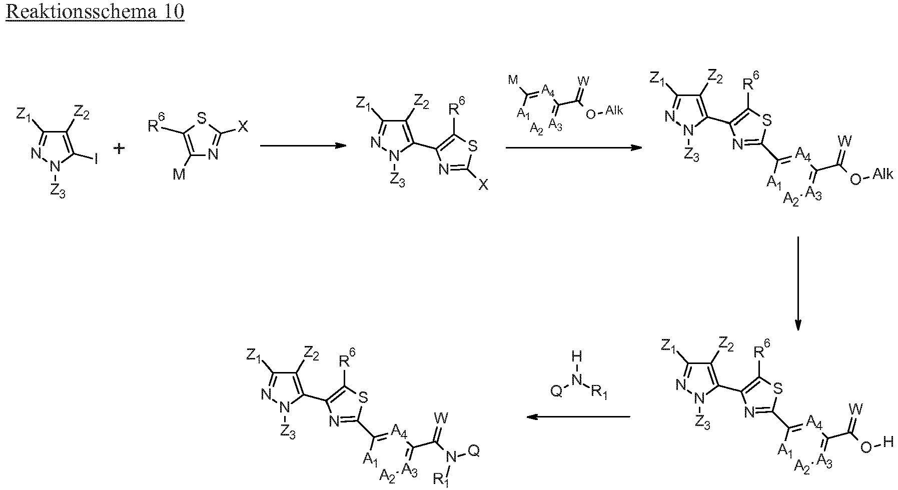

Die Reste A₁-A₄, Q, W, R¹ und Z¹-Z³ haben die oben beschriebenen Bedeutungen. R⁶ hat die oben beschriebenen Bedeutungen, steht bevorzugt aber für Wasserstoff. "X" steht für Chlor oder Brom, bevorzugt für Chlor. "M" steht für eine Boronsäure, einen Boronsäureester oder ein Trifluorboronat.

"Alk" steht für eine Alkylgruppe, z.B. Methyl oder Ethyl.

### 1. Stufe des Darstellungsverfahren J zur Herstellung der erfindungsgemäßen Verbindungen der allgemeinen Struktur (Iat).

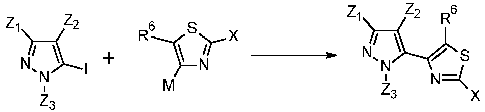

Die Reste Z¹-Z³ haben die oben beschriebenen Bedeutungen. R⁶ hat die oben beschriebenen Bedeutungen, steht bevorzugt aber für Wasserstoff. "X" steht für Chlor oder Brom, bevorzugt für Chlor. "M" steht für eine Boronsäure, einen Boronsäureester oder ein Trifluorboronat.

Verbindungen der allgemeinen Struktur **51** können in Analogie zu literaturbekannten Verfahren aus Verbindungen mit der allgemeinen Struktur **49** und **50** durch Übergangsmetall-katalysierte Kreuzkupplung hergestellt werden [WO2012142504].

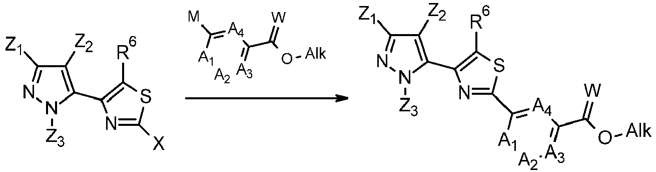

Die Reste A₁-A₄, W und Z¹-Z³ haben die oben beschriebenen Bedeutungen. R⁶ hat die oben beschriebenen Bedeutungen, steht bevorzugt aber für Wasserstoff. "X" steht für Chlor oder Brom, bevorzugt für Chlor. "M" steht für eine Boronsäure, einen Boronsäureester oder ein Trifluorboronat.

"Alk" steht für eine Alkylgruppe, z.B. Methyl oder Ethyl.

Verbindungen der allgemeinen Struktur **53** können in Analogie zu literaturbekannten Verfahren aus Verbindungen mit der allgemeinen Struktur **51** und **52** durch Übergangsmetall-katalysierte Kreuzkupplung hergestellt werden [European Journal of Organic Chemistry 2012, 31, 6248-6259].

Die letzten Stufen zur Herstellung der erfindungsgemäßen Verbindungen (**Iat**), Hydrolyse des Carbonsäureesters **53** und Amidierung der Carbonsäure **54** mit Aminen der allgemeinen Struktur **25** können in Analogie zu literaturbekannten Verfahren durchgeführt werden [WO2010-051926; WO2010-133312].

Oxidationsmittel für die Oxidation alkoholischer Gruppen sind bekannt (vgl. z. B. Oxidationsreagenzien in Organic Synthesis by Oxidation with Metal Compounds, Mijs, de Jonge, Plenum Verlag, New York, 1986; Manganese Compounds as Oxidizing Agents in Organic Chemistry, Arndt, Open Court Publishing Company, La Salle, IL, 1981; The Oxidation of Organic Compounds by Permanganate Ion and Hexavalent Chromium, Lee, Open Court Publishing Company, La Salle, IL, 1980). Eine Oxidation kann beispielsweise in Gegenwart von Permanganaten (z.B. Kaliumpermanganat), Metalloxiden (z.B. Mangandioxid, Chromoxide die beispielsweise in Dipyridin-chrom(VI)-oxid als Collins Reagenz (vgl. J. C. Collins et al., Tetrahedron Lett. 30, 3363-3366, 1968) verwendet werden) durchgeführt werden. Ebenfalls in Gegenwart von Pyridiniumchlorochromat (z.B. Corey's Reagenz) (vgl. auch R. O. Hutchins et al., Tetrahedron Lett. 48, 4167-4170, 1977; D. Landini et al. Synthesis 134-136, 1979) oder Ruthenium-tetroxid (vgl. S.-I. Murahashi, N. Komiya Ruthenium-catalyzed Oxidation of Alkenes, Alcohols, Amines, Amides, β-Lactams, Phenols and Hydrocarbons, in: Modern Oxidation Methods, Baeckvall, Jan-Erling (Eds.), Wiley-VCH-Verlag GmbH & Co. KGaA, 2004). Ebenfalls geeignet sind Ultraschall-induzierte Oxidationsreaktionen, sowie die Verwendung von Kaliumpermanganat (vgl. J. Yamawaki et al., Chem. Lett. 3, 379-380, 1983).

Zur Deblockierung/Abspaltung der Schutzgruppe SG können alle bekannten geeigneten sauren oder basischen Reaktionshilfsmittel nach den in der Literatur beschriebenen Verfahrensweises verwendet werden. Bei Verwendung von Schutzgruppen für Aminogruppen des Carbamat-Typs werden bevorzugt saure Reaktionshilfsmittel verwendet. Bei Verwendung der t-Butylcarbamat-Schutzgruppe (BOC-Gruppe) werden beispielsweise Mischungen von Mineralsäuren wie Salzsäure, Bromwasserstoffsäure, Salpetersäure, Schwefelsäure, Phosphorsäure oder organischen Säuren wie Benzoesäure, Ameisensäure, Essigsäure, Trifluoressigsäure, Methansulfonsäure, Benzolsulfonsäure oder Toluolsulfonsäure und einem geeigneten Verdünnungsmittel wie Wasser und/oder einem organischen Lösungsmittel wie Tetrahydrofuran, Dioxan, Dichlormethan, Chloroform, Essigester, Ethanol oder Methanol verwendet. Bevorzugt sind Mischungen von Salzsäure oder Essigsäure mit Wasser und/oder einem organischen Lösungsmittel wie Essigester.

Es ist bekannt, dass manche Reaktionen und Herstellungsverfahren besonders gut in Gegenwart von Verdünnungs- bzw. Lösungsmittel und basischer oder saurer Reaktionshilfsmitteln durchführbar sind. Mischungungen der Verdünnungs- bzw. Lösungsmittel sind ebenfalls einsetzbar. Die Verdünnungs- bzw. Lösungsmittel werden vorteilhafterweise in einer solchen Menge eingesetzt, dass das Reaktionsgemisch während des ganzen Verfahrens gut rührbar ist.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-t-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, *N,N-*Dimethyl-formamid, *N*,*N*-Dipropyl-formamid, *N,N*-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, *N,N'*-1,4-Diformyl-piperazin) und Ketone (z.B. Aceton, Acetophenon, Methylethylketon, Methylbutylketon).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Na-triums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), *N,N,N,N*-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, *N,N-*Dimethylanilin, *N,N-*Dimethyl-toluidin, *N,N-*Dimethyl-p-aminopyridin, N-Methyl-pyrrolidin, N-Methylpiperidin, N-Methyl-imidazol, N-Methyl-pyrazol, N-Methyl-morpholin, N-Methylhexamethylendiamin, Pyridin, 4-Pyrrolidinopyridin, 4-Dimethylamino-pyridin, Chinolin, α-Picolin, β-Picolin, Isochinolin, Pyrimidin, Acridin, *N,N*,N',N'-Tetramethylendiamin, *N,N,*N'*,*N'-Tetraethylendiamin, Chinoxalin, N-Propyl-diisopropylamin, N-Ethyl-diisopropylamin, *N,N'*-Dimethylcyclohexylamin, 2,6-Lutidin, 2,4-Lutidin oder Triethyldiamin).

Als saure Reaktionshilfsmittel zur Durchführung der erfindungsgemäßen Verfahren können alle Mineralsäuren (z.B. Halogenwasserstoff-säuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)-chlorid, Bortrifluorid oder sein Etherat, Titan-(V)chlorid, Zinn(V)-chlorid, und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure eingesetzt werden.

Sofern in den Reaktionsschemata Schutzgruppen vorgesehen sind, können alle allgemein bekannten Schutzgruppen verwendet werden. Insbesondere solche, die von Greene T. W., Wuts P. G. W. in Protective Groups in Organic Synthesis; John Wiley & Sons, Inc. 1999, "Protection for the hydroxyl group including 1,2- and 1,3-diols" beschrieben sind.

### Weiterhin eignen sich auch Schutzgruppen

vom Typ eines substituierten Methylethers (z.B. Methoxymethylether (MOM), Methylthiomethylether (MTM), (Phenyl-dimethylsilyl)methoxymethylether (SNOM-OR), Benzyloxymethylether (BOM-OR) para-Methoxybenzyloxymethylether (PMBM-OR), para-Nitrobenzyloxymethyl-ether, ortho-Nitrobenzyloxymethylether (NBOM-OR), (4-Methoxyphenoxy)-methylether (p-AOM-OR), Guaiacolmethylether (GUM-OR), t-Butoxymethylether, 4-Pentyloxy-methylether (POM-OR), Silyloxymethylether, 2-Methoxy-ethoxy-methylether (MEM-OR), 2,2,2-Trichlorethoxymethylether, Bis(2-chlorethoxy)-methylether, 2-(Trimethyl-silyl)ethoxymethylether (SEM-OR), Methoxymethylether (MM-OR));
vom Typ eines substituierten Ethylethers (z.B. 1-Ethoxyethylether (EE-OR), 1-(2-Chlorethoxy)ethylether (CEE-OR), 1-[2-(Trimethylsilyl)ethoxy]ethylether (SEE-OR), 1-Methyl-1-methoxyethylether (MIP-OR), 1-Methyl-1-benzyloxyethylether (MBE-OR), 1-Methyl-1-benzyloxy-2-fluor-ethylether (MIP-OR), 1-Methyl-1-phenoxyethylether, 2,2,-Trichlorethylether, 1,1-Dianisyl-2,2,2-trichlorethylether (DATE-OR), 1,1,1,3,3,3-Hexafluor-2-phenylisopropylether (HIP-OR), 2-Trimethylsilylethylether, 2-(Benzylthio)ethylether, 2-(Phenylselenyl)ethylether), eines Ethers (z.B. Tetrahydropyranylether (THP-OR), 3-Brom-tetrahydropyranylether (3-BrTHP-OR), Tetrahydrothiopyranylether, 1-Methoxy-cyclohexylether, 2- und 4-Picolylether, 3-Methyl-2-picolyl-N-oxido-ether, 2-Quinolinylmethylether (Qm-OR), 1-Pyrenylmethylether, Dipenylmethylether (DPM-OR), para, para'-Dinitrobenzhydrylether (DNB-OR), 5-Dibenzosuberylether, Triphenylmethylether (Tr-OR), alpha-Naphthyldiphenylmethylether, para-Methoxy-phenyldiphenylmethylether (MMTrOR), Di(para-methoxy-phenyl)phenylmethylether (DMTr-OR), Tri(para-methoxy-phenyl)phenylmethylether (TMTr-OR), 4-(4'-Brom-phenacyloxy) phenyldiphenylmethylether, 4,4',4"-Tris(4,5-dichlorphthalimido-phenyl)methylether (CPTr-OR), 4,4',4"-Tris(benzoyloxyphenyl)-methylether (TBTr-OR), 4,4'-Dimethoxy-3"-[N-(imidazolylmethyl)]-tritylether (IDTr-OR), 4,4'-Dimethoxy-3"-[N-(imidazolylethyl)carbamoyl]tritylether (IETr-OR), 1,1-Bis(4-methoxy-phenyl)-1'-pyrenyl-methylether (Bmpm-OR), 9-Anthrylether, 9-(9-Phenyl)xanthenylether (Pixyl-OR), 9-(9-Phenyl-10-oxo)anthryl (Tritylon-Ether), 4-Methoxy-tetrahydropyranylether (MTHP-OR), 4-Methoxy-tetrahydrothiopyranylether, 4-Methoxy-tetrahydrothiopyranyl-S,S-dioxid, 1-[(2-Chlor-4-methyl)phenyl]-4-methoxypiperidin-4-yl-ether (CTMP-OR), 1-(2-Fluorphenyl)-4-methoxy-piperidin-4-yl-ether (Fpmp-OR), 1,4-Dioxan-2-yl-ether, Tetrahydrofuranylether, Tetrahydrothiofuranylether, 2,3,3a,4,5,6,7,7a-Octahydro-7,8,8-trimethyl-4,7-methanbenzofuran-2-yl-ether (MBF-OR), t-Butylether, Allylether, Propargylether, para-Chlorphenylether, para-Methoxy-phenylether, para-Nitro-phenylether, para-2,4-Dinitro-phenylether (DNP-OR), 2,3,5,6-Tetrafluor-4-(trifluormethyl)phenylether, Benzylether (Bn-OR));
vom Typ eines sustituierten Benzylethers (z.B. para-Methoxy-benzylether (MPM-OR), 3,4-Dimethoxybenzylether (DMPM-OR), ortho-Nitro-benzylether, para-Nitro-benzylether, para-Halo-benzylether, 2,6-Dichlor-benzylether, para-Aminoacyl-benzylether (PAB-OR), para-Azido-benzylether (Azb-OR), 4-Azido-3-chlor-benzylether, 2-Trifluormethyl-benzylether, para-(Methylsulfinyl)benzylether (Msib-OR));
vom Typ eines Silylethers (z.B. Trimethylsilylether (TMS-OR), Triethylsilylether (TES-OR), Triisopropylsilylether (TIPS-OR), Dimethylisopropylsilylether (IPDMS-OR), Diethylisopropylsilylether (DEIPS-OR), Dimethylhexylsilylether (TDS-OR), t-Butyldimethylsilylether (TBDMS-OR), t-Butyldiphenylsilylether(TBDPS-OR), Tribenzylsilylether, Tri-para-xylylsilylether, Triphenylsilylether (TPS-OR), Diphenylmethylsilylether (DPMS-OR), Di-t-butylmethylsilylether (DTBMS-OR), Tris(trimethylsilyl)silylether (Sisylether), Di-t-butylmethylsilylether (DTBMS-OR), Tris(trimethylsilyl)silylether (Sisylether), (2-Hydroxystyryl)-dimethylsilylether (HSDMS-OR), (2-Hydroxystyryl)diisopropylsilylether (HSDIS-OR), t-Butylmethoxyphenyl-silylether (TBMPS-OR), t-Butoxydiphenylsilylether (DPTBOS-OR));
vom Typ eines Esters (z.B. Formiatester, Benzoylformiatester, Acetatester (Ac-OR), Chloracetatester, Dichloracetatester, Trichloracetatester, Trifluoracetatester, (TFA-OR), Methoxyacetatester, Triphenylmethoxyacetatester, Phenoxyacetatester, para-Chlor- phenoxyacetatester, Phenylacetatester, Diphenylacetatester (DPA-OR), Nicotinatester, 3-Phenyl-propionatester, 4-Pentoatester, 4-Oxopentoatester (Levulinate) (Lev-OR) 4,4-(Ethylendithio)-pentanoatester (LevS-OR), 5-[3-Bis(4-methoxyphenyl)hydroxy-methoxyphenoxy]-levulinatester, Pivaloatester (Pv-OR), 1-Adamantanoatester, Crotonatester, 4-Methoxy-crotonatester, Benzoatester (Bz-OR), para-Phenyl-benzoatester, 2,4,6-Trimethyl-benzoatester (Mesitoate), 4-(Methylthiomethoxy)-butyratester (MTMB-OR), 2-(Methylthiomethoxymethyl)-benzoatester (MTMT-OR),
vom Typ eines Carbonats (z.B. Methylcarbonat, Methoxymethylcarbonat, 9-Fluorenylmethylcarbonat (Fmoc-OR), Ethylcarbonat, 2,2,2-Trichlorethylcarbonat (Troc-OR), 1,1-Dimethyl-2,2,2-trichlorethylcarbonat (TCBOC-OR), 2-(Trimethylsilyl)ethylcarbonat (TMS-OR), 2-(Phenylsulfonyl)-ethylcarbonat (Ps-OR), 2-(Triphenylphosphonio)-ethylcarbonat (Peoc-OR), t-Butylcarbonat (Boc-OR), Isobutylcarbonat, Vinylcarbonat, Allylcarbonat (Alloc-OR), para-Nitro-phenylcarbonat, Benzylcarbonat (Z-OR), para-Methoxy-benzylcarbonat, 3,4-Dimethoxy-benzylcarbonat, ortho-Nitro-benzylcarbonat, para-Nitro-benzylcarbonat, 2-Dansylethylcarbonat (Dnseoc-OR), 2-(4-Nitrophenyl)ethylcarbonat (Npeoc-OR), 2-(2,4-Dinitrophenyl)ethylcarbonat (Dnpeoc)), und
vom Typ eines Sulfats (z.B. Allylsulfonat (Als-OR), Methansulfonat (Ms-OR), Benzylsulfonat, Tosylat (Ts-OR), 2-[(4-Nitrophenyl)ethyl]sulfonat (Npes-OR)).

Als Katalysatoren zur Durchführung einer katalytischen Hydrierung im erfindungsgemäßen Verfahren sind alle üblichen Hydrierkatalysatoren, wie beispielsweise Platin-Katalysatoren (z.B. Platin-Platte, Platin-Schwamm, Platin-Schwarz, kolloidales Platin, Platinoxid, Platindraht), Palladium-Katalysatoren (z.B. Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid , NickelKatalysatoren (z.B. reduziertes Nickel, Nickeloxid, Raney-Nickel), Ruthenium-Katalysatoren, Cobalt-Katalysatoren (z.B. reduziertes Cobalt, Raney-Cobalt), Kupfer-Katalysatoren (z.B. reduziertes Kupfer, Raney-Kupfer, Ullmann-Kupfer) geeignet. Bevorzugt werden Edelmetalllkatalysatoren (z.B. Platin- und Palladium- oder Ruthenium-Katalysatoren) verwendet, die gegebenenfalls auf einem geeigneten Träger (z.B. Kohlenstoff oder Silizium) aufgebraucht sind, Rhodium-Katalysatoren (z.B. Tris(triphenylphosphin)rhodium(I)-chlorid in Gegenwart von Triphenylphosphin). Ferner können "chiralen Hydrierkatalysatoren" (z. B. solche die chirale Diphosphinliganden enhalten wie (2S,3S)-(-)-2,3-Bis(diphenylphosphino)-butan [(S,S)-Chiraphos] oder (R)-(+)-2,2'- bzw. (S)-(-)-2,2'-Bis(diphenylphosphino)-1,1'-binaphthalin [R(+)-BINAP bzw. S(-)-BINAP]) verwendet werden, wodurch der Anteil eines Isomers im Isomerengemisch erhöht wird bzw. das Entstehen eines anderen Isomers nahezu vollständig unterdrückt wird.

Die Herstellung von Salzen der erfindungsgemäßen Verbindungen erfolgt nach Standardverfahren. Repräsentative Säureadditionssalze sind beispielsweise solche die durch Reaktion mit anorganischen Säuren, wie beispielsweise Schwefelsäure, Salzsäure, Bromwasserstoffsäure, Phosphorsäure oder organischen Carbonsäuren wie Essigsäure, Trifluoressigsäure, Zitronensäure, Bernsteinsäure, Buttersäure, Milchsäure, Ameisensäure, Fumarsäure, Maleinsäure, Malonsäure, Camphersäure, Oxalsäure, Phthalsäure, Propionsäure, Glycolsäure, Glutarsäure, Stearinsäure, Salicylsäure, Sorbinsäure, Weinsäure, Zimtsäure, Valeriansäure, Pikrinsäure, Benzoesäure oder organischen Sulfonsäuren wie Methansulfonsäure und 4-Toluolsulfonsäure gebildet werden.

Repräsentativ sind auch Salze von erfindungsgemäßen Verbindungen, die aus organischen Basen, wie beispielsweise Pyridin oder Triethylamine gebildet werden oder solche, die aus anorganischen Basen, wie beispielsweise Hydride, Hydroxide oder Karbonate des Natriums, Lithiums, Calciums, Magnesiums oder Bariums, gebildet werden, wenn die Verbindungen der allgemeinen Formel (I) ein zu dieser Salzbildung geeignetes Strukturelement aufweist.

Synthesemethoden zur Darstellung heterocyclischer N-Oxide und t-Aminen sind bekannt. Sie können mit Peroxysäuren (z.B. Peressigsäure und meta-Chlor-perbenzoesäure (MCPBA), Wasserstoffperoxid), Alkylhydroperoxide (z.B. t-Butylhydroperoxid), Natriumperborat und Dioxiranen (z.B. Dimethyldioxiran) erhalten werden. Diese Methoden sind beispielsweise von T. L. Gilchrist, in Comprehensive Organic Synthesis, Vol. 7, S. 748-750, 1992, S. V. Ley, (Ed.), Pergamon Press; M. Tisler, B. Stanovnik, in Comprehensive Heterocyclic Chemistry, Vol. 3, S. 18-20, 1984, A. J. Boulton, A. McKillop, (Eds.), Pergamon Press; M. R. Grimmett, B. R. T. Keene in Advances in Heterocyclic Chemistry, Vol. 43, S. 149-163, 1988, A. R. Katritzky, (Ed.), Academic Press; M. Tisler, B. Stanovnik, in Advances in Heterocyclic Chemistry, Vol. 9, S. 285-291, 1968, A. R. Katritzky, A. J. Boulton (Eds.), Academic Press; G. W. H. Cheeseman, E. S. G. Werstiuk in Advances in Heterocyclic Chemistry, Vol. 22, S. 390-392, 1978, A. R. Katritzky, A. J. Boulton, (Eds.), Academic Press beschrieben.

### Experimenteller Teil

### Darstellungsverfahren A

### Versuchsbeschreibungen:

### Beispiel Iap-1:

Die Herstellung von 4-Chlor-3-carbomethoxy-benzaldehyd ist bereits in der Literatur beschrieben (WO2010011584, Seiten 19-20; Molinaro, Carmela; Roy, Amelie; Lau, Stephen; Trinh, Thao; Angelaud, Remy; O'Shea, Paul D.; Shultz, Scott; Cameron, Mark; Corley, Ed; Steinhuebel, Dietrich; Weisel, Mark; Krska, Shane; Abele, Stefan; Funel, Jacques-Alexis Journal of Organic Chemistry, 76 (2011) 1062 - 1071; WO2012114268, Seite 137).

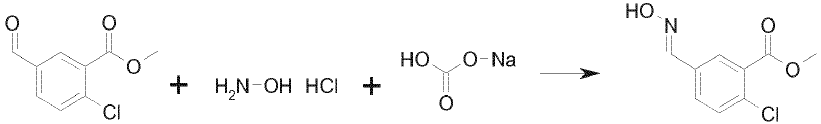

4,1 g (20,6 mmol) 4-Chlor-3-carbomethoxy-benzaldehyd wurden in 82 ml Methanol gelöst, 1,734 g (20,6 mmol) Natriumhydrogencarbonat zugegeben und auf 0 °C gekühlt. Dann wurden 5,738 g (82,5 mmol) Hydroxylamin Hydrochlorid zugegeben und bei 2,5 Stunden bei Raumtemperatur gerührt. Zur Aufarbeitung wurde danach im Vakuum am Rotationsverdampfer eingedampft und in 100 ml Essigester aufgenommen. Der Feststoff wurde abfiltriert und das Filtrat im Vakuum am Rotationsverdampfer eingedampft. Der Rückstand wurde dann zur Reinigung über eine Kartusche mit 120 g Kieselgel mittels eines Gradienten in Cyclohexan/Essigester 9:1 bis 7:3 (v/v) chromatogrphiert und ergab 2,68 g Methyl-2-chlor-5-[(hydroxyimino)methyl]benzoat.

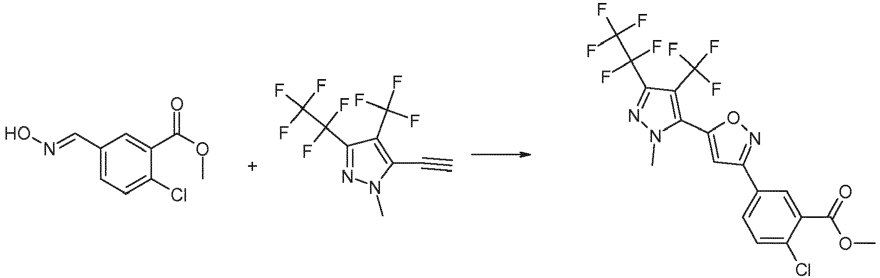

1,1 g (5,14 mmol) Methyl-2-chlor-5-[(hydroxyimino)methyl]benzoat wurden in 15 ml Dimethylformamid vorgelegt und 756 mg (5,66 mmol) N-Chlor-succinimid zugegeben. Es wurde 3,5 Stunden bei Raumtemperatur gerührt, danach weitere 190 mg (1,42 mmol) N-Chlor-succinimid nachgesetzt und weitere 2 Stunden bei Raumtemperatur gerührt. Die Mischung wurde dann auf 0 °C gekühlt und eine Lösung von 1,5 g (5,14 mmol) 5-Ethinyl-1-methyl-3-(pentafluorethyl)-4-(trifluormethyl)-1H-pyrazol in 5 ml Dimethylformamid zugetropft, und anschliessend 1,15 g (11,39 mmol) Triethylamin zugegeben. Der Ansatz wurde über Nacht bei Raumtemperatur gerührt. Zur Aufarbeitung wurde Wasser zugefügt und zweimal mit Dichlormethan extrahiert. Die vereinigten Extrakte wurden viermal mit Wasser gewaschen, mit Natriumsulfat getrocknet und im Vakuum am Rotationsverdampfer eingedampft. Der Rückstand wurde über eine Kartusche mit 40 g Kieselgel und einem Gradienten von reinem Cyclhexan nach Cyclohexan/Essigester 8:2 (v/v) gereinigt. Es wurden 0,57 g Methyl-2-chlor-5-{5-[1-methyl-3-(pentafluorethyl)-4-(trifluormethyl)-1H-pyrazol-5-yl]-1,2-oxazol-3-yl}benzoat erhalten.

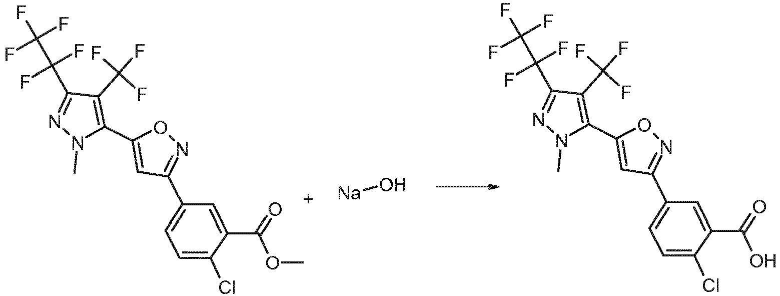

0,5 g (1,11 mmol) Methyl-2-chlor-5-15-[1-methyl-3-(pentafluorethyl)-4-(trifluormethyl)-1H-pyrazol-5-yl]-1,2-oxazol-3-yl}benzoat wurden in 28 ml Methanol vorgelegt und 1,11 ml (1,11 mmol) 1 M Natronlauge zugegeben. Es wurde dann 6 Stunden unter Rückfluss erhitzt. Anschliessend wurde am Rotationsverdampfer im Vakuum eingedampft. Der Rückstand wurde mit verdünnter wässriger Salzsäure angesäuert und dreimal mit Essigester extrahiert, die vereinigten Extrakte danach einmal mit gesättigter Kochsalzlösung gewaschen, mit Natriumsulfat getrocknet und am Rotationsverdampfer im Vakuum eingedampft. Als Rückstand wurden 0,54 g 2-Chlor-5-{5-[1-methyl-3-(pentafluorethyl)-4-(trifluormethyl)-1H-pyrazol-5-yl]-1,2-oxazol-3-yl}benzoesäure erhalten.

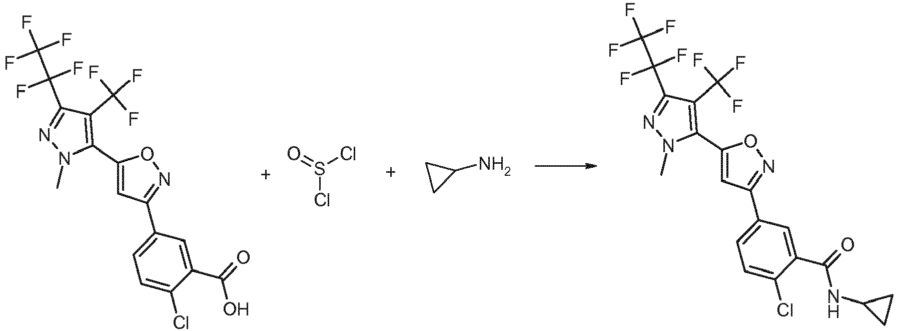

0,1 g (0,2 mmol) 2-Chlor-5-{5-[1-methyl-3-(pentafluorethyl)-4-(trifluormethyl)-1H-pyrazol-5-yl]-1,2-oxazol-3-yl}benzoesäure wurden in 1 ml Toluol gelöst und 122 mg (1,02 mmol) Thionylchlorid zugegeben. Der Ansatz wurde 2 Stunden auf 80 °C erhitzt und danach im Vakuum am Rotationsverdampfer eingedampft. Zur Entfernung von Resten an Thinylchlorid wurden dann 1 ml trockenes Toluol zugefügt und erneut eingedampft. Den Rückstand löste man dann in 0,5 ml Dichlormethan und tropfte die Lösung zu einer Lösung von 29 mg (51 mmol) Cycloproylamin in 0,5ml Dichlormethan bei 0 °C. Danach wurde eine Stunde ohne Kühlung nachgerührt. Zur Aufarbeitung wurde die Mischung mit 5 %iger wässriger NaH2PO4-Lösung gewaschen, mit Natriumsulfat getrocknet und im Vakuum am Rotationsverdampfer eingedampft. Der Rückstand wurde über eine 40 g Kartusche mit Kieselgel und einem Gradienten von Cyclohexan/Essigester 9:1 bis Cyclohexan/Essigester 7:3 (v/v) gereinigt. Es wurden 63 mg 2-Chlor-N-cyclopropyl-5-{5-[1-methyl-3-(pentafluorethyl)-4-(trifluormethyl)-1H-pyrazol-5-yl]-1,2-oxazol-3-yl}benzamid erhalten.
HPLC-MS^{a)}: logP = 4,06, Masse (m/z) = 529 [M+H]+.
¹H-NMR (400 MHz, d₃-Acetonitril): δ = 7,97 (s, 1 H), 7,95-7,96 (dd, J1=7,4 Hz, J2=1,5 Hz, 1 H), 7,6-7,62 (dd, J1=7,4 Hz, J2=1,5 Hz, 1H), 7,31 (s, 1 H), 7,02 (s (breit), 1 H (N-H)), 3,97 (s, 3 H), 2,83-2,88 (m, 1H), 0,76-0,8 (m, 2 H), 0,586-0,625 (m, 2 H).

### Darstellungsverfahren B

### Synthese von N-Benzyl-3-{5-[1-methyl-3-(pentafluorethyl)-4-(trifluormethyl)-1H-pyrazol-5-yl]-1H-1,2,4-triazol-3-yl}benzamid (Ibg-1), N-Benzyl-3-{1-methyl-5-[1-methyl-3-(pentafluorethyl)-4-(trifluormethyl)-1H-pyrazol-5-yl]-1H-1,2,4-triazol-3-yl}benzamid (Ibg-15) und N-Benzyl-3-{1-methyl-3-[1-methyl-3-(pentafluorethyl)-4-(trifluormethyl)-1H-pyrazol-5-yl]-1H-1,2,4-triazol-5-yl}benzamid (Ibh-2).

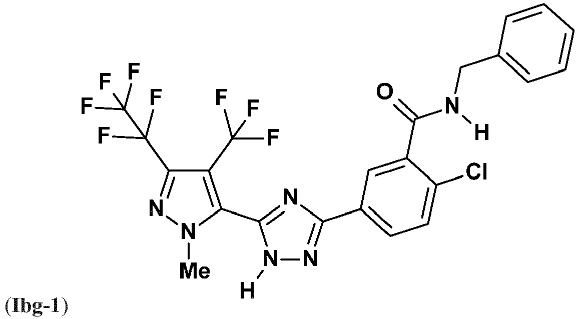

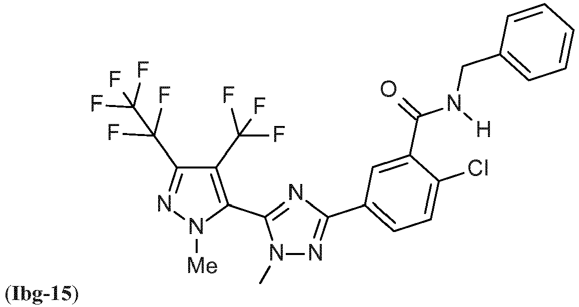

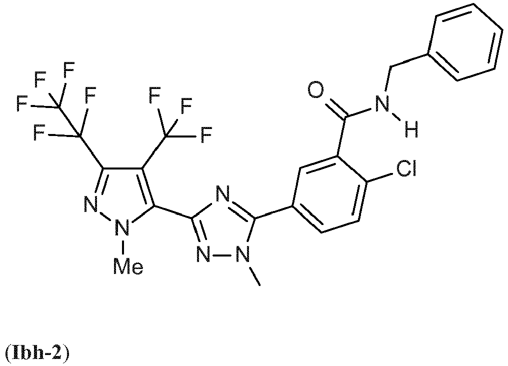

### Stufe 1

8,4 g Methyl-1-methyl-3-(pentafluorethyl)-4-(trifluormethyl)-1H-pyrazol-5-carboxylat (25,7 mmol) wurde in 50,0 mL Ethanol p.a. gelöst und dann trofpenweise mit 12,5 mL Hydrazin Hydrat (257 mmol) versetzt. Das Reaktionsgemisch wurde 16h unter Rückfluß erhitzt. Das abgekühlte Reaktionsgemisch wurde mit 50,0 mL Wasser verdünnt. Das Ethanol wurde am Rotationsverdampfer unter vermindertem Druck entfernt. Die wässrige Phase wurde anschließend dreimal mit je 50,0 mL Ethylacetat extrahiert. Die vereinigten organischen Phasen wurden über Magnesiumsulfat anhyd. getrocknet, filtriert und anschließend am Rotationsverdampfer bis zur Trocken eingeengt.

Es wurden 7,80g 1-Methyl-3-(pentafluorethyl)-4-(trifluormethyl)-1H-pyrazol-5-carbohydrazid als farbloser Feststoff isoliert. Das Rohprodukt wurde ohne Aufreinigung weiter umgesetzt.
HPLC-MS^{a)}: logP = 2,12, Masse (m/z) = 327 [M+H]+.
¹H-NMR (400 MHz, d3-Acetonitril): δ = 10,15 (s, 1H), 4,81 (s, 2H), 3,92 (s, 3H).

### Stufe2

200 mg 1-Methyl-3-(pentafluorethyl)-4-(trifluormethyl)-1H-pyrazol-5-carbohydrazid (0.61 mmol), 290 mg N-Benzyl-3-cyanbenzamid (1,22 mmol) und 42mg Kaliumcarbonat (0,3 mmol) wurden in 4,00 mL n-Butan-1-ol suspendiert und anschließend 2h bei 150°C in der Mikrowelle erhitzt. Die Reaktionslösung wurde am Rotationsverdampfer zur Trockene eingeengt. Der Rückstand wurde zweimal mit Toluol versetzt und wieder bis zur Trockene eingeengt. Das Rohprodukt wurde mittels MPLC an Silica aufgereinigt.

Es wurden 130 mg N-Benzyl-3-{5-[1-methyl-3-(pentafluorethyl)-4-(trifluormethyl)-1H-pyrazol-5-yl]-1H-1,2,4-triazol-3-yl}benzamid (Ibg-1) als farbloser Feststoff isoliert.
HPLC-MS^{a)}: logP = 3,80, Masse (m/z) = 545 [M+H]+.
¹H-NMR (400 MHz, d6-DMSO): δ = 15,37 (s, 1H), 9,24 (t, 1H), 8,58 (s, 1H), 8,20 (d, 1H), 8,07 (d, 1H), 7,71 (t, 1H), 7,22-7,36 (m. 5H), 4,52 (d, 2H), 4,08 (s, 3H).

### Stufe 3

102 mg (0,18 mmol) N-Benzyl-3-{5-[1-methyl-3-(pentafluorethyl)-4-(trifluormethyl)-1H-pyrazol-5-yl]-1H-1,2,4-triazol-3-yl}benzamid (**Ibg-1**) wurden in 4 mL THF p.a. gelöst und dann unter Eisbadkühlung nacheinander mit 52 mg (0,36 mmol) Kaliumcarbonat und 26,6 mg (0,18 mmol) Methyliodid versetzt. Das Reaktionsgemisch wurde bei Raumtemperatur 14 h gerührt und dann mit Wasser verdünnt. Die wässrige Phase wurde mehrfach mitEthylacetat extrahiert. Die vereinigten organischen Phasen wurden über Natriumsulfat getrocknet, abfiltriert und am Rotationsverdampfer unter vermindertem Druck eingeengt. Das Rohprodukt wurde mittels MPLC an Kieselgel aufgereinigt.

Es wurden 35 mg N-Benzyl-3-{1-methyl-5-[1-methyl-3-(pentafluorethyl)-4-(trifluormethyl)-1H-pyrazol-5-yl]-1H-1,2,4-triazol-3-yl}benzamid (**Ibg-15**) isoliert.
HPLC-MS^{a)}: logP = 4,24, Masse (m/z) = 559 [M+H]+.
¹H-NMR (400 MHz, d3-Acetonitril): δ =8,53 (d, 1H), 8,23 (d, 1H), 7,80 (d, 1H), 7,67 (s, 1H), 7,59 (t, 1H), 7,39-7,32 (m, 3H) 7,29-7,24 (m, 1H), 4,57 (d, 2H), 3,87 (s, 3H), 3,85 (s, 3H).

Es wurden 11 mg N-Benzyl-3-{1-methyl-3-[1-methyl-3-(pentafluorethyl)-4-(trifluormethyl)-1H-pyrazol-5-yl]-1H-1,2,4-triazol-5-yl}benzamid (**Ibh-2**) isoliert.
HPLC-MS^{a)}: logP = 4,16, Masse (m/z) = 559 [M+H]+.
¹H-NMR (400 MHz, d3-Acetonitril): δ =8,23-8.22 (m, 1H), 8,02-8,00 (m, 1H), 7,96-7,93 (m, 1H), 7,70-7,66 (m, 2H), 7,40-7,34 (m, 3H), 7,29-7,25 (m, 1H), 4,58 (d, 2H), 4,06 (s, 3H), 4,03 (s, 3H).

### Darstellungsverfahren C

### Synthese von 2-Chlor-N-(1-cyancyclopropyl)-5-{4-[1-methyl-3-(pentafluorethyl)-4-(trifluormethyl)-1H-pyrazol-5-yl]-2-furyl}benzamid (Iac-1)

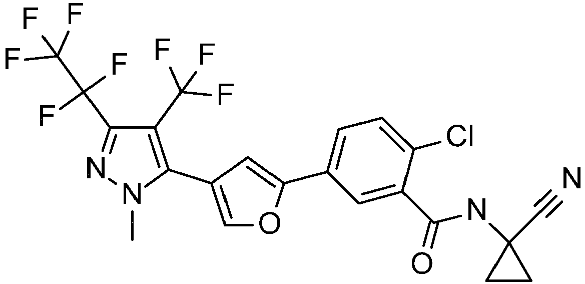

### Stufe 1

6,50 g (44,2 mmol) 3-Bromfuran wurden bei -78°C in 65,0 mL Tetrahydrofuran abs.vorgelegt und dann mit 27,6 mL 1,6M (44,2 mmol) n-Butyllithium Lösung versetzt. Die Reaktionsmischung wurde 15 Minuten bei -65°C gerührt und anschließend bei -65°C mit einer Lösung von 12,7 g (44,2 mmol) 5-Fluor-1-methyl-3-(pentafluorethyl)-4-(trifluormethyl)-1*H*-pyrazol in 20,0 mL Tetrahydrofuran abs. versetzt. Das Reaktionsgemisch wurde auf Raumtemperatur erwärmt. Nach 2h bei Raumtemperatur wurde das Reaktionsgemisch vorsichtig mit Wasser gequencht. Das Tetrahydrofuran wurde unter vermindertem Druck am Rotationsverdampfer entfernt. Die wässrige Phase wurde anschließend mehrfach mit Chloroform extrahiert. Die vereinigten org. Phasen wurde über Natriumsulftat getrocknet, filtiriert und am Rotationsverdampfer unter vermindertem Druck eingeengt. Das Rohprodukt wurde mittels MPLC an Silica aufgereinigt.

Es wurden 4,00 g 5-(3-Furyl)-1-methyl-3-(pentafluorethyl)-4-(trifluormethyl)-*1H*-pyrazol isoliert.
GC-MS: Index = 1276, Masse (m/z) = 334 [M]+.
¹H-NMR (400 MHz, d6-DMSO): δ =8,15 (s, 1H), 7,93-7,95 (m, 1H), 6,81 (s, 1H), 3,85 (s, 3H).

### Stufe 2

2,00 g (5,99 mmol) 5-(3-Furyl)-1-methyl-3-(pentafluorethyl)-4-(trifluormethyl)-*1H*-pyrazol gelöst in 20,0 mL THF pa wurden mit 1,28 g (7,19 mmol) N-Bromsuccinimid versetzt. Das Reaktionsgemisch wurde 2 h auf 50°C erwärmt. Nach dem Abkühlen auf Raumtemperatur wurde die Reaktionsmischung mit einer wässrigen Natriumhydrogensulfit Lösung versetzt. Das Gemisch wurde mehrfach mit

Chloroform extrahiert. Die vereinigten organischen Phasen wurde am Rotationsverdampfer eingeegnt und das erhaltene Rohprodukt mittels MPLC an Silica gereinigt.

Es wurden 1,00 g 5-(5-Brom-3-furyl)-1-methyl-3-(pentafluorethyl)-4-(trifluormethyl)-1*H*-pyrazol mit einer Reinheit von 55% nach LCMS isoliert.
HPLC-MS^{a)}: logP = 4,49, Masse (m/z) = 415 [M+H]+.

### Stufe 3

1,04 g (2.52 mmol) 5-(5-Brom-3-furyl)-1-methyl-3-(pentafluorethyl)-4-(trifluormethyl)-1*H-*pyrazol, 747 mg (2,52 mmol) Methyl-2-chlor-5-(4,4,5,5-tetramethyl-1,3,2-dioxaborolan-2-yl)benzoate und 145 mg (0,13 mmol) Tetrakis(triphenylphosphin)palladium(0) wurden unter Schutzgasatmosphäre mit 31,2 mL 2-Propanol und 7,68 mL 1M wässriger Natriumhydrogencarbonat versetzt. Beide Lösungsmittel wurde vor ihrer Verwendung mit Argon gesättigt, indem mindestens 15 Minuten Argon druch die Lösungsmittel geleitet wurde. Das Reaktionsgemisch wurde 7h bei 90°C und danach 14h bei Raumtemperatur gerührt. Das Reaktionsgemisch wurde mittels Rotationsverdampfer unter vermindertem Druck zur Trockene eingeengt. Der Rückstand wurde in einem Gemisch aus Chloroform und Wasser aufgenommen. Die wässrige Phase wurde dreimal mit Chloroform extrahiert. Die vereinigten organischen Phasen wurden über Natriumsulfat getrocknet, filtriert und am Rotationsverdampfer unter vermindertem Durck eingeengt. Das Rohprodukt wurde mittels MPLC an Silica gereinigt.

Es wurden 350 mg Methyl-2-chlor-5-{4-[1-methyl-3-(pentafluorethyl)-4-(trifluormethyl)-1*H*-pyrazol-5-yl]-2-furyl}benzoat isoliert.
HPLC-MS^{a)}: logP = 5,20, Masse (m/z) = 503 [M+H]+.

### Stufe 4

200 mg (0,40 mmol) Methyl-2-chlor-5-{4-[1-methyl-3-(pentafluorethyl)-4-(trifluormethyl)-1*H*-pyrazol-5-yl]-2-furyl}benzoate wurde in einer Mischung aus 1,43 mL Wasser und 11,4 mL Methanol gelöst. Die Lösung wurde mit 19 mg (0,79 mmol) Lithiumhydroxid versetzt und 30 Minuten auf 50°C erwärmt. Das Reaktionsgemisch wurde anschließend 14h bei Raumtemperatur gerührt. Das Methanol wurde am Rotationsverdampfer unter vermindertem Druck entfernt. Der pH Wert des Reaktionsgemischs wurde anschließend mit 10%-iger Salzsäure auf 1-2 eingestellt. Der ausfallende Niederschlag wurde 1h bei Raumtemperatur gealtert und dann filtriert. Der Feststoff wurde dreimal mit Wasser gewaschen und anschließend getrocknet.

Es wurden 170 mg 2-Chlor-5-{4-[1-methyl-3-(pentafluorethyl)-4-(trifluormethyl)-1*H*-pyrazol-5-yl]-2-furyl}benzoesäure als farbloser Feststoff isoliert.
HPLC-MS^{a)}: logP = 4,13, Masse (m/z) = 489 [M+H]+.
¹H-NMR (400 MHz, d6-DMSO): δ = 8,29 (s, 1H), 8,15 (d, 1H), 7,91 (dd, 1H), 7,66 (d, 1H), 7,46 (s, 1H), 3,92 (s, 3H).

### Stufe 5

85 mg (0.17 mmol) 2-Chlor-5-{4-[1-methyl-3-(pentafluorethyl)-4-(trifluormethyl)-1*H*-pyrazol-5-yl]-2-furyl}benzoesäure wurde in 2 mL N,N-Dimethylformamid pa gelöst und mit 61,4 mg (0,19 mmol) TBTU versetzt. Nach fünf Minuten wurden 91 µL (0,52 mmol) N,N-Diisopropyl-ethylamin und 22,7 mg (0,19 mmol) 1-Aminocyclopropancarbonitril Hydrochlorid (1:1) zugefügt. Das Reaktionsgemisch wurde 1h bei Raumtemperatur und fünf Minuten bei 50°C gerührt. Das Reaktionsgemisch wurde ohne weitere Aufarbeitung mittels MPLC an RP18 Kieselgel gereinigt.

Es wurden 40 mg 2-Chlor-N-(1-cyancyclopropyl)-5-14-[1-methyl-3-(pentafluorethyl)-4-(trifluormethyl)-1*H*-pyrazol-5-yl]-2-furyl}benzamid (Iac-1) isoliert.
HPLC-MS^{a)}: logP = 4,02, Masse (m/z) = 553 [M+H]+.
¹H-NMR (600 MHz, d3-Acetonitril): δ = 7,86 (s, 1H), 7,83 (d, 1H), 7,80 (dd, 1H), 7,58 (s, 1H), 7,55 (d, 1H), 7,05 (s, 1H), 3,83 (s, 3H), 1,56-1,59 (m, 2H), 1,34-1,36 (m, 2H).

### Darstellungsverfahren D

### Synthese von N-Cyclopropyl-3-{5-[1-methyl-3-(pentafluorethyl)-4-(trifluormethyl)-1H-pyrazol-5-yl]-1,3,4-thiadiazol-2-yl}benzamid (Iaj-1)

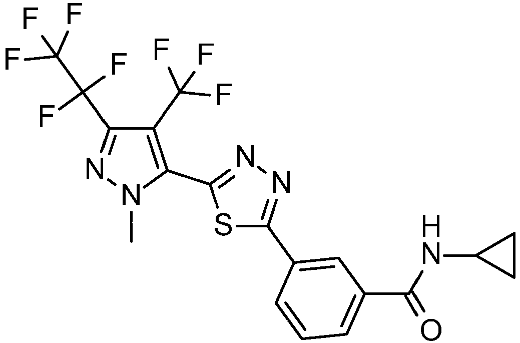

### Stufe 1

300 mg (0,92 mmol) 1-Methyl-3-(pentafluorethyl)-4-(trifluormethyl)-1*H*-pyrazol-5-carbohydrazid und 166 mg (0,92 mmol) 3-(Methoxycarbonyl)benzoesäure wurden in 10,5 mL Dichlormethan gelöst und auf 0°C gekühlt. Es wurden 135 mg (1,10 mmol) DMAP und 194 mg (1,01 mmol) EDCl dazugegeben und anschließend 2h unter Eiskühlung und 14h bei Raumtemperatur gerührt. Das Reaktionsgemisch wurde mit Dichlormethan verdünnt und nacheinander mit wässriger Natriumhydrogencarbonat Lösung, 10%-iger wässriger Citronensäure Lösung gewaschen. Das ausfallende Produkt wurde abfiltriert und getrocknet.

Es wurden 230 mg Methyl-3-[(2-{[1-methyl-3-(pentafluorethyl)-4-(trifluormethyl)-1*H*-pyrazol-5-yl]carbonyl}hydrazino)carbonyl]benzoat als farbloser Feststoff isoliert.
HPLC-MS^{f}): logP = 2,06, Masse (m/z) = 489 [M+H]+.
¹H-NMR (400 MHz, d6-DMSO): δ = 8,55 (s, 1H), 8,19-8,21 (m, 2H), 7,72 (t, 1H), 4,17 (s, 3H), 3,92 (s, 3H).

### Stufe 2

200 mg (0,41 mmol) Methyl-3-[(2-{[1-methyl-3-(pentafluorethyl)-4-(trifluormethyl)-1*H*-pyrazol-5-yl]carbonyl}hydrazino)carbonyl]benzoate und 190 mg (0,47 mmol) 2,4-Bis(4-methoxyphenyl)-1,3,2,4-dithiadiphosphetan-2,4-disulfid (Lawessons Reagenz) wurden in einem Mikrowellengefäß in 2,6 mL Toluol gelöst und 12 h auf 120°C Ölbadtemperatur erhitzt. Das Reaktionsgemisch wurde filtriert und dasFlitrat mit 5 mL Dichlormethan verdünnt. Die organische Phase wurde einmal mit gesättigter wässriger Natriumhydrogencarbonat Lösung gewaschen. Das Reaktionsgemisch wurde am Rotationsverdampfer bis zur Trockene eingeengt. Das Rohprodukt wurde mittels MPLC an Kieselgel aufgereinigt.

Es wurden 140 mg Methyl-3-{5-[1-methyl-3-(pentafluorethyl)-4-(trifluormethyl)-1*H*-pyrazol-5-yl]-1,3,4-thiadiazol-2-yl}benzoat als nahezu farbloser Feststoff erhalten.
HPLC-MS^{a)}: logP = 3,93, Masse (m/z) = 487 [M+H]+.
¹H-NMR (400 MHz, d6-DMSO): δ = 8,61 (s, 1H), 8,38 (d, 1H), 8,21 (d, 1H), 7,80 (t, 1H), 3,98 (s, 3H), 3,94 (s, 3H).

### Stufe 3

120 mg (0,25 mmol) Methyl-3-{5-[1-methyl-3-(pentafluorethyl)-4-(trifluormethyl)-1*H*-pyrazol-5-yl]-1,3,4-thiadiazol-2-yl}benzoat wurden in einem 1:1 Gemisch aus THF und Wasser gelöst und mit einer Lösung von 21 mg (0,49 mmol) Lithiumhydroxid-monohydrat in 1 mL Wasser versetzt. Das Reaktionsgemisch wurde 14h bei Raumtemperatur gerührt und anschließend am Rotationsverdampfer eingeengt. Der Rückstand wurde mehrfach mit tert-Butyl-methylether extrahiert. Die vereinigten organischen Phasen wurden am Rotationsverdampfer bis Trockene eingeengt.

Es wurden 114 mg 3-{5-[1-Methyl-3-(pentafluorethyl)-4-(trifluormethyl)-1H-pyrazol-5-yl]-1,3,4-thiadiazol-2-yl}benzoesäure isoliert.
HPLC-MS^{a)}: logP = 3,58, Masse (m/z) = 473 [M+H]+.
¹H-NMR (400 MHz, d6-DMSO): δ = 8,49 (s, 1H), 8,10 (d, 1H), 8,06 (d, 1H), 7,53 (t, 1H), 3,98 (s, 3H).

### Stufe 4

90 mg (0,19 mmol) 3-{5-[1-Methyl-3-(pentafluorethyl)-4-(trifluormethyl)-1H-pyrazol-5-yl]-1,3,4-thiadiazol-2-yl}benzoesäure und 11 mg (0,19 mmol) Cyclopropylamin wurden in 1,5 mL Dichlormethan gelöst und auf 0°C gekühlt. Es wurden 28 mg (0,23 mmol) DMAP und 40 mg (0,21 mmol) EDCl dazugegeben und anschließend 2h unter Eiskühlung und 14h bei Raumtemperatur gerührt. Das Reaktionsgemisch wurde mit Dichlormethan verdünnt und nacheinander mit wässriger Natriumhydrogencarbonat Lösung, 10%-iger wässriger Citronensäure Lösung gewaschen. Das Rohprodukt wurde mittels MPLC an Kieselgel gereinigt..

Es wurden 42 mg *N*-Cyclopropyl-3-{5-[1-methyl-3-(pentafluorethyl)-4-(trifluormethyl)-1*H*-pyrazol-5-yl]-1,3,4-thiadiazol-2-yl}benzamid (**Iaj-1**) isoliert.HPLC-MS^{a)}: logP = 3,72, Masse (m/z) = 512 [M+H]+.
¹H-NMR (400 MHz, d6-DMSO): δ = 8,74 (d, 1H), 8,49 (s, 1H), 8,25 (d, 1H), 8,08 (d, 1H), 7,71 (t, 1H), 3,97 (s, 3H), 2,87-2,92 (m, 1H), 0,71-0,76 (m, 2H), 0,58-0,63 (m, 2H).

### Darstellungsverfahren E

### Synthese von N-(1-Cyancyclopropyl)-3-{2-[1-methyl-3-(pentafluorethyl)-4-(trifluormethyl)-1H-pyrazol-5-yl]-1,3-thiazol-5-yl}benzamid (Iaq-1)

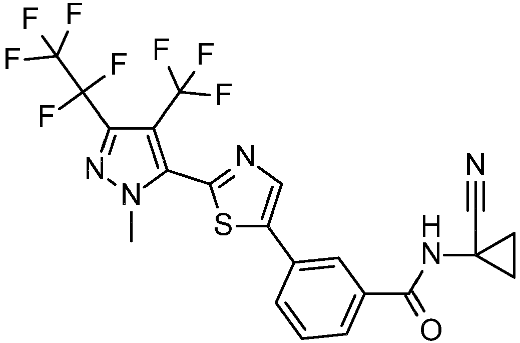

Stufe 1 5,00 g (16,0 mmol) 1-Methyl-3-(pentafluorethyl)-4-(trifluormethyl)-1*H-*pyrazol-5-carbonsäure wurden in 50 mL Dichlormethan suspendiert und mit 2 Tropfen Dimethylformamid versetzt. Eine Lösung aus 2,10 mL (24,0 mmol) Oxalylchlorid und 10 mL Dichlormethan wurden zu der Reaktionsmischung getropft. Die Reaktionsmischung wurde erst 3h bei Raumtemperatur und dann noch 5 Minuten auf 40°C erwärmt. Die Lösungsmittel wurden mit dem Rotationsverdampfer entfernt. Der Rückstand wurde in 5 mL Dichlormethan gelöst und zu einer mit einem Eisbad gekühlten 33%-igen Lösung von Ammoniak in Wasser getropft. Das Reaktionsgemisch wurde eine Stunde bei Raumtemperatur gerührt und dann auf die Hälfte des Volumens am Rotationsverdampfer eingeengt. Der entstandene Feststoff wurde abfiltriert und getrocknet.

Es wurden 3,80 g 1-Methyl-3-(pentafluorethyl)-4-(trifluormethyl)-1*H*-pyrazol-5-carboxamid als farbloser Feststoff isoliert.
HPLC-MS^{a)}: logP = 2,33, Masse (m/z) = 312 [M+H]+.
¹H-NMR (400 MHz, d6-DMSO): δ = 8,55 (s, 1H), 8,43 (s, 1H), 3,95 (s, 3H).

### Stufe 2

500 mg (1,61 mmol) 1-Methyl-3-(pentafluorethyl)-4-(trifluormethyl)-1*H*-pyrazol-5-carboxamid wurden in 12 mL Toluol vorgelegt und auf 80°C erwärmt. Zu der Reaktionsmischung wurden 390 mg (0,96 mmol) 2,4-Bis(4-methoxyphenyl)-1,3,2,4-dithiadiphosphetan-2,4-disulfid (Lawessons Reagenz) zugegeben. Nach 3h wurde das Reaktionsgemisch von 80°C auf 95°C erwärmt und für 14h bei dieser Temperatur nachgerührt. Nach dem Abkühlen wurde das Reaktionsgemisch mit Ethylacetat verdünnt und einmal mit gesättigter wässriger Natriumhydrogencarbonat Lösung gewaschen. Die organische Phase wurde am Rotationsverdampfer zur Trockene eingeengt. Das Rohprodukt wurde mittels MPLC an Kieselgel aufgereinigt.

Es wurden 300 mg 1-Methyl-3-(pentafluorethyl)-4-(trifluormethyl)-1*H*-pyrazol-5-carbothioamid isoliert.
HPLC-MS^{a)}: logP = 2,92, Masse (m/z) = 328 [M+H]+.
¹H-NMR (400 MHz, dl-Chloroform): δ = 7,97 (s, 1H), 7,34 (s, 1H), 4,03 (s, 3H).

### Stufe 3

100 mg (0,30 mmol) 1-Methyl-3-(pentafluorethyl)-4-(trifluormethyl)-1*H*-pyrazol-5-carbothioamid, 119 mg (0,30 mmol) Methyl-3-(bromacetyl)benzoat und 24 mg (0,30 µmοl) Pyridin p.a. wurden unter Schutzgas Atmosphäre in Ethanol p.a. 6h unter Rückfluß erhitzt. Das Reaktionsgemisch wurde am Rotationsverdampfer zur Trockene eingeengt und das erhaltene Rohprodukt wurde mittels MPLC an Kieselgel aufgereinigt.

Es wurden 95 mg Methyl-3-{2-[1-methyl-3-(pentafluorethyl)-4-(trifluormethyl)-1H-pyrazol-5-yl]-1,3-thiazol-5-yl}benzoat isoliert.
HPLC-MS^{a)}: logP = 4,96, Masse (m/z) = 486 [M+H]+.
¹H-NMR (400 MHz, dl-Chloroform): δ = 8,59 (s, 1H), 8,17 (d, 1H), 8,08 (d, 1H), 7,93 (s, 1H), 7,56 (t, 1H), 4,04 (s, 3H), 3,97 (s, 3H).

### Stufe 4

85 mg (0,18 mmol) Methyl-3-{2-[1-methyl-3-(pentafluorethyl)-4-(trifluormethyl)-1H-pyrazol-5-yl]-1,3-thiazol-5-yl}benzoat wurden in einer Mischung aus 4 mL THF p.a. und 2 mL dest. Wasser gelöst und mit einer Lösung von 15 mg (0,35 mmol) Lithiumhydroxid-monohydrat in 1,0 mL dest. Wasser versetzt. Das Reaktionsgemisch wurde 14h bei Raumtemperatur gerührt. Das THF wurde am Rotationsverdampfer unter vermindertem Druck entfernt. Das wässrige Reaktionsgemisch wurde unter Eiskühlung mit 1N Salzsäure angesäuert. Der erhaltene Niederschlag wurde abfiltriert und dann getrocknet.

Es wurden 60 mg 3-{2-[1-Methyl-3-(pentafluorethyl)-4-(trifluormethyl)-1H-pyrazol-5-yl]-1,3-thiazol-5-yl}benzoesäure isoliert.
HPLC-MS^{a)}: logP = 4,04, Masse (m/z) = 472 [M+H]+.
¹H-NMR (400 MHz, d6-DMSO): δ = 8,76 (s, 1H), 8,58 (s, 1H), 8,27 (d, 1H), 7,98 (d, 1H), 7,64 (t, 1H), 3,99 (s, 3H).

### Stufe 5

40 mg (0,09 mmol) 3-{2-[1-Methyl-3-(pentafluorethyl)-4-(trifluormethyl)-1H-pyrazol-5-yl]-1,3-thiazol-5-yl}benzoesäure und 12 mg (0,10 mmol) 1-Aminocyclopropancarbonitrilhydrochlorid (1:1) wurden in 10,0 mL Dichlormethan suspendiert/gelöst und auf 0°C gekühlt. Es wurden 25 mg (0,20 mmol) DMAP und 18 mg (0,09 mmol) EDCl dazugegeben und anschließend 2h unter Eiskühlung und 14h bei Raumtemperatur gerührt. Das Reaktionsgemisch wurde nacheinander mit 1N Salzsäure und 1N Natronlauge gewaschen. Die organische Phase wurde über Natriumsulfat getrocknet, filtriert und am Rotationsverdampfer unter vermindertem Druck bis zur Trockene eingeengt. Das Rohprodukt wurde mittels MPLC an Kieselgel gereinigt.

Es wurden 23 mg *N*-(1-Cyancyclopropyl)-3-{2-[1-methyl-3-(pentafluorethyl)-4-(trifluormethyl)-1*H-*pyrazol-5-yl]-1,3-thiazol-5-yl}benzamid (**Iaq-1**) isoliert.
HPLC-MS^{a)}: logP = 3,93, Masse (m/z) = 536 [M+H]+.
¹H-NMR (400 MHz, d6-DMSO): δ = 9,46 (s, 1H), 8,69 (s, 1H), 8,47 (s, 1H), 8,22 (d, 1H), 7,87 (d, 1H), 7,62 (t, 1H), 3,99 (s, 3H), 1,56-1,61 (m, 2H), 1,29-1,33 (m, 2H).

### Darstellungsverfahren F

### Synthese von N-Cyclopropyl-3-[2,2'-dimethyl-5'-(pentafluorethyl)-4'-(trifluormethyl)-2H,2'H-3,3'-bipyrazol-5-yl]-N-methylbenzamid (Iaw-1) und N-Cyclopropyl-3-[1,2'-dimethyl-5'-(pentafluorethyl)-4'-(trifluormethyl)-1H,2'H-3,3'-bipyrazol-5-yl]-N-methylbenzamid (Iax-1)

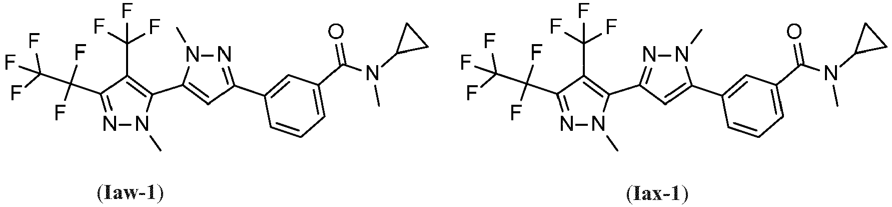

### Stufe 1

250 mg (1,15 mmol) 3-Acetyl-*N*-cyclopropyl-*N*-methylbenzamid wurden in 5 mL THF p.a. unter Schutzgasatmosphäre gelöst und auf -78°C gekühlt. Die Reaktionslösung wurde innerhalb von 20 Minuten mit 1,44 mL einer 2 M Lösung von Lithiumdiisopropylamid in THF/Heptan/Ethylbenzol versetzt. Die Reaktionsmischung wurde 30 Minuten bei -78°C gerührt und anschließend mit einer Lösung von 418 mg frisch hergestelltem und mehrfach mit Toluol co-destilliertem 1-Methyl-3-(pentafluorethyl)-4-(trifluormethyl)-1*H-*pyrazol-5-carbonylchlorid in THF p.a. versetzt. Die Reaktionsmischung wurde 1h und bei -78°C gerührt und dann auf Raumtemperatur erwärmt. Nach 30 Minuten bei Raumtemperatur wurde die Reaktion durch die Zugabe von gesättigter Ammoniumchlorid Lösung gequencht. Das Reaktionsgemisch wurde mehrfach mit Dichlormethan extrahiert. Die vereinigten org. Phasen wurden mit gesättigter Kochsalzlösung gewaschen, über Natriumsulfat getrocknet und filtriert. Das Lösungsmittel wurde am Rotationsverdampfer eingeengt. Das Rohprodukt wurde mittels MPLC an Kieselgel gereinigt.

Es wurden 160 mg *N*-Cyclopropyl-*N*-methyl-3-{3-[1-methyl-3-(pentafluorethyl)-4-(trifluormethyl)-1*H-*pyrazol-5-yl]-3-oxopropanoyl}benzamid in 86%-iger Reinheit (laut LCMS) isoliert.
HPLC-MS^{a)}: logP = 4,39, Masse (m/z) = 512 [M+H]+.

### Stufe 2

100 mg (0,20 mmol) *N*-Cyclopropyl-*N*-methyl-3-{3-[1-methyl-3-(pentafluorethyl)-4-(trifluormethyl)-1*H*-pyrazol-5-yl]-3-oxopropanoyl}benzamid und 9,01 mg (0,20 mmol) *N*-Methylhydrazin wurden in 10 mL Ethanol unter Rückfluß erhitzt. Das Lösungsmittel wurde am Rotationsverdampfer unter vermindertem Druck entfernt. Das Rohprodukt wurde mittels MPLC an Kieselgel gereinigt.

Es wurden 16,0 mg *N*-Cyclopropyl-3-[2,2'-dimethyl-5'-(pentafluorethyl)-4'-(trifluormethyl)-2*H*,2'*H*-3,3'-bipyrazol-5-yl]-*N*-methylbenzamid (**Iaw-1**) isoliert.
HPLC-MS^{a)}: logP = 3,94, Masse (m/z) = 522 [M+H]+.
¹H-NMR (400 MHz, d6-DMSO): δ = 7,85-7,92 (m, 2H), 7,47-7,51 (m, 2H), 7,27 (s, 1H), 3,86 (s, 3H), 3,76 (s, 3H), 2,99 (s, 3H), 0,33-0,63 (m, 4H).

Des Weiteren wurden 11,0 mg *N*-Cyclopropyl-3-[1,2'-dimethyl-5'-(pentafluorethyl)-4'-(trifluormethyl)-1*H*,2'*H*-3,3'-bipyrazol-5-yl]-*N*-methylbenzamid (**Iax-1**) isoliert.
HPLC-MS^{a)}: logP = 4,13, Masse (m/z) = 522 [M+H]+.
¹H-NMR (400 MHz, d6-DMSO): δ = 7,65-7,74 (m, 2H), 7,55-7,60 (m, 2H), 6,89 (s, 1H), 4,00 (s, 3H), 3,97 (s, 3H), 3,04 (s, 3H), 0,33-0,63 (m, 4H).

### Darstellungsverfahren G

### Synthese von 2-Chlor-N-cyclopropyl-5-{5-[1-methyl-3-(pentafluorethyl)-4-(trifluormethyl)-1H-pyrazol 5-yl]-1,2,4-thiadiazol-3-yl}benzamid (Ibd-3)

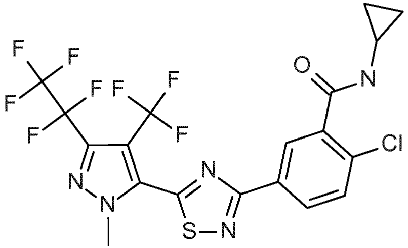

### Stufe 1

Es wurden nacheinander 5,00 g (21,0 mmol) 4-Chlor-*N*-cyclopropylisophthalamid, 120 mL einer 3:1 Toluol/1,4-Dioxan Mischung, 2,50 g (18,09 mmol) Kaliumcarbonat und 5,50 g (42,0 mmol) Chlor(chlorsulfanyl)oxomethan in einem 250 mL Dreihalskolben vorgelegt. Das Reaktionsgemisch wurde 2 h bei 100°C gerührt. Nach dem Abkühlen auf Raumtemperatur wurde die Reaktion durch die Zugabe von 200 mL Wasser gequencht und der entstehende Niederschlag abfiltirert und getrocknet.

Es wurden 2,99 g 2-Chlor-*N*-cyclopropyl-5-(2-oxo-1,3,4-oxathiazol-5-yl)benzamid erhalten.
¹H-NMR (300 MHz, d6-DMSO): δ = 8.67-8.68 (m, 1H), 7.92-7.96 (m, 1H), 7.84-7.85 (m, 1H), 7.69-7.72 (m, 1H), 2.79-2.86 (m, 1H), 0.68-0.75 (m, 2H), 0.53-0.57 (m, 2H).

### Stufe 2

593 mg (2 mmol) 2-Chlor-*N*-cyclopropyl-5-(2-oxo-1,3,4-oxathiazol-5-yl)benzamid wurden in 20 mL Decan vorgelegt und mit 2,93 g (10 mmol) 1-Methyl-3-(pentafluorethyl)-4-(trifluormethyl)-1H-pyrazol-5-carbonitril versetzt. Das Reaktionsgemisch wurde 1h bei 200°C in der Mikrowelle erhitzt. Das Reaktionsgemisch wurde auf Eiswasser gegoßen und die wässrige Phase dreimal mit je 20 mL Ethylacetat extrahiert. Die vereinigten organischen Phasen wurden dreimal mit je 10 mL gesättigter Kochsalzlösung gewaschen, über Natriumsulfat getrocknet, abfiltriert und am Rotationsverdampfer unter vermindertem Druck eingeengt. Das Rohprodukt wurde mittels präparativer HPLC (C18) aufgereinigt.

Es wurden 35 mg 2-Chlor-*N*-cyclopropyl-5-{5-[1-methyl-3-(pentafluorethyl)-4-(trifluormethyl)-1*H-*pyrazol-5-yl]-1,2,4-thiadiazol-3-yl}benzamid (**Ibd-3**) als farbloser Feststoff isoliert.
HPLC-MS^{b)}: Retentionszeit = 2,31 min., Masse (m/z) = 546 [M+H]+.
¹H-NMR (300 MHz, d6-DMSO): δ = 8.64-8.66 (m, 1H), 8.26-8.29 (m, 1H), 8.18-8.19 (m, 1H), 7.71-7.73 (m, 1H), 3.97 (s, 3H), 2.80-2.87 (m, 1H), 0.70-0.73 (m, 2H), 0.54-0.56 (m, 2H).

### Darstellungsverfahren H

### Synthese von 2-Chlor-N-cyclopropyl-5-{3-[1-methyl-3-(pentafluorethyl)-4-(trifluormethyl)-1H-pyrazol-5-yl]-1,2,4-oxadiazol-5-yl}benzamid (Ibe-1).

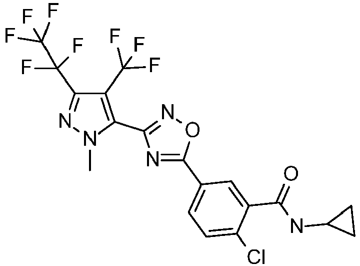

### Stufe 1

6,0 g (20,5 mmol) 1-Methyl-3-(pentafluorethyl)-4-(trifluormethyl)-1*H*-pyrazol-5-carbonitril wurden in 150 mL Ethanol gelöst und mit 50%-igen Lösung von N-Hydroxylamin in Wasser versetzt. Die Reaktionslösung wurde 3 h bei Raumtemperatur gerührt und dann im Vakuum am Rotationsverdampfer zur Trockene eingeengt.

Es wurden 2,8 g N'-Hydroxy-1-methyl-3-(pentafluorethyl)-4-(trifluormethyl)-1H-pyrazol-5-carboximidamid als gelber Feststoff isoliert.
HPLC-MS^{d)}: Retentionszeit = 0,93 min., Masse (m/z) = 237 [M+H]+.

### Stufe 2

Es wurden nacheinander 2,8 g (8,59 mmol) N'-Hydroxy-1-methyl-3-(pentafluorethyl)-4-(trifluormethyl)-1*H*-pyrazol-5-carboximidamid, 100 mL Dichlormethan, 2,6 g (25,7 mmol) Triethylamin und zuletzt 4,7 g (17,1 mmol) tert-Butyl-2-chlor-5-(chlorcarbonyl)benzoat in einem Rundkolben vorgelegt. Nach 1h bei Raumtemperatur wurde das Lösungsmittel am Rotationsverdampfer entfernt. Der Rückstand wurde in 20 mL DMSO gelöst und mit 2,6 g (25,7 mmol) Triethylamin versetzt. Die Reaktionsmischung wurde 1h bei 140°C in der Mikrowelle erhitzt. Das Reaktionsgemisch wurde auf Raumtemperatur abgekühlt und mit 50 mL Wasser verdünnt. Die resultierende Lösung wurde dreimal mit je 50 mL Ethylacetat extrahiert. Die vereinigten organischen Phasen wurden mit gesättigter Kochsalzlösung gewaschen, über Natriumsulfat getrocknet und filtriert. Das Rohprodukt wurde säulenchromatographisch an Kieselgel aufgereinigt.

Es wurden 960 mg tert-Butyl-2-chlor-5-{3-[1-methyl-3-(pentafluorethyl)-4-(trifluormethyl)-1H-pyrazol-5-yl]-1,2,4-oxadiazol-5-yl}benzoat als gelber Feststoff isloiert.
HPLC-MS^{d)}: Retentionszeit = 1,77 min., Masse (m/z) = 547 [M+H]+.

### Stufe 3

960 mg (1,76 mmol) tert-Butyl-2-chlor-5-{3-[1-methyl-3-(pentafluorethyl)-4-(trifluormethyl)-1H-pyrazol-5-yl]-1,2,4-oxadiazol-5-yl}benzoat wurde in 30 mL Trifluoressigsäure gelöst und 12h bei Raumtemperatur gerührt. Das Reaktionsgemisch wurde am Rotationsverdampfer unter vermindertem Druck eingeengt. Der ausfallende Feststoff wurde abfiltriert und getrocknet.

Es wurden 800 mg 2-Chlor-5-{3-[1-methyl-3-(pentafluorethyl)-4-(trifluormethyl)-1H-pyrazol-5-yl]-1,2,4-oxadiazol-5-yl}benzoesäure als gelber Feststoff isoliert.
HPLC-MS^{e)}: Retentionszeit = 1,88 min., Masse (m/z) = 489 [M-H]-.

### Stufe 4

Nacheinander wurden 200 mg (0,41 mmol) mg 2-Chlor-5-{3-[1-methyl-3-(pentafluorethyl)-4-(trifluormethyl)-1H-pyrazol-5-yl]-1,2,4-oxadiazol-5-yl}benzoesäure, 20 mL Dimethylformamid, 158 mg (1,23 mmol) N,N-Diisopropylethylamin, 188 mg (0,5 mmol) HATU und 46 mg (0,82 mmol) Cyclopropylamin in einem Rundkolben vorgelegt. Die Reaktionsmischung wurde 2h bei 50°C gerührt. Das Reaktionsgemisch wurde mit 5 mL Wasser verdünnt und die resultierende Lösung wurde dreimal mit je 5 mL Ethylacetat extrahiert. Die vereinigten organischen Phasen wurden mit 10 mL gesättigter Kochsalzlösung gewaschen, über Natriumsulfat getrocknet, filtriert und am Rotationsverdampfer unter vermindertem Druck eingeengt. Das Rohprodukt wurde mittels präparativer HPLC aufgereinigt.

Es wurden 26 mg 2-Chlor-*N*-cyclopropyl-5-{3-[1-methyl-3-(pentafluorethyl)-4-(trifluormethyl)-1*H-*pyrazol-5-yl]-1,2,4-oxadiazol-5-yl}benzamid (**Ibe-1**) als farbloser Feststoff isoliert.
HPLC-MS^{a)}: logP = 4,16, Masse (m/z) = 530 [M+H]+.
¹H-NMR (300 MHz, d6-DMSO): δ = 8.71-8.73 (m, 1H), 8.22-8.26 (m, 1H), 8.16-8.17 (m, 1H), 7.83-7.86 (m, 1H), 4.15 (s, 3H), 2.83-2.89 (m, 1H), 0.73-0.74 (m, 2H), 0.56-0.59 (m, 2H).

### Darstellungsverfahren I

### Synthese von 2-Chlor-N-cyclopropyl-5-{5-[1-methyl-3-(pentafluorethyl)-4-(trifluormethyl)-1H-pyrazol 5-yl]-1,2,4-oxadiazol-3-yl}benzamid (Ibf-1).

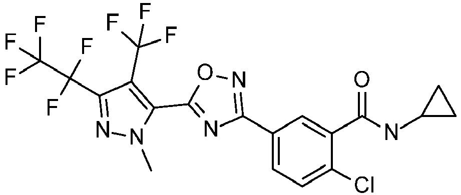

### Stufe 1

10,0 g (51,1 mmol) Methyl-2-chlor-5-cyanbenzoat wurden in 150 mL Ethanol gelöst und mit 2,6 g (255 mmol) einer 50%-igen Lösung von N-Hydroxylamin in Wasser versetzt. Das Reaktiongemisch wurde über Nacht bei Raumtemperatur gerührt. Das Reaktionsgemisch wurde am Rotatiosverdampfer unter vermindertem Druck aufkonzentriert. Der Rückstand wurde in 100 mL Wasser aufgenommen und die wässrige Phase anschließend dreimal mit je 100 mL Ethylacetat extrahiert. Die vereinigten organischen Phase wurden über Natriumsulfat getrocknet und anschließend filtriert. Das Filtrat wurde am Rotationsverdampfer unter vermindertem Druck zur Trockene eingeengt.

Es wurden 6,0 g Methyl-2-chlor-5-(N'-hydroxycarbamimidoyl)benzoat als farbloser Feststoff isoliert.
1H-NMR (300 MHz, d6-DMSO): δ 9.86 (s, 1H), 8.1 (d, 1H), 7.85,(dd, 1H), 7.60 (d, 3H), 5.98 (brs, 2H), 3.90 (s, 3H).

### Stufe 2

3,12 g (10,0 mmol) 1-Methyl-3-(pentafluorethyl)-4-(trifluormethyl)-1H-pyrazol-5-carbonsäure, 4,41 g (15,0 mmol) EDCI, 2,03 g (10,0 mmol) HOBt und 2,97 g (15,0 mmol) N,N-Diisopropylethylamin wurden in 80 mL THF aufgenommen und 1h bei Raumtemperatur gerührt. Es wurden 3,43 g (15,0 mmol) Methyl-2-chlor-5-(N'-hydroxycarbamimidoyl)benzoat zu der Reaktionslösung hinzugegeben und 14 h bei Raumtemperatur gerührt. Das Reaktionsgemisch wurde am Rotationsverdampfer bis zut Trockene eingeengt. Der Rückstand wurde dreimal mit je 50 mL Ethylacetat extrahiert, die vereinigten organischen Phasen wurden dreimal mit je 50 mL Wasser und einmal mit 50 mL gesättigter Kochsalzlösung gewaschen. Die organische Phase wurde über Natriumsulfat getrocknet, filtriert und am Rotationsverdampfer zur Trockene eingeengt. Das Rohprodukt wurde an Kieselgel säulenchromatographisch gereinigt.

Es wurden 5,26 g Methyl-2-chlor-5-(N'-hydroxy-N-{[1-methyl-3-(pentafluorethyl)-4-(trifluormethyl)-1H-pyrazol-5-yl]carbonyl}carbamimidoyl)benzoat isoliert.
HPLC-MS^{d)}: Retentionszeit = 1,64 min., Masse (m/z) = 523 [M+H]+.

### Stufe 3

5,23 g (10,0 mmol) Methyl-2-chlor-5-(N'-hydroxy-N-{[1-methyl-3-(pentafluorethyl)-4-(trifluormethyl)-1H-pyrazol-5-yl]carbonyl}carbamimidoyl)benzoat wurden in 60 mL Toluol 14 h unter Rückfluß erhitzt. Das Reaktionsgemisch wurde am Rotationsverdampfer unter vermindertem Druck eingeengt. Das Rohprodukt wurde an Kieselgel säulenchromatographisch aufgereinigt.

Es wurden 2,02 g Methyl-2-chlor-5-{5-[1-methyl-3-(pentafluorethyl)-4-(trifluormethyl)-1H-pyrazol-5-yl]-1,2,4-oxadiazol-3-yl}benzoat isoliert.
HPLC-MS^{d)}: Retentionszeit = 1,80 min., Masse (m/z) = nicht detektierbar.

### Stufe 4

228 mg (0,45 mmol) Methyl-2-chlor-5-{5-[1-methyl-3-(pentafluorethyl)-4-(trifluormethyl)-1H-pyrazol-5-yl]-1,2,4-oxadiazol-3-yl}benzoat wurden in 70 mL Methanol gelöst und dann mit 15 mL (15 mmol) 1 M Natronlauge versetzt. Das Reaktionsgemisch wurde 14 h bei Raumtemperatur gerührt. Der pH Wert der Lösung wurde mit 1 N Salzsäure auf pH 3 eingestellt. Die Reaktionslösung wurde am Rotationsverdampfer unter vermindertem Druck eingeengt. Der Rückstand wurde dreimal mit je 25 mL Ethylacetat extrahiert. Die vereinigten organischen Phasen wurden mit gesättigter Kochsalzlösung gewaschen, über Natriumsulfat getrocknet und filtriert. Das Lösungsmittel wurde am Rotationsverdampfer unter vermindertem Druck entfernt.

Es wurden 198 mg 2-Chlor-5-{5-[1-methyl-3-(pentafluorethyl)-4-(trifluormethyl)-1H-pyrazol-5-yl]-1,2,4-oxadiazol-3-yl}benzoesäure isoliert.
HPLC-MS^{e)}: Retentionszeit = 1,70 min., Masse (m/z) = 489 [M+H]+.

### Stufe 5

Es wurden nacheinander 196 mg (0,4 mmol) 2-Chlor-5-{5-[1-methyl-3-(pentafluorethyl)-4-(trifluormethyl)-1H-pyrazol-5-yl]-1,2,4-oxadiazol-3-yl}benzoesäure, 10 mL Dichlormethan, 10 mL THF, 115 mg (0,6 mmol) EDCI, 54 mg (0,4 mmol) HOBt, 77 mg (0,6 mmol) und N,N-Diisopropylethylamin in einem Rundkolben vorgelegt und eine 1h bei Raumtemperatur gerührt. Das Reaktionsgemisch wurde mit einem Eis/Wasser Bad gekült und dann mit 48 mg (0,48 mmol) Cyclopropylamin versetzt. Die Reaktionslösung wurde bei Raumtemperatur 14 h gerührt und anschließend am Rotationsverdampfer unter vermindertem Druck eingeengt. Der Rückstand wurde dreimal mit je 25 mL Ethylacetat extrahiert. Die vereinigten organischen Phasen wurden dreimal mit gesättigter Kochsalzlösung gewaschen, über Natriumsulfat getrocknet und filtriert. Das Lösungsmittel wurde am Rotationsverdampfer unter vermindertem Druck entfernt und das Rohprodukt anschließend säulenchromatographisch an Kieselgel aufgereinigt.

Es wurden 62,9 mg 2-Chlor-*N*-cyclopropyl-5-{5-[1-methyl-3-(pentafluorethyl)-4-(trifluormethyl)-1H-pyrazol-5-yl]-1,2,4-oxadiazol-3-yl}benzamid (Ibf-2) als farbloser Feststoff isoliert.
HPLC-MSb): Retentionszeit = 5,04 min., Masse (m/z) = 530 [M+H]+.
1H-NMR (300 MHz, d6-DMSO): δ 8.69 (s, 1H), 8.15 (dd, 1H), 8.06 (d, 1H), 7.78 (d, 1H), 4.33 (s, 3H), 2.90-2.81(m, 1H), 0.76-0.50 (m, 4H).

### Darstellungsverfahren J

### Synthese von N-Cyclopropyl-2-fluor-5-{4-[1-methyl-3-(pentafluorethyl)-4-(trifluormethyl)-1H-pyrazol-5-yl]-1,3-thiazol-2-yl}benzamid (Iat-1).

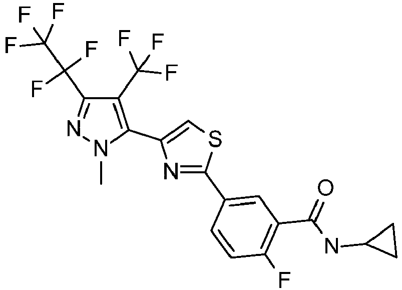

### Stufe 1

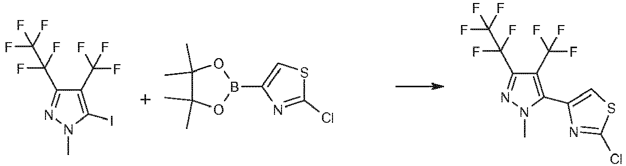

0,5g (1,27 mmol) 5-iodo-1-methyl-3-(pentafluoroethyl)-4-(trifluoromethyl)-1H-pyrazole wurden mit 0.375g (1,53 mmol) 2-chloro-4-(4,4,5,5-tetramethyl-1,3,2-dioxaborolan-2-yl)-1,3-thiazole, 0,31g (2,04 mmol) Caesiumfluorid und 0,071 g (0,061 mmol) Tetrakis(triphenylphosphin)palladium(0) in 10 ml Dimethoxyethan in einem Mikrowellenvial gelöst. Mit einer Kanüle wurden 3 min Ar durch die Lösung geleitet. Danach wurde das Reaktionsgefäss mit einem Krimpdeckel verschlossen und in der Mikorwelle bei 100°C für 4 h umgesetzt. Die Reaktionsmischung wurde über Celite filtriert und eingeengt. Das Rohprodukt wurde in Acetonitril gelöst und über eine präparative HPLC mit RP18 Säule mit einem Wasser / Acetonitrilgradienten 9/1 bis 1/9 gereinigt.

Die Zielproduktfraktionen wurden per LC-MS identifiziert, vereinigt und eingeengt. Dies ergab 0,031g 2-chloro-4-[1-methyl-3-(pentafluoroethyl)-4-(trifluoromethyl)-1H-pyrazol-5-yl]-1,3-thiazol.

### Stufe 2

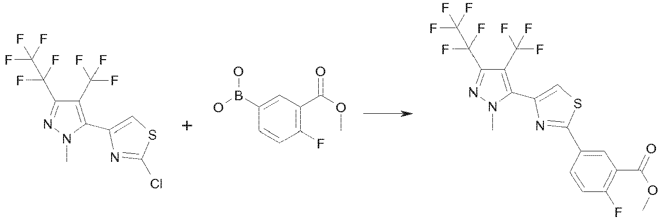

35,0 mg (0,087 mmol) 2-chloro-4-[1-methyl-3-(pentafluoroethyl)-4-(trifluoromethyl)-1H-pyrazol-5-yl]-1,3-thiazol wurden mit 17,2 mg (0,087 mmol) 4-Fluoro-3-(methoxycarbonyl)phenylboronsäure, 8,1 mg (0,017 mmol) 2-Dicyclohexylphosphino-2',6'-diisopropoxybiphenyl und 14,22 mg (0,017 mmol) Chloro(2-dicyclohexylphosphino-2',6'-diisopropyl-1,1'-biphenyl)(2-(2-aminoethyl)phenyl)palladium(II) und 27,7 mg (0,13 mmol) Kaliumphosphat in 2 ml Dioxan in einem Mikrowellenvial gelöst. Mit einer Kanüle wurden 3 min Ar durch die Lösung geleitet. Danach wurde das Reaktionsgefäss mit einem Krimpdeckel verschlossen und in der Mikorwelle bei 120°C für 3 h umgesetzt. Die Reaktionsmischung wurde über Celite filtriert und eingeengt und mit 2 ml Wasser versetzt. Die Reaktionsmischung wurde mit 3 mal 5 ml Ethylacetat extrahiert. Die org Phase wurde mit ges. NaCl Lösung extrahiert und über Na₂SO₄ getrocknet. Sie wurde zur Trockene eingeengt. Das Rohprodukt wurde in Acetonitril gelöst und über eine präparative HPLC mit RP18 Säule mit einem Wasser / Acetonitrilgradienten 9/1 bis 1/9 gereinigt.

Die Zielproduktfraktionen wurden per LC-MS identifiziert, vereinigt und eingeengt. Dies ergab 10,4 mg Methyl-2-fluor-5-{4-[1-methyl-3-(pentafluorethyl)-4-(trifluormethyl)-1H-pyrazol-5-yl]-1,3-thiazol-2-yl}benzoat.

### Stufe 3

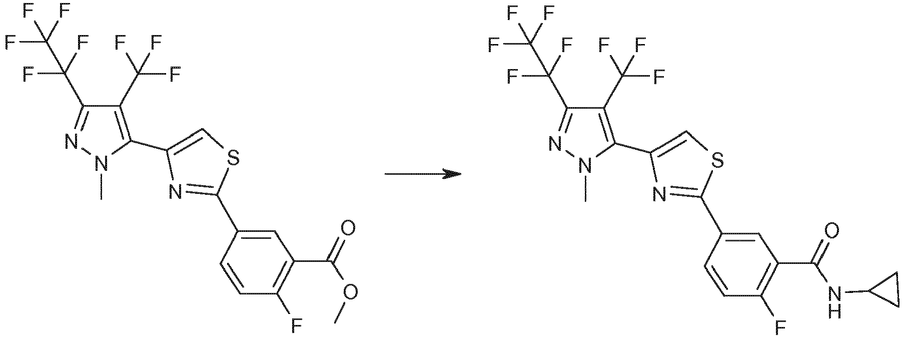

10,4 mg (0.13 mmol) Methyl-2-fluor-5-{4-[1-methyl-3-(pentafluorethyl)-4-(trifluormethyl)-1H-pyrazol-5-yl]-1,3-thiazol-2-yl}benzoat wurden in 3 ml Dioxan gelöst und es wurden 5mg LiOH gelöst in ca. 0,2 ml Wasser wurden zugegeben und die Reaktionsmischung wurden für 12 h gerührt. Dies Lösung wurde mit 1N HCL Lösung neutral gestellt Die Reaktionsmischung wurde mit 3 mal 5 ml Ethylacetat extrahiert. Die org Phase wurde mit ges. NaCl Lösung extrahiert und über Na₂SO₄ getrocknet. Sie wurde zur Trockene eingeengt. Der Rückstand wurde in 1ml Dichlormethan aufgenommen und mit 1µl Cycloporpylamin, 6 mg HATU und 3 µl DIEPA versetzt. Nach Umsetzung bei 60°C in der Mikrowelle für 30 min wurde die Reaktionsmischung eingeengt und in 7ml Acetonitril gelöst und über eine präparative HPLC mit RP18 Säule mit einem Wasser / Acetonitrilgradienten 9/1 bis 1/9 gereinigt.

Die Zielproduktfraktionen wurden per LC-MS identifiziert, vereinigt und eingeengt. Dies ergab 2,5 mg 2-chloro-4-[1-methyl-3-(pentafluoroethyl)-4-(trifluoromethyl)-1H-pyrazol-5-yl]-1,3-thiazol.

N-Cyclopropyl-2-fluor-5-{4-[1-methyl-3-(pentafluorethyl)-4-(trifluormethyl)-1H-pyrazol-5-yl]-1,3-thiazol-2-yl}benzamid (Iat-1)
HPLC-MS^{c)}: rt = 1,24 min, Masse (m/z) = 529 [M+H]+.
¹H-NMR s.u. Peakliste

Mit Hilfe der oben beschriebenen Darstellungsverfahren A bis I wurden neben den bereits beschriebenen Verbindungen die in dem Tabelle 1 - 11 aufgeführten Verbindungen dargestellt.

**Tabelle 1**

| | | | | | | | | | | | | | | |
|---|---|---|---|---|---|---|---|---|---|---|---|---|---|---|
| 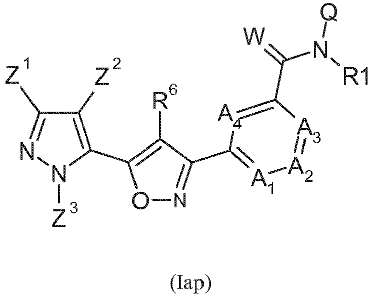 | | | | | | | | | | | | | | |

| **Bsp. -Nr.** | **Z¹** | **Z²** | **Z³** | **R¹** | **R⁶** | **A₄** | **A₃** | **A₂** | **A₁** | **W** | **Q** | **logP^{a})** | **Masse [m/z]^{a)1)}** | **Darstellungs -verfahren** |
|---|---|---|---|---|---|---|---|---|---|---|---|---|---|---|
| Iap-2 | C₂F₅ | CF₃ | CH₃ | H | H | CH | CC l | CH | CH | O | CH₂CF₃ | 4,42 | 571 | A |
| Iap-3 | C₂F₅ | CF₃ | CH₃ | H | H | CH | CC l | CH | CH | O | 1-(cyano)cyclopropyl | 3,96 | 554 | A |
| Iap-4 | C₂F₅ | CF₃ | CH₃ | H | H | CH | CC l | CH | CH | O | thiethan-3-yl | 4,37 | 561 | A |
| Iap-5 | C₂F₅ | CF₃ | CH₃ | H | H | CH | CC l | CH | CH | O | 1-(trifluoromethyl)cyclopropyl | 4,55 | 597 | A |
| Iap-6 | C₂F₅ | CF₃ | CH₃ | H | H | CH | CC l | CH | CH | O | 2-oxo-2-(2,2,2-trifluoroethylamino)ethyl | 3,87 | 628 | A |

**Tabelle 2**

| | | | | | | | | | | | | | | |
|---|---|---|---|---|---|---|---|---|---|---|---|---|---|---|
| 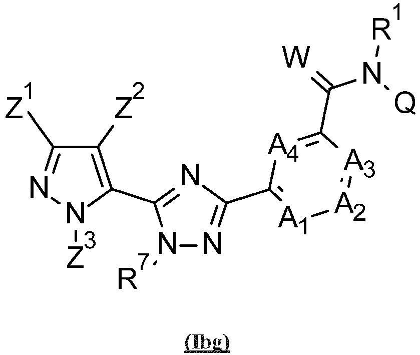 | | | | | | | | | | | | | | |

| **Bsp.-Nr.** | **Z¹** | **Z²** | **Z³** | **R¹** | **R7** | **A₄** | **A₃** | **A₂** | **A₁** | **W** | **Q** | **logP^{a)}** | **Masse [m/z] ^{a)1)}** | **Darstellungs -verfahren** |
|---|---|---|---|---|---|---|---|---|---|---|---|---|---|---|
| Ibg-2 | C₂F₅ | CF₃ | CH₃ | H | H | CH | CH | CH | CH | O | Cyclopropyl | 3,24 | 495 | B |
| Ibg-3 | C₂F₅ | CF₃ | CH₃ | H | H | CH | CH | CH | CH | O | 2,2,2-Trifluorethyl | 3,54 | 537 | B |
| Ibg-4 | C₂F₅ | CF₃ | CH₃ | H | CH₃ | CH | CC l | CH | CH | O | 2,2,2-Trifluorethyl | 4,14 | 585 | B |
| Ibg-5 | C₂F₅ | CF₃ | CH₃ | H | Et | CH | CC l | CH | CH | O | Cyclopropyl | 4,09 | 557 | B |
| Ibg-6 | C₂F₅ | CF₃ | CH₃ | H | Et | CH | CC l | CH | CH | O | 2,2,2-Trifluorethyl | 4,41 | 599 | B |
| Ibg-7 | C₂F₅ | CF₃ | CH₃ | H | CH₃ | CH | CC l | CH | CH | O | Cyclopropyl | 3,80 | 543 | B |
| Ibg-8 | C₂F₅ | CF₃ | CH₃ | H | iPr | CH | CC l | CH | CH | O | Benzyl | 4,93 | 621 | B |
| Ibg-9 | C₂F₅ | CF₃ | CH₃ | H | Et | CH | CC l | CH | CH | O | Benzyl | 4,71 | 607 | B |
| Ibg-10 | C₂F₅ | CF₃ | CH₃ | H | Me | CH | CC l | CH | CH | O | Benzyl | 4,43 | 593 | B |
| Ibg-11 | C₂F₅ | CF₃ | CH₃ | H | iPr | CH | CC l | CH | CH | O | Phenyl | 5,13 | 607 | B |
| Ibg-12 | C₂F₅ | CF₃ | CH₃ | H | Et | CH | CC l | CH | CH | O | Phenyl | 4,85 | 593 | B |
| Ibg-13 | C₂F₅ | CF₃ | CH₃ | H | iPr | CH | CC l | CH | CH | O | Cyclopropyl | 4,35 | 571 | B |
| Ibg-14 | C₂F₅ | CF₃ | CH₃ | H | Me | CH | CC l | CH | CH | O | Phenyl | 4,56 | 579 | B |

**Tabelle 3**

| | | | | | | | | | | | | | | | |
|---|---|---|---|---|---|---|---|---|---|---|---|---|---|---|---|
| 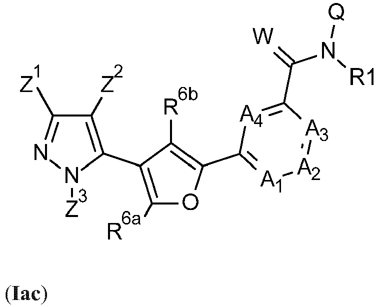 | | | | | | | | | | | | | | | |

| **Bsp.-Nr.** | **Z¹** | **Z²** | **Z³** | **R¹** | **R^{6a}** | **R^{6b}** | **A₄** | **A₃** | **A₂** | **A₁** | **W** | **Q** | **logP^{a)}** | **Masse [m/z] ^{a)1)}** | **Darstellungs -verfahren** |
|---|---|---|---|---|---|---|---|---|---|---|---|---|---|---|---|
| Iac-3 | C₂F₅ | CF₃ | CH₃ | H | H | H | CH | CC l | CH | CH | O | Cyclopropyl | 4,08 | 528 | C |

**Tabelle 4**

| | | | | | | | | | | | | | | |
|---|---|---|---|---|---|---|---|---|---|---|---|---|---|---|
| 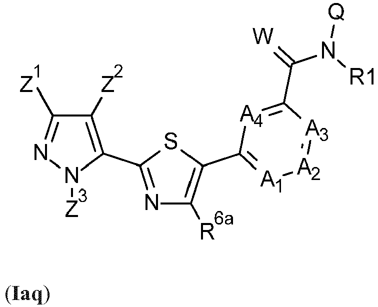 | | | | | | | | | | | | | | |

| **Bsp.-Nr.** | **Z¹** | **Z²** | **Z³** | **R¹** | **R^{6a}** | **A₄** | **A₃** | **A₂** | **A₁** | **W** | **Q** | **logP^{a)}** | **Masse [m/z] ^{a)1)}** | **Darstellungs -verfahren** |
|---|---|---|---|---|---|---|---|---|---|---|---|---|---|---|
| Iaq-2 | C₂F₅ | CF₃ | CH₃ | H | H | CH | CH | CH | CH | O | H | 3,38 | 471 | E |

**Tabelle 5**

| | | | | | | | | | | | | | |
|---|---|---|---|---|---|---|---|---|---|---|---|---|---|
| 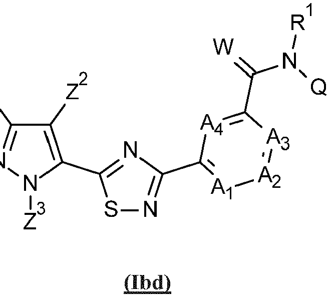 | | | | | | | | | | | | | |

| **Bsp.-Nr.** | **Z¹** | **Z²** | **Z³** | **R¹** | **A₄** | **A₃** | **A₂** | **A₁** | **W** | **Q** | **logP^{a)}** | **Masse [m/z]^{a)1)}** | **Darstellungs -verfahren** |
|---|---|---|---|---|---|---|---|---|---|---|---|---|---|
| Ibd-1 | C₂F₅ | CF₃ | CH₃ | H | CH | CCl | CH | CH | O | Benzyl | 5,00 | 596 | G |
| Ibd-2 | C₂F₅ | CF₃ | CH₃ | H | CH | CCl | CH | CH | O | 4-Fluorphenyl | 5,17 | 600 | G |

**Tabelle 6**

| | | | | | | | | | | | | | |
|---|---|---|---|---|---|---|---|---|---|---|---|---|---|
| 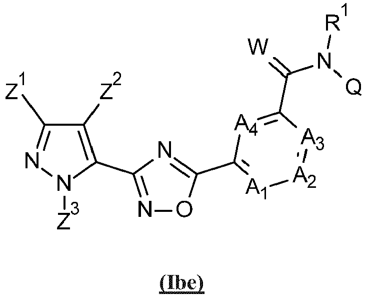 | | | | | | | | | | | | | |

| **Bsp.-Nr.** | **Z¹** | **Z²** | **Z³** | **R¹** | **A₄** | **A₃** | **A₂** | **A₁** | **W** | **Q** | **logP^{a)}** | **Masse [m/z] ^{a)1)}** | **Darstellungs -verfahren** |
|---|---|---|---|---|---|---|---|---|---|---|---|---|---|
| Ibe-2 | C₂F₅ | CF₃ | CH₃ | H | CH | CCl | CH | CH | O | Benzyl | 4,76 | 580 | H |
| Ibe-3 | C₂F₅ | CF₃ | CH₃ | H | CH | CCl | CH | CH | O | 4-Fluorphenyl | 4,91 | 584 | H |
| Ibe-4 | C₂F₅ | CF₃ | CH₃ | H | CH | CCl | CH | CH | O | 1-(cyano)cyclopropyl | 4,01 | 555 | H |

**Tabelle 7**

| | | | | | | | | | | | | | |
|---|---|---|---|---|---|---|---|---|---|---|---|---|---|
| 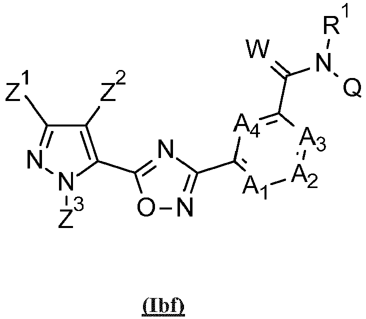 | | | | | | | | | | | | | |

| **Bsp.-Nr.** | **Z¹** | **Z²** | **Z³** | **R¹** | **A₄** | **A₃** | **A₂** | **A₁** | **W** | **Q** | **logP^{a)}** | **Masse [m/z] ^{a)1)}** | **Darstellungs -verfahren** |
|---|---|---|---|---|---|---|---|---|---|---|---|---|---|
| Ibf-2 | C₂F₅ | CF₃ | CH₃ | H | CH | CCl | CH | CH | O | 1 -(cyano)cyclopropyl | 4,14 | 555 | **I** |
| Ibf-3 | C₂F₅ | CF₃ | CH₃ | H | CH | CCl | CH | CH | O | cyclopropyl | 4,26 | 530 | **I** |

**Tabelle 8**

| | | | | | | | | | | | | | | |
|---|---|---|---|---|---|---|---|---|---|---|---|---|---|---|
| 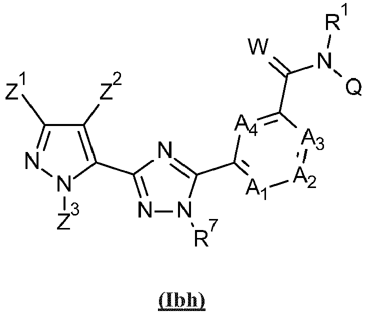 | | | | | | | | | | | | | | |

| **Bsp.-Nr.** | **Z¹** | **Z²** | **Z³** | **R¹** | **R⁷** | **A₄** | **A₃** | **A₂** | **A₁** | **W** | **Q** | **logP^{a)}** | **Masse [m/z]^{a)1)}** | **Darstellungs -verfahren** |
|---|---|---|---|---|---|---|---|---|---|---|---|---|---|---|
| Ibh-3 | C₂F₅ | CF₃ | CH₃ | H | Me | CH | CCl | CH | CH | O | 2,2,2-Trifluorethyl | 3,99 | 585 | B |
| Ibh-4 | C₂F₅ | CF₃ | CH₃ | H | Et | CH | CCl | CH | CH | O | Cyclopropyl | 3,99 | 557 | B |
| Ibh-5 | C₂F₅ | CF₃ | CH₃ | H | Et | CH | CCl | CH | CH | O | 2,2,2-Trifluorethyl | 4,32 | 599 | B |
| Ibh-6 | C₂F₅ | CF₃ | CH₃ | H | iPr | CH | CCl | CH | CH | O | Cyclopropyl | 4,39 | 571 | B |
| Ibh-7 | C₂F₅ | CF₃ | CH₃ | H | iPr | CH | CCl | CH | CH | O | 2,2,2-Trifluorethyl | 4,68 | 613 | B |
| Ibh-8 | C₂F₅ | CF₃ | CH₃ | H | Me | CH | CCl | CH | CH | O | Cyclopropyl | 3,67 | 543 | B |
| Ibh-9 | C₂F₅ | CF₃ | CH₃ | H | iPr | CH | CCl | CH | CH | O | Benzyl | 4,98 | 621 | B |
| Ibh-10 | C₂F₅ | CF₃ | CH₃ | H | Et | CH | CCl | CH | CH | O | Benzyl | 4,59 | 607 | B |
| Ibh-11 | C₂F₅ | CF₃ | CH₃ | H | Me | CH | CCl | CH | CH | O | Benzyl | 4,29 | 593 | B |
| Ibh-12 | C₂F₅ | CF₃ | CH₃ | H | iPr | CH | CCl | CH | CH | O | Phenyl | 5,23 | 607 | B |
| Ibh-13 | C₂F₅ | CF₃ | CH₃ | H | Et | CH | CCl | CH | CH | O | Phenyl | 4,73 | 593 | B |
| Ibh-14 | C₂F₅ | CF₃ | CH₃ | H | Me | CH | CCl | CH | CH | O | Phenyl | 4,42 | 579 | B |

**Tabelle 9**

| | | | | | | | | | | | | | | |
|---|---|---|---|---|---|---|---|---|---|---|---|---|---|---|
| 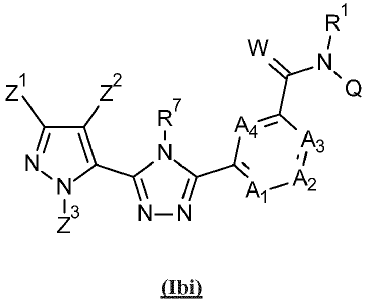 | | | | | | | | | | | | | | |

| **Bsp.-Nr.** | **Z¹** | **Z²** | **Z³** | **R¹** | **R⁷** | **A₄** | **A₃** | **A₂** | **A₁** | **W** | **Q** | **logP^{a)}** | **Masse [m/z]^{a)1)}** | **Darstellungs -verfahren** |
|---|---|---|---|---|---|---|---|---|---|---|---|---|---|---|
| Ibi-1 | C₂F₅ | CF₃ | CH₃ | H | H | CH | CCl | CH | CH | O | Benzyl | 4,00 | 579 | B |
| Ibi-2 | C₂F₅ | CF₃ | CH₃ | H | H | CH | CCl | CH | CH | O | 2,2,2-Trifluorethyl | 3,73 | 571 | B |
| Ibi-3 | C₂F₅ | CF₃ | CH₃ | H | H | CH | CCl | CH | CH | O | Cyclopropyl | 3,40 | 529 | B |
| Ibi-4 | C₂F₅ | CF₃ | CH₃ | H | H | CH | CCl | CH | CH | O | Phenyl | 4,13 | 565 | B |

**Tabelle 10**

| | | | | | | | | | | | | | | | |
|---|---|---|---|---|---|---|---|---|---|---|---|---|---|---|---|
| 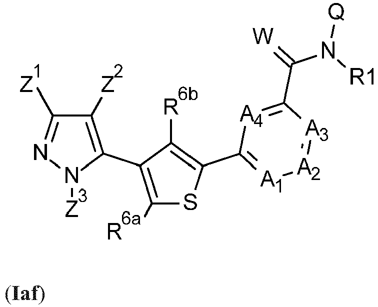 | | | | | | | | | | | | | | | |

| **Bsp.-Nr.** | **Z¹** | **Z²** | **Z³** | **R¹** | **R^{6a}** | **R^{6b}** | **A₄** | **A₃** | **A₂** | **A₁** | **W** | **Q** | **logP^{a)}** | **Masse [m/z] ^{a)1)}** | **Darstellungs -verfahren** |
|---|---|---|---|---|---|---|---|---|---|---|---|---|---|---|---|
| Iaf-1 | C₂F₅ | CF₃ | CH₃ | H | H | H | CH | CC l | CH | CH | O | Cyclopropyl | 4,31 | 544 | C |

**Tabelle 11**

| | | | | | | | | | | | | | | | |
|---|---|---|---|---|---|---|---|---|---|---|---|---|---|---|---|
| 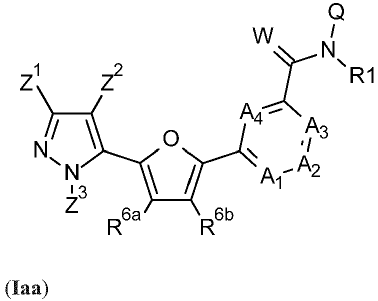 | | | | | | | | | | | | | | | |

| **Bsp.-Nr.** | **Z¹** | **Z²** | **Z³** | **R¹** | **R^{6a}** | **R^{6b}** | **A₄** | **A₃** | **A₂** | **A₁** | **W** | **Q** | **logP^{a)}** | **Masse [m/z] ^{a)1)}** | **Darstellungs -verfahren** |
|---|---|---|---|---|---|---|---|---|---|---|---|---|---|---|---|
| Iaa-1 | C₂F₅ | CF₃ | CH₃ | H | H | H | CH | CC l | CH | CH | O | 1-(cyano)cyclopropyl | 4,08 | 553 | C |

| | | | | | | | | | | | | | | | |
|---|---|---|---|---|---|---|---|---|---|---|---|---|---|---|---|
| ¹Bei der angegebenen Masse handelt es sich um den Peak des Isotopenmusters des [M+H]⁺ Ions mit der höchsten Intensität; falls das [M-H]⁻ Ion detektiert wurde, ist die Massenangabe mit² gekennzeichnet. ²Bei der angegebenen Masse handelt es sich um den Peak des Isotopenmusters des [M-H]⁻ Ions mit der höchsten Intensität. ^{a)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 micron; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System. ^{b)} Anmerkung zur Bestimmung der Retentionszeit und Massendetektion: Die Bestimmung der angegebenen Retentionszeiten erfolgte an einer Phasenumkehrsäule (C18). Shimadzu LCMS 2020; 50*3,0 Shimadzu shim-pack XR-ODS 2,2 micron; Eluent A: Wasser (0,05 % TFA); Eluent B: Acetonitril; linearer Gradient von 5 % Acetonitril bis 95 % Acetonitril in 6,00 min, dann 95% Acetonitril für weitere 1,1 min; Ofentemperatur 40 °C; Fluß:1,0 mL/min. ^{c)} Anmerkung zur Bestimmung der Retentionszeit und Massendetektion: Instrument: Waters ACQUITY SQD UPLC System; Säule: Waters Acquity UPLC HSS T3 1.8 µ 50 x 1 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 208 - 400 nm. ^{d)} Anmerkung zur Bestimmung der Retentionszeit und Massendetektion: Die Bestimmung der angegebenen Retentionszeiten erfolgte an einer Phasenumkehrsäule (C18). Shimadzu LCMS 2020; 50*3,0 Shimadzu shim-pack XR-ODS 2,2 microm; Eluent A: Wasser (0,05 % TFA); Eluent B: Acetonitril; linearer Gradient von 5 % Acetonitril bis 100 % Acetonitril in 1,2 min, dann 100% Acetonitril für weitere 1,1 min; Ofentemperatur 40 °C; Fluß:1,0 mL/min. ^{e)} Anmerkung zur Bestimmung der Retentionszeit und Massendetektion: Die Bestimmung der angegebenen Retentionszeiten erfolgte an einer Phasenumkehrsäule (C18). Shimadzu LCMS 2020; 50*3,0 Shimadzu shim-pack XR-ODS 2,2 micron; Eluent A: Wasser (0,1 % Ameisensäure); Eluent B: Acetonitril (0,1% Ameisensäure); linearer Gradient von 5 % Acetonitril bis 100 % Acetonitril in 1,2 min, dann 100% Acetonitril für weitere 1,1 min; Ofentemperatur 40 °C; Fluß: 1,0 mL/min. ^{f)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18). Agilent 1100 LC-System; 50*4,6 Zorbax Eclipse Plus C18 1,8 microm; Eluent A: Acetonitril; Eluent B: Wasser (+79mg Ammoniumhydrogencarbonat/l); linearer Gradient von 10 % Acetonitril bis 95 % Acetonitril in 4,25 min, dann 95% Acetonitril für weitere 1,55 min; Ofentemperatur 55 °C; Fluß:2,0 mL/min. Die Massendetektion erfolgt über ein Agilend MSD-System. | | | | | | | | | | | | | | | |

### NMR-Daten ausgewählter Beispiele

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾; ......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| Beispiel laa-1: ¹H-NMR(600,1 MHz, CD3CN): δ= 7,832(3,2);7,828(4,1);7,811(2,4);7,807(1,7);7,797(2,5);7,793(2,1);7,603(1,5);7.545(3,8);7, 531(3,4);7,089(4,0);7,083(4,6);7,011(3,7);7,005(3,2);5,447(1,2);3,995(16,0);2,142(7,2);1,957(0,6);1,953(0,7);1,949(3,8);1,945(6,7);1,941(9, 8);1,937(6,6);1,933(3,3);1,594(1,7);1,585(4,1);1,580(4,1);1,571(2,0);1,360(2,1);1,350(4,1);1,346(4,3);1,336(1,7);0,000(1,3) |
| Beispiel lac-1: ¹H-NMR(600,1 MHz, CD3CN): δ= 7,863(5,5);7,827(3,2);7,823(4,0);7,805(2,1);7,801(1,6);7,790(2,2);7,787(1,9);7,782(0,3);7, 582(1,6);7,552(3,7);7,538(3,3);7,424(0,4);7,048(5,0);3,835(16,0);3,810(4,4);3,638(0,9);3,412(0,3);3,282(1,7);3,273(1,7);2,142(1,6);1,964(0, 8);1,956(2,3);1,952(2,8);1,948(15,4);1,944(26,8);1,939(39,4);1,935(26,8);1,931(13,6);1,591(1,6);1,581(4,2);1,577(4,2);1,568(2,0);1,364(2,0) ;1,354(4,1);1,350(4,3);1,340(1,7);1,272(0,3);0,000(5,2) |
| Beispiel lac-3: ¹H-NMR(400,0 MHz, d₆-DMSO): δ= 8,582(2,5);8,571(2,4);8,315(1,2);8,270(6,0);8,078(0,4);8,074(0,4);7,813(1,8);7,807(2,4); 7,792(2,1);7,784(6,0);7,602(3,8);7,592(0,5);7,582(3,1);7,551(0,4);7,529(0,4);7,421(5,8);7,178(0,4);6,924(0,4);6,919(0,4);5,755(2,7);3,907(1 6,0);3,710(1,4);3,324(129,5);3,301(1,6);2.861(0,8);2,852(1,1);2,843(1,6);2,833(1,5);2,824(1,1);2,815(0,7);2,676(0,6);2,671(0,7);2,667(0,5);2 ,506(84,3);2,502(105,1);2,498(79,3);2,333(0,6);2,329(0,7);0,735(1,0);0,723(2,9);0,718(3,7);0,705(3,6);0,700(3,0);0,688(1,4);0,672(0,4);0,66 7(0,4);0,565(1,4);0,555(8,8);0,548(8,7);0,589(8,1);0,527(0,9);0,464(0,5);0457(04);0,146(0,5);0,009(10,9);0,000(109,4);-0,149(0,6) |
| Beispiel laf-1: ¹H-NMR(400,0 MHz, d₆-DMSO): δ= 8,578(2,3);8,567(2,4);8,051(4,9);7,842(4,8);7,752(1,5);7,746(2,1);7,725(7,0);7,572(3,6);7 ,552(2,9);3,862(0,7);3,846(16,0);3,565(0,5);3,327(38,2);2,854(0,6);2,844(1,0);2,836(1,4);2,826(1,4);2,817(1,0);2,808(0,7);2,671(0,5);2,502( 71,9);2,329(0,5);2,086(0,4);1,398(4,4);0,731(0,8);0,713(3,6);0,701(3,4);0,695(3,0);0,684(1,2);0,563(1,1);0,552(3,6);0,545(3,7);0,537(3,2);0, 524(0,9);0,000(41,8) |
| Beispiel laj-1: ¹H-NMR(400,0 MHz, d₆-DMSO): δ= 8,749(1,8);8,739(1,8);8,486(3,8);8,316(0,4);8,263(1,8);8,243(1,9);8,086(1,9);8,066(2,1);7 ,727(1,9);7,708(3,5);7,688(1,6);5,756(0,8);4,288(1,0);3,973(16,0);3,324(145,3);2,917(0,6);2,908(0,9);2,899(1,4);2,889(1,4);2,881(0,9);2,871 (0,7);2,675(0,6);2,671(0,9);2,667(0,6);2,524(1,9);2,506(96,8);2,502(127,5);2,498(94,2);2,333(0,6);2,329(0,9);2,324(0,6);0,760(0,8);0,746(2, 3);0,742(8,4);0,780(8,0);0,728(2,7);0,718(1,2);0,674(0,4);0,684(1,2);0,628(8,7);0,616(8,8);0,608(2,6);0,595(0,8);0,008(1,8);0,000(56,9);-0,008(2,4) |
| Beispiel lap-1: ¹H-NMR(400,0 MHz, CD3CN): δ= 7,969(8,9);7,965(3,7);7,950(2,8);7,945(1,6);7,621(2,7);7,617(1,5);7,602(1,6);7,598(2,4);7, 312(7,0);7,019(1,0);5,448(0,9);3,967(16,0);2,879(0,7);2,870(1,0);2,861(1,6);2,851(1,6);2,843(1,1);2,833(0,8);2,473(0,4);2,468(0,8);2,464(1, 0);2,459(0,7);2,454(0,4);2,167(275,2);2,120(0,7);2,114(1,0);2,108(1,2);2,101(0,9);2,095(0,5);1,964(7,0);1,958(20,9);1,952(84,6);1,946(148, 5);1,940(190,5);1,934(131,6);1,928(67,1);1,781(0,5);1,775(0,9);1,769(1,1);1,763(0,8);1,756(0,4);1,270(1,2);0,804(0,8);0,791(2,8);0,786(3,4) ;0,773(3,7);0,768(2,6);0,756(1,1);0,625(1,1);0,615(3,0);0,613(3,1);0,608(3,3);0,604(3,0);0,599(2,7);0,586(0,8);0,146(2,4);0,027(0,3);0,000(5 39,7);-0,009(31,0);-0,037(0,4);-0,039(0,4);-0,041(0,4);-0,042(0,4);-0,045(0,4);-0,150(2,4) |
| Beispiel lap-2: ¹H-NMR(400,0 MHz, CD3CN): δ= 8,020(7,6);8,001(2,4);7,996(1,5);7,671(2,3);7,649(2,1);7,485(0,8);7,328(7,0);4,153(0,8);4, 137(0,9);4,130(2,4);4,113(2,4);4,106(2,5);4,090(2,4);4,083(1,0);4,066(0,9);3,969(16,0);2,467(0,3);2,463(0,4);2,458(0,3);2,165(115,1);2,113( 0,4);2,107(0,4);1,971(1,0);1,964(2,6);1,958(6,5);1,952(29,7);1,946(52,8);1,940(69,5);1,934(47,9);1,928(24,4);1,768(0,4);1,437(0,9);1,270(0, 8);1,203(0,3);0,146(1,5);0,007(17,8);-0,001(270,7);-0,150(1,5) |
| Beispiel lap-3: ¹H-NMR(601,6 MHz, CD3CN): δ= 8,020(9,9);8,018(3,7);8,008(3,1);8,004(1,9);7,654(2,7);7,652(1,8);7,641(1,9);7,639(3,0);7, 634(1,5);7,324(8,4);3,970(16,0);2,146(107,9);2,060(0,3);2,056(0,6);2,052(0,9);2,048(0,6);1,973(1,5);1,966(3,5);1,958(9,3);1,954(11,2);1,95 0(58,9);1,946(104,1);1,942(152,2);1,937(102,4);1,933(51,4);1,917(0,4);1,831(0,6);1,827(0,9);1,823(0,6);1,601(1,8);1,591(4,2);1,587(4,3);1, 578(2,1);1,437(0,4);1,375(2,2);1,366(4,1);1,362(4,4);1,352(1,8);1,216(0,4);1,204(0,7);1,192(0,4);0,005(2,1);0,000(64,8);-0,006(2,3) |
| Beispiel lap-4: ¹H-NMR(601,6 MHz, CD3CN): δ= 8,008(3,1);8,005(4,1);7,991(2,6);7,987(1,7);7,977(2,6);7,973(2,1);7,640(3,7);7,626(3,4);7, 489(0,6);7,477(0,6);7,321(8,1);5,330(0,8);5,316(1,6);5,303(1,6);5,288(0,9);3,970(16,0);3,542(2,3);3,540(1,3);3,526(3,8);3,514(1,5);3,512(2, 8);3,374(2,8);3,371(1,6);3,360(4,0);3.357(3,8);3,346(1,3);3,344(2,3);3,279(0,8);3,270(0,8);2,141(321,1);2,110(0,3);2,059(0,9);2,055(1,5);2,0 51(2,2);2,047(1,5);2,043(0,8);1,965(9,0);1,957(23,2);1,952(26,0);1,949(154,0);1,945(264,8);1,940(384,0);1,936(266,4);1,932(137,2);1,924( 2,4);1,834(0,8);1,830(1,5);1,826(2,2);1,822(1,4);1,817(0,7);1,270(0,4);0,005(1,6):0,000(57,9);-0,006(1,9) |
| Beispiel lap-5: ¹H-NMR(601,6 MHz, CD3CN): δ= 7,999(2,0);7,996(2,6);7,985(1,9);7,982(3,1);7,971(4,2);7,968(3,0);7,643(3,7);7,629(3,4);7, 535(1,3);7,317(8,5);3,968(16,0);2,141(240,0);2,059(0,8);2,055(1,3);2,051(1,9);2,047(1,2);2,043(0,6);1,965(7,2);1,957(19,0);1,953(21,7);1,9 49(124,7);1,945(218,2);1,940(315,9);1,936(211,0);1,932(109,2);1,924(1,6);1,834(0,6);1,830(1,2);1.826(1,8);1,822(1,2);1,818(0,6);1,399(1,5 );1,389(3,5);1,386(3,8);1,376(2,1);1,269(1,6);1,259(2,9);1,248(0,7);1,217(0,5);0,005(1,4);0.000(48,0);-0.006(1,4) |
| Beispiel lap-6: ¹H-NMR(601,6 MHz, CD3CN): δ= 8,111(3,3);8,108(3,3);8,002(2,1);7,999(2,0);7,988(2,4);7,985(2,2);7,663(3,7);7,650(3,5);7, 362(0,7);7,324(6,5);7,095(0,5);5,448(0,3);4,058(6,7);4,048(6,7);3,977(0,8);3,969(16,0);3,961(2,3);3,950(2,3);3,946(2,4);3,935(2,3);3,930(0, 9);3,919(0,8);3,891(0,9);3,270(0,3);2,141(353,9);2,108(0,3);2,059(0,9);2,055(1,5);2,051(2,2);2,047(1,5);2,043(0,7);1,972(1,1);1,965(9,1);1,9 57(23,6);1,953(27,2);1,949(155,9);1,945(269,4);1,940(394,3);1,936(268,2);1,932(136,9);1,834(0,9);1,830(1,5);1,826(2,2);1,822(1,5);1,817( 0,8);1,285(0,6);1,271(1,2);1,204(0,5);0,005(1,7);0,000(57,4);-0,006(1,8) |
| Beispiel laq-1: ¹H-NMR(400,0 MHz, d₆-DMSO): δ= 9,455(4,1);8,692(7,3);8,466(4,0);8,316(0,4);8,231(2,1);8,211(2,2);7,876(2,0);7,856(2,3); 7,6421,8;7,6223,1;7,6031,5;7,2500,8;7,2320,7;7,1820,9;7,1640,8;7,1440,4;3,98916,0;3,32350,6;2,6710,8;2,502114,6;2,329(0,7);2,300(3,4);1,605(1,5);1,591(4,2);1,584(4,7);1,571(2,0);1,329(1,9);1,316(4,3);1,309(4,6);1,295(1,6);1,234(0,4);1,056(0,5);0,146(0,4); 0,000(78,0);-0,150(0,4) |
| Beispiel laq-2: ¹H-NMR(400,0 MHz, d₆-DMSO): δ= 8,670(8,8);8,509(3,7):8,316(0,8);8,181(1,9);8,162(2,0);8,094(1,6);7,910(1,8);7,890(2,0); 7,599(1.9);7,579(3,4);7,560(1,6);7,479(1,6);5,756(0,6);3,995(16,0);3,322(87,9);2,676(0,9);2,671(1,2);2,667(0,9);2,564(0,3);2,524(3,4);2,511 (65,5);2,506(132,2);2,502(176,6);2,498(132,7);2,333(0,7);2,329(1,1);2,324(0,8);1,235(0,9);0,146(0,8);0,008(6,3);0,000(164,7);-0,008(8,4);-0,150(0,8) |
| Beispiel lat-1: ¹H-NMR (400 MHz, CHLOROFORM-d) δ [ppm]: 0.000 (1.72), 0.645 (0.48), 0.658 (1.74), 0.662 (1.89), 0.668 (1.94), 0.672 (1.77), 0.675 (1.43), 0.685 (0.59), 0.897 (0.61), 0.910 (1.68), 0.915 (1.96), 0.929 (2.03), 0.933 (1.46), 0.946 (0.46), 1.255 (0.42), 1.570 (16.00), 2.968 (0.54), 2.977 (0.69), 2.979 (0.64), 2.986 (0.68), 2.995 (0.50), 3.998 (9.61), 6.808 (0.46), 6.840 (0.46), 7.216 (0.90), 7.237 (1.12), 7.244 (1.18), 7.639 (2.98), 8.138 (0.57), 8.144 (0.65), 8.150 (0.66), 8.156 (0.74), 8.159 (0.72), 8.165 (0.64), 8.171 (0.61), 8.177 (0.51), 8.641 (1.00), 8.647 (0.96), 8.658 (1.02), 8.665 (0.90). |
| Beispiel law-1: ¹H-NMR(601,6 MHz, CDCl₃): δ= 7,946(0,7);7,886(1,0);7,884(1,3);7,872(1,4);7,869(1,0);7,476(0,6);7,464(1,5);7,452(1,9);7,4 39(0,7);7,265(6,2);6,770(6,3);5,671(1,1);5,300(2,4);4,266(1,3);4,005(0,7);3,979(1,2);3,846(0,7);3,821(11,9);3,780(0,9);3,767(16,0);3,123(1, 2);3,114(1,3);2,890(0,6);2,875(0,6);2,869(0,8);2,863(1,2);2,857(0,9);2,851(0,7);2,845(0,4);2,371(0,4);1,966(0,5);1,644(1,1);1,428(0,4);1,334 (0,4);1,318(0,4);1,285(0,6);1,255(1,5);0,621(0,4);0,505(0,5);0,000(2,6) |
| Beispiel lax-1: ¹H-NMR(400,0 MHz, d₆-DMSO): δ= 8,318(0,3);7,733(1,4);7,703(0,8);7,698(0,9);7,688(1,2);7,681(1,4);7,676(1,3);7,599(3,3); 7,586(2,2);7,567(0,4);7,472(0,3);7,461(0,4);7,267(0,5);6,891(4,9);5,757(0,4);4,038(0,6);4,020(0,5);3,995(16,0);3,974(12,2);3,934(0,6);3,887 (0,3);3,879(0,8);3,858(1,1);3,754(1,3);3,330(99,3);3,036(0,4);2,996(4,1);2,931(0,6);2,919(0,5);2,811(1,0);2,676(0,6);2,672(0,9);2,667(0,7);2 ,507(96,6);2,503(128,0);2,498(97,5);2,333(0,6);2,329(0,8);2,325(0,6);1,989(1,6);1,706(0,6);1,398(5,4);1,351(0,4);1,336(0,4);1,287(0,5);1,27 1(0,6);1,259(0,5);1,249(0,7);1,233(1,8);1,216(0,5);1,193(0,5);1,175(0,9);1,157(0,5);0,539(1,1);0,451(1,0);0,000(1,3) |
| Beispiel lbd-1: ¹H-NMR(400,0 MHz, d₆-DMSO): δ= 9,171(1,0);9,156(2,1);9,141(1,0);8,315(2,0);8,310(2,3);8,294(1,9);8,289(2,7);8,266(4,6); 8,261(3,6);7,757(3,9);7,736(3,7);7,392(1,3);7,376(11,0);7,359(5,0);7,339(1,4);7,291(0,8);7,286(1,3);7,270(1,5);7,254(0,7);4,501(4,7);4,486( 4,7);3,977(16,0);3,901(0,6);3,368(0,3);3,323(127,1);2,671(0,7);2,667(0,5);2,506(86,7);2,502(110,7);2,498(83,2);2,333(0,5);2,329(0,7);2,324 (0,5);1,236(0,4);0,000(3,3) |
| Beispiel lbd-2: ¹H-NMR(400,0 MHz, d₆-DMSO): δ= 10,716(4,1);8,391(3,3);8,386(4,4);8,373(2,4);8,368(1,5);8,352(2,3);8,347(1,9);7,825(3,4) ;7,804(3,2);7,764(2,4);7,752(2,8);7,742(2,9);7,729(2,6);7,238(2,6);7,216(4,8);7,194(2,4);3,975(16,0);3,902(0,5);3,366(0,4);3,325(92,4);2,67 2(0,6);2,503(91,0);2,329(0,5);1,235(0,4);0,000(2,2) |
| Beispiel lbd-3: ¹H-NMR(400,0 MHz, d₆-DMSO): δ= 8,656(2,1);8,646(2,1);8,314(0,3);8,291(2,0);8,286(2,3);8,270(2,1);8,265(2,5);8,197(4,3); 8,191(8,9);7,728(4,0);7,707(8,7);4,000(0,5);8,967(16,0);8,902(1,4);8,828(180,7);2,868(0,6);2,858(0,9);2,849(1,4);2,839(1,4);2,881(0,9);2,82 1(0,7);2,676(0,5);2,671(0,7);2,667(0,5);2,541(0,5);2,507(88,6);2,502(115,2);2,498(86,8);2,333(0,5);2,329(0,7);2,325(0,5);1,260(0,4);1,235(1 ,1);0,740(0,8);0,727(2,4);0,722(3,4);0,710(3,2);0,704(2,7);0,693(1,1);0,567(1,1);0,557(3,3);0,550(3,1);0,547(3,0);0,541(2,8);0,529(0,8);0,00 0(3,6) |
| Beispiel lbe-1: ¹H-NMR(601,6 MHz, d₆-DMSO): δ= 8,741(2,0);8,734(2,0);8,243(2,1);8,240(2,3);8,229(2,2);8,226(2,5);8,166(4,1);8,162(3,7); 7,887(0,5);7,883(0,4);7,845(3,9);7,831(3,6);7,665(0,4);7,651(0.4);4,163(0,6);4,150(16,0);3,872(2,3);3,330(624,0);3,051(0,5);2,869(0,6);2,86 2(0,9);2,857(1,3);2,850(1,3);2,844(0,9);2,838(0,7);2,831(0,4);2,828(0,7);2,617(1,7);2,614(2,3);2,611(1,7);2,523(4,2);2,520(5,4);2,517(5,6);2 ,508(133,3);2,505(273,9);2,502(372,9);2,499(282,1);2,474(1,3);2,390(1,7);2,387(2,3);2,384(1,7);1,639(0,8);1,235(1,6);0,854(0,4);0,744(0,8) ;0,735(2,4);0,732(3,3);0,724(3,1);0,720(2,7);0,712(1,4);0,699(0,6);0,582(1,0);0,574(2,9);0,570(2,9);0,567(2,7);0,564(2,8);0,556(0,8);0,544(0 ,5);0,540(0,5);0,537(0,5);0,534(0,4);0,000(4,9) |
| Beispiel lbe-2: ¹H-NMR(400,0 MHz, d₆-DMSO): δ= 9,232(0,9):9,217(1,9);9,202(1,0);8,313(0,5);8,272(1,7);8,266(2,2);8,251(1,8);8,245(2,6); 8,216(4,2);8,211(3,6);7,875(3,7);7,854(3,4);7,403(1,3);7,383(9,7);7,367(4,6);7,355(0,6);7,347(1,6);7,298(0,8);7,293(1,2);7,288(0,7);7,277(1, 5);7,265(0,5);7,260(0,7);7,255(0,4);4,516(4,5);4,501(4,5);4,151(16,0);4,134(0,4);3,901(1,7);3,390(0,3);3,325(329,8);3,279(0,6);2,675(0,8);2, 671(1,1);2,667(0,9);2,506(139,0);2,502(186,0);2,498(145,3);2,333(0,8);2,329(1,1);2,324(0,9);1,258(0,4);1,235(1,3);0,913(0,3);0,000(4,9) |
| Beispiel lbe-3: ¹H-NMR(400,0 MHz, d₆-DMSO): δ= 10,764(3,7);8,396(3,4);8,390(3,9);8,328(1,9);8,323(1,7);8,314(0,6);8,307(2,1);8,301(1,9) ;7,938(3,5);7,917(3,2);7,765(2,4);7,752(2,6);7,747(1,9);7,742(2,8);7,730(2,6);7,251(2,5);7,229(4,7);7,207(2,4);4,162(16,0);3,902(1,0);3,325( 256,2);2,671(1,0);2,668(0,8);2,507(135,0);2,503(174,0);2,498(133,6);2,329(1,1);2,325(0,9);1,336(0,3);1,277(0,5);1,261(1,0);1,245(0,9);1,23 5(0,9);0,000(4,3) |
| Beispiel lbe-4: ¹H-NMR(400,0 MHz, d₆-DMSO): δ= 9,628(4,2);8,297(1,6);8,292(2,4);8,273(9,0);7,892(2,6);7,870(2,4);4,143(16,0);3,377(0,4) ;3,325(314,7);3,280(0,5);2,671(1,2);2,666(1,0):2,541(23,4);2,506(148,1);2,502(190,5);2,498(147,5);2,328(1,2);2,324(0,9);1,618(1,4);1,604( 3,9);1,597(4,2);1,584(1,8);1,357(1,8);1,344(4,0);1,337(4,2);1,323(1,5);1,297(0,3);1,286(0,3);1,258(0,6);1,235(2,2);0,854(0,4);0,000(1,5) |
| Beispiel lbf-1: ¹H-NMR(400,0 MHz, d₆-DMSO): δ= 10,753(3,7);8,314(0,4);8,275(3,5);8,270(4,5);8,246(2,5);8,240(1,8);8,225(2,6);8,219(2,2); 7,883(4,0);7,862(3,6);7,771(2,5);7,758(2,8);7,753(1,7);7,748(2,9);7,735(2,7);7,248(2,7);7,242(1,0);7,226(4,9);7,209(0,9);7,203(2,5);4,340(1 6,0);3,902(1,7);3,323(249,1);3,286(0,4);2,675(0,9);2,671(1,2);2,666(0,9);2,524(4,4);2,511(79,5);2,506(155,9);2,502(201,8);2,497(147,7);2,4 93(72,7);2,333(0,9);2,329(1,2);2,324(0,9);1,234(1,2);0,000(6,4) |
| Beispiel lbf-2: ¹H-NMR(400,0 MHz, d₆-DMSO): δ= 9,595(4,1);8,313(0,4);8,215(1,8);8,211(1,9);8,195(1,9);8,190(2,2);8,146(4,0);8,141(3,4);7 ,838(3,4);7,817(3,1);4,329(16,0);3,901(1,5);3,382(0,4);3,322(189,7);2,671(1,2);2,502(195,8);2,328(1,2);1,619(1,4);1,605(3,9);1,598(4,1);1,5 85(1,7);1,344(1,7);1,330(4,0);1,324(4,1);1,309(1,4);1,235(0,5);0,000(4,5) |
| Beispiel lbf-3: ¹H-NMR(400,0 MHz, d₆-DMSO): δ= 8,694(2,3);8,684(2,2);8,164(2,0);8,159(2,1);8,143(2,3);8,138(2,3);8,063(4,3);8,058(3,7);7 ,790(3,5):7,769(3,2);4,332(16,0);3,901(0,4);3,327(145,2);2,882(0,8);2,872(1,1):2,864(1,5);2,854(1,5);2,846(1,1);2,836(0,7);2,826(0,3);2,672 (0,8);2,506(103,4);2,502(120,8);2,329(0,7);0,751(1,0);0,734(3,6);0,721(3,6);0,716(3,0);0,704(1,2);0,577(1,4);0,566(3,9);0,560(3,9);0,551(3, 2);0,539(0,9);0,000(2,6) |
| Beispiel lbg-1: ¹H-NMR(400,0 MHz, d₆-DMSO): δ= 15,366(3,2);9,256(1,1);9,241(2,2);9,226(1,0);8,584(3,7);8,316(0,7);8,209(1,9);8,189(1,9) ;8,078(1,8);8,059(1,9);7,734(1,7);7,714(3,1);7,695(1,5);7,352(16,0);7,344(8,1);7,339(9,2);7,327(1,4);7,318(0,9);7,275(1,0);7,267(1,3);7,262( 1,3);7,260(1,3);7,253(1,6);7,247(1,1);7,241(0,8);7,232(0,4);4,530(4,9);4,515(5,0);4,494(0,3);4,076(15,4);3,996(0,3);3,903(3,9);3,892(0,3);3, 331(332,8);2,681(0,4);2,676(0,9);2,672(1,3);2,667(0,9);2,663(0,4);2,542(0,7);2,525(3,9);2,512(78,5);2,507(156,8);2,503(204,8);2,498(146,8 );2,494(70,0);2,339(0,4);2,334(0,9);2,329(1,2);2,325(0,9);1,259(0,3);1,245(0,3);1,234(0,6);0,941(0,5);0,876(0,4);0,000(0,8) |
| Beispiel lbg-2: ¹H-NMR(400,0 MHz, CD3CN): δ= 13,369(0,4);8,515(5,2);8,172(1,6);8,153(1,7);7,910(2,1);7,890(2,4);7,655(1,6);7,636(2,8);7 ,617(1,4);7,588(0,4);7,288(1,2);4,031(16,0);2,919(0,3);2,910(1,0);2,900(1,5);2,892(2,1);2,882(2,1);2,873(1,5);2,864(1,0);2,855(0,4);2,473(0, 5);2,469(0,7);2,464(0,6);2,460(0,3);2,159(219,6);2,126(1,7);2,120(1,5);2,114(1,7);2,108(2,2);2,102(1,5);2,095(0,9);2,087(0,6);2,024(0,4);2,0 10(0,4);1,996(0,4);1,965(7,4);1,959(18,5);1,953(96,5);1,947(173,4);1,940(233,2);1,934(161,4);1,928(83,8);1,884(0,3);1,781(0,7);1,775(1,1); 1,769(1,5);1,763(1,1);1,756(0,6);1,546(0,4);1,527(0,4);1,508(0,4);1,487(0,4);1,429(0,4);1,419(0,4);1,410(0,3);1,400(0,4);1,380(0,4);1,340(0, 8);1,270(13,3);1,217(1,0);1,177(0,5);1,161(0,4);1,151(0,4);1,126(0,4);1,116(0,4);1,104(0,4);0,974(0,4);0,932(0,4);0,913(0,5);0,881(2,6);0,86 4(1,7);0,857(1,6);0,836(1,2);0,817(0,6);0,798(1,4);0,786(3,7);0,781(5,1);0,768(4,9);0,763(4,0);0,751(1,9);0,730(0,5);0,712(0,4);0,696(0,4);0 ,686(0,4);0,657(1,8);0,646(4,8);0,639(4,8);0,636(4,4);0,630(3,9);0,617(1,3);0,146(1,9);0,079(3,5);0,008(15,8);0,000(413,3);-0,009(19,0);-0,150(2,0) |
| Beispiel lbg-3: ¹H-NMR(400,0 MHz, d₆-DMSO): δ= 15,396(3,1);9,328(1,6);9,313(3,1);9,297(1,5);8,582(5,4);8,316(0,7);8,249(2,5);8,230(2,6) ;8,070(2,1);8,051(2,4);7,763(1,7);7,744(2,9);7,724(1,4);4,184(0,8);4,160(2,6):4,144(2,8);4,136(2,9):4,120(2,7);4,095(1,4);4,077(16,0);3,903( 2,5);3,332(329,6);2,677(1,0);2,672(1,3);2,668(1,0);2,542(0,8);2,525(4,1);2,512(84,7);2,508(167,9);2,503(218,4);2,499(158,5);2,494(77,6);2, 335(1,0);2,330(1,3);2,325(1,0);1,235(0,4);0,000(0,8) |
| Beispiel lbg-4: ¹H-NMR(400,0 MHz, d₆-DMSO): δ= 9,321(1,0);9,305(1,9);9,289(0,9);8,122(1,7);8,117(1,8);8,101(1,8);8,096(2,0);8,051(0,4); 8,046(0,4);8,027(3,8);8,022(3,4);7,703(3,2);7,682(2,9);4,286(1,4);4,153(0,6);4,130(1,7);4,114(1,8);4,106(1,9);4,089(1,7);4,066(0,6);3,930(1 4,7);3,911(16,0);3,323(100,6);2,672(0,6):2,506(82,4);2,502(100,9);2,329(0,6);0,000(2,4) |
| Beispiel lbg-5: ¹H-NMR(400,0 MHz, d₆-DMSO): δ= 8,617(2,4);8,606(2,3);8,074(1,9);8,070(2,0);8,053(2,1);8,049(2,2);7,982(4,1);7,977(3,7); 7,646(3,5);7,625(3,2);4,212(0,7);4,194(1,4);4,176(1,6);4,167(1,6);4,149(1,4);4,132(0,7);3,899(16,0);3,323(124,1);2,857(0,7);2,847(1,0);2,83 8(1,4);2,829(1,4);2,820(1,0);2,811(0,6);2,671(0,7);2,502(125,5);2,329(0,7);1,429(4,5);1,411(9,2);1,393(4,4);0,732(0,8);0,714(3,5);0,701(3,4 );0,697(3,0);0,685(1,1);0,562(1,2);0,551(3,7);0,545(3,8);0,537(3,2);0,524(0,9);0,000(2,5) |
| Beispiel lbg-6: ¹H-NMR(400,0 MHz, d₆-DMSO): δ= 9,319(1,0);9,303(2,1);9,287(1,0);8,313(0,4);8,132(2,0);8,127(2,1);8,111(2,2);8,106(2,4); 8,034(4,2);8,029(3,8);7,701(3,9);7,680(3,6);4,219(0,5);4,201(1,2);4,183(1,3);4,175(1,3);4,156(1,5);4,138(1,2);4,130(1,9);4,114(1,8);4,106(1, 9);4,090(1,8);4,065(0,6);3,902(16,0);3,365(0,4);3,322(232,0);3,282(0,4);2,675(0,8);2,671(1,0);2,667(0,8);2,506(135,5);2,502(173,4);2,497(1 29,5);2,333(0,8);2,329(1,1);2,324(0,8);1,429(4,6);1,411(9,7);1,393(4,5);0,000(5,1) |
| Beispiel lbg-7: ¹H-NMR(400,0 MHz, d₆-DMSO): δ= 8,615(1,9);8,604(1,9);8,065(1,7);8,059(1,9);8,044(1,8);8,038(2,1);7,974(3,7);7,969(3,3); 7,648(8,4);7,627(8,1);4,282(0,6);8,942(0,6);8,928(18,6);8,904(16,0);8,824(76,1);2,856(0,6);2,846(0,8);2,888(1,2);2,828(1,2);2,819(0,8);2,81 0(0,6);2,676(0,3);2,672(0,4);2,667(0,4);2,507(57,9);2,502(73,9);2,498(55,8);2,329(0,5);0,731(0,7);0,718(2,2);0,713(2,9);0,701(2,8);0,696(2, 3);0,684(0,9);0,560(1,0);0,549(3,0);0,543(2,8);0,534(2,5);0,521(0,7);0,000(2,1) |
| Beispiel lbg-8: ¹H-NMR(400,0 MHz, d₆-DMSO): δ= 9,181(0,9);9,166(1,9);9,151(0,9);7,836(1,2);7,830(1,4);7,815(2,2);7,810(3,3);7,791(4,5); 7,787(3,5);7,780(4,9);7,760(2,2);7,398(1,1);7,393(1,6);7,377(5,9);7,370(64);7,352(4,7);7,332(1,7);7,286(0,9);7,282(1,4);7,278(0,8);7,271(0, 8);7,265(1,7);7,252(0,5);7,248(0,7);4,875(0,4);4,858(1,1);4,842(1,6);4,826(1,1);4,810(0,4);4,501(4,3);4,487(4,4);4,096(15,6);3,902(3,1);3,36 5(0,3);3,325(137,0);2,676(0,5);2,671(0,6);2,667(0,5);2,542(0,6);2,525(1,8);2,511(40,8);2,507(79,3);2,502(102,4);2,498(75,9);2,494(38,4);2, 334(0,4);2,329(0,6);2,325(0,4);1,501(16,0);1,485(15,9);1,236(0,6);0,000(3,5) |
| Beispiel lbg-9: ¹H-NMR(400,0 MHz, d₆-DMSO): δ= 9,128(0,9);9,114(2,0);9,099(1,0);8,100(1,9);8,095(2,3);8,079(2,0);8,074(2,7);8,047(4,6); 8,042(3,7);7,676(3,9);7,655(3,6);7,388(0,9);7,374(13,6);7,368(6,0);7,358(5,3);7,338(1,1);7,287(0,8);7,282(1,2);7,273(1,1);7,266(1,4);7,251( 0,7);7,245(0,4);4,487(4,6);4,472(4,6);4,213(0,5);4,196(1,1);4,177(1,3);4,169(1,3);4,151(1,2);4,133(0,5);3,900(16,0);3,324(140,1);3,290(0,4); 2,675(0,4);2,671(0,6);2,667(0,4);2,524(1,9);2,506(79,5);2,502(102,9);2,497(77,7);2,333(0,5);2,329(0,6);2,324(0,5);1,427(4,7);1,409(9,9);1,3 91(4,6);0,000(2,9) |
| Beispiel lbg-10: ¹H-NMR(400,0 MHz, d₆-DMSO): δ= 9,130(0,8);9,115(1,7);9,100(0,8);8,090(1,7);8,084(2,0);8,069(1,8);8,064(2,5);8,039(4,1) ;8,034(3,3);7,677(3,4);7,656(3,1);7,373(12,2);7,366(5,4);7,358(5,0);7,343(0,5);7,337(0,9);7,288(0,7);7,281(1,0);7,274(1,0);7,266(1,2);7,258( 0,8);7,252(0,6);4,486(4,0);4,471(4,0);3,928(13,2);3,904(16,0);3,397(0,6);3,390(0,4);3,384(0,6);3,323(87,8);2,690(0,4);2,675(0,3);2,671(0,5); 2,667(0,4);2,511(30,1);2,506(59,5);2,502(77,9);2,497(59,2);2,333(0,3);2,329(0,5);2,324(0,4);0,000(2,5) |
| Beispiel lbg-11: ¹H-NMP(400,0 MHz, d₆-DMSO): δ= 10,684(0,9);10,628(3,9);8,325(0,4);8,169(3,3);8,164(4,8);8,153(2,7);8,148(1,7);8,133(2 ,5);8,127(2,1):7,947(1,0);7,942(1,1):7,886(0,3);7,865(0,8):7,860(0,8);7,838(1,3):7,817(0,5);7,736(7,8);7,716(7,6);7,388(3,1);7,369(5,5):7,34 9(3,5);7,148(1,8);7,130(3,0);7,111(1,3);4,868(0,4);4,554(0,4);4,538(1,2);4,521(1,7);4,505(1,3);4,489(0,5);4,100(4,3);3,902(6,6);3,883(16,0); 3,330(291,3);3,174(0,3);2,677(0,8);2,672(1,2);2,668(0,9);2,542(0,6);2,508(153,1);2,503(199,2);2,499(155,8);2,334(0,9);2,330(1,2);2,325(1, 0);1,512(4,2);1,496(4,4);1,480(6,4);1,464(6,5);1,443(6,5);1,427(6,2);1,258(0,4);1,235(0,8);0,000(5,4) |
| Beispiel lbg-12: ¹H-NMR(400,0 MHz, d₆-DMSO): δ= 10,615(4,0);8,169(3,3);8,164(4,9);8,154(2,8);8,149(1,7);8,134(2,5);8,128(2,1);7,740(4, 6);7,733(4,5);7,719(5,0);7,714(5,1);7,387(2,5);7,368(4,4);7,348(2,7);7,147(1,4);7,129(2,4);7,110(1,1);4,223(0,6);4,207(1,3);4,189(1,5);4,178 (1,5);4,160(1,3);4,143(0,6);3,908(16,0);3,326(121,7);3,282(0,4);2,676(0,5);2,672(0,6);2,668(0,5);2,507(78,8);2,503(102,2);2,498(80,4);2,33 4(0,5);2,330(0,6);2,325(0,5);1,439(4,8);1,421(10,0);1,403(4,7);1,245(0,3);1,235(0,4);0,000(2,6) |
| Beispiel lbg-13: ¹H-NMR(400,0 MHz, d₆-DMSO): δ= 8,657(2,0);8,646(2,0);7,805(1,2);7,800(1,4);7,784(2,6);7,779(3,1);7,750(4,7);7,728(5,4) ;7,722(8,8);4,884(04);4,818(1,1);4,802(1,6);4,786(1,2);4,770(0,4);4,111(0,5);4,090(15,8);8,902(0,4)3,825(105,2);2,862(0,6);2,852(0,9);2,8 44(1,3);2,834(1,3);2,825(0,9);2,816(0,6);2,676(0,4);2,672(0,5);2,667(0,4);2,507(62,6);2,503(82,0);2,498(62,5);2,334(0,4);2,330(0,5);2,325(0 ,4);1,495(16,0);1,479(16,0);1,235(0,7);0,738(0,8);0,725(2,4);0,720(3,3);0,708(3,0);0,702(2,7);0,691(1,0);0,560(1,1);0,550(3,1);0,544(3,1);0, 540(3,0);0,534(2,8);0,522(0,8);0,000(2,4) |
| Beispiel lbg-14: ¹H-NMR(400,0 MHz, d₆-DMSO): δ= 10,614(3,2);8,161(2,7);8,156(4,0);8,145(2,2);8,140(1,3);8,125(2,1);8,119(1,7);7,742(3, 3);7,731(3,5);7,721(3.5);7,712(3,8);7,386(2,1);7,367(3,6);7,346(2,3);7,146(1,2);7,128(2,0);7,109(0,9);4,146(0,9);4,085(0,8);4,067(0,3);3,937 (13,7);3,917(16,0);3,902(2,0);3,872(0,4);3,427(0,4);3,410(0,4);3,324(62,1);2,676(0,4);2,672(0,5);2,667(0,4);2,542(0,5);2,538(0,5);2,507(59, 0);2,503(75,2);2,498(56,3);2,329(0,4);2,325(0,3);1,236(0,7);1,161(0,5);0,000(2,5) |
| Beispiel lbg-15: ¹H-NMR(600,1 MHz, CD3CN): δ= 8,532(1,4);8,530(2,5);8,527(1,4);8,240(1,0);8,238(1,3);8,235(0,9);8,227(1,0);8,225(1,4);8 ,222(0,9);7,909(0,9);7,907(1,2);7,906(1,1);7,904(0,9);7,896(1,0);7,894(1,3);7,893(1,3);7,891(0,9);7,659(0,5);7,601(1,3);7,588(2,4);7,576(1,2 );7,384(1,0);7,382(1,4);7,370(3,1);7,359(2,6):7,355(0,6);7,346(3,0);7,336(0,7);7,333(1,3);7,281(0,5);7,278(0,8);7,276(0,5);7,266(1,2);7,254( 0,5);4,579(3,6);4,569(3,6);3,873(11,8);3,853(16,0);2,139(4,6);1,971(1,2);1,964(0,6);1,955(1,6);1,951(2,0);1,947(10,6);1,943(18,4);1,939(26, 8);1,935(18,3);1,931(9,1);1,216(0,3);1,204(0,7);1,192(0,3);0,000(7,4) |
| Beispiel lbh-2: ¹H-NMR(600,1 MHz, CD3CN): δ= 8,225(1,3);8,223(2,2);8,220(1,3);8,020(0,8);8,018(1,1);8,016(0,8);8,007(0,9);8,0054(1,1);8 ,0045(1,1);8,003(0,8);7,953(0,9);7,951(1,1);7,950(1,0);7,948(0,8);7,940(1,0);7,938(1,1);7,937(1,1);7,935(0,8);7,693(1,2);7,680(2,3);7,667(1, 3);7,388(1,3);7,376(2,7);7,375(2,6);7,360(2,0);7,356(0,5);7,348(2,6);7,335(1,1);7,280(0,7);7,268(1,1);7,256(0,4);4,588(3,1);4,578(3,1);4,062 (16,0);4,033(10,1);3,935(0,6);2,137(45,4);1,971(0,6);1,963(0,6);1,955(1,6);1,951(2,0);1,947(10,6);1,943(18,5);1,939(26,9);1,935(18,2);1,93 1(9,2);1,285(0,4);1,277(0,3);1,272(0,4);0,000(6,0) |
| Beispiel lbh-3: ¹H-NMR(400,0 MHz, d₆-DMSO): δ= 9,355(0,7);9,339(1,5);9,324(0,7);7,990(1,3);7,984(1,5);7,969(1,6);7,963(1,9);7,902(3,1); 7,896(2,7);7,802(3,0);7,782(2,5);4,164(0,4);4,140(1,4);4,121(16,0);4,100(1,4);4,091(0,8);4,076(12,0);3,902(0,5);3,323(102,8);2,676(0,4);2,6 71(0,6);2,667(0,4);2,511(36,8);2,507(70,9);2,502(91,4):2,498(68,1);2,494(34,9);2,333(0,4);2,329(0,5);2,324(0,4);0,000(2,8) |
| Beispiel lbh-4: ¹H-NMR(400,0 MHz, d₆-DMSO): δ= 8,646(2,0);8,635(2,0);8,313(0,6);7,859(1,8);7,853(2,0);7,838(2,4);7,833(2,9);7,784(4,4); 7,779(3,8);7,747(4,5);7,726(3,2);4,421(1,4);4,403(4,4);4,384(4,4);4,366(1,4);4,104(0,6);4,084(16,0);3,901(1,9);3,322(227,3);2,862(0,6);2,85 2(0,9);2,844(1,3);2,834(1,3);2,825(0,9);2,816(0,6);2,680(0,5);2,676(0,9);2,671(1,2);2,667(0,9);2,524(3,8);2,511(76,7);2,507(151,3);2,502(19 7,3);2,498(147,9);2,493(75,9);2,333(0,9);2,329(1,2);2,324(0,9);1,461(5,0);1,443(11,1);1,425(5,0);1,411(0,4);1,236(0,4);0,738(0,8);0,725(2,4 );0,720(3.4);0,708(3,2);0,702(2,8);0,691(1,1);0,559(1,1);0,549(3,2);0,542(3,2);0,539(3,0);0,533(2,8);0,521(0,9);0,000(6,0) |
| Beispiel lbh-5: ¹H-NMR(400,0 MHz, d₆-DMSO): δ= 9,359(0,9);9,344(2,0);9,328(1,0);8,035(0,4);8,030(0,4);7,923(1,6);7,918(1,8);7,902(2,2); 7,897(2,6);7,823(4,1);7,818(3,8);7,802(4,3);7,781(3,2);7,701(0,4);7,680(0,4);4,437(1,3);4,419(4,1);4,401(4,2);4,383(1,3);4,165(0,6);4,141(1, 8);4,125(1,8);4,116(2,1);4,100(2,0);4,088(16,0);3,902(2,1);3,350(0,6);3,323(88,4);2,676(0,4);2,672(0,5);2,667(0,4);2,542(0,4):2,511(33,9);2, 507(66,6);2,503(87,4);2,498(66,3);2,494(34,9);2,333(0,4);2,329(0,5);2,325(0,4); 1,472(4,7); 1,454(10,3); 1,436(4,7); 1,412(1,1); 1,394(0,5);0,0 00(2,5) |
| Beispiel lbh-6: ¹H-NMR(400,0 MHz, d₆-DMSO): δ= 8,643(0,3);8,618(2,4);8,608(2,4);8,076(1,9);8,071(2,1);8,055(2,1);8,050(2,3);7,984(4,2); 7,979(3,9);7,779(0,4);7,749(0,5);7,728(0,7);7,642(3,7);7,621(3,4);4,542(0,5);4,526(1,3);4,510(1,7);4,493(1,3);4,478(0,5);4,090(1,9);3,902(3, 2);3,874(16,0);3,323(125,9);3,268(0,3);3,170(0,5);2,859(0,7);2,850(1,1);2,841(1,6);2,831(1,6);2,823(1,1);2,813(0,8):2,671(0,8);2,502(130,6) ;2,445(0,4);2,329(0,8);1,495(2,2);1,475(7,5);1,459(7,0);1,436(6,9);1,420(6,6);1,265(0,4);1,259(0,4);1,247(0,5);1,236(0,7);0,734(0,9);0,716(3 ,7);0,703(3,6);0,699(3,1);0,687(1,1);0,565(1,2);0,554(3,7);0,548(4,1);0,540(3,5);0,527(1,0);0,000(2,9) |
| Beispiel lbh-7: ¹H-NMR(400,0 MHz, d₆-DMSO): δ= 9,316(1,0);9,301(2,1);9,285(1,0);8,135(2,3);8,130(2,5);8,114(2,5);8,109(2,9);8,035(4,8); 8,030(4,3);7,843(0,3);7,804(0,5);7,765(0,4);7,760(0,4);7,697(4,4);7,676(4,1);4,548(0,5);4,531(1,3);4,515(1,8);4,499(1,3);4,483(0,5);4,156(0, 6);4,140(0,8);4,132(1,8);4,116(1,9);4,107(1,9);4,092(3,3);4,067(0,6);3,902(2,1);3,878(16,0);3,323(129,0);3,294(0,4);2,676(0,5);2,671(0,7);2, 667(0,5);2,542(0,4);2,525(2,2);2,511(45,3);2,507(90,1);2,502(117,6);2,498(87,5);2493(44,1);2,334(0,5);2,329(0,7);2,324(0,5);1,503(1,9);1, 487(2,4);1,478(6,3);1,462(6,4);1,437(6,3);1,421(6,1);0,000(4,2) |
| Beispiel lbh-8: ¹H-NMR(400,0 MHz, d₆-DMSO): δ= 8,643(1,5);8,632(1,5);7,926(1,4);7,920(1,6);7,905(1,7);7,899(2,1);7,859(3,4);7,854(2,8); 7,748(3,2);7,727(2,6);4,128(0,6);4,109(16,0);4,092(0,6);4,074(12,1);3,902(0,5):3,349(0,6);3,323(77,8):2,866(0,5);2,857(0,7);2,848(1,0);2,83 8(1,0);2,829(0,6);2,820(0,5);2,676(0,4);2,671(0,5);2,667(0,4);2,511(31,8);2,507(60,0);2,502(76,6);2,498(56,9);2,494(29,1);2,333(0,3);2,329( 0,5);2,325(0,3);0,739(0,6);0,727(1,9);0,722(2,5);0,709(2,4);0,704(2,0);0,692(0,8);0,562(0,8);0,552(2,4);0,546(2,4);0,542(2,2);0,536(2,1);0,5 24(0,6);0,000(2,5) |
| Beispiel lbh-10: ¹H-NMR(400,0 MHz, d₆-DMSO): δ= 9,172(0,9);9,157(1,9);9,142(0,9);8,312(0,5);7,887(1,5);7,882(2,0);7,867(1,9);7,861(3,4) ;7,851(4,7);7,846(3,1);7,791(0,7);7,777(4,3);7,756(2,9);7,397(1,0);7,393(1,6);7,376(6,1);7,371(8,3);7,353(4,7);7,333(1,7);7,287(0,9);7,283(1 ,4);7,278(0,8);7,266(1,6);7,253(0,5);7,249(0,7);7,245(0,4);7,197(0,6);7,168(0,6);6,793(0,3);6,765(0,4);6,749(0,4);6,721(0,4);5,979(0,5);5,93 5(0,5);5,441(0,6);5,411(0,5);4,500(4,5);4,485(4,5);4,440(1,3);4,422(4,3);4,403(4,3);4,385(1,4);4,090(16,0);3,985(0,4);3,959(1,1);3,933(1,1); 3,907(0,5);3,901(1,2);3,373(0,9);3,329(435,0);3,280(0,6);3,270(0,5);3,254(0,3);2,676(0,9);2,671(1,2);2,667(0,9);2,541(0,7);2,525(3,6);2,511 (77,3);2,507(153,6);2,502(200,6);2,498(149,2);2,493(75,5);2,457(0,5);2,383(4,1);2,334(1,0);2,329(1,3);2,325(1,0);1,470(5,0);1,452(11,0);1, 434(4,9);0,000(8,9);-0,008(0,3) |
| Beispiel lbh-11: ¹H-NMR(400,0 MHz, d₆-DMSO): δ= 9,165(0,8);9,150(1,6);9,135(0,8);7,953(1,1);7,948(1,9);7,936(2,8);7,931(7,1);7,778(2,7) ;7,772(0,9);7,761(0,7);7,755(2,2);7,395(1,3);7,378(4,7);7,373(6,1);7,354(3,7);7,335(1,4);7,283(1,1);7,266(1,3);7,249(0,5);4,502(3,6);4,487(3 ,6);4,125(16,0);4,080(12,7);3,901(0,8);3,365(0,4);3,325(252,8);3,295(0,8);2,675(0,6);2,671(0,8);2,667(0,6);2,524(2,6);2,506(110,0);2,502(1 41,9);2,498(106,6);2,333(0,6);2,329(0,9);2,324(0,6);0,000(3,9) |
| Beispiel lbh-12: ¹H-NMR(400,0 MHz, d₆-DMSO): δ= 10,618(4,0);8,168(3,2);8,163(4,7);8,154(2,8);8,149(1,6);8,133(2,5);8,128(2,1);7,737(6, 0);7,734(4,8);7,717(6,4);7,391(2,6);7,371(4,4);7,351(2,7);7,151(1,4);7,132(2,4);7,114(1,1);4,554(0,5);4,538(1,2);4,522(1,7);4,505(1,3);4,489 (0,5);3,882(16,0);3,440(0,7);3,427(0,6);3,420(0,5);3,398(1,5);3,343(1284,7);3,292(1,6);3,282(1,1);3,269(1,2);3,252(0,6);3,237(0,4);3,225(0, 4);3,192(0,4);3,184(0,3);2,676(1,1);2,672(1,5);2,667(1,2);2,542(50,5);2,525(3,9);2,507(188,0);2,503(247,6);2,498(187,6);2,433(0,4);2,368(0 ,3);2,334(1,2):2,329(1,6);2,325(1,2);1,650(0,3):1,479(6,3):1,463(6,5):1,441(6,5):1,425(6,2):1,298(0,6);1,279(0,6):1,261(1,2);1,259(1,1):1,23 6(1,8);0,853(0,4);0,000(1,9) |
| Beispiel lbh-13: ¹H-NMR(400,0 MHz, d₆-DMSO): δ= 10,652(3,7);8,313(0,4);8,005(3,7);8,000(4,1);7,941(1,9);7,935(1,6);7,920(2,6);7,914(2, 4);7,838(4,3);7,817(3,0);7,732(4,0);7,713(4,2);7,389(2,6);7,370(4,3);7,350(2,7);7,149(1,5);7,131(2,4);7,112(1,1);4,461(1,4);4,443(4,2);4,425 (4,2);4,407(1,4);4,094(16,0);3,921(0,5);3,901(1,0);3,323(218,2);2,676(0,8);2,671(1,1);2,667(0,8);2,511(73,2);2,507(1384);2,502(179,0);2,4 97(133,8);2,493(69,0);2,333(0,8);2,329(1,1);2,324(0,8);1,483(4,9);1,465(10,4);1,447(4,7);1,437(0,5);1,234(0,3);0,000(5,9) |
| Beispiel lbh-14: ¹H-NMR(400,0 MHz, d₆-DMSO): δ= 10,656(3,1);8,314(0,4);8,075(3,0);8,070(3,4);8,006(1,6);8,001(1,4);7,985(1,9);7,980(1, 8);7,839(3,3);7,818(2,6);7,736(3,3);7,717(3,6);7,390(2,1);7,371(3,6);7,351(2,2);7,150(1,2);7,131(2,0);7,113(0,9);4,145(16,0);4,084(13,5);3,9 01(0,9);3,323(240,5);2,676(0,8);2,671(1,0);2,667(0,8);2,506(126,1);2,502(164,7);2,498(125,8);2,333(0,7);2,329(1,0);2,324(0,7);0,000(4,5) |
| Beispiel Ibh-9: ¹H-NMR(400,0 MHz, d₆-DMSO): δ= 9,127(1,1);9,112(2,3);9,097(1,1);8,102(1,8);8,097(2,2);8,081(2,0);8,076(2,6);8,049(4,5); 8,044(3,7);7,672(4,0);7,651(3,7);7,391(1,2);7,375(12,0);7,358(5,3);7,338(1,4);7,287(0,8);7,282(1,3);7,272(1,0);7,266(1,6);7,250(0,7);4,541( 0,5);4,525(1,3);4,509(1,8);4,490(5,6);4,475(5,2);4,095(0,6);3,901(1,5);3,876(16,0);3,389(0,4);3,359(0,4);3,322(132,8);2,671(0,7);2,666(0,5); 2,540(0,5);2,506(86,3);2,502(112,9);2,498(87,2);2,333(0,5);2,329(0,7);2,325(0,5);1,501(0,7);1,475(6,2);1,459(6,3);1,435(6,3);1,419(6,1);1,2 35(0,7);0,000(3,1) |
| Beispiel Ibi-1: ¹H-NMR(400,0 MHz, d₆-DMSO): δ= 15,389(2,6);9,171(1,2);9,157(2,4);9,142(1,3);8,313(0,6);8,118(7,0);8,097(3,8);8,092(2,9); 7,783(2,5);7,763(2,3);7,403(2,1);7,384(16,0);7,367(7,9);7,347(2,5);7,298(1,3);7,293(2,0);7,287(1,2);7,276(2,5);7,265(0,9);7,260(1,1);4,506( 6,9);4,491(6,9);4,107(1,0);4,075(13,0);3,901(1,1);3,325(266,4);3,262(0,4);3,169(0,4);2,676(1,1);2,671(1,4);2,667(1,1);2,542(1,0);2,511(97,3 );2,507(184,9);2,502(236,9);2,498(176,5);2,494(90,6);2,435(0,4);2,334(1,1);2,329(1,4);2,325(1,1);0,008(0,3);0,000(7,8) |
| Beispiel lbi-2: ¹H-NMR(400,0 MHz, d₆-DMSO): δ= 15,412(3,0);9,356(1,9);9,341(3,5);9,326(1,8);8,313(1,1);8,148(3,9);8,143(4,5);8,127(4,1); 8,122(5,5);8,099(6,3);7,809(3,0);7,788(2,7);4,176(1,2);4,152(3,8);4,136(4,0);4,128(4,2);4,112(4,1);4,070(16,0);3,902(2,8);3,509(0,4);3,406( 0,5);3,323(628,3);3,260(0,3);3,175(0,6);3,164(0,6);2,676(2,3);2,671(3,1);2,667(2,3);2,541(3,4);2,511(208,3);2,507(394,7);2,502(503,7);2,49 8(374,4);2,493(190,0);2,333(2,2);2,329(2,9);2,324(2,2);1,235(0,5);0,008(0,7);0,000(15,5);-0,008(0,6) |
| Beispiel lbi-3: ¹H-NMR(400,0 MHz, d₆-DMSO): δ= 15,339(0,8);8,654(3,1);8,644(3,0);8,314(0,3);8,094(2,6);8,089(3,5);8,073(2,6);8,068(4,4); 8,056(5,9);8,051(4,2);7,747(2,8);7,726(2,5);4,281(0,6);4,085(0,9);4,063(16,0);3,902(1,1);3,391(0,4);3,328(198,2);3,278(0,4);3,176(0,4);3,16 3(0,4);2,891(0,3);2,881(0,9);2,872(1,4);2,863(2,0);2,853(2,0);2,844(1,3);2,835(1,0);2,825(0,4):2,676(0,7);2,672(0,9);2,668(0,7);2,542(0,7);2 ,511(61,3);2,507(117,2);2,503(151,2);2,498(113,6);2,334(0,7);2,330(0,9);2,325(0,7);0,753(1,2);0,740(3,8);0,736(5,1);0,723(4,8);0,718(4,1); 0,706(1,6);0,565(1,7);0,554(5,0);0,548(4,8);0,544(4,6);0,539(4,3);0,526(1,3);0,000(4,5) |
| Beispiel lbi-4: ¹H-NMR(400,0 MHz, d₆-DMSO): δ= 15,391(3,5);10,670(4,2);8,247(4,2);8,242(4,0);8,225(0,4);8,177(3,0);8,171(2,4);8,156(3,1 );8,150(2,5);7,852(3,4);7,831(3,0);7,744(5,7);7,741(6,4);7,722(6,8);7,402(3,7);7,383(6,0);7,362(3,4);7,161(2,0);7,143(3,1);7,125(1,4);4,291( 0,6);4,084(16,0);4,000(0,5);3,902(2,3);3,330(89,2);3,171(1,0);2,677(0,6);2,673(0,7);2,668(0,5);2,512(68,5);2,508(104,9);2,503(1194);2,499 (82,4);2,495(38,3);2,335(0,6);2,330(0,7);2,326(0,5);1,259(0,4);1,235(0,7);0,000(4,1) |

### Herstellung der Ausgangsverbindungen

Alle eingesetzten Ausgangsverbindungen sind entweder nach oder in Analogie zu literaturbekannten Verfahren darstellbar oder sind kommerziell erhältlich.

### Synthese von Methyl-1-methyl-3-(pentafluorethyl)-4-(trifluormethyl)-1H-pyrazol-5-carboxylat

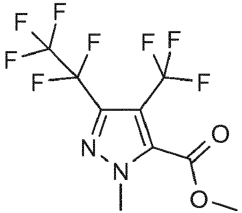

10,0 g 1-Methyl-3-(pentafluorethyl)-4-(trifluormethyl)-1H-pyrazol-5-carbonsäure (32 mmol) wurden in 250 mL Dichlormethan p.a. vorgelegt und dann unter rühren mit 8,39 mL Oxalylchlorid (96,1 mmol) versetzt. Das Reaktionsgemisch wurde 30 Minuten bei Raumtemperatur und anschließend 30 Minuten unter Rückfluß gerührt. Das Reaktionsgemisch wurde am Rotationsverdampfer bis zur Trockene eingeengt und in 100 mL Chloroform aufgenommen. Die so erhaltene Lösung wurde dann tropfenweise zu einer Suspension aus 6,43 g Silber(I)cyanid, 65 mL Methanol p.a. und 150 mL Chloroform getropft. Die Reaktionsmischung wurde dann 16h unter Rückfluß erhitzt und auf Raumtemperatur abgekühlt Die Suspension wurde dann über Silica filtriert und mit Dichlormethan nachgespült. Das Lösungsmittel wurde am Rotationsverdampfer bis zur Trockene eingeengt.

Es wurden 8,40 g Methyl-1-methyl-3-(pentafluorethyl)-4-(trifluormethyl)-1H-pyrazol-5-carboxylat als farblose Flüssigkeit isoliert.
HPLC-MS^{a)}: logP = 3,42, Masse (m/z) = 327 [M+H]+.
¹H-NMR (400 MHz, d3-Acetonitril): δ = 4,08 (s, 3H), 3,97 (s, 3H).

### Biologische Ausführungsbeispiele für Verwendungen im Bereich Pflanzenschutz und Tiergesundheit

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis,* wenn in diesem Test bei einer Aufwandmenge von 5 µg/cm² mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 µg/cm² (500 g/ha): Iac-1, Iaf-1, Iap-1, Iap-3

### Rhipicephalus sanguineus - in-vitro Kontakttests mit Adulten der braunen Hundezecke

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Hundezecken (*Rhipicephalus sanguineus*) besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt. Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus,* wenn in diesem Test bei einer Aufwandmenge von 5 µg/cm² mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 100% bei einer Aufwandmenge von 5 µg/cm² (500 g/ha): Iaf-1, Iap-1, Iap-3, lap-4

Bei diesem Test zeigen z.B. die folgenden Verbindungen der Herstellungsbeispiele eine Wirkung von 80% bei einer Aufwandmenge von 5 µg/cm² (500 g/ha): Ibf-2

Bei diesem Test zeigen z.B. die folgenden Verbindungen der Herstellungsbeispiele eine Wirkung von 80% bei einer Aufwandmenge von 1 µg/cm² (100 g/ha): Iap-2

### Amblyomma hebaraeum -Test (AMBYHE)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Zeckennymphen (*Amblyomma hebraeum*) werden in perforierte Plastikbecher gesetzt und in der gewünschten Konzentration eine Minute getaucht. Die Zecken werden auf Filterpapier in eine Petrischale überführt und in einem Klimaschrank gelagert.

Nach 42 Tagen wird die Abtötung in % bestimmt. Dabei bedeutet 100 %, dass alle Zecken abgetötet wurden; 0 % bedeutet, dass keine der Zecken abgetötet wurde.

Bei diesem Test zeigen z. B. die folgenden Verbindungen der Herstellungsbeispiele eine Wirkung von 100% bei einer Aufwandmenge von 100ppm: Iac-1, Iac-3, Iaf-1, Iap-1

### Boophilus microplus - Diptest

| | |
|---|---|
| Testtiere: | Rinderzecken (*Boophilus microplus*) Stamm Parkhurst,SP-resistent |
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken (*Boophilus microplus*) werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 100ppm: Iac-1, Iac-3, Iaf-1, Iap-1

### Boophilus microplus -Injektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken (*Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20µg/Tier: Iaa-1, Iac-1, Iac-3, Iaf-1, Iap-1, Iap-2, Iap-3, Iap-4, Iap-5, Ibe-4, Ibf-2, Ibf-3, Ibh-4

### Ctenocephalides felis - Oraltest

| | |
|---|---|
| Lösungsmittel: | Dimethylsulfoxid |

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100ppm: Iaa-1, Iac-1, Iac-3, Iaf-1, Iap-1, Iap-2, Iap-3, Iap-4, Iap-5, Ibe-4, Ibf-2, Ibf-3

Bei diesem Test zeigen z.B. die folgenden Verbindungen der Herstellungsbeispiele eine Wirkung von 90% bei einer Aufwandmenge von 100ppm: Ibh-4

### Lucilia cuprina - Test

| | |
|---|---|
| Lösungsmittel: | Dimethy lsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L11-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: Iaa-1, Iac-1, Iac-3, Iaf-1, Iap-1, Iap-2, Iap-3, Iap-4, Iap-5, Ibe-4, Ibf-2, Ibf-3, Ibh-4

### Musca domestica-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen (*Musca domestica*) besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 100% bei einer Aufwandmenge von 100ppm: Iaa-1, Iac-1, Iac-3, Iap-1, Iap-2, Iap-3, Iap-5, Ibe-4, Ibf-2

Bei diesem Test zeigen z.B. die folgenden Verbindungen der Herstellungsbeispiele eine Wirkung von 90% bei einer Aufwandmenge von 100ppm: Ibh-4

Bei diesem Test zeigen z.B. die folgenden Verbindungen der Herstellungsbeispiele eine Wirkung von 80% bei einer Aufwandmenge von 100ppm: Ibf-3

### Mvzus persicae - Sprühtest

| | | |
|---|---|---|
| Lösungsmittel: | 78 Gewichtsteile | Aceton |
| | 1,5 Gewichtsteile Dimethylformamid | |
| | | |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 90% bei einer Aufwandmenge von 100g/ha: Iaf-1

### Phaedon cochleariae - Sprühtest

| | | |
|---|---|---|
| Lösungsmittel: | 78,0 Gewichtsteile | Aceton |
| | 1,5 Gewichtsteile Dimethylformamid | |
| | | |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: Iaj-1, Iap-4, Ibd-3, Ibe-1, Ibe-3, Ibe-4, Ibf-2, Ibf-3, Ibg-13, Ibg-5, Ibg-7, Ibg-8, Ibg-9, Ibh-3, Ibh-4, Ibh-5, Ibh-6, Ibh-8

Bei diesem Test zeigen z. B. die folgenden Verbindungen der Herstellungsbeispiele Wirkung von 100% bei einer Aufwandmenge von 100g/ha: Iaa-1, Iac-1, Iac-3, Iaf-1, Iap-1, Iap-2, Iap-3, Iap-5, Iap-6, Iaq-1, Iax-1

### Spodoptera frugiperda - Sprühtest

| | | |
|---|---|---|
| Lösungsmittel: | 78,0 Gewichtsteile | Aceton |
| | 1,5 Gewichtsteile Dimethylformamid | |
| | | |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500g/ha: Ibe-1, Ibe-4, Ibf-2, Ibf-3, Ibh-3

Bei diesem Test zeigen z. B. die folgenden Verbindungen der Herstellungsbeispiele Wirkung von 83% bei einer Aufwandmenge von 500g/ha: Ibh-8

Bei diesem Test zeigen z. B. die folgenden Verbindungen der Herstellungsbeispiele Wirkung von 80% bei einer Aufwandmenge von 500g/ha: Iap-4

Bei diesem Test zeigen z. B. die folgenden Verbindungen der Herstellungsbeispiele Wirkung von 100% bei einer Aufwandmenge von 100g/ha: Iaa-1, Iac-1, Iac-3, Iaf-1, Iap-1, Iap-2, Iap-3

### Tetranychus urticae - Sprühtest, OP-resistent

| | |
|---|---|
| Lösungsmittel: | 78,0 GewichtsteileAceton |
| | 1,5 Gewichtsteile Dimethylformamid |
| | |
| Emulgator: | Alky lary lpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 500g/ha: Ibh-4

Bei diesem Test zeigen z. B. die folgenden Verbindungen der Herstellungsbeispiele eine Wirkung von 90% bei einer Aufwandmenge von 500g/ha: Iap-4, Ibe-4, Ibg-5, Ibh-3

Bei diesem Test zeigen z. B. die folgenden Verbindungen der Herstellungsbeispiele eine Wirkung von 100% bei einer Aufwandmenge von 100g/ha: Iac-1, Iac-3, Iaf-1, Iap-3

### Tetranychus urticae- Sprühtest; OP-resistent

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Bohnenpflanzen (*Phaseolus vulgaris*), die stark von allen Stadien der Gemeinen Spinnmilbe (*Tetranychus urticae*) befallen sind, werden durch Spritzen mit der Wirkstoffzubereitung in der gewünschten Konzentration behandelt.

Nach 7 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 20ppm: Iap-1

## Patentansprüche

1. Verbindungen der allgemeinen Formel (I) 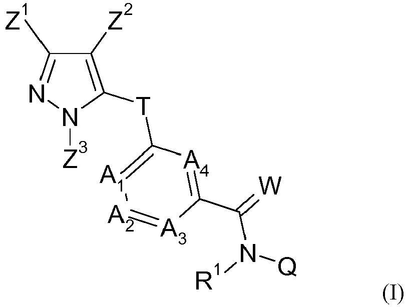 in denen
R¹ für Wasserstoff, gegebenenfalls substituiertes Alkyl, Alkenyl, Alkinyl, Cycloalkyl, Cycloalkyl- alkyl, Alkylcarbonyl, Alkoxycarbonyl, Arylalkyl, Heteroarylalkyl,
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, Cyano, Nitro, gegebenenfalls substituiertes Alkyl, Cycloalkyl, Alkoxy, *N*-Alkoxy-imino-alkyl, Alkylsulfanyl, Alkylsulfinyl, Alkylsulfonyl, *N*-Alkylamino oder *N,N*-Dialkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
W für Sauerstoff oder Schwefel steht;
Q für Wasserstoff, Formyl, Hydroxy, Amino oder eine der gegebenenfalls substituierten Gruppierungen Alkyl, Alkyloxy, Alkenyl, Alkinyl, Cycloalkyl, Heterocycloalkyl, Cycloalkylalkyl, Heterocycloalkylalkyl, Arylalkyl, Heteroarylalkyl oder für eine Gruppierung *N*-Alkylamino, *N*-Alkylcarbonylamino, *N,N-*Dialkylamino steht; oder
Q für einen gegebenenfalls einfach bis mehrfach mit V substituierten, einfach bis mehrfach ungesättigten 5- bis 6-gliedrigen Carbozyklus steht, der gegebenenfalls durch Heteroatome unterbrochen sein kann, wobei
V für Halogen, Cyano, Nitro, gegebenenfalls substituiertes Alkyl, Alkenyl, Alkinyl, Cycloalkyl, Alkoxy, *N*-Alkoxyiminoalkyl, Alkylsulfanyl, Alkylsulfinyl, Alkylsulfonyl, *N,N*-Dialkylamino steht
T für einen der nachfolgend aufgeführten 5 gliedrigen Heteroaromaten T1-T35 steht, wobei die Bindung zur Pyrazolkopfgruppe mit einem Sternchen gekennzeichnet ist, 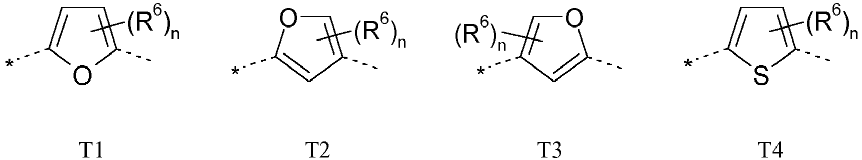 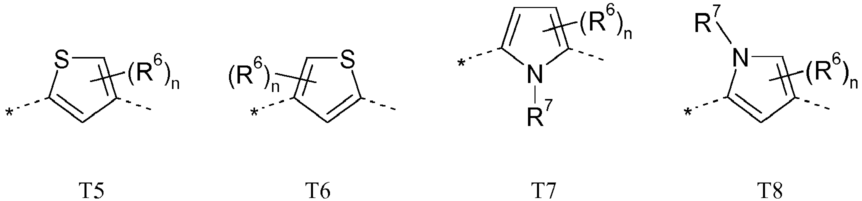 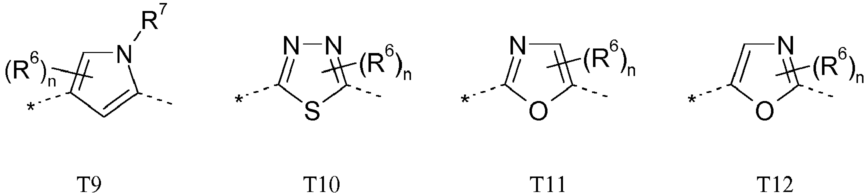 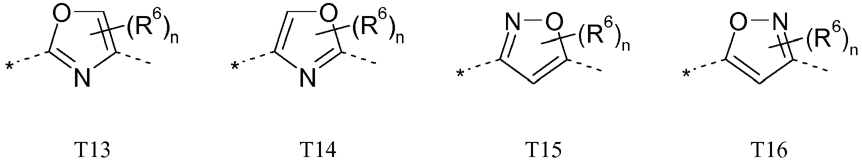 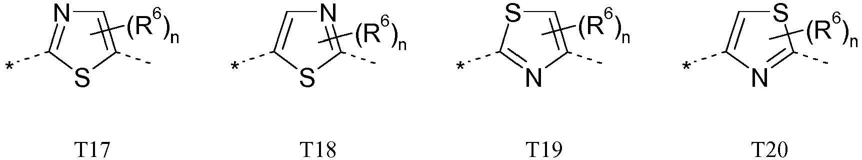 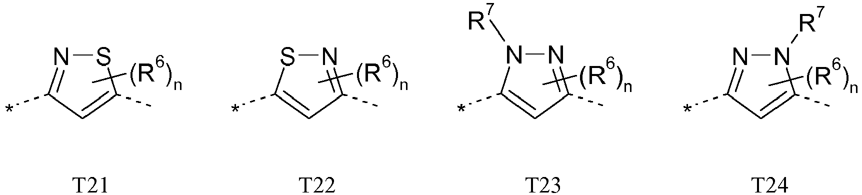 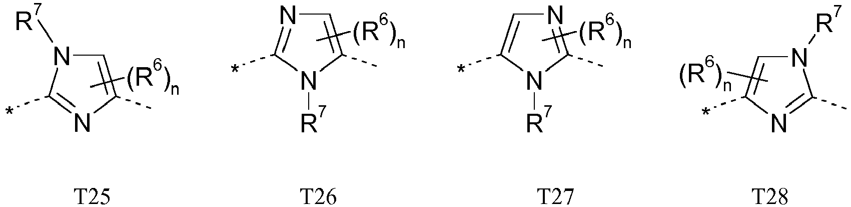 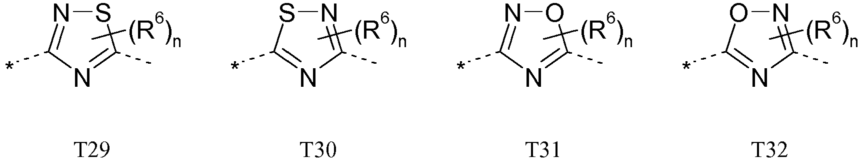 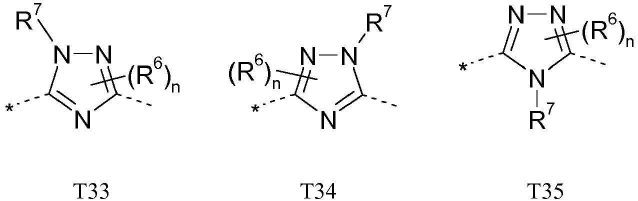
wobei
R⁶ unabhängig voneinander für Halogen, Cyano, Nitro, Amino oder ein gegebenenfalls substituiertes Alkyl, Alkyloxy, Alkylcarbonyl, Alkylsulfanyl, Alkylsulfinyl, Alkylsulfonyl, und
n für die Werte 0-2 stehen;
R⁷ für Wasserstoff, oder ein gegebenenfalls substituiertes Alkyl oder Cycloalkyl, in welchem ggf. eine Methylengruppe durch ein Heteroatom substituiert ist, steht;
Z¹ für ein gegebenenfalls substituiertes Halogenalkyl oder Halogencycloalkyl, und
Z² für Wasserstoff, Halogen, Cyano, Nitro, Amino oder ein gegebenenfalls substituiertes Alkyl, Alkylcarbonyl, Alkylsulfanyl, Alkylsulfinyl, Alkylsulfonyl, und
Z³ für Wasserstoff oder ein gegebenenfalls substituiertes Alkyl, Cycloalkyl, Heterocycloalkyl, Alkenyl, Alkinyl stehen;, wobei für den Fall, dass T gleich T23 oder T24 ist, einer der Reste Z¹, Z² oder Z³ mit mindestens 3 Halogenatomen substituiert ist.

2. Verbindungen gemäß Anspruch 1, in denen
R¹ für Wasserstoff, gegebenenfalls substituiertes C₁-C₆-Alkyl, C₃-C₆-Alkenyl, C₃-C₆-Alkinyl, C₃-C₇-Cycloalkyl, C₃-C₇-Cycloalkyl-C₁-C₃-alkyl, C₁-C₆-Alkylcarbonyl, C₁-C₆-Alkoxycarbonyl, Aryl(C₁-C₃)-alkyl, Heteroaryl(C₁-C₃)-alkyl,
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆-alkylamino, stehen, oder
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
W für Sauerstoff oder Schwefel steht;
Q für Wasserstoff, Formyl, Hydroxy, Amino oder eine der gegebenenfalls substituierten Gruppierungen C₁-C₆-Alkyl, C₃-C₆-Alkenyl, C₃-C₆-Alkinyl, C₃-C₆-Cycloalkyl, C₁-C₅-Heterocycloalkyl, C₁-C₄-Alkoxy, C₃-C₆-Cycloalkyl-C₁-C₆-alkyl, Aryl-(C₁-C₃)-alkyl, Heteroaryl-(C₁-C₃)-alkyl oder für eine Gruppierung *N*-C₁-C₄-Alkylamino, *N-*C₁-C₄-Alkylcarbonylamino, *N,N*-Di-C₁-C₄-alkylamino steht; oder
Q für einen gegebenenfalls einfach bis mehrfach mit V substituierten, einfach bis dreifach ungesättigten 5 bis 6-gliedrigen Carbozyklus steht, oder für einen gegebenenfalls mehrfach mit V substituierten, einfach bis dreifach ungesättigten 5- bzw. 6-gliedrigen, heterozyklischen Ring steht, wobei
V unabhängig voneinander für Halogen, Cyano, Nitro, gegebenenfalls substituiertes C₁-C₆-Alkyl, C₁-C₄-Alkenyl, C₁-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N-*Di-(C₁-C₆-alkyl)aminosteht;
T für einen der nachfolgend aufgeführten 5 gliedrigen Heteroaromaten T1-T35 steht, wobei die Bindung zur Pyrazolkopfgruppe mit einem Sternchen gekennzeichnet ist, 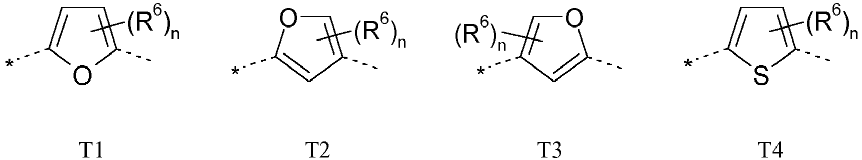 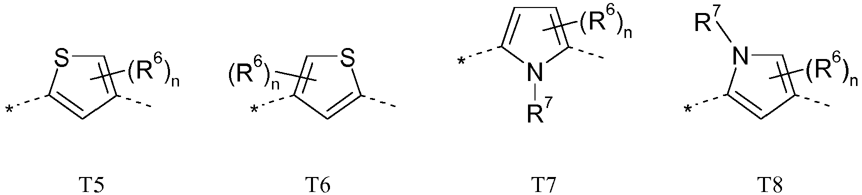 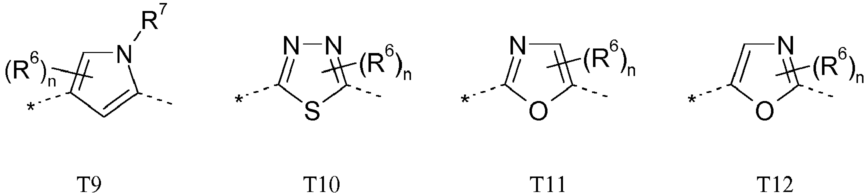 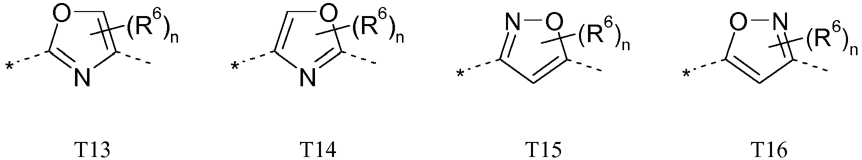 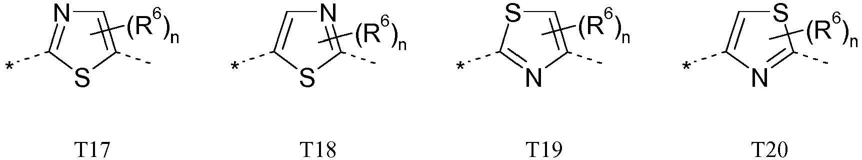 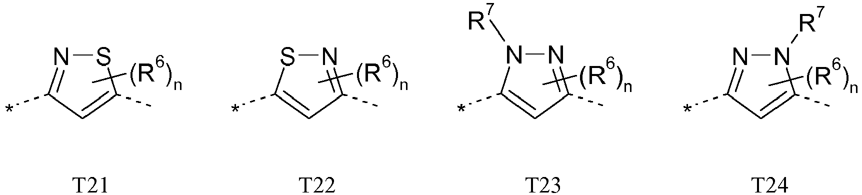 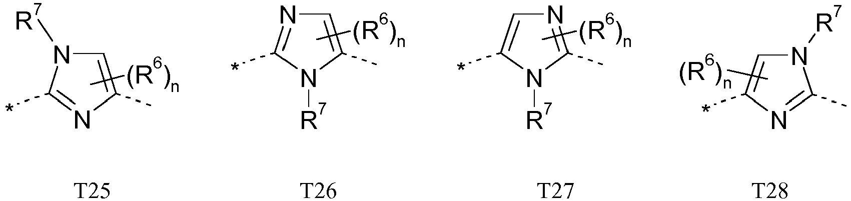 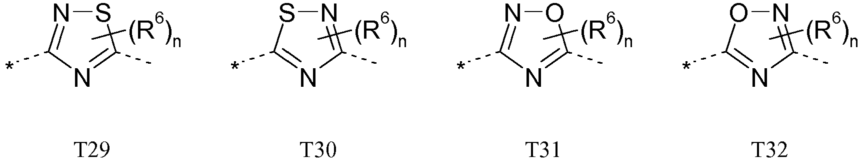 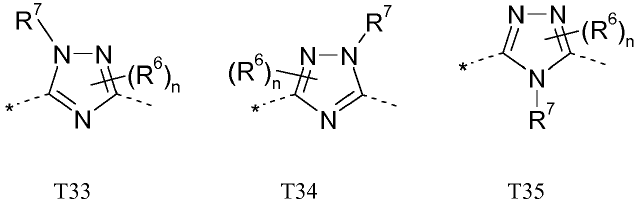
wobei
R⁶ unabhängig voneinander für Halogen, Cyano, Nitro, Amino oder ein gegebenenfalls mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
n für die Werte 0-1 stehen;
R⁷ für Wasserstoff, oder ein gegebenenfalls substituiertes C₁-C₆-Alkyl oder C₃-C₆-Cycloalkyl, in welchem ggf. eine Methylengruppe durch Heteroatome substituiert sein kann, steht;
Z¹ für ein gegebenenfalls substituiertes C₁-C₆-Halogenalkyl, C₃-C₆-Halogencycloalkyl, und
Z² für Wasserstoff Halogen, Cyano, Nitro, Amino oder ein gegebenenfalls substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
Z³ für Wasserstoff oder ein gegebenenfalls substituiertes C₁-C₆-Alkyl, C₃-C₆-Cycloalkyl, C₃-C₅-Heterocycloalkyl, C₃-C₆-Alkenyl, C₃-C₆-Alkinyl, stehen,
wobei für den Fall, dass T gleich T23 oder T24 ist, einer der Reste Z¹, Z² oder Z³ mit mindestens 3 Halogenatomen substituiert ist.

3. Verbindungen gemäß Anspruch 1 oder 2, in denen
R¹ für Wasserstoff, gegebenenfalls ein- bis fünffach unabhängig voneinander mit Fluor, Chlor, Cyano, Alkoxy und Alkoxycarbonyl substituiertes C₁-C₆-Alkyl, C₃-C₆-Alkenyl, C₃-C₆-Alkinyl, C₃-C₇-Cycloalkyl, C₃-C₇-Cycloalkyl-C₁-C₃-alkyl,C₁-C₆-Alkylcarbonyl, C₁-C₆-Alkoxycarbonyl, Aryl(C₁-C₃)-alkyl, Heteroaryl(C₁-C₃)-alkyl,
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Fluor, Chlor, Brom, Iod, Cyano, Nitro, gegebenenfalls durch einfach bis fünffach mit Fluor, Chlor, Cyano oder C₁-C₄-Alkoxy substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino stehen;
W für Sauerstoff oder Schwefel steht;
Q für Wasserstoff, Hydroxy, Formyl, Amino oder eine der gegebenenfalls unabhängig voneinander ein- oder mehrfach mit Hydoxy, Nitro, Amino, Fluor, Chlor, Brom, Iod, C₁-C₄-Alkoxy, Cyano, Hydroxycarbonyl. C₁-C₄-Alkoxycarbonyl, Carbamoyl, Thiocarbamoyl, C₁-C₄-Alkylcarbamoyl, C₃-C₆-Cycloalkylcarbamoyl, Phenyl substituierten Gruppierungen C₁-C₆-Alkyl, C₃-C₆-Alkenyl, C₃-C₆-Alkinyl, C₃-C₆-Cycloalkyl, C₂-C₆-Heterocycloalkyl, C₁-C₄-Alkoxy, C₁-C₆-Alkyl-C₃-C₆-cycloalkyl, C₃-C₆-Cycloalkyl-C₁-C₆-alkyl, C₁-C₆-Hydroxyalkyl, Aryl(C₁-C₃)-alkyl, Heteroaryl(C₁-C₃)-alkyl, *N*-C₁-C₄-Alkylamino, *N*-C₁-C₄-Alkylcarbonylamino oder *N,N*-Di-C₁-C₄-alkylamino steht; oder
Q für ein mit 0, 1,2 oder 3 Substituenten V substituiertes Aryl oder für ein mit 0, 1,2 oder 3 Substituenten V substituierten 5 bzw. 6 gliedrigen Heteroaromaten steht, wobei
V unabhängig voneinander für Fluor, Chlor, Brom, Iod, Cyano, Nitro, gegebenenfalls substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N*-Di-(C₁-C₆-alkyl)amino steht;
T für einen der nachfolgend aufgeführten 5 gliedrigen Heteroaromaten T1-T35 steht, wobei die Bindung zur Pyrazolkopfgruppe mit einem Sternchen gekennzeichnet ist, 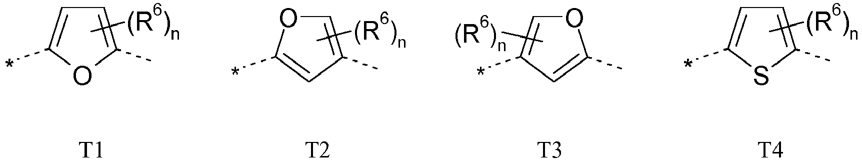 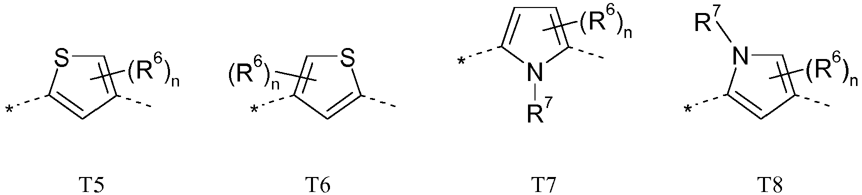 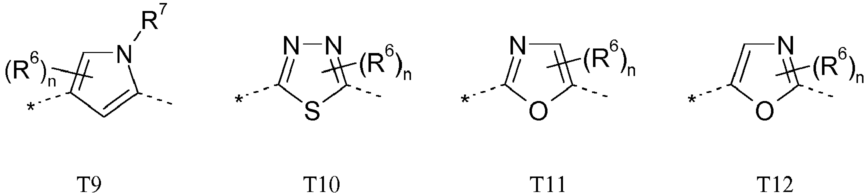 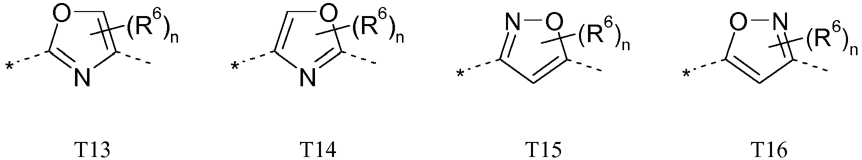 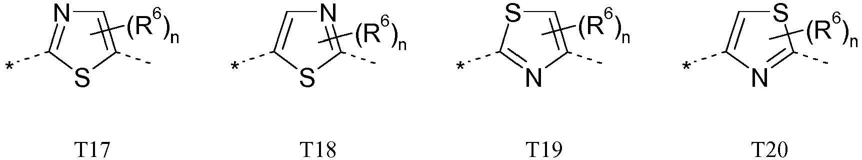 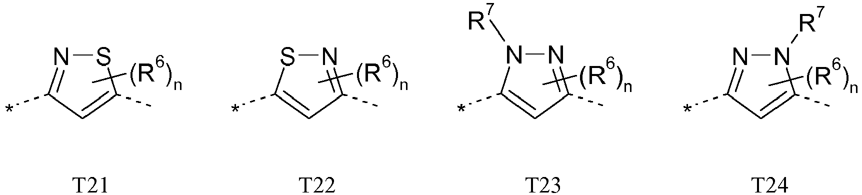 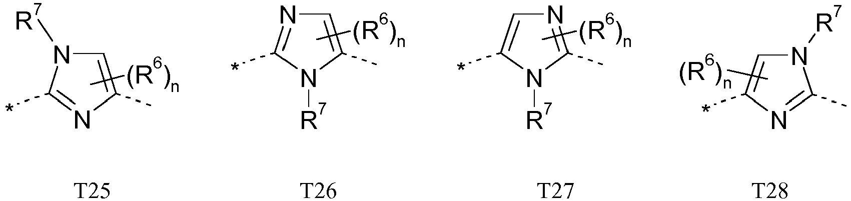 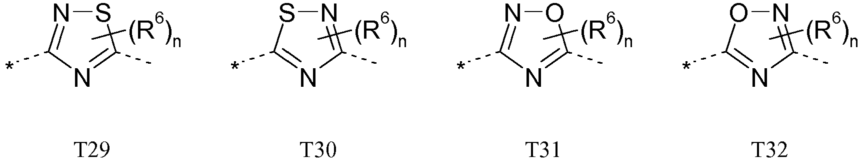 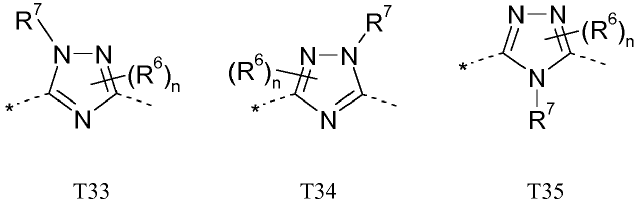
wobei
R⁶ unabhängig voneinander für Fluor, Chlor, Brom, Iod, Cyano, Nitro, Amino oder ein gegebenenfalls einfach bis fünffach mit Fluor oder Chlor substituiertes C₁-C₆-Alkyl, C₁-C₆-Alkoxy, C₁-C₆-Alkylcarbonyl, C₁-C₆-Alkylsulfanyl, C₁-C₆-Alkylsulfinyl, C₁-C₆-Alkylsulfonyl, und
n für die Werte 0-1 stehen;
R⁷ für Wasserstoff, oder ein gegebenenfalls einfach bis fünffach mit Fluor, Chlor, Cyano oder C₁-C₄-Alkoxy substituiertes C₁-C₆-Alkyl oder C₃-C₆-Cycloalkyl, in welchem ggf. eine Methylengruppe durch Heteroatome substituiert sein kann, steht; Z¹ für ein gegebenenfalls substituiertes C₁-C₆-Haloalkyl, C₃-C₆-Halocycloalkyl, und
Z² für Wasserstoff, Halogen, Cyano, Nitro, Amino oder ein gegebenenfalls einfach bis fünffach substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
Z³ für Wasserstoff oder ein gegebenenfalls einfach bis fünffach mit Fluor, Chlor, Cyano oder C₁-C₄-Alkoxy substituiertes C₁-C₆-Alkyl, C₃-C₆-Cycloalkyl, C₁-C₄-Alkenyl, C₁-C₄-Alkinyl, stehen;
wobei für den Fall, dass T gleich T23 oder T24 ist, einer der Reste Z¹, Z² oder Z³ mit mindestens 3 Halogenatomen substituiert ist.

4. Verbindungen gemäß einem der Ansprüche 1, 2 oder 3, in denen
R¹ für Wasserstoff, Methyl, Ethyl, n-Propyl, Isopropyl, n-Butyl, Isobutyl, s-Butyl, t-Butyl, 2,2,-Difluorethyl, 2,2,2-Trifluorethyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht;
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R² und R⁵ unabhängig voneinander für Wasserstoff, Methyl, Fluor und Chlor stehen und
R³ und R⁴ unabhängig voneinander für Wasserstoff, Fluor, Chlor, Brom, Iod, Cyano, Nitro, Methyl, Ethyl, Cyclopropyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl stehen
W für Sauerstoff oder Schwefel steht;
Q für für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxyethyl, 2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Methyl-cyclopropyl, 1-Trifluormethyl-cyclopropyl, 1-Carbamoyl-cyclopropyl, 1-Thiocarbamoyl-cyclopropyl, 1-Methoxycarbonyl-cyclopropyl, 1-(N-Methylcarbamoyl)cyclopropyl, 1-(*N-*Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl,, 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 4-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N-*Dimethylamino, *N,N*-Diethylamino steht; oder
Q für ein mit 0, 1, 2 oder 3 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
V unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino steht;
T für einen der nachfolgend aufgeführten 5 gliedrigen Heteroaromaten T1-T35 steht, wobei die Bindung zur Pyrazolkopfgruppe mit einem Sternchen gekennzeichnet ist, 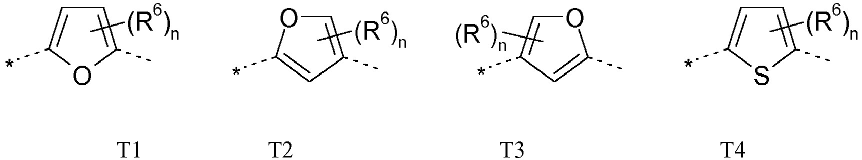 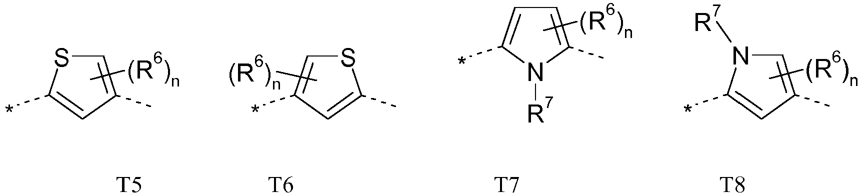 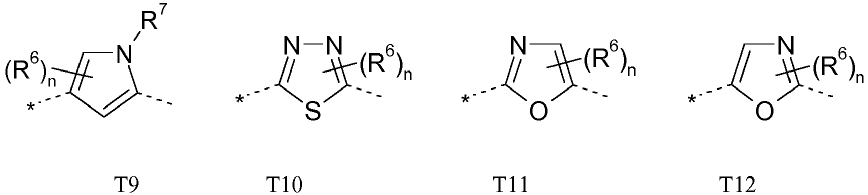 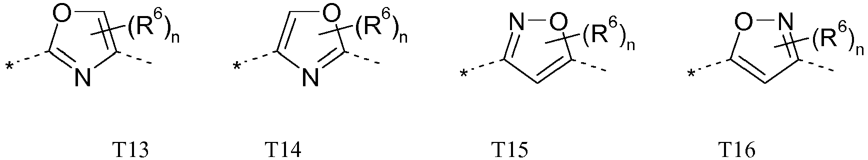 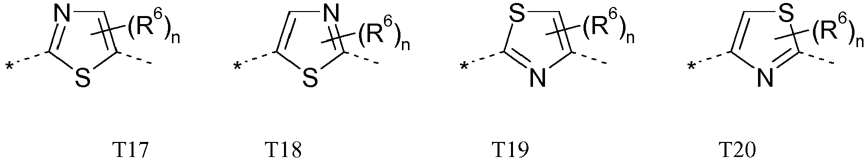 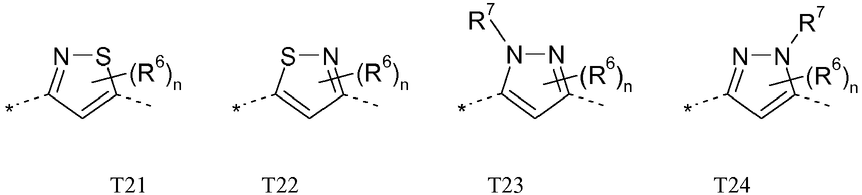 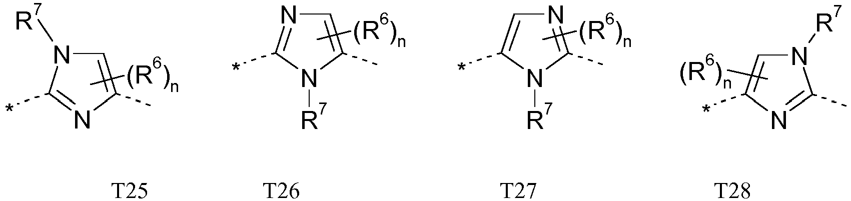 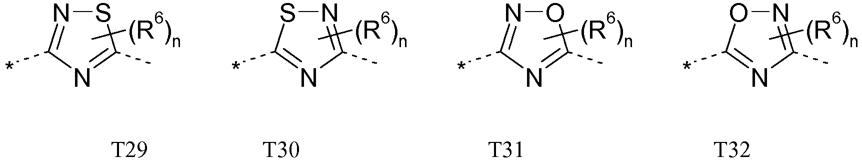 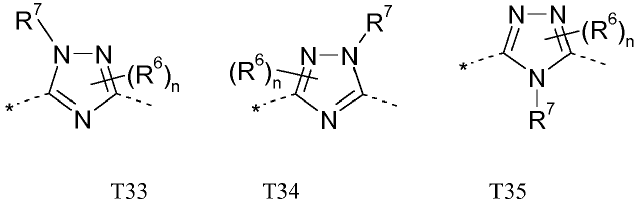
wobei
R⁶ unabhängig voneinander für Fluor, Chlor, Brom, Iod, Cyano, Nitro, Amino, Methyl, Ethyl, n-Propyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trifuormethylsulfinyl, und
n für die Werte 0-1 stehen;
R⁷ für Wasserstoff, Methyl, Ethyl, n-Propyl, Isopropyl, n-Butyl, Isobutyl, s-Butyl, t-Butyl, Ethenyl, 1-Propenyl, 2-Propenyl, 1-Propinyl, 1-Butinyl, Difluormethyl, Trifluormethyl, 1-Fluorethyl, 1-Fluor-1-methylethyl, 2-Fluorethyl, 2,2-Difluorethyl, 2,2,2-Trifluorethyl, Cyclopropyl, Cyclobutyl, Cyclopentyl, Cyclohexyl stehen;
Z¹ für Difluormethyl, Trichlormethyl, Chlordifluormethyl, Dichlorfluormethyl, Trifluormethyl, Bromdichlormethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl, Heptafluor-n-propyl, Heptafluor-isopropyl, Nonafluor-n-butyl, 1-Chlorcyclopropyl, 1-Fluorcyclopropyl, 1-Bromcyclopropyl, 1-Trifluormehtyl-cyclopropyl, und 2,2-Difluor-1-methyl-cyclopropyl, und
Z² für Wasserstoff, Fluor, Chlor, Brom, Iod, Cyano, Nitro, Amino, Methyl, Ethyl, n-Propyl, 1-Methylethyl, 1,1-Dimethylethyl, Difluormethyl, Trichlormethyl, Chlordifluormethyl, Dichlorfluormethyl, Trifluormethyl, Bromdichlormethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-t-butyl, Heptafluor-n-propyl, Heptafluor-isopropyl, Nonafluor-n-butyl, Methylsulfanyl, Methylsulfinyl, Methylsulfonyl, Ethylsulfanyl, Ethylsulfinyl, Ethylsulfonyl, Trifluormethylsulfanyl, Trifluor-methylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl und
Z³ für Wasserstoff, Methyl, Ethyl, n-Propyl, Isopropyl, n-Butyl, Isobutyl, s-Butyl, t-Butyl, Prop-2-enyl, Prop-2-inyl, But-3-inyl, Difluormethyl, Trifluormethyl, 1-Fluorethyl, 1-Fluor-1-methylethyl, 2-Fluorethyl, 2,2-Difluorethyl, 2,2,2-Trifluorethyl, stehen;
wobei für den Fall, dass T gleich T23 oder T24 ist, einer der Reste Z¹, Z² oder Z³ mit mindestens 3 Halogenatomen substituiert ist.

5. Verbindungen gemäß einem der Ansprüche 1, 2, 3 oder 4, in denen
Z¹ für Trifluormethyl, 1-Chlor-cyclopropyl, 1-Fluor-cyclopropyl oder Pentafluorethyl steht,
Z² für Trifluormethyl, Nitro, Methylsulfanyl, Methylsulfinyl, Methylsulfonyl, Fluor, Chlor, Brom, Cyano oder Iod steht,
Z³ für Methyl, Ethyl, n-Propyl oder Wasserstoff steht,
R¹ für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht,
A¹ und A⁴ für CH stehen und A2 für CH oder N steht
A₃ für CR⁴ und
R⁴ für Fluor, Chlor, Brom, Iod oder Methyl steht
T für einen der nachfolgend aufgeführten 5 gliedrigen Heteroaromaten T1-T35 steht, wobei die Bindung zur Pyrazolkopfgruppe mit einem Sternchen gekennzeichnet ist, 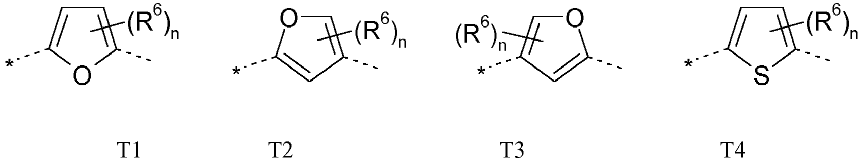 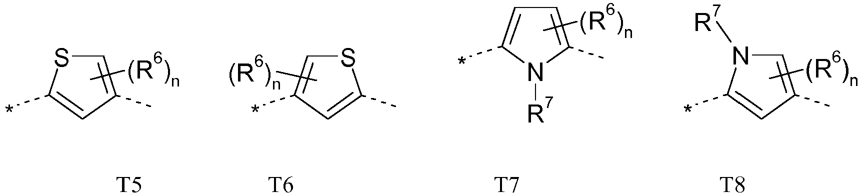 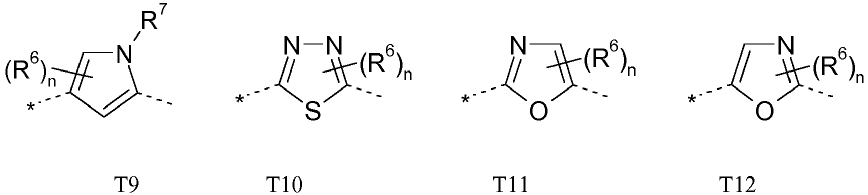 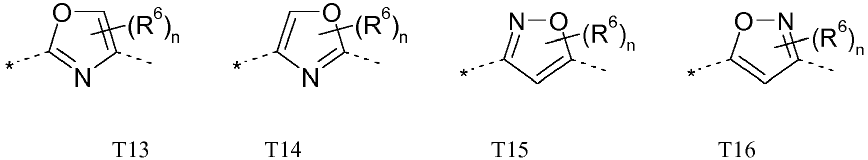 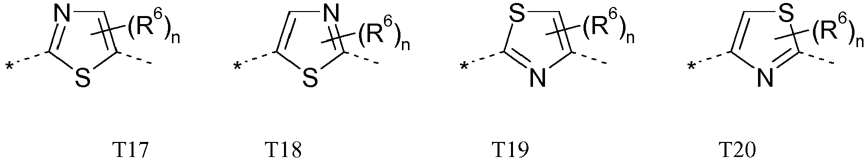 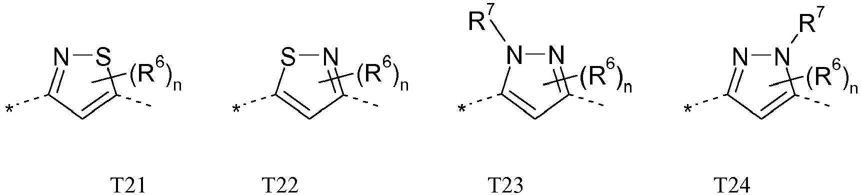 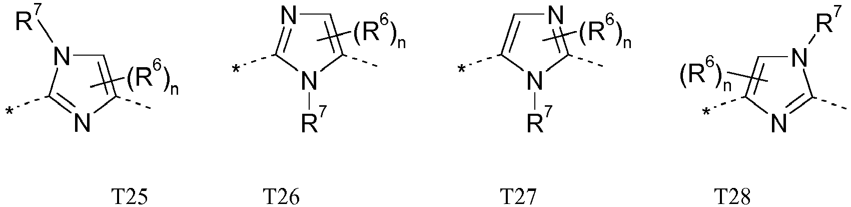 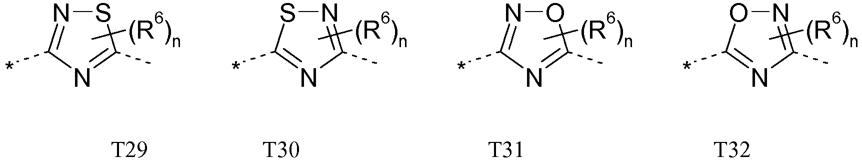 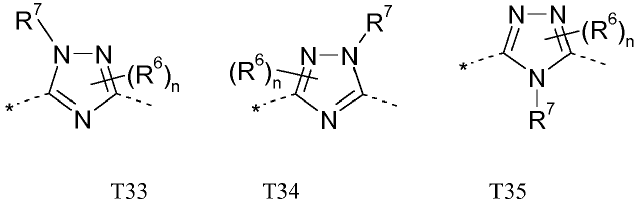
wobei
R⁶ für Wasserstoff, Methyl, Ethyl, 2-Methylethyl, 2,2-Dimethylethyl, Fluor, Chlor, Brom, Iod, Nitro, Trifluormethyl, Amino, und
n für die Werte 0-1 steht;
W für Sauerstoff steht und
Q für für Wasserstoff, Methyl, Ethyl, n-Propyl, 1-Methylethyl, 1,1-Dimethylethyl, n-Butyl, 1-Methylpropyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Methyl-cyclopropyl, 1-Trifluormethyl-cyclopropyl, 1-Carbamoyl-cyclopropyl, 1-Thiocarbamoyl-cyclopropyl, 1-Methoxycarbonyl-cyclopropyl, 1-(*N*-Methylcarbamoyl)cyclopropyl, 1-(*N-*Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl" 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 4-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, (6-Chlor-pyridin-3-yl)methyl, 5-Fluorpyridin-2-ylmethyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N-*Dimethylamino, *N,N-*Diethylamino steht; oder
Q für ein mit 0, 1, 2 oder 3 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
V unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht; wobei für den Fall, dass T gleich T23 oder T24 ist, einer der Reste Z¹, Z² oder Z³ mit mindestens 3 Halogenatomen substituiert ist.

6. Verbindungen gemäß einem der Ansprüche 1 bis 5, und gemäß einer der folgenden allgemeinen Formeln (Iaa) - (Ibi)

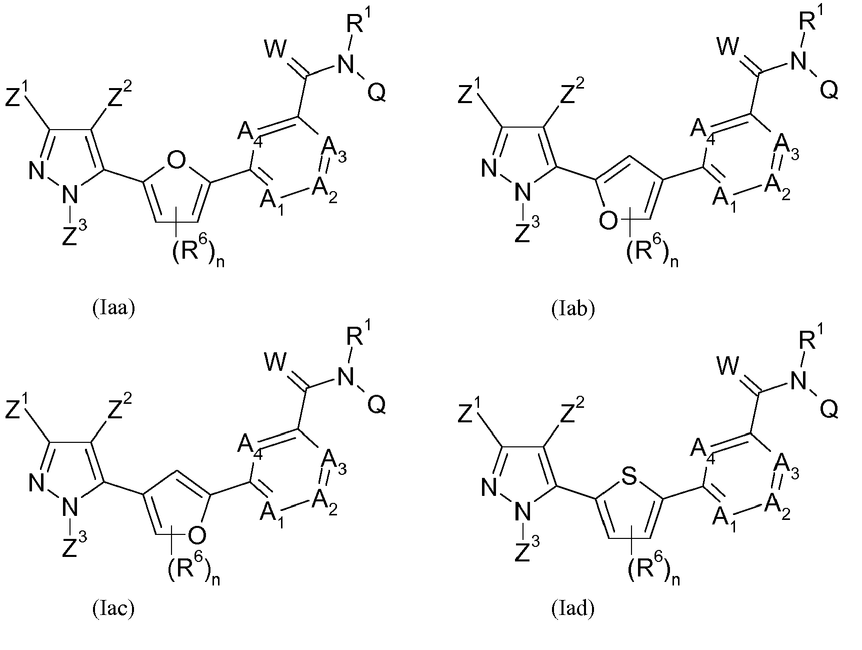

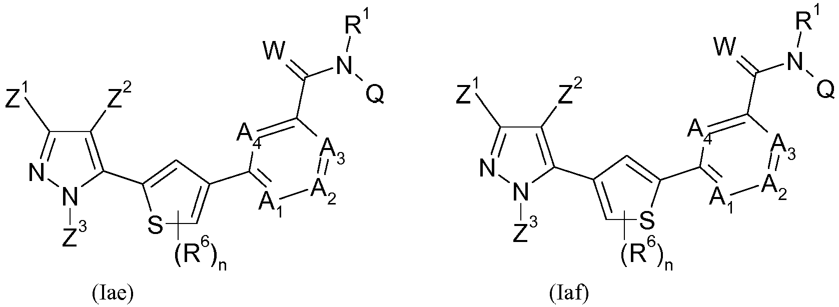

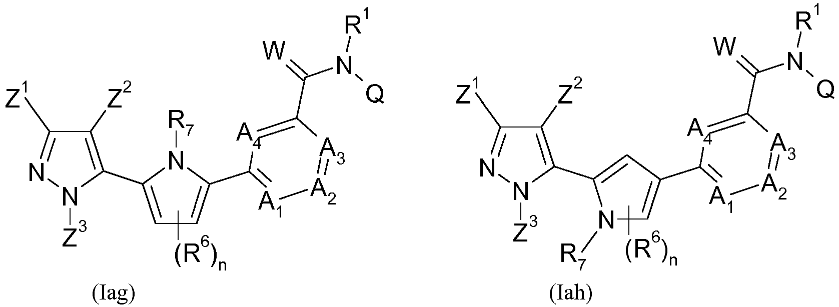

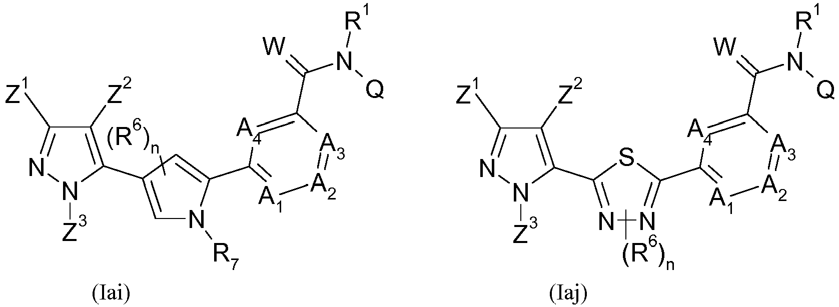

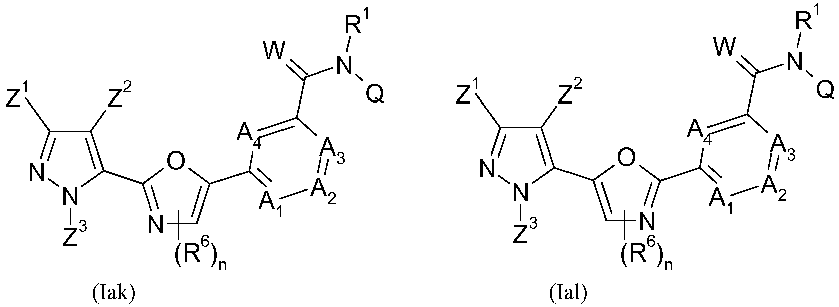

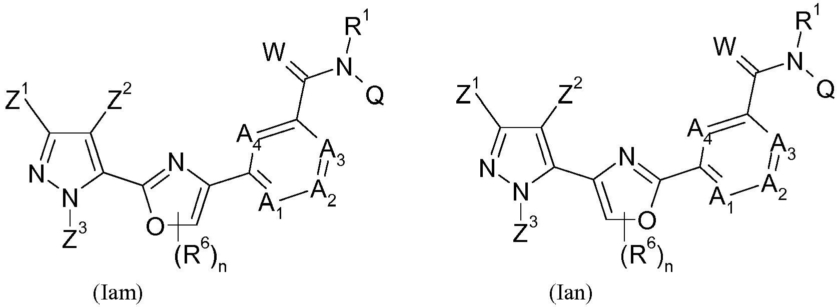

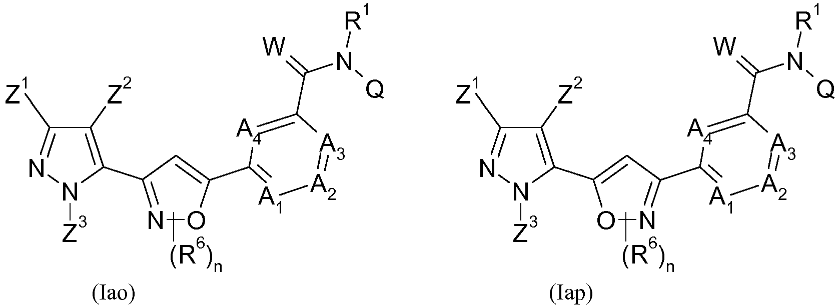

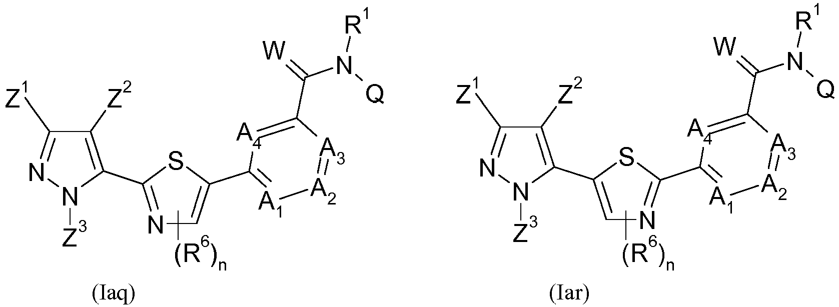

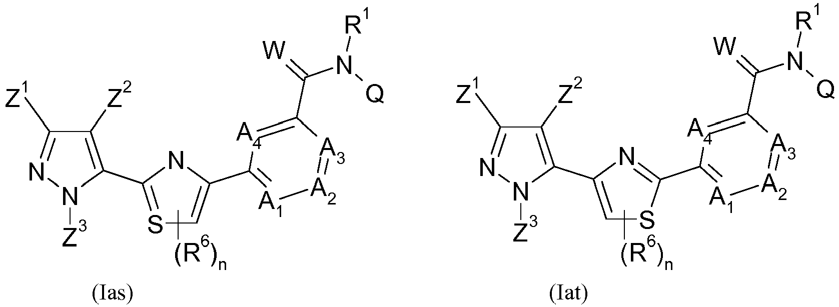

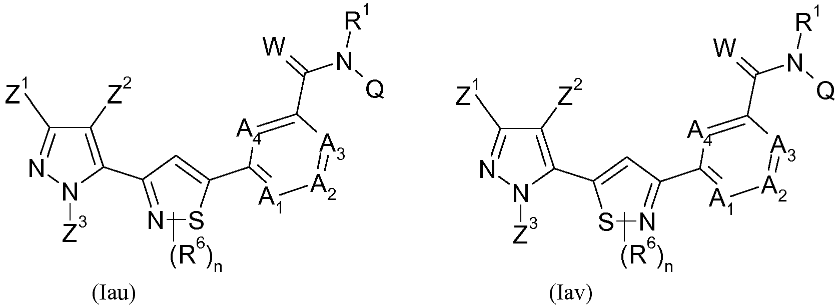

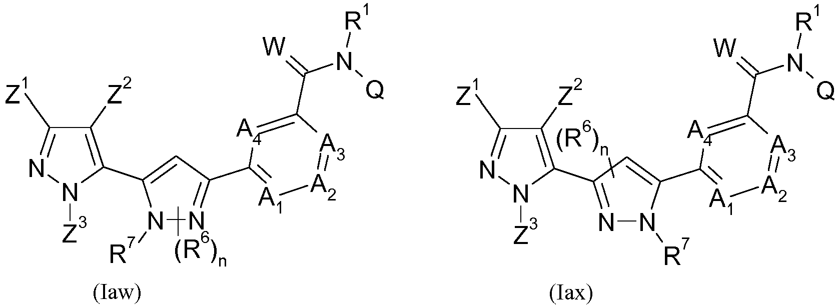

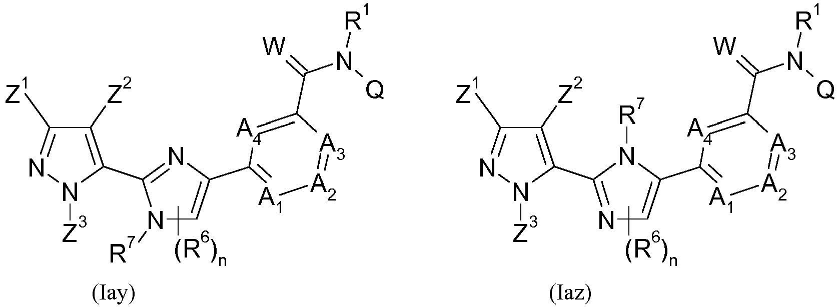

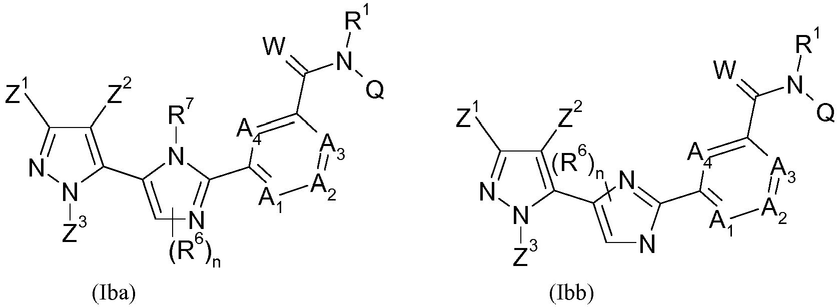

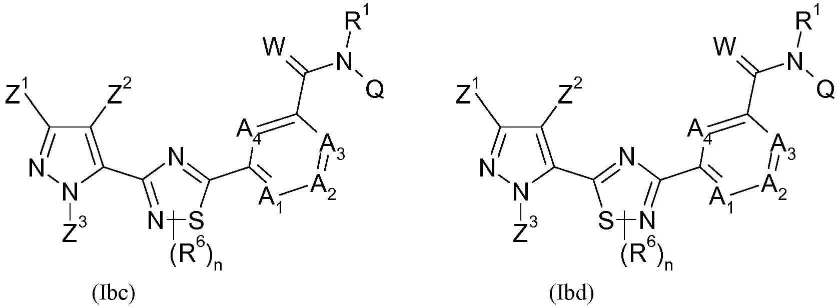

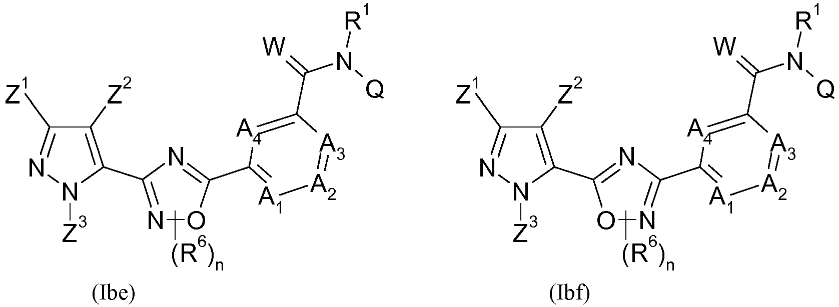

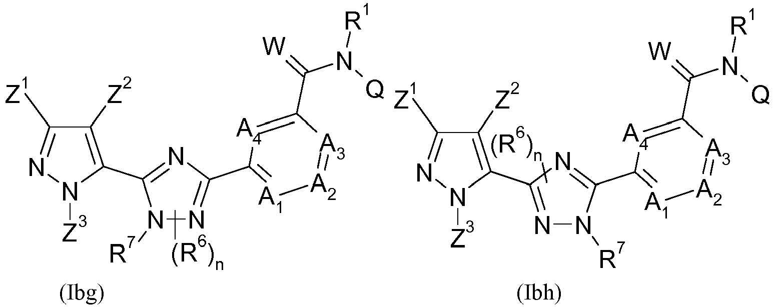

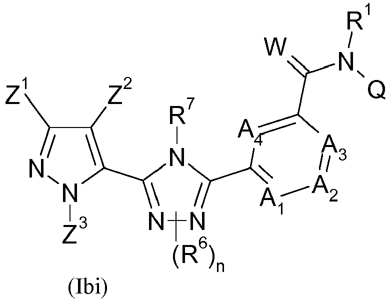

7. Verbindugen gemäß Anspruch 6, in denen Z¹ für CF₂CF₃ steht, Z² für CF₃, Z³ für CH₃, die Reste R¹ und R⁶ für Wasserstoff stehen, A¹ und A⁴ für CH stehen und A² für CH oder N steht, A³ für C-Cl, W für Sauerstoff und Q für 1-Cyano-cyclopropyl oder Cyclopropyl stehen.

8. Verwendung von Verbindungen der allgemeinen Formel (I) gemäß einem der Ansprüche 1 bis 5 sowie der Verbindungen gemäß Anspruch 6 oder 7 zur Bekämpfung von Insekten, Spinnentieren und Nematoden, wobei die chirurgische, therapeutische und diagnostische Behandlung des menschlichen oder tierischen Körpers ausgeschlossen ist.

9. Pharmazeutische Zusammensetzungen, enthaltend wenigstens eine Verbindung gemäß einem der Ansprüche 1 bis 7.

10. Verbindungen gemäß einem der Ansprüche 1 bis 7 zur Verwendung als Arzneimittel.

11. Verwendung von Verbindungen gemäß einem der Ansprüche 1 bis 7 zur Herstellung pharmazeutischer Zusammensetzungen zur Bekämpfung von Parasiten auf Tieren.

12. Verfahren zur Herstellung von Pflanzenschutzmitteln enthaltend Verbindungen gemäß einem der Ansprüche 1 bis 7, sowie übliche Streckmittel und/oder oberflächenaktive Substanzen.

13. Verfahren zum Bekämpfen von Schädlingen, **dadurch gekennzeichnet, dass** man eine Verbindung gemäß einem der Ansprüche 1 bis 7 auf die Schädlinge und/oder ihren Lebensraum einwirken lässt, wobei die chirurgische, therapeutische und diagnostische Behandlung des menschlichen oder tierischen Körpers ausgeschlossen ist.

14. Verwendung von Verbindungen gemäß einem der Ansprüche 1 bis 7 zum Schutz des Vermehrungsmaterials von Pflanzen.

## Claims

1. Compounds of the general formula (I) 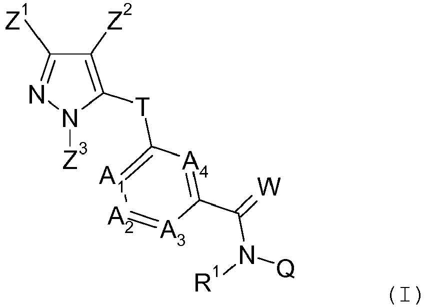 in which
R¹ represents hydrogen, optionally substituted alkyl, alkenyl, alkynyl, cycloalkyl, cycloalkylalkyl, alkylcarbonyl, alkoxycarbonyl, arylalkyl, heteroarylalkyl,
the chemical moieties
A₁ represents CR² or nitrogen,
A₂ represents CR³ or nitrogen,
A₃ represents CR⁴ or nitrogen, and
A₄ represents CR⁵ or nitrogen, but where not more than three of the chemical moieties A₁ to A₄ simultaneously represent nitrogen;
R², R³, R⁴ and R⁵ independently of one another represent hydrogen, halogen, cyano, nitro, optionally substituted alkyl, cycloalkyl, alkoxy, *N*-alkoxyiminoalkyl, alkylsulphanyl, alkylsulphinyl, alkylsulphonyl, *N*-alkylamino or *N,N*-dialkylamino;
if neither of the A₂ and A₃ moieties is nitrogen, R³ and R⁴ together with the carbon atom to which they are bonded may form a 5- or 6-membered ring containing 0, 1 or 2 nitrogen atoms and/or 0 or 1 oxygen atom and/or 0 or 1 sulphur atom, or
if neither of the A₁ and A₂ moieties is nitrogen, R² and R³ together with the carbon atom to which they are bonded may form a 6-membered ring containing 0, 1 or 2 nitrogen atoms;
W represents oxygen or sulphur;
Q represents hydrogen, formyl, hydroxy, amino or one of the optionally substituted moieties alkyl, alkyloxy, alkenyl, alkynyl, cycloalkyl, heterocycloalkyl, cycloalkylalkyl, heterocycloalkylalkyl, arylalkyl, heteroarylalkyl or represents a moiety *N*-alkylamino, *N*-alkylcarbonylamino, *N,N-*dialkylamino; or
Q represents a mono- to polyunsaturated 5- to 6-membered carbocycle which may optionally be interrupted by heteroatoms and is optionally mono- to polysubstituted by V, where
V represents halogen, cyano, nitro, optionally substituted alkyl, alkenyl, alkynyl, cycloalkyl, alkoxy, N-alkoxyiminoalkyl, alkylsulphanyl, alkylsulphinyl, alkylsulphonyl, N,N-dialkylamino;
T represents one of the 5-membered heteroaromatic systems T1-T35 shown below, where the bond to the pyrazolyl head group is indicated by an asterisk, 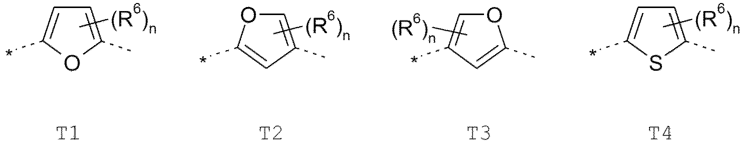 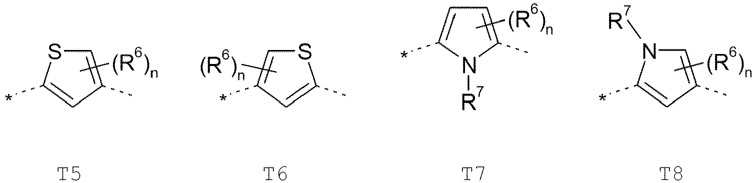 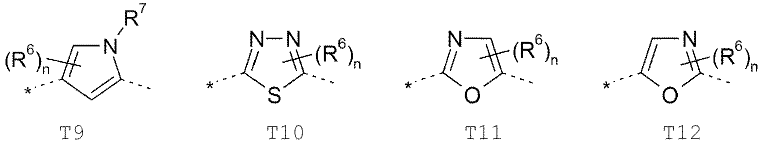 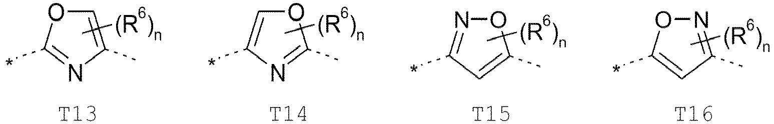 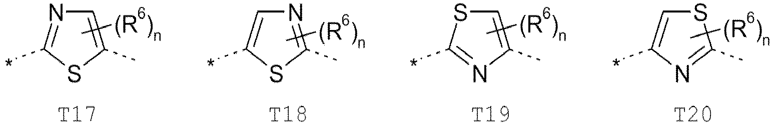 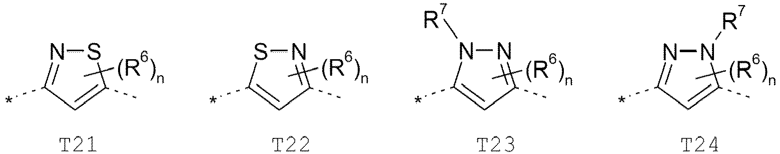 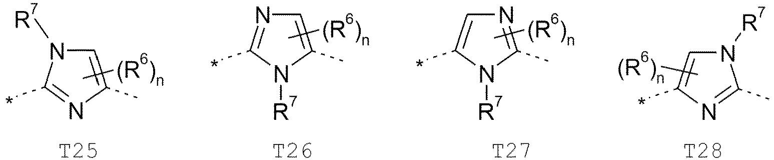 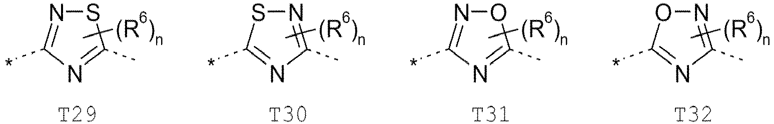 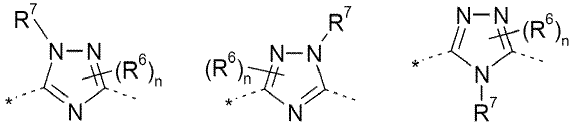
where
R⁶ independently of one another represent halogen, cyano, nitro, amino or optionally substituted alkyl, alkyloxy, alkylcarbonyl, alkylsulphanyl, alkylsulphinyl, alkylsulphonyl, and
n represents the values 0-2;
R⁷ represents hydrogen, or optionally substituted alkyl or cycloalkyl in which optionally one methylene group is substituted by a heteroatom;
Z¹ represents optionally substituted haloalkyl or halocycloalkyl, and
Z² represents hydrogen, halogen, cyano, nitro, amino or optionally substituted alkyl, alkylcarbonyl, alkylsulphanyl, alkylsulphinyl, alkylsulphonyl, and
Z³ represents hydrogen or optionally substituted alkyl, cycloalkyl, heterocycloalkyl, alkenyl, alkynyl, where
if T is T23 or T24, one of the radicals Z¹, Z² or Z³ is substituted by at least 3 halogen atoms.

2. Compounds according to Claim 1 in which
R¹ represents hydrogen, optionally substituted C₁-C₆-alkyl, C₃-C₆-alkenyl, C₃-C₆-alkynyl, C₃-C₇-cycloalkyl, C₃-C₇-cycloalkyl-C₁-C₃-alkyl, C₁-C₆-alkylcarbonyl, C₁-C₆-alkoxycarbonyl, aryl-(C₁-C₃)-alkyl, heteroaryl-(C₁-C₃)-alkyl,
the chemical moieties
A₁ represents CR² or nitrogen,
A₂ represents CR³ or nitrogen,
A₃ represents CR⁴ or nitrogen, and
A₄ represents CR⁵ or nitrogen,
but where not more than three of the chemical moieties A₁ to A₄ simultaneously represent nitrogen;
R², R³, R⁴ and R⁵ independently of one another represent hydrogen, halogen, cyano, nitro, optionally substituted C₁-C₆-alkyl, C₃-C₆-cycloalkyl, C₁-C₆-alkoxy, *N*-C₁-C₆-alkoxyimino-C₁-C₃-alkyl, C₁-C₆-alkylsulphanyl, C₁-C₆-alkylsulphinyl, C₁-C₆-alkylsulphonyl, *N*-C₁-C₆-alkylamino, *N,N*-di-C₁-C₆-alkylamino, or
if neither of the A₂ and A₃ moieties is nitrogen, R³ and R⁴ together with the carbon atom to which they are bonded may form a 5-or 6-membered ring containing 0, 1 or 2 nitrogen atoms and/or 0 or 1 oxygen atom and/or 0 or 1 sulphur atom, or
if neither of the A₁ and A₂ moieties is nitrogen, R² and R³ together with the carbon atom to which they are bonded may form a 6-membered ring containing 0, 1 or 2 nitrogen atoms;
W represents oxygen or sulphur;
Q represents hydrogen, formyl, hydroxy, amino or one of the optionally substituted moieties C₁-C₆-alkyl, C₃-C₆-alkenyl, C₃-C₆-alkynyl, C₃-C₆-cycloalkyl, C₁-C₅-heterocycloalkyl, C₁-C₄-alkoxy, C₃-C₆-cycloalkyl-C₁-C₆-alkyl, aryl- (C₁-C₃)-alkyl, heteroaryl-(C₁-C₃)-alkyl or represents a moiety *N*-C₁-C₄-alkylamino, *N*-C₁-C₄-alkylcarbonylamino, *N,N*-di-C₁-C₄-alkylamino; or
Q represents a mono- to triunsaturated 5- to 6-membered carbocycle which is optionally mono- to polysubstituted by V or a mono- to triunsaturated 5- or 6-membered heterocyclic ring which is optionally polysubstituted by V, where
V independently of one another represent halogen, cyano, nitro, optionally substituted C₁-C₆-alkyl, C₁-C₄-alkenyl, C₁-C₄-alkynyl, C₃-C₆-cycloalkyl, C₁-C₆-alkoxy, *N*-C₁-C₆-alkoxyimino-C₁-C₃-alkyl, C₁-C₆-alkylsulphanyl, C₁-C₆-alkylsulphinyl, C₁-C₆-alkylsulphonyl, *N,N*-di-(C₁-C₆-alkyl)amino;
T represents one of the 5-membered heteroaromatic systems T1-T35 shown below, where the bond to the pyrazolyl head group is indicated by an asterisk, 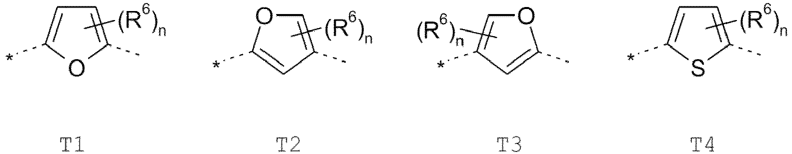 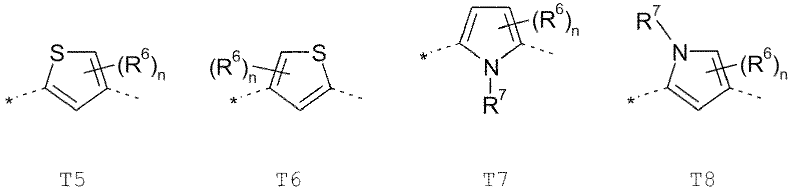 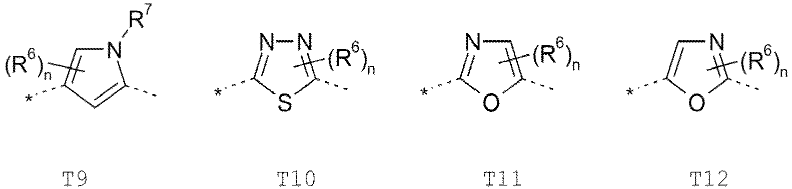 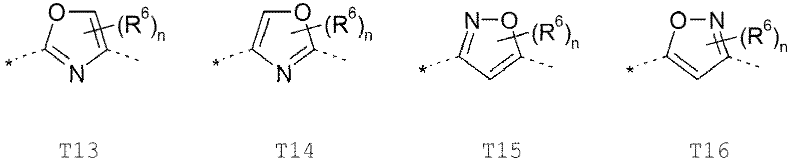 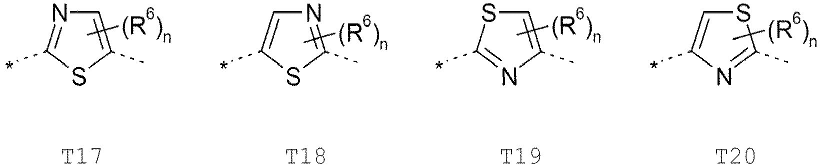 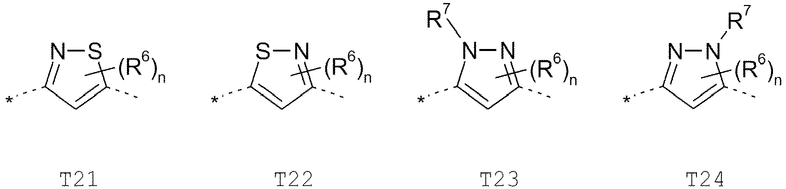 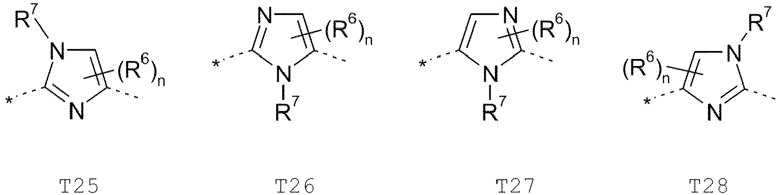 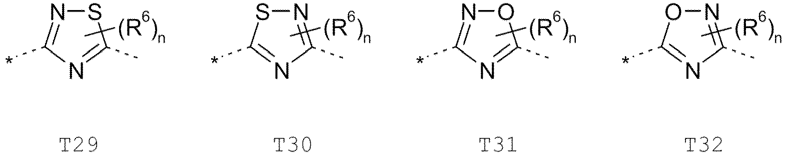 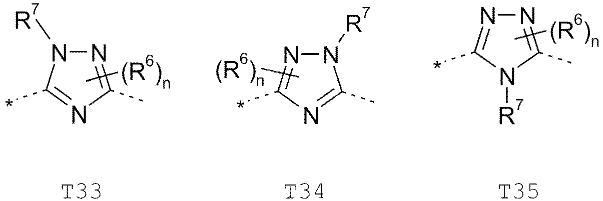
where
R⁶ independently of one another represent halogen, cyano, nitro, amino or optionally halogen-substituted C₁-C₆-alkyl, C₁-C₆-alkyloxy, C₁-C₆-alkylcarbonyl, C₁-C₆-alkylsulphanyl, C₁-C₆-alkylsulphinyl, C₁-C₆-alkylsulphonyl, and
n represents the values 0-1;
R⁷ represents hydrogen, or optionally substituted C₁-C₆-alkyl or C₃-C₆-cycloalkyl in which optionally one methylene group may be substituted by heteroatoms;
Z¹ represents optionally substituted C₁-C₆-haloalkyl, C₃-C₆-halocycloalkyl, and
Z² represents hydrogen, halogen, cyano, nitro, amino or optionally substituted C₁-C₆-alkyl, C₁-C₆-alkylcarbonyl, C₁-C₆-alkylsulphanyl, C₁-C₆-alkylsulphinyl, C₁-C₆-alkylsulphonyl, and
Z³ represents hydrogen or optionally substituted C₁-C₆-alkyl, C₃-C₆-cycloalkyl, C₃-C₅-heterocycloalkyl, C₃-C₆-alkenyl, C₃-C₆-alkynyl,
where if T is T23 or T24, one of the radicals Z¹, Z² or Z³ is substituted by at least 3 halogen atoms.

3. Compounds according to Claim 1 or 2 in which
R¹ represents hydrogen or represents C₁-C₆-alkyl, C₃-C₆-alkenyl, C₃-C₆-alkynyl, C₃-C₇-cycloalkyl, C₃-C₇-cycloalkyl-C₁-C₃-alkyl, C₁-C₆-alkylcarbonyl, C₁-C₆-alkoxycarbonyl, aryl-(C₁-C₃)-alkyl, heteroaryl-(C₁-C₃)-alkyl which are optionally mono- to pentasubstituted independently of one another by fluorine, chlorine, cyano, alkoxy and alkoxycarbonyl,
the chemical moieties
A₁ represents CR² or nitrogen,
A₂ represents CR³ or nitrogen,
A₃ represents CR⁴ or nitrogen, and
A₄ represents CR⁵ or nitrogen,
but where not more than three of the chemical moieties A₁ to A₄ simultaneously represent nitrogen;
R², R³, R⁴ and R⁵ independently of one another represent hydrogen, fluorine, chlorine, bromine, iodine, cyano, nitro or represent C₁-C₆-alkyl, C₃-C₆-cycloalkyl, C₁-C₆-alkoxy, *N*-C₁-C₆-alkoxyimino-C₁-C₃-alkyl, C₁-C₆-alkylsulphanyl, C₁-C₆-alkylsulphinyl, C₁-C₆-alkylsulphonyl, *N*-C₁-C₆-alkylamino or *N,N*-di-C₁-C₆-alkylamino which are optionally mono- to pentasubstituted by fluorine, chlorine, cyano or C₁-C₄-alkoxy;
W represents oxygen or sulphur;
Q represents hydrogen, hydroxy, formyl, amino or one of the moieties C₁-C₆-alkyl, C₃-C₆-alkenyl, C₃-C₆-alkynyl, C₃-C₆-cycloalkyl, C₂-C₆-heterocycloalkyl, C₁-C₄-alkoxy, C₁-C₆-alkyl-C₃-C₆-cycloalkyl, C₃-C₆-cycloalkyl-C₁-C₆-alkyl, C₁-C₆-hydroxyalkyl, aryl- (C₁-C₃)-alkyl, heteroaryl- (C₁-C₃)-alkyl, *N*-C₁-C₄-alkylamino, *N*-C₁-C₄-alkylcarbonylamino or *N,N*-di-C₁-C₄-alkylamino which are optionally mono- or polysubstituted independently of one another by hydroxy, nitro, amino, fluorine, chlorine, bromine, iodine, C₁-C₄-alkoxy, cyano, hydroxycarbonyl, C₁-C₄-alkoxycarbonyl, carbamoyl, thiocarbamoyl, C₁-C₄-alkylcarbamoyl, C₃-C₆-cycloalkylcarbamoyl, phenyl; or
Q represents aryl substituted by 0, 1, 2 or 3 substituents V or a 5- or 6-membered heteroaromatic system substituted by 0, 1, 2 or 3 substituents V, where
V independently of one another represent fluorine, chlorine, bromine, iodine, cyano, nitro, optionally substituted C₁-C₆-alkyl, C₂-C₄-alkenyl, C₂-C₄-alkynyl, C₃-C₆-cycloalkyl, C₁-C₆-alkoxy, *N*-C₁-C₆-alkoxyimino-C₁-C₃-alkyl, C₁-C₆-alkylsulphanyl, C₁-C₆-alkylsulphinyl, C₁-C₆-alkylsulphonyl, *N,N*-di-(C₁-C₆-alkyl)amino;
T represents one of the 5-membered heteroaromatic systems T1-T35 shown below, where the bond to the pyrazolyl head group is indicated by an asterisk, 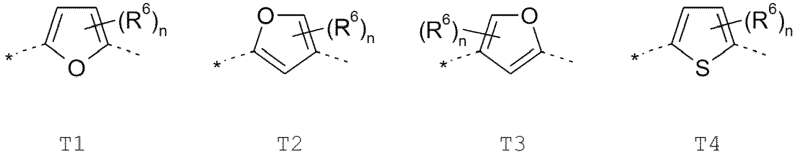 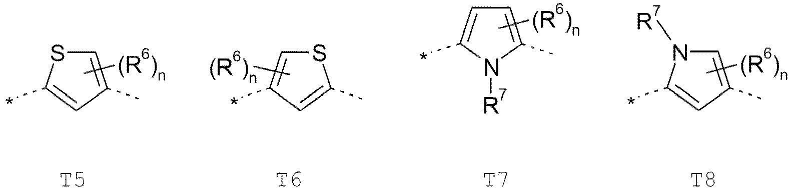 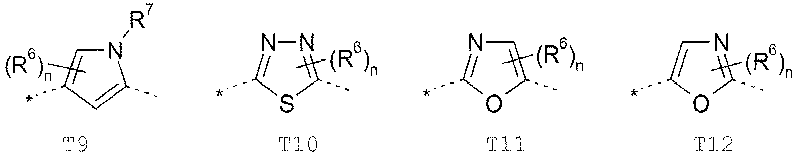 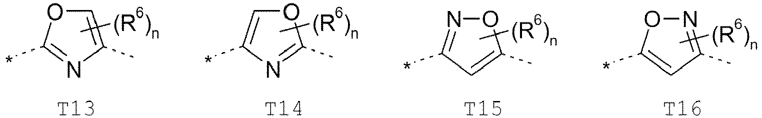 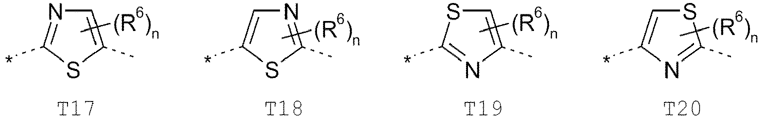 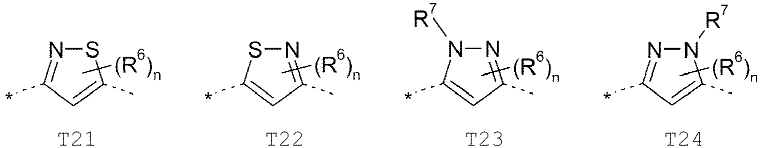 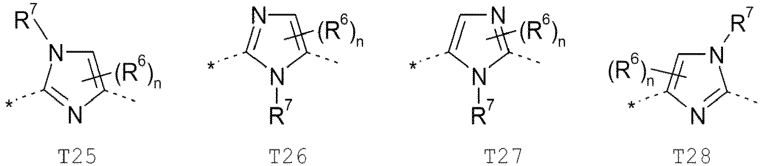 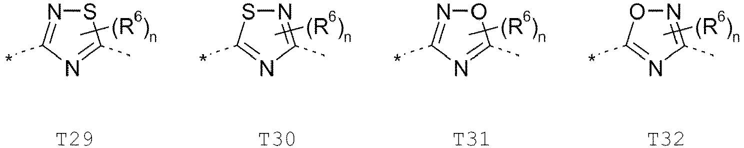 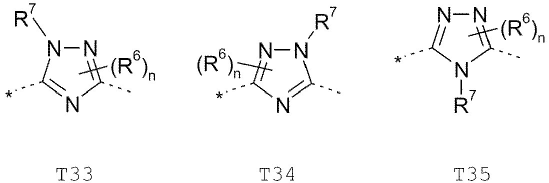
where
R⁶ independently of one another represent fluorine, chlorine, bromine, iodine, cyano, nitro, amino or represent C₁-C₆-alkyl, C₁-C₆-alkoxy, C₁-C₆-alkylcarbonyl, C₁-C₆-alkylsulphanyl, C₁-C₆-alkylsulphinyl, C₁-C₆-alkylsulphonyl which are optionally mono- to pentasubstituted by fluorine or chlorine, and
n represents the values 0-1;
R⁷ represents hydrogen, or C₁-C₆-alkyl or C₃-C₆-cycloalkyl in which optionally one methylene group may be substituted by heteroatoms and which are optionally mono- to pentasubstituted by fluorine, chlorine, cyano or C₁-C₄-alkoxy; Z¹ represents optionally substituted C₁-C₆-haloalkyl, C₃-C₆-halocycloalkyl, and
Z² represents hydrogen, halogen, cyano, nitro, amino or optionally mono- to pentasubstituted C₁-C₆-alkyl, C₁-C₆-alkylcarbonyl, C₁-C₆-alkylsulphanyl, C₁-C₆-alkylsulphinyl, C₁-C₆-alkylsulphonyl, and
Z³ represents hydrogen or represents C₁-C₆-alkyl, C₃-C₆-cycloalkyl, C₁-C₄-alkenyl, C₁-C₄-alkynyl which are optionally mono- to pentasubstituted by fluorine, chlorine, cyano or C₁-C₄-alkoxy;
where if T is T23 or T24, one of the radicals Z¹, Z² or Z³ is substituted by at least 3 halogen atoms.

4. Compounds according to any of Claims 1, 2 or 3 in which
R¹ represents hydrogen, methyl, ethyl, n-propyl, isopropyl, n-butyl, isobutyl, s-butyl, t-butyl, 2,2-difluoroethyl, 2,2,2-trifluoroethyl, methoxymethyl, ethoxymethyl, propoxymethyl, methylcarbonyl, ethylcarbonyl, n-propylcarbonyl, isopropylcarbonyl, s-butylcarbonyl, t-butylcarbonyl, methoxycarbonyl, ethoxycarbonyl, n-propoxycarbonyl, isopropoxycarbonyl, s-butoxycarbonyl, t-butoxycarbonyl, cyanomethyl, 2-cyanoethyl, benzyl, 4-methoxybenzyl, pyrid-2-ylmethyl, pyrid-3-ylmethyl, pyrid-4-ylmethyl, 6-chloropyrid-3-ylmethyl;
the chemical moieties
A₁ represents CR² or nitrogen,
A₂ represents CR³ or nitrogen,
A₃ represents CR⁴ or nitrogen, and
A₄ represents CR⁵ or nitrogen,
but where not more than three of the chemical moieties A¹ to A₄ simultaneously represent nitrogen;
R² and R⁵ independently of one another represent hydrogen, methyl, fluorine or chlorine and
R³ and R⁴ independently of one another represent hydrogen, fluorine, chlorine, bromine, iodine, cyano, nitro, methyl, ethyl, cyclopropyl, fluoromethyl, difluoromethyl, chlorodifluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N-*methoxyiminomethyl, 1-(*N*-methoxyimino)ethyl, methylsulphanyl, trifluoromethylsulphanyl, methylsulphonyl, methylsulphinyl, trifluoromethylsulphonyl, trifluoromethylsulphinyl;
W represents oxygen or sulphur;
Q represents hydrogen, methyl, ethyl, n-propyl, 1-methylethyl, 1,1-dimethylethyl, 1-methylpropyl, n-butyl, 2-methylpropyl, 2-methylbutyl, hydroxyethyl, 2-hydroxypropyl, cyanomethyl, 2-cyanoethyl, 2-fluoroethyl, 2,2-difluoroethyl, 2,2,2-trifluoroethyl, 1-trifluoromethylethyl, 2,2-difluoropropyl, 3,3,3-trifluoropropyl, 2,2-dimethyl-3-fluoropropyl, cyclopropyl, 1-cyanocyclopropyl, 1-methylcyclopropyl, 1-trifluoromethylcyclopropyl, 1-carbamoylcyclopropyl, 1-thiocarbamoylcyclopropyl, 1-methoxycarbonylcyclopropyl, 1-(*N-*methylcarbamoyl)cyclopropyl, 1-(*N-*cyclopropylcarbamoyl)cyclopropyl, cyclopropylmethyl, cyclobutyl, cyclopentyl, cyclohexyl, 1-cyclopropylethyl, bis(cyclopropyl)methyl, 2,2-dimethylcyclopropylmethyl, 2-phenylcyclopropyl, 2,2-dichlorocyclopropyl, trans-2-chlorocyclopropyl, cis-2-chlorocyclopropyl, 2,2-difluorocyclopropyl, trans-2-fluorocyclopropyl, cis-2-fluorocyclopropyl, trans-4-hydroxycyclohexyl, 4-trifluoromethylcyclohexyl, prop-2-enyl, 2-methylprop-2-enyl, prop-2-ynyl, 1,1-dimethylbut-2-ynyl, 3-chloroprop-2-enyl, 3,3-dichloroprop-2-enyl, 3,3-dichloro-1,1-dimethylprop-2-enyl, oxetan-3-yl, thietan-3-yl, 1-oxidothietan-3-yl, 1,1-dioxidothietan-3-yl, isoxazol-3-ylmethyl, 1,2,4-triazol-3-ylmethyl, 3-methyloxetan-3-ylmethyl, benzyl, 2,6-difluorophenylmethyl, 3-fluorophenylmethyl, 2-fluorophenylmethyl, 4-fluorophenylmethyl, 2,5-difluorophenylmethyl, 1-phenylethyl, 4-chlorophenylethyl, 2-trifluoromethylphenylethyl, 1-pyridin-2-ylethyl, pyridin-2-ylmethyl, 5-fluoropyridin-2-ylmethyl, (6-chloropyridin-3-yl)methyl, pyrimidin-2-ylmethyl, methoxy, 2-ethoxyethyl, 2-(methylsulphanyl)ethyl, 1-methyl-2-(ethylsulphanyl)ethyl, 2-methyl-1-(methylsulphanyl)propan-2-yl, methoxycarbonyl, methoxycarbonylmethyl, NH₂, *N*-ethylamino, *N*-allylamino, *N,N-*dimethylamino, *N,N*-diethylamino; or
Q represents phenyl, naphthyl, pyridazine, pyrazine, pyrimidine, triazine, pyridine, pyrazole, thiazole, isothiazole, oxazole, isoxazole, triazole, imidazole, furan, thiophene, pyrrole, oxadiazole, thiadiazole each substituted by 0, 1, 2 or 3 substituents V, where
V independently of one another represent fluorine, chlorine, bromine, iodine, cyano, nitro, methyl, ethyl, difluoromethyl, trichloromethyl, chlorodifluoromethyl, dichlorofluoromethyl, trifluoromethyl, 1-fluoroethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, heptafluoro-n-propyl, heptafluoroisopropyl, nonafluoro-n-butyl, cyclopropyl, cyclobutyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N-*methoxyiminomethyl, 1-(*N*-methoxyimino)ethyl, methylsulphanyl, methylsulphonyl, methylsulphinyl, trifluoromethylsulphonyl, trifluoromethylsulphinyl, trifluoromethylsulphanyl, *N,N*-dimethylamino;
T represents one of the 5-membered heteroaromatic systems T1-T35 shown below, where the bond to the pyrazolyl head group is indicated by an asterisk, 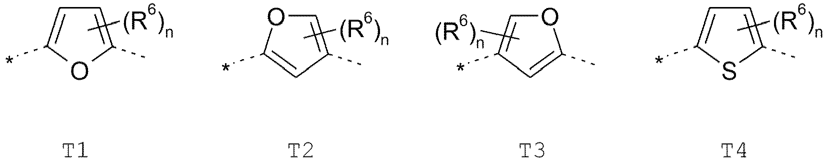 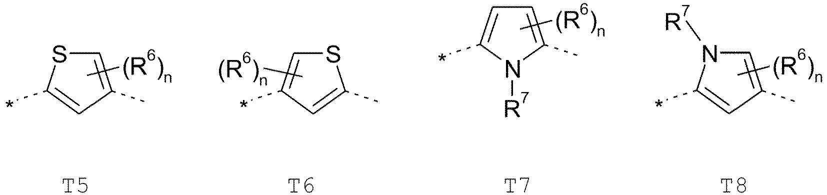 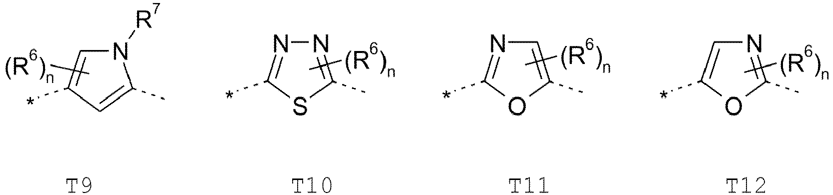 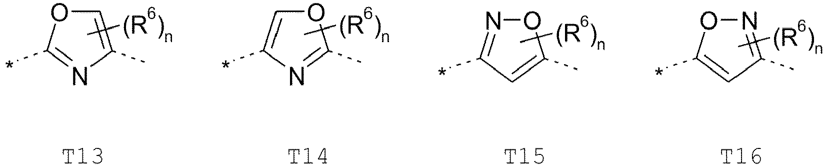 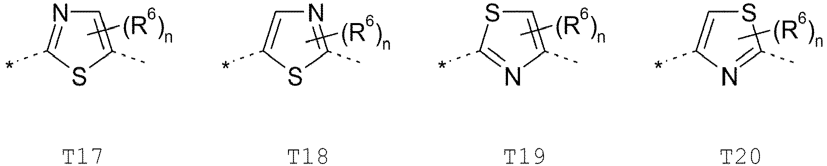 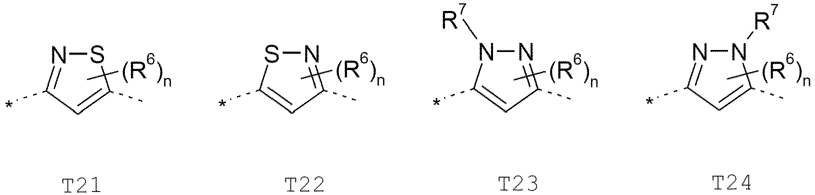 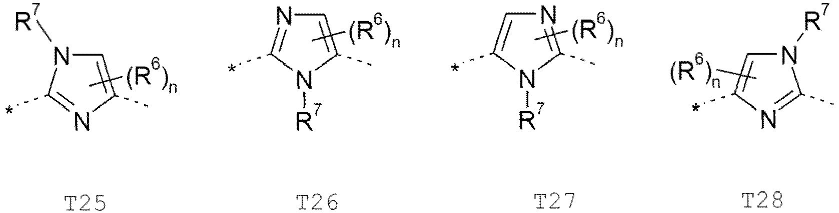 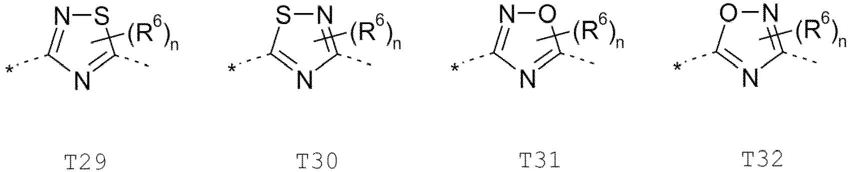 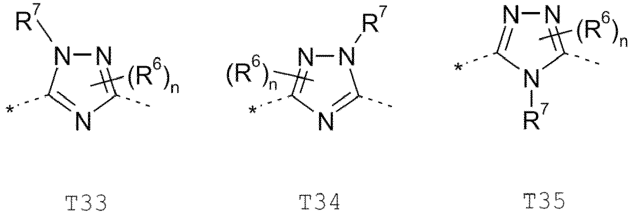
where
R⁶ independently of one another represent fluorine, chlorine, bromine, iodine, cyano, nitro, amino, methyl, ethyl, n-propyl, 1-methylethyl, tert-butyl, trifluoromethyl, difluoromethyl, methoxy, ethoxy, trifluoromethoxy, 2,2-difluoroethoxy, 2,2,2-trifluoroethoxy, methylcarbonyl, ethylcarbonyl, trifluoromethylcarbonyl, methylsulphanyl, methylsulphinyl, methylsulphonyl, trifluoromethylsulphonyl, trifluoromethylsulphanyl, trifluoromethylsulphinyl, and
n represents the values 0-1;
R⁷ represents hydrogen, methyl, ethyl, n-propyl, isopropyl, n-butyl, isobutyl, s-butyl, t-butyl, ethenyl, 1-propenyl, 2-propenyl, 1-propynyl, 1-butynyl, difluoromethyl, trifluoromethyl, 1-fluoroethyl, 1-fluoro-1-methylethyl, 2-fluoroethyl, 2,2-difluoroethyl, 2,2,2-trifluoroethyl, cyclopropyl, cyclobutyl, cyclopentyl, cyclohexyl;
Z¹ represents difluoromethyl, trichloromethyl, chlorodifluoromethyl, dichlorofluoromethyl, trifluoromethyl, bromodichloromethyl, 1-fluoroethyl, 1-fluoro-1-methylethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, heptafluoro-n-propyl, heptafluoroisopropyl, nonafluoro-n-butyl, 1-chlorocyclopropyl, 1-fluorocyclopropyl, 1-bromocyclopropyl, 1-trifluoromethylcyclopropyl or 2,2-difluoro-1-methylcyclopropyl, and
Z² represents hydrogen, fluorine, chlorine, bromine, iodine, cyano, nitro, amino, methyl, ethyl, n-propyl, 1-methylethyl, 1,1-dimethylethyl, difluoromethyl, trichloromethyl, chlorodifluoromethyl, dichlorofluoromethyl, trifluoromethyl, bromodichloromethyl, 1-fluoroethyl, 1-fluoro-1-methylethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-t-butyl, heptafluoro-n-propyl, heptafluoroisopropyl, nonafluoro-n-butyl, methylsulphanyl, methylsulphinyl, methylsulphonyl, ethylsulphanyl, ethylsulphinyl, ethylsulphonyl, trifluoromethylsulphanyl, trifluoromethylsulphinyl, trifluoromethylsulphonyl, chlorodifluoromethylsulphanyl, chlorodifluoromethylsulphinyl, chlorodifluoromethylsulphonyl, dichlorofluoromethylsulphanyl, dichlorofluoromethylsulphinyl, dichlorofluoromethylsulphonyl and
Z³ represents hydrogen, methyl, ethyl, n-propyl, isopropyl, n-butyl, isobutyl, s-butyl, t-butyl, prop-2-enyl, prop-2-ynyl, but-3-ynyl, difluoromethyl, trifluoromethyl, 1-fluoroethyl, 1-fluoro-1-methylethyl, 2-fluoroethyl, 2,2-difluoroethyl, 2,2,2-trifluoroethyl;
where if T is T23 or T24, one of the radicals Z¹, Z² or Z³ is substituted by at least 3 halogen atoms.

5. Compounds according to any of Claims 1, 2, 3 or 4 in which
Z1 represents trifluoromethyl, 1-chlorocyclopropyl, 1-fluorocyclopropyl or pentafluoroethyl,
Z2 represents trifluoromethyl, nitro, methylsulphanyl, methylsulphinyl, methylsulphonyl, fluorine, chlorine, bromine, cyano or iodine;
Z3 represents methyl, ethyl, n-propyl or hydrogen,
R1 represents hydrogen, methyl, ethyl, n-propyl, isopropyl, n-butyl, isobutyl, s-butyl, t-butyl, methoxymethyl, ethoxymethyl, propoxymethyl, methylcarbonyl, ethylcarbonyl, n-propylcarbonyl, isopropylcarbonyl, s-butylcarbonyl, t-butylcarbonyl, methoxycarbonyl, ethoxycarbonyl, n-propoxycarbonyl, isopropoxycarbonyl, s-butoxycarbonyl, t-butoxycarbonyl, cyanomethyl, 2-cyanoethyl, benzyl, 4-methoxybenzyl, pyrid-2-ylmethyl, pyrid-3-ylmethyl, pyrid-4-ylmethyl, 6-chloropyrid-3-ylmethyl,
A1 and A4 represent CH and A2 represents CH or N,
A3 represents CR4 and
R4 represents fluorine, chlorine, bromine, iodine or methyl,
T represents one of the 5-membered heteroaromatic systems T1-T35 shown below, where the bond to the pyrazolyl head group is indicated by an asterisk, 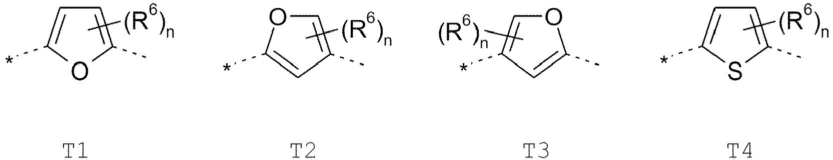 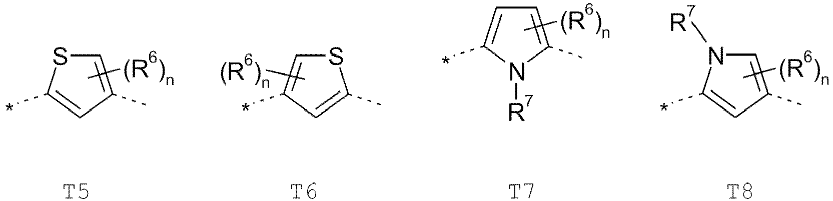 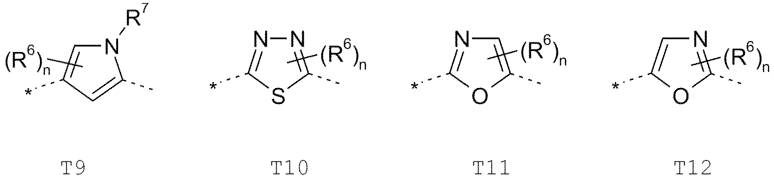 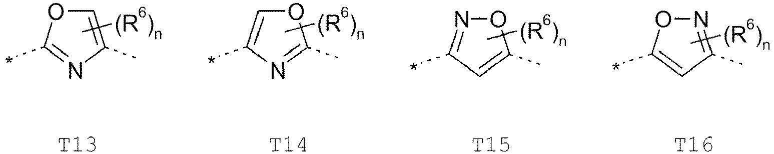 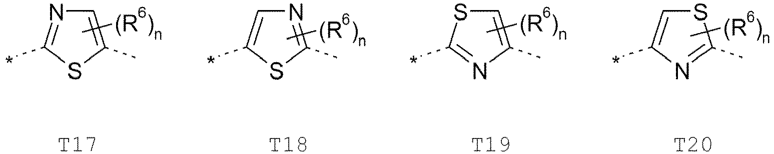 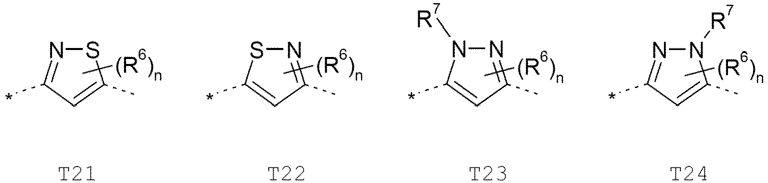 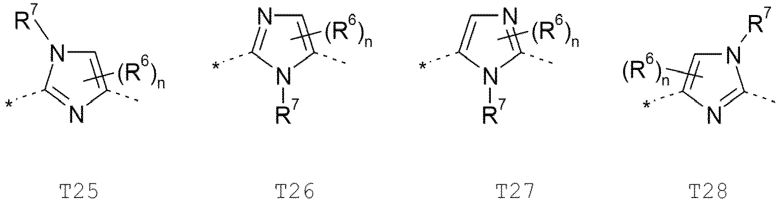 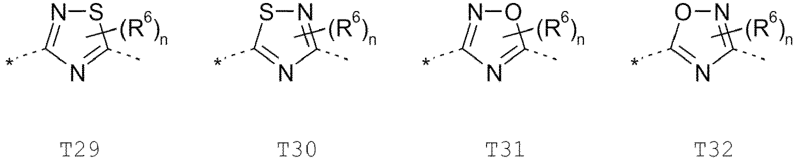 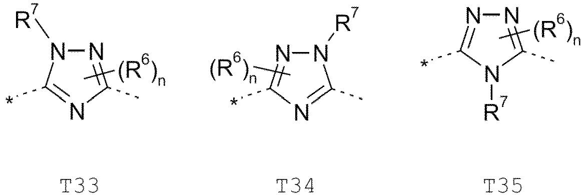
where
R6 represents hydrogen, methyl, ethyl, 2-methylethyl, 2,2-dimethylethyl, fluorine, chlorine, bromine, iodine, nitro, trifluoromethyl, amino, and
n represents the values 0-1;
W represents oxygen and
Q represents hydrogen, methyl, ethyl, n-propyl, 1-methylethyl, 1,1-dimethylethyl, n-butyl, 1-methylpropyl, 2-methylpropyl, 2-methylbutyl, hydroxymethyl, 2-hydroxypropyl, cyanomethyl, 2-cyanoethyl, 2-fluoroethyl, 2,2-difluoroethyl, 2,2,2-trifluoroethyl, 1-trifluoromethylethyl, 2,2-difluoropropyl, 3,3,3-trifluoropropyl, 2,2-dimethyl-3-fluoropropyl, cyclopropyl, 1-cyanocyclopropyl, 1-methylcyclopropyl, 1-trifluoromethylcyclopropyl, 1-carbamoylcyclopropyl, 1-thiocarbamoylcyclopropyl, 1-methoxycarbonylcyclopropyl, 1-(N-methylcarbamoyl)cyclopropyl, 1-(N-cyclopropylcarbamoyl)cyclopropyl, cyclopropylmethyl, cyclobutyl, cyclopentyl, cyclohexyl, 1-cyclopropylethyl, bis(cyclopropyl)methyl, 2,2-dimethylcyclopropylmethyl, 2-phenylcyclopropyl, 2,2-dichlorocyclopropyl, trans-2-chlorocyclopropyl, cis-2-chlorocyclopropyl, 2,2-difluorocyclopropyl, trans-2-fluorocyclopropyl, cis-2-fluorocyclopropyl, trans-4-hydroxycyclohexyl, 4-trifluoromethylcyclohexyl, prop-2-enyl, 2-methylprop-2-enyl, prop-2-ynyl, 1,1-dimethylbut-2-ynyl, 3-chloroprop-2-enyl, 3,3-dichloroprop-2-enyl, 3,3-dichloro-1,1-dimethylprop-2-enyl, oxetan-3-yl, thietan-3-yl, 1-oxidothietan-3-yl, 1,1-dioxidothietan-3-yl, isoxazol-3-ylmethyl, 1,2,4-triazol-3-ylmethyl, 3-methyloxetan-3-ylmethyl, benzyl, 2,6-difluorophenylmethyl, 3-fluorophenylmethyl, 2-fluorophenylmethyl, 4-fluorophenylmethyl, 2,5-difluorophenylmethyl, 1-phenylethyl, 4-chlorophenylethyl, 2-trifluoromethylphenylethyl, 1-pyridin-2-ylethyl, pyridin-2-ylmethyl, (6-chloropyridin-3-yl)methyl, 5-fluoropyridin-2-ylmethyl, pyrimidin-2-ylmethyl, methoxy, 2-ethoxyethyl, 2-(methylsulphanyl)ethyl, 1-methyl-2-(ethylsulphanyl)ethyl, 2-methyl-1-(methylsulphanyl)propan-2-yl, methoxycarbonyl, methoxycarbonylmethyl, NH2, N-ethylamino, N-allylamino, N,N-dimethylamino, N,N-diethylamino; or
Q represents phenyl, naphthyl, pyridazine, pyrazine, pyrimidine, triazine, pyridine, pyrazole, thiazole, isothiazole, oxazole, isoxazole, triazole, imidazole, furan, thiophene, pyrrole, oxadiazole, thiadiazole substituted by 0, 1, 2 or 3 substituents V, where
V independently of one another represent fluorine, chlorine, bromine, iodine, cyano, nitro, methyl, ethyl, difluoromethyl, trichloromethyl, chlorodifluoromethyl, dichlorofluoromethyl, trifluoromethyl, 1-fluoroethyl, 2-fluoroethyl, 2,2-difluoroethyl, 2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-tert-butyl, heptafluoro-n-propyl, heptafluoroisopropyl, nonafluoro-n-butyl, cyclopropyl, cyclobutyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, N-methoxyiminomethyl, 1-(N-methoxyimino)ethyl, methylsulphanyl, methylsulphonyl, methylsulphinyl, trifluoromethylsulphonyl, trifluoromethylsulphinyl, trifluoromethylsulphanyl, N,N-dimethylamino;
where if T is T23 or T24, one of the radicals Z1, Z2 or Z3 is substituted by at least 3 halogen atoms.

6. Compounds according to any of Claims 1 to 5 and according to one of the general formulae (Iaa) - (Ibi) below

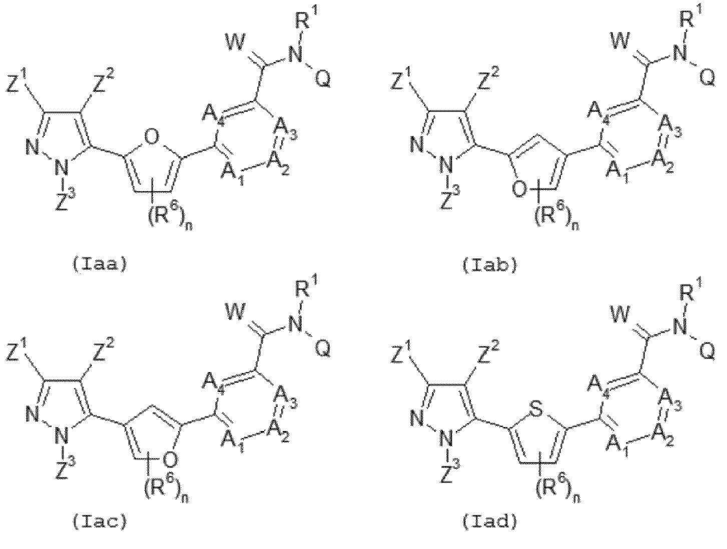

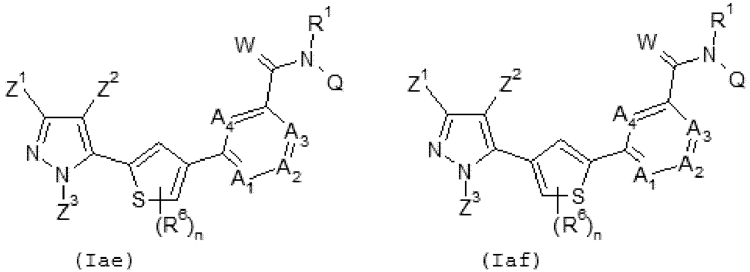

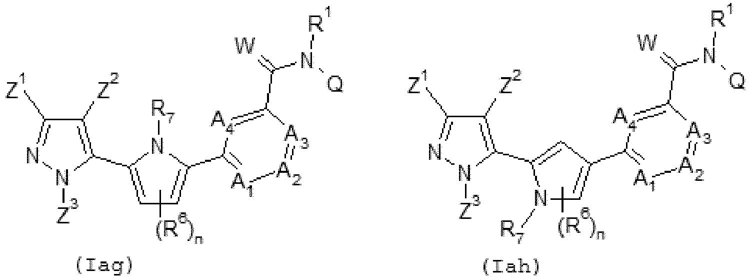

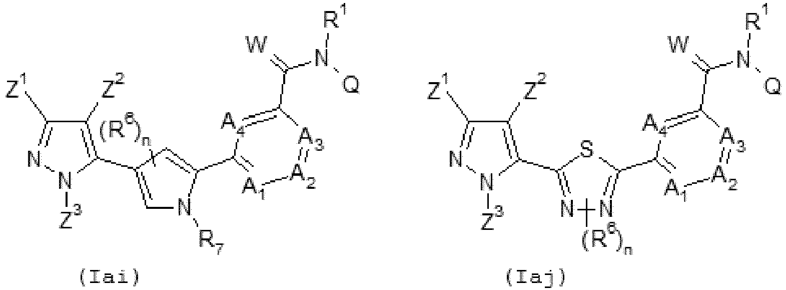

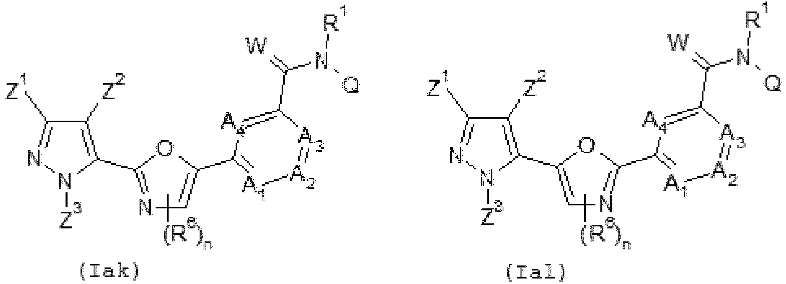

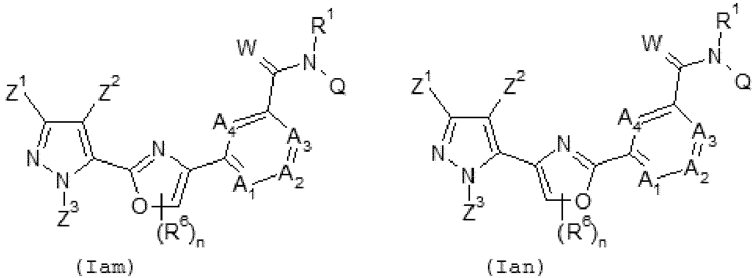

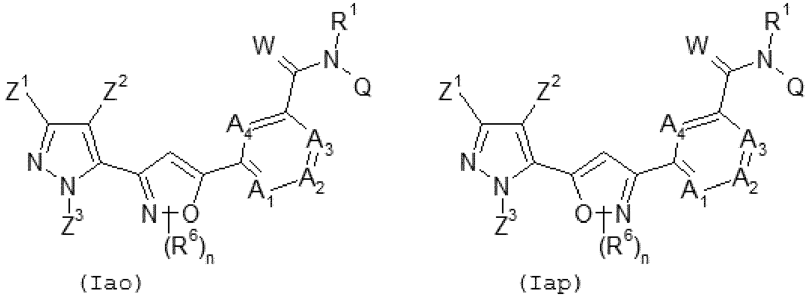

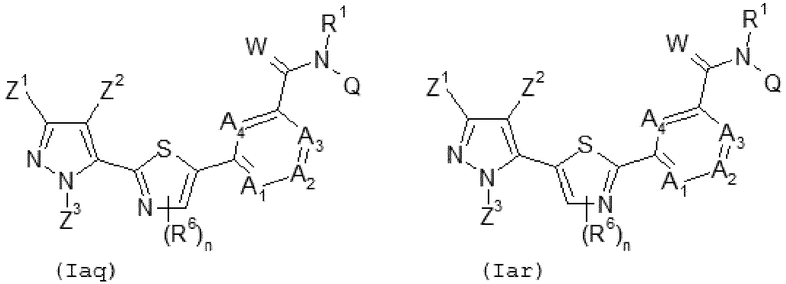

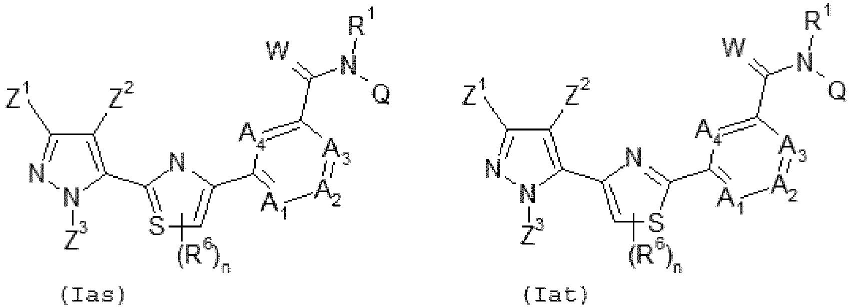

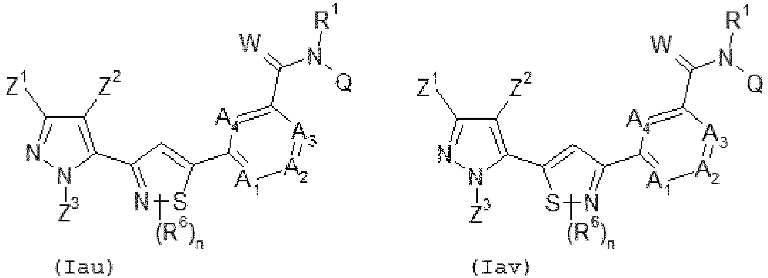

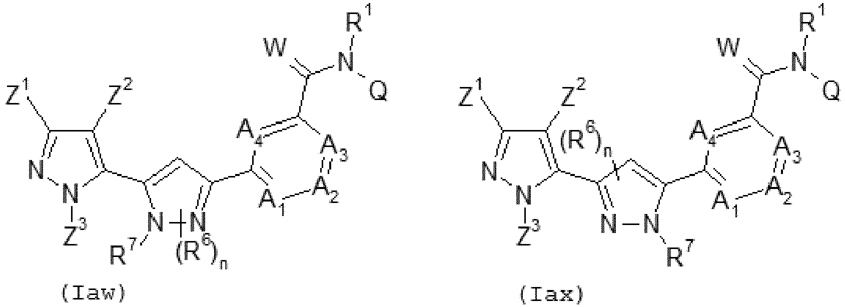

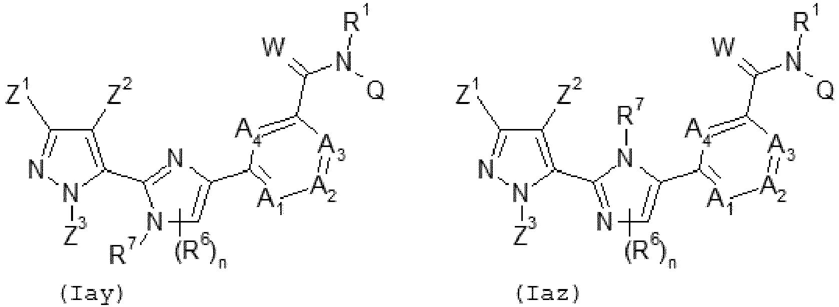

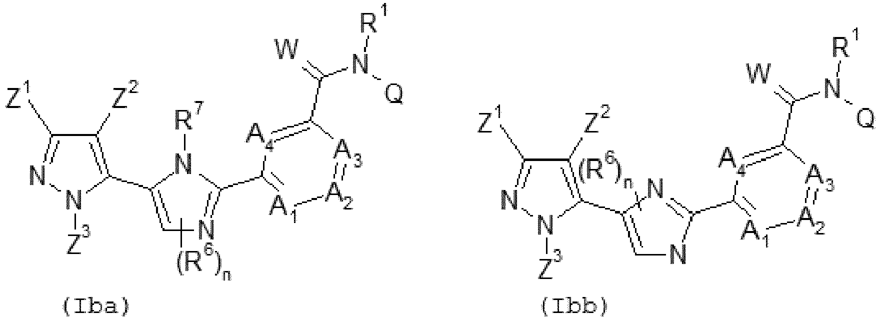

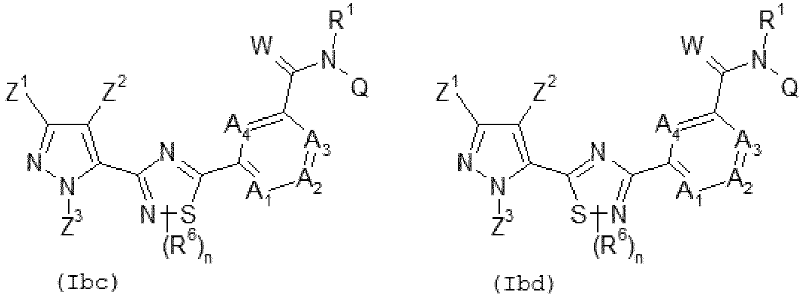

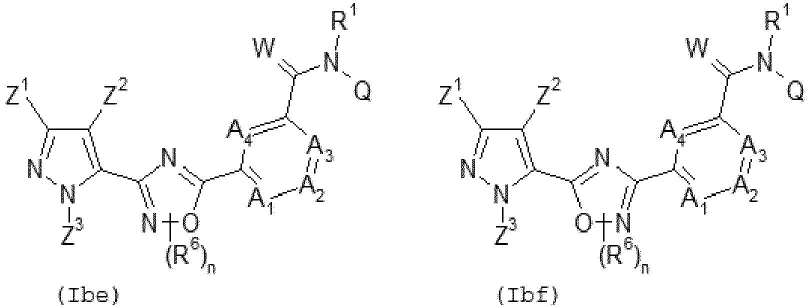

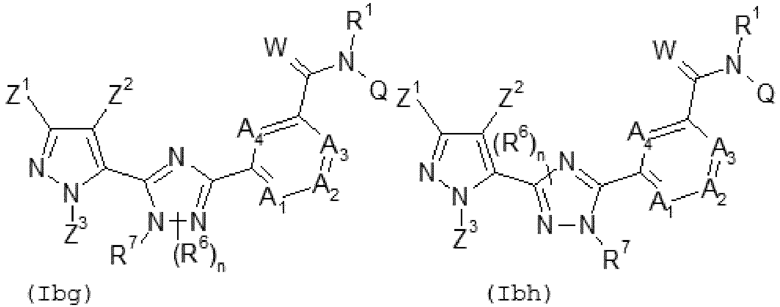

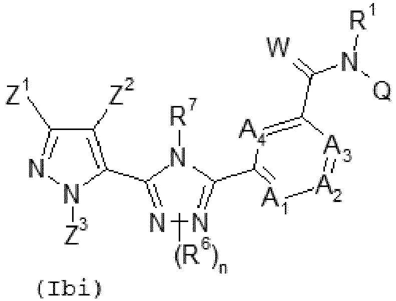

7. Compounds according to Claim 6 in which Z1 represents CF2CF3, Z2 represents CF3, Z3 represents CH3, the radicals R1 and R6 represent hydrogen, A1 and A4 represent CH, A2 represents CH or N, A3 represents C-Cl, W represents oxygen and Q represents 1-cyanocyclopropyl or cyclopropyl.

8. Use of compounds of the general formula (I) according to any of Claims 1 to 5 and of the compounds according to Claim 6 or 7 for controlling insects, arachnids and nematodes, where the surgical, therapeutic and diagnostic treatment of the human or animal body is excluded.

9. Pharmaceutical compositions comprising at least one compound according to any of Claims 1 to 7.

10. Compounds according to any of Claims 1 to 7 for use as medicaments.

11. Use of compounds according to any of Claims 1 to 7 for producing pharmaceutical compositions for controlling parasites on animals.

12. Process for producing crop protection compositions comprising compounds according to any of Claims 1 to 7 and customary extenders and/or surfactants.

13. Method for controlling pests, **characterized in that** a compound according to any of Claims 1 to 7 is allowed to act on the pests and/or their habitat, where the surgical, therapeutic and diagnostic treatment of the human or animal body is excluded.

14. Use of compounds according to any of Claims 1 to 7 for protecting the propagation material of plants.

## Revendications

1. Composés de formule générale (I) 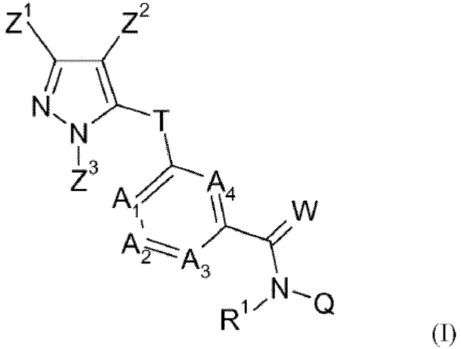 dans lesquels
R¹ représente hydrogène, alkyle éventuellement substitué, alcényle, alcynyle, cycloalkyle, cycloalkylalkyle, alkylcarbonyle, alcoxycarbonyle, arylalkyle, hétéroarylalkyle,
les groupes chimiques représentent
A₁ CR² ou azote,
A₂ CR³ ou azote,
A₃ CR⁴ ou azote, et
A₄ CR⁵ ou azote,
pas plus de trois des groupes chimiques A₁ à A₄ représentant toutefois simultanément l'azote ;
R², R³, R⁴ et R⁵ représentent indépendamment les uns des autres hydrogène, halogène, cyano, nitro, alkyle éventuellement substitué, cycloalkyle, alcoxy, *N-*alcoxy-imino-alkyle, alkylsulfanyle, alkylsulfinyle, alkylsulfonyle, *N*-alkylamino ou *N,N*-dialkylamino, lorsqu'aucun des groupes A₂ et A₃ ne représentent l'azote, R³ et R⁴ pouvant former ensemble avec l'atome de carbone auquel ils sont reliés un cycle à 5 ou 6 chaînons, qui contient 0, 1 ou 2 atomes d'azote et/ou 0 ou 1 atome d'oxygène et/ou 0 ou 1 atome de soufre, ou lorsqu'aucun des groupes A₁ et A₂ ne représente l'azote, R² et R³ pouvant former ensemble avec l'atome de carbone auquel ils sont reliés un cycle à 6 chaînons qui contient 0, 1 ou 2 atomes d'azote ;
W représente oxygène ou soufre ;
Q représente hydrogène, formyle, hydroxy, amino ou un des groupes éventuellement substitués alkyle, alkyloxy, alcényle, alcynyle, cycloalkyle, hétérocycloalkyle, cycloalkylalkyle, hétérocycloalkylalkyle, arylalkyle, hétéroarylalkyle, ou un groupe *N*-alkylamino, *N*-alkylcarbonylamino, *N,N-*dialkylamino ; ou
Q représente un carbocycle de 5 à 6 chaînons, insaturé une fois à plusieurs fois, éventuellement substitué une fois à plusieurs fois avec V, qui peut éventuellement être interrompu par des hétéroatomes,
V représentant halogène, cyano, nitro, alkyle éventuellement substitué, alcényle, alcynyle, cycloalkyle, alcoxy, *N*-alcoxyiminoalkyle, alkylsulfanyle, alkylsulfinyle, alkylsulfonyle, *N,N-*dialkylamino,
T représente un des radicaux hétéroaromatiques à 5 chaînons T1 à T35 listés ci-après, la liaison au groupe de tête pyrazole étant **caractérisée par** un astérisque, 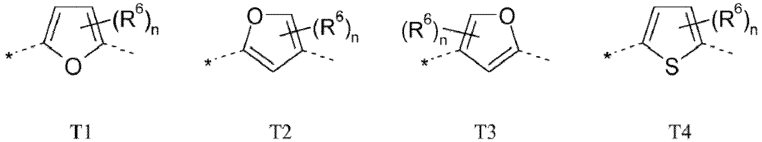 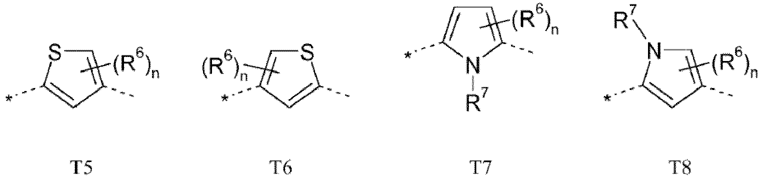 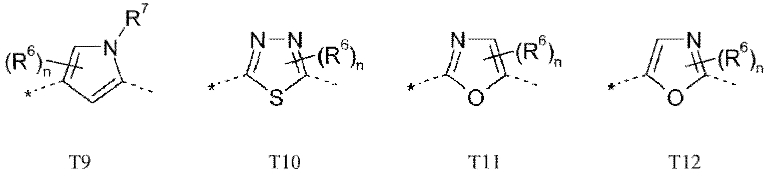 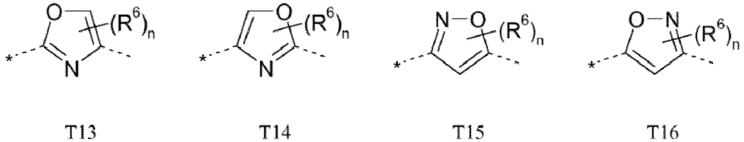 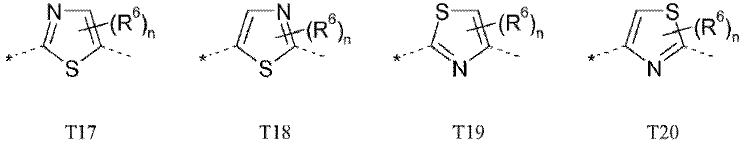 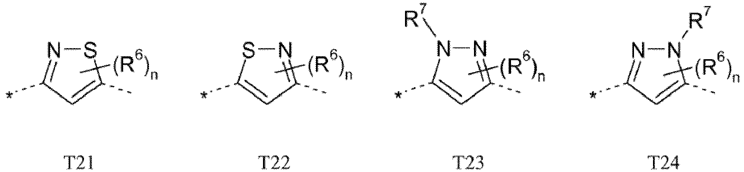 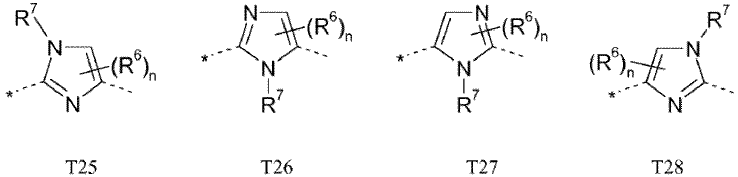 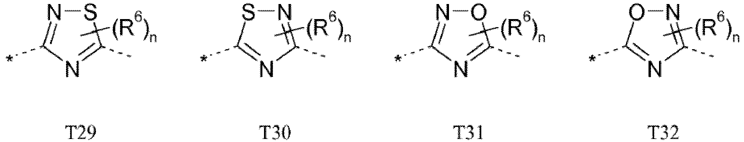 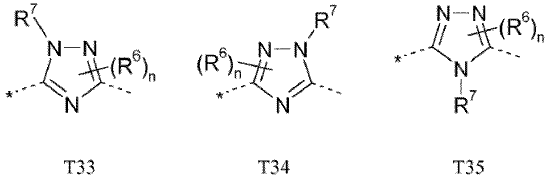
dans lesquels
les R⁶ représentent indépendamment les uns des autres halogène, cyano, nitro, amino ou un alkyle, alkyloxy, alkylcarbonyle, alkylsulfanyle, alkylsulfinyle, alkylsulfonyle éventuellement substitué, et
n représente les valeurs 0 à 2 ;
R⁷ représente l'hydrogène, ou un alkyle ou cycloalkyle éventuellement substitué, dans lequel un groupe méthylène est éventuellement substitué par un hétéroatome ;
Z¹ représente un halogénoalkyle ou halogénocycloalkyle éventuellement substitué, et
Z² représente hydrogène, halogène, cyano, nitro, amino ou un alkyle, alkylcarbonyle, alkylsulfanyle, alkylsulfinyle, alkylsulfonyle éventuellement substitué, et
Z³ représente l'hydrogène ou un alkyle, cycloalkyle, hétérocycloalkyle, alcényle, alcynyle éventuellement substitué ;
lorsque T est T23 ou T24, un des radicaux Z¹, Z² ou Z³ étant substitué avec au moins 3 atomes d'halogène.

2. Composés selon la revendication 1, dans lesquels R¹ représente hydrogène, alkyle en C₁-C₆ éventuellement substitué, alcényle en C₃-C₆, alcynyle en C₃-C₆, cycloalkyle en C₃-C₇, cycloalkyle en C₃-C₇-alkyle en C₁-C₃, alkylcarbonyle en C₁-C₆, alcoxycarbonyle en C₁-C₆, aryl-alkyle en C₁-C₃, hétéroaryl-alkyle en C₁-C₃,
les groupes chimiques représentent
A₁ CR² ou azote,
A₂ CR³ ou azote,
A₃ CR⁴ ou azote, et
A₄ CR⁵ ou azote,
pas plus de trois des groupes chimiques A₁ à A₄ représentant toutefois simultanément l'azote ;
R², R³, R⁴ et R⁵ représentent indépendamment les uns des autres hydrogène, halogène, cyano, nitro, alkyle en C₁-C₆ éventuellement substitué, cycloalkyle en C₃-C₆, alcoxy en C₁-C₆, *N*-alcoxy en C₁-C₆-imino-alkyle en C₁-C₃, alkylsulfanyle en C₁-C₆, alkylsulfinyle en C₁-C₆, alkylsulfonyle en C₁-C₆, *N*-alkylamino en C₁-C₆ ou *N*,*N*-di-alkylamino en C₁-C₆, ou
lorsqu'aucun des groupes A₂ et A₃ ne représentent l'azote, R³ et R⁴ pouvant former ensemble avec l'atome de carbone auquel ils sont reliés un cycle à 5 ou 6 chaînons, qui contient 0, 1 ou 2 atomes d'azote et/ou 0 ou 1 atome d'oxygène et/ou 0 ou 1 atome de soufre, ou lorsqu'aucun des groupes A₁ et A₂ ne représente l'azote, R² et R³ pouvant former ensemble avec l'atome de carbone auquel ils sont reliés un cycle à 6 chaînons qui contient 0, 1 ou 2 atomes d'azote ;
W représente oxygène ou soufre ;
Q représente hydrogène, formyle, hydroxy, amino ou un des groupes éventuellement substitués alkyle en C₁-C₆, alcényle en C₃-C₆, alcynyle en C₃-C₆, cycloalkyle en C₃-C₆, hétérocycloalkyle en C₁-C₅, alcoxy en C₁-C₄, cycloalkyle en C₃-C₆-alkyle en C₁-C₆, aryl-alkyle en C₁-C₃, hétéroaryl-alkyle en C₁-C₃, ou un groupe *N-*alkylamino en C₁-C₄, *N*-alkylcarbonylamino en C₁-C₄, *N,N-*di-alkylamino en C₁-C₄ ; ou
Q représente un carbocycle de 5 à 6 chaînons, insaturé une fois à trois fois, éventuellement substitué une fois à plusieurs fois avec V, ou un cycle hétérocyclique à 5 ou 6 chaînons, insaturé une fois à trois fois, éventuellement substitué plusieurs fois avec V,
les V représentant indépendamment les uns des autres halogène, cyano, nitro, alkyle en C₁-C₆ éventuellement substitué, alcényle en C₁-C₄, alcynyle en C₁-C₄, cycloalkyle en C₃-C₆, alcoxy en C₁-C₆, *N*-alcoxy en C₁-C₆-imino-alkyle en C₁-C₃, alkylsulfanyle en C₁-C₆, alkylsulfinyle en C₁-C₆, alkylsulfonyle en C₁-C₆, *N*,*N*-di-(alkyle en C₁-C₆)-amino,
T représente un des radicaux hétéroaromatiques à 5 chaînons T1 à T35 listés ci-après, la liaison au groupe de tête pyrazole étant **caractérisée par** un astérisque, 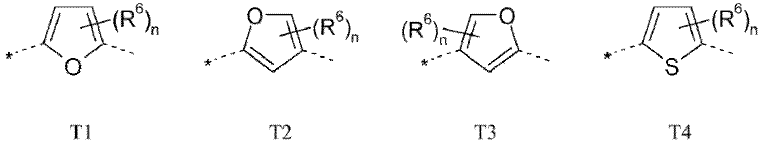 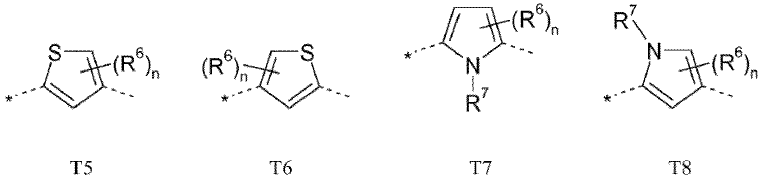 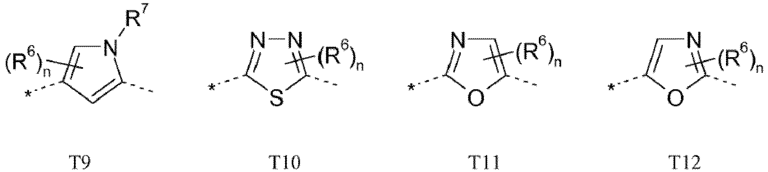 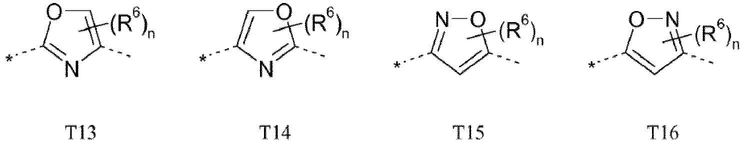 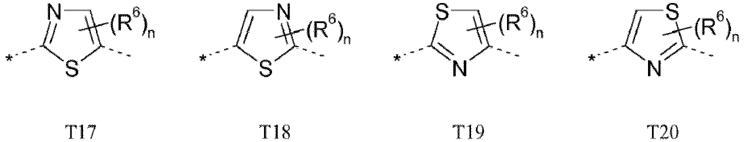 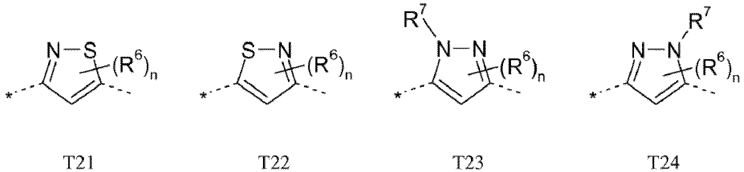 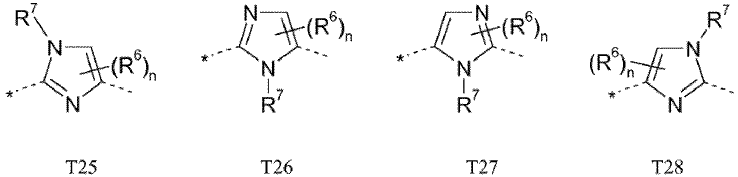 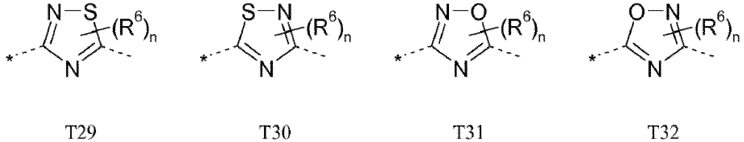 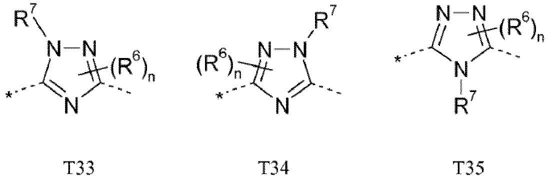 dans lesquels
les R⁶ représentent indépendamment les uns des autres halogène, cyano, nitro, amino ou un alkyle en C₁-C₆, alkyloxy en C₁-C₆, alkylcarbonyle en C₁-C₆, alkylsulfanyle en C₁-C₆, alkylsulfinyle en C₁-C₆, alkylsulfonyle en C₁-C₆ éventuellement substitué avec halogène, et
n représente les valeurs 0 à 1 ;
R⁷ représente hydrogène, ou un alkyle en C₁-C₆ ou cycloalkyle en C₃-C₆ éventuellement substitué, dans lequel un groupe méthylène peut éventuellement être substitué par des hétéroatomes ;
Z¹ représente un halogénoalkyle en C₁-C₆, halogénocycloalkyle en C₃-C₆ éventuellement substitué, et
Z² représente hydrogène, halogène, cyano, nitro, amino ou un alkyle en C₁-C₆, alkylcarbonyle en C₁-C₆, alkylsulfanyle en C₁-C₆, alkylsulfinyle en C₁-C₆, alkylsulfonyle en C₁-C₆ éventuellement substitué, et
Z³ représente hydrogène ou un alkyle en C₁-C₆, cycloalkyle en C₃-C₆, hétérocycloalkyle en C₃-C₅, alcényle en C₃-C₆, alcynyle en C₃-C₆ éventuellement substitué,
lorsque T est T23 ou T24, un des radicaux Z¹, Z² ou Z³ étant substitué avec au moins 3 atomes d'halogène.

3. Composés selon la revendication 1 ou 2, dans lesquels
R¹ représente hydrogène, alkyle en C₁-C₆ éventuellement substitué une à cinq fois indépendamment les unes des autres avec fluor, chlore, cyano, alcoxy et alcoxycarbonyle ; alcényle en C₃-C₆, alcynyle en C₃-C₆, cycloalkyle en C₃-C₇, cycloalkyle en C₃-C₇-alkyle en C₁-C₃, alkylcarbonyle en C₁-C₆, alcoxycarbonyle en C₁-C₆, aryl-alkyle en C₁-C₃, hétéroaryl-alkyle en C₁-C₃,
les groupes chimiques représentent
A₁ CR² ou azote,
A₂ CR³ ou azote,
A₃ CR⁴ ou azote, et
A₄ CR⁵ ou azote,
pas plus de trois des groupes chimiques A₁ à A₄ représentant toutefois simultanément l'azote ;
R², R³, R⁴ et R⁵ représentent indépendamment les uns des autres hydrogène, fluor, chlore, brome, iode, cyano, nitro, alkyle en C₁-C₆ éventuellement substitué une fois à cinq fois par fluor, chlore, cyano ou alcoxy en C₁-C₄; cycloalkyle en C₃-C₆, alcoxy en C₁-C₆, *N*-alcoxy en C₁-C₆-imino-alkyle en C₁-C₃, alkylsulfanyle en C₁-C₆, alkylsulfinyle en C₁-C₆, alkylsulfonyle en C₁-C₆, *N-*alkylamino en C₁-C₆ ou *N*,*N*-di-alkylamino en C₁-C₆ ;
W représente oxygène ou soufre ;
Q représente hydrogène, hydroxy, formyle, amino ou un des groupes alkyle en C₁-C₆, alcényle en C₃-C₆, alcynyle en C₃-C₆, cycloalkyle en C₃-C₆, hétérocycloalkyle en C₂-C₆, alcoxy en C₁-C₄, alkyle en C₁-C₆-cycloalkyle en C₃-C₆, cycloalkyle en C₃-C₆-alkyle en C₁-C₆, hydroxyalkyle en C₁-C₆, aryl-alkyle en C₁-C₃, hétéroaryl-alkyle en C₁-C₃, *N*-alkylamino en C₁-C₄, *N-*alkylcarbonylamino en C₁-C₄ ou *N*,*N*-di-alkylamino en C₁-C₄, éventuellement substitués une ou plusieurs fois indépendamment les unes des autres avec hydroxy, nitro, amino, fluor, chlore, brome, iode, alcoxy en C₁-C₄, cyano, hydroxycarbonyle, alcoxycarbonyle en C₁-C₄, carbamoyle, thiocarbamoyle, alkylcarbamoyle en C₁-C₄, cycloalkylcarbamoyle en C₃-C₆, phényle ; ou
Q représente un aryle substitué avec 0, 1, 2 ou 3 substituants V ou un radical hétéroaromatique à 5 ou 6 chaînons substitué avec 0, 1, 2 ou 3 substituants V, les V représentant indépendamment les uns des autres fluor, chlore, brome, iode, cyano, nitro, alkyle en C₁-C₆ éventuellement substitué, alcényle en C₂-C₄, alcynyle en C₂-C₄, cycloalkyle en C₃-C₆, alcoxy en C₁-C₆, *N*-alcoxy en C₁-C₆-imino-alkyle en C₁-C₃, alkylsulfanyle en C₁-C₆, alkylsulfinyle en C₁-C₆, alkylsulfonyle en C₁-C₆, *N,N*-di-(alkyle en C₁-C₆)-amino,
T représente un des radicaux hétéroaromatiques à 5 chaînons T1 à T35 listés ci-après, la liaison au groupe de tête pyrazole étant **caractérisée par** un astérisque, 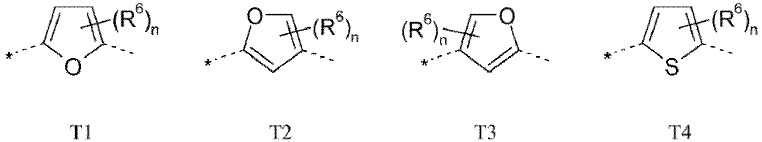 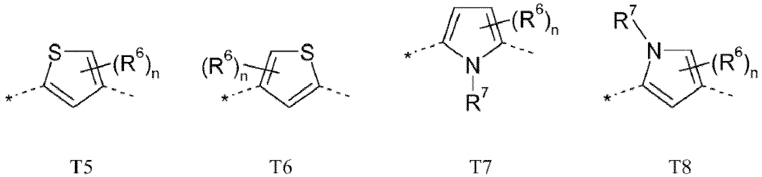 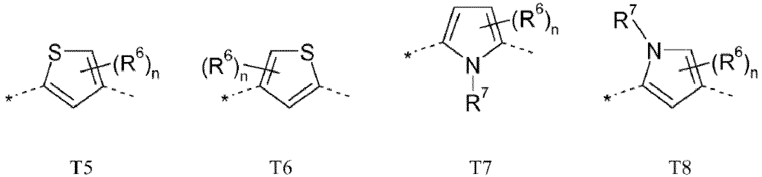 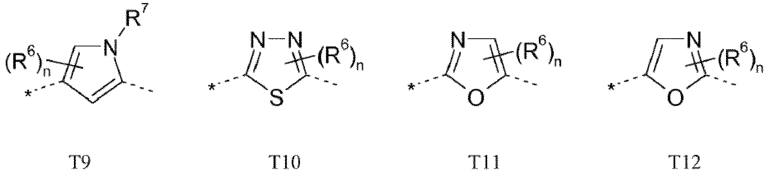 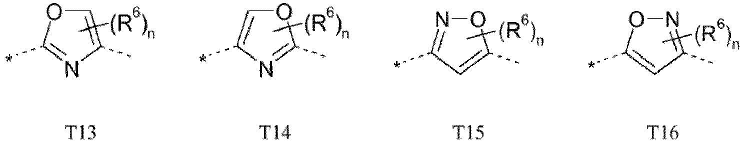 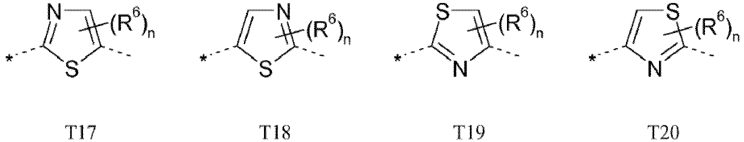 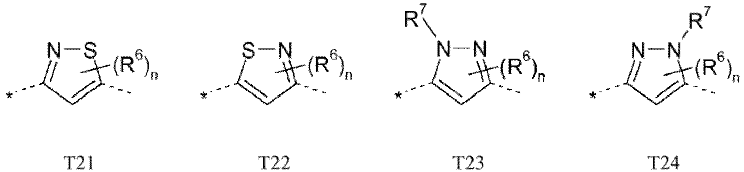 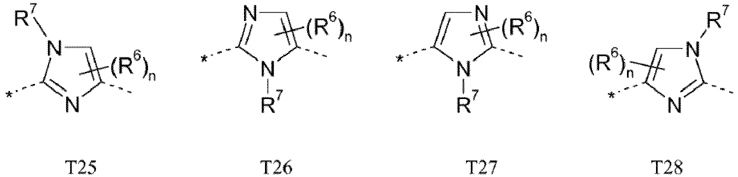 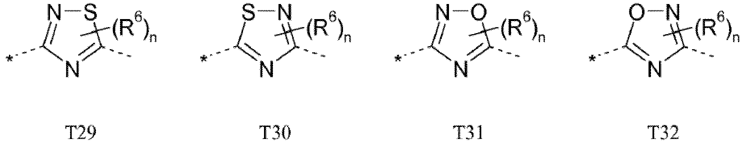 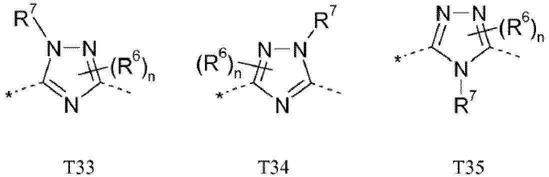 dans lesquels
les R⁶ représentent indépendamment les uns des autres fluor, chlore, brome, iode, cyano, nitro, amino ou un alkyle en C₁-C₆, alkyloxy en C₁-C₆, alkylcarbonyle en C₁-C₆, alkylsulfanyle en C₁-C₆, alkylsulfinyle en C₁-C₆, alkylsulfonyle en C₁-C₆ éventuellement substitué une fois à cinq fois avec fluor ou chlore, et
n représente les valeurs 0 à 1 ;
R⁷ représente hydrogène, ou un alkyle en C₁-C₆ ou cycloalkyle en C₃-C₆ éventuellement substitué une fois à cinq fois avec fluor, chlore, cyano ou alcoxy en C₁-C₄, dans lequel un groupe méthylène peut éventuellement être substitué par des hétéroatomes ;
Z¹ représente un haloalkyle en C₁-C₆, halocycloalkyle en C₃-C₆ éventuellement substitué, et
Z² représente hydrogène, halogène, cyano, nitro, amino ou un alkyle en C₁-C₆, alkylcarbonyle en C₁-C₆, alkylsulfanyle en C₁-C₆, alkylsulfinyle en C₁-C₆, alkylsulfonyle en C₁-C₆ éventuellement substitué une fois à cinq fois, et
Z³ représente hydrogène ou un alkyle en C₁-C₆, cycloalkyle en C₃-C₆, alcényle en C₁-C₄, alcynyle en C₁-C₄ éventuellement substitué une fois à cinq fois avec fluor, chlore, cyano ou alcoxy en C₁-C₄ ;
lorsque T est T23 ou T24, un des radicaux Z¹, Z² ou Z³ étant substitué avec au moins 3 atomes d'halogène.

4. Composés selon l'une quelconque des revendications 1, 2 ou 3, dans lesquels
R¹ représente hydrogène, méthyle, éthyle, n-propyle, isopropyle, n-butyle, isobutyle, s-butyle, t-butyle, 2,2,-difluoroéthyle, 2,2,2-trifluoroéthyle, méthoxyméthyle, éthoxyméthyle, propoxyméthyle, méthylcarbonyle, éthylcarbonyle, n-propylcarbonyle, isopropylcarbonyle, s-butylcarbonyle, t-butylcarbonyle, méthoxycarbonyle, éthoxycarbonyle, n-propoxycarbonyle, isopropoxycarbonyle, s-butoxycarbonyle, t-butoxycarbonyle, cyanométhyle, 2-cyanoéthyle, benzyle, 4-méthoxybenzyle, pyrid-2-yl-méthyle, pyrid-3-yl-méthyle, pyrid-4-yl-méthyle, 6-chloro-pyrid-3-yl-méthyle ;
les groupes chimiques représentent
A₁ CR² ou azote,
A₂ CR³ ou azote,
A₃ CR⁴ ou azote, et
A₄ CR⁵ ou azote,
pas plus de trois des groupes chimiques A₁ à A₄ représentant toutefois simultanément l'azote ;
R² et R⁵ représentent indépendamment l'un de l'autre hydrogène, méthyle, fluor et chlore, et
R³ et R⁴ représentent indépendamment l'un de l'autre hydrogène, fluor, chlore, brome, iode, cyano, nitro, méthyle, éthyle, cyclopropyle, fluorométhyle, difluorométhyle, chlorodifluorométhyle, trifluorométhyle, 2,2,2-trifluoroéthyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, *N-*méthoxyiminométhyle, 1-(*N*-méthoxyimino)-éthyle, méthylsulfanyle, trifluorométhylsulfanyle, méthylsulfonyle, méthylsulfinyle, trifluorométhylsulfonyle, trifluorométhylsulfinyle ;
W représente oxygène ou soufre ;
Q représente hydrogène, méthyle, éthyle, n-propyle, 1-méthyléthyle, 1,1-diméthyléthyle, 1-méthylpropyle, n-butyle, 2-méthylpropyle, 2-méthylbutyle, hydroxyéthyle, 2-hydroxypropyle, cyanométhyle, 2-cyanoéthyle, 2-fluoroéthyle, 2,2-difluoroéthyle, 2,2,2-trifluoroéthyle, 1-trifluorométhyléthyle, 2,2-difluoropropyle, 3,3,3-trifluoropropyle, 2,2-diméthyl-3-fluoropropyle, cyclopropyle, 1-cyano-cyclopropyle, 1-méthyl-cyclopropyle, 1-trifluorométhyl-cyclopropyle, 1-carbamoyl-cyclopropyle, 1-thiocarbamoyl-cyclopropyle, 1-méthoxycarbonyl-cyclopropyle, 1-(*N-*méthylcarbamoyl)cyclopropyle, 1-*(N-*cyclopropylcarbamoyl)cyclopropyle, cyclopropyl-méthyle, cyclobutyle, cyclopentyle, cyclohexyle, 1-cyclopropyléthyle, bis(cyclopropyl)méthyle, 2,2-diméthylcyclopropyl-méthyle, 2-phénylcyclopropyle, 2,2-dichlorocyclopropyle, trans-2-chlorocyclopropyle, cis-2-chlorocyclopropyle, 2,2-difluorocyclopropyle, trans-2-fluorocyclopropyle, cis-2-fluorocyclopropyle, trans-4-hydroxycyclohexyle, 4-trifluorométhylcyclohexyle, prop-2-ényle, 2-méthylprop-2-ényle, prop-2-inyle, 1,1-diméthylbut-2-inyle, 3-chloro-prop-2-ényl, 3,3-dichloro-prop-2-ényle, 3,3-dichloro-1,1-diméthylprop-2-ényle, oxétan-3-yle, thiétan-3-yle, 1-oxydo-thiétan-3-yle, 1,1-dioxydo-thiétan-3-yle, isoxazol-3-ylméthyle, 1,2,4-triazol-3-ylméthyle, 3-méthyloxétan-3-ylméthyle, benzyle, 2,6-difluorophénylméthyle, 3-fluorophénylméthyle, 2-fluorophénylméthyle, 4-fluorophénylméthyle, 2,5-difluorophénylméthyle, 1-phényléthyle, 4-chlorophényléthyle, 2-trifluorométhylphényléthyle, 1-pyridin-2-yléthyle, pyridin-2-ylméthyle, 5-fluoropyridin-2-ylméthyle, (6-chloro-pyridin-3-yl)méthyle, pyrimidin-2-ylméthyle, méthoxy, 2-éthoxyéthyle, 2-(méthylsulfanyl)éthyle, 1-méthyl-2-(éthylsulfanyl)éthyle, 2-méthyl-1-(méthylsulfanyl)propan-2-yle, méthoxycarbonyle, méthoxycarbonylméthyle, NH₂, *N*-éthylamino, *N*-allylamino, *N*,*N*-diméthylamino, *N,N*-diéthylamino ; ou
Q représente un phényle, un naphtyle, une pyridazine, une pyrazine, une pyrimidine, une triazine, une pyridine, un pyrazole, un thiazole, un isothiazole, un oxazole, un isoxazole, un triazole, un imidazole, un furane, un thiophène, un pyrrole, un oxadiazole, un thiadiazole substitué avec 0, 1, 2 ou 3 substituants V, les V représentant indépendamment les uns des autres fluor, chlore, brome, iode, cyano, nitro, méthyle, éthyle, difluorométhyle, trichlorométhyle, chlorodifluorométhyle, dichlorofluorométhyle, trifluorométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, heptafluoro-n-propyle, heptafluoro-isopropyle, nonafluoro-n-butyle, cyclopropyle, cyclobutyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, *N-*méthoxyiminométhyle, 1-(*N*-méthoxyimino)-éthyle, méthylsulfanyle, méthylsulfonyle, méthylsulfinyle, trifluorométhylsulfonyle, trifluorométhylsulfinyle, trifluorométhylsulfanyle, *N,N*-diméthylamino ;
T représente un des radicaux hétéroaromatiques à 5 chaînons T1 à T35 listés ci-après, la liaison au groupe de tête pyrazole étant **caractérisée par** un astérisque, 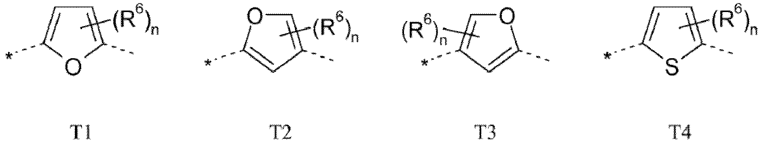 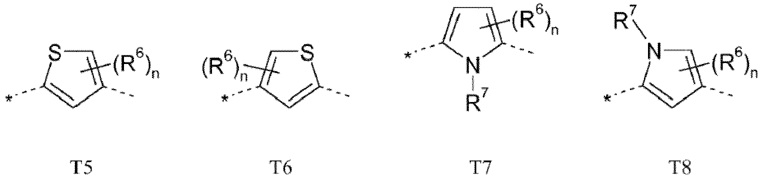 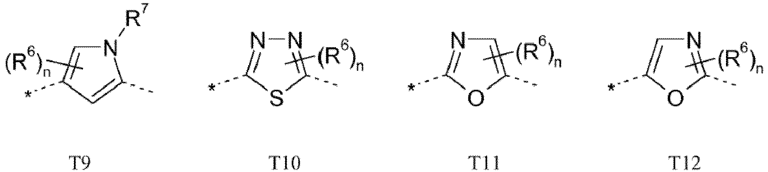 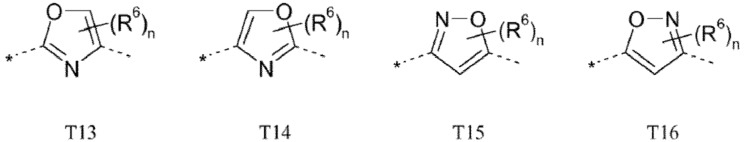 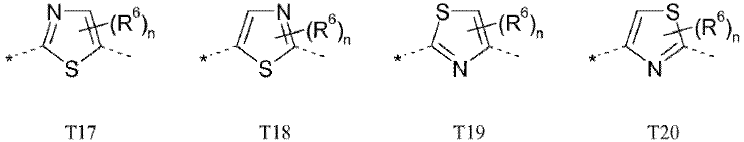 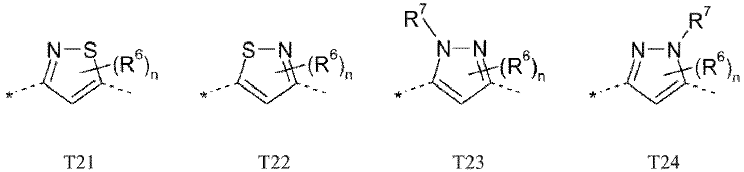 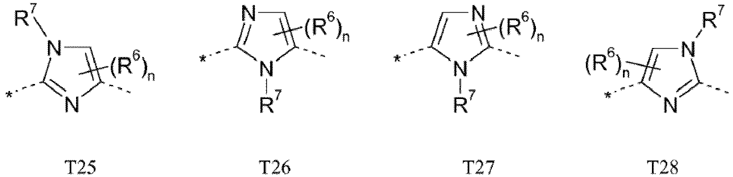 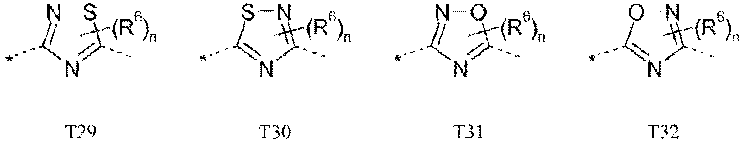 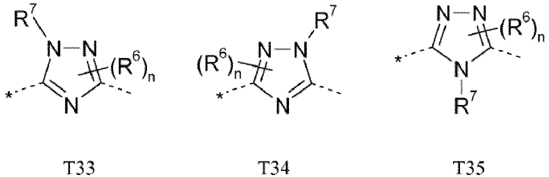 dans lesquels
les R⁶ représentent indépendamment les uns des autres fluor, chlore, brome, iode, cyano, nitro, amino, méthyle, éthyle, n-propyle, 1-méthyléthyle, tert-butyle, trifluorométhyle, difluorométhyle, méthoxy, éthoxy, trifluorométhoxy, 2,2-difluoroéthoxy, 2,2,2-trifluoroéthoxy, méthylcarbonyle, éthylcarbonyle, trifluorométhylcarbonyle, méthylsulfanyle, méthylsulfinyle, méthylsulfonyle, trifluorométhylsulfonyle, trifluorométhylsulfanyle, trifuorméthylsulfinyle, et
n représente les valeurs 0 à 1 ;
R⁷ représente hydrogène, méthyle, éthyle, n-propyle, isopropyle, n-butyle, isobutyle, s-butyle, t-butyle, éthényle, 1-propényle, 2-propényle, 1-propinyle, 1-butinyle, difluorométhyle, trifluorométhyle, 1-fluoroéthyle, 1-fluoro-1-méthyléthyle, 2-fluoroéthyle, 2,2-difluoroéthyle, 2,2,2-trifluoroéthyle, cyclopropyle, cyclobutyle, cyclopentyle, cyclohexyle ; Z¹ représente difluorométhyle, trichlorométhyle, chlorodifluorométhyle, dichlorofluorométhyle, trifluorométhyle, bromodichlorométhyle, 1-fluoroéthyle, 1-fluoro-1-méthyléthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, heptafluoro-n-propyle, heptafluoro-isopropyle, nonafluoro-n-butyle, 1-chlorocyclopropyle, 1-fluorocyclopropyle, 1-bromocyclopropyle, 1-trifluorométhyl-cyclopropyle et 2,2-difluoro-1-méthyl-cyclopropyle, et
Z² représente hydrogène, fluor, chlore, brome, iode, cyano, nitro, amino, méthyle, éthyle, n-propyle, 1-méthyléthyle, 1,1-diméthyléthyle, difluorométhyle, trichlorométhyle, chlorodifluorométhyle, dichlorofluorométhyle, trifluorométhyle, bromodichlorométhyle, 1-fluoroéthyle, 1-fluoro-1-méthyléthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-t-butyle, heptafluoro-n-propyle, heptafluoro-isopropyle, nonafluoro-n-butyle, méthylsulfanyle, méthylsulfinyle, méthylsulfonyle, éthylsulfanyle, éthylsulfinyle, éthylsulfonyle, trifluorométhylsulfanyle, trifluorométhylsulfinyle, trifluorométhylsulfonyle, chloro-difluorométhylsulfanyle, chloro-difluorométhylsulfinyle, chloro-difluorométhylsulfonyle, dichloro-fluorométhylsulfanyle, dichloro-fluorométhylsulfinyle, dichloro-fluorométhylsulfonyle et
Z³ représente hydrogène, méthyle, éthyle, n-propyle, isopropyle, n-butyle, isobutyle, s-butyle, t-butyle, prop-2-ényle, prop-2-inyle, but-3-inyle, difluorométhyle, trifluorométhyle, 1-fluoroéthyle, 1-fluoro-1-méthyléthyle, 2-fluoroéthyle, 2,2-difluoroéthyle, 2,2,2-trifluoroéthyle ;
lorsque T est T23 ou T24, un des radicaux Z¹, Z² ou Z³ étant substitué avec au moins 3 atomes d'halogène.

5. Composés selon l'une quelconque des revendications 1, 2, 3 ou 4, dans lesquels
Z¹ représente trifluorométhyle, 1-chlorocyclopropyle, 1-fluoro-cyclopropyle ou pentafluoroéthyle,
Z² représente trifluorométhyle, nitro, méthylsulfanyle, méthylsulfinyle, méthylsulfonyle, fluor, chlore, brome, cyano ou iode,
Z³ représente méthyle, éthyle, n-propyle ou hydrogène,
R¹ représente hydrogène, méthyle, éthyle, n-propyle, isopropyle, n-butyle, i-butyle, s-butyle, t-butyle, méthoxyméthyle, éthoxyméthyle, propoxyméthyle, méthylcarbonyle, éthylcarbonyle, n-propylcarbonyle, isopropylcarbonyle, s-butylcarbonyle, t-butylcarbonyle, méthoxycarbonyle, éthoxycarbonyle, n-propoxycarbonyle, isopropoxycarbonyle, s-butoxycarbonyle, t-butoxycarbonyle, cyanométhyle, 2-cyanoéthyle, benzyle, 4-méthoxybenzyle, pyrid-2-yl-méthyle, pyrid-3-yl-méthyle, pyrid-4-yl-méthyle, 6-chloro-pyrid-3-yl-méthyle ;
A₁ et A⁴ représentent CH et A² représente CH ou N,
A³ représente CR⁴, et
R⁴ représente fluor, chlore, brome, iode ou méthyle,
T représente un des radicaux hétéroaromatiques à 5 chaînons T1 à T35 listés ci-après, la liaison au groupe de tête pyrazole étant **caractérisée par** un astérisque, 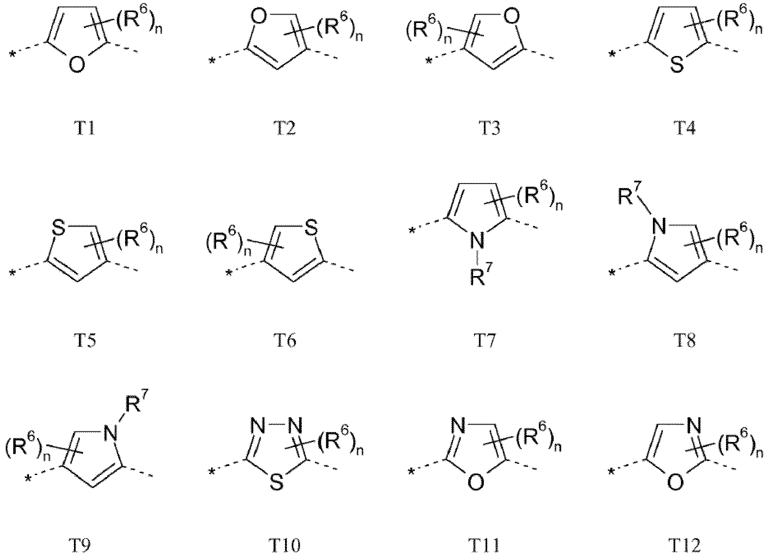 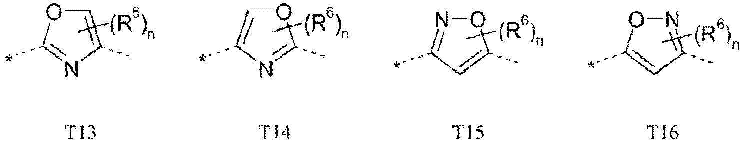 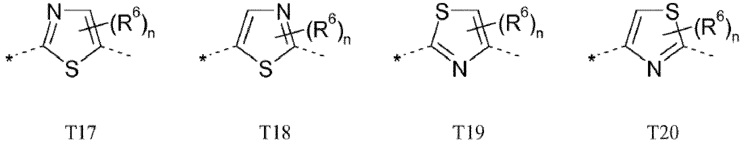 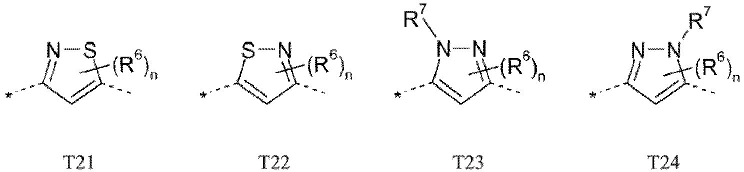 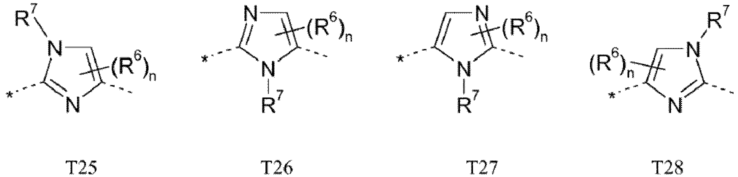 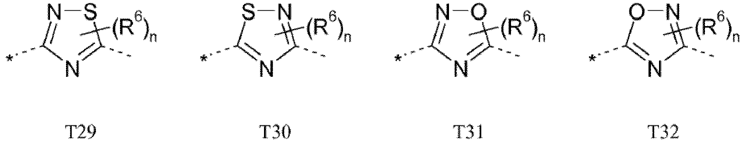 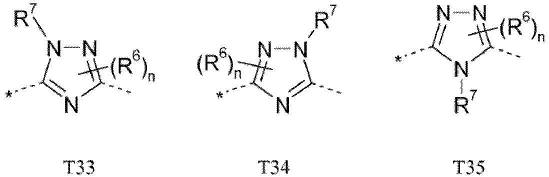 dans lesquels
les R⁶ représentent hydrogène, méthyle, éthyle, 2-méthyléthyle, 2,2-diméthyléthyle, fluor, chlore, brome, iode, nitro, trifluorométhyle, amino, et
n représente les valeurs 0 à 1 ;
W représente oxygène, et
Q représente hydrogène, méthyle, éthyle, n-propyle, 1-méthyléthyle, 1,1-diméthyléthyle, n-butyle, 1-méthylpropyle, 2-méthylpropyle, 2-méthylbutyle, hydroxyméthyle, 2-hydroxypropyle, cyanométhyle, 2-cyanoéthyle, 2-fluoroéthyle, 2,2-difluoroéthyle, 2,2,2-trifluoroéthyle, 1-trifluorométhyléthyle, 2,2-difluoropropyle, 3,3,3-trifluropropyle, 2,2-diméthyl-3-fluoropropyle, cyclopropyle, 1-cyano-cyclopropyle, 1-méthyl-cyclopropyle, 1-trifluorométhyl-cyclopropyle, 1-carbamoyl-cyclopropyle, 1-thiocarbamoyl-cyclopropyle, 1-méthoxycarbonyl-cyclopropyle, 1-(*N-*méthylcarbamoyl)cyclopropyle, 1-*(N-*cyclopropylcarbamoyl)cyclopropyle, cyclopropyl-méthyle, cyclobutyle, cyclopentyle, cyclohexyle, 1-cyclopropyléthyle, bis(cyclopropyl)méthyle, 2,2-diméthylcyclopropyl-méthyle, 2-phénylcyclopropyle, 2,2-dichlorcyclopropyle, trans-2-chlorcyclopropyle, cis-2-chlorcyclopropyle, 2,2-difluorocyclopropyle, trans-2-fluorocyclopropyle, cis-2-fluorocyclopropyle, trans-4-hydroxycyclohexyle, 4-trifluorométhylcyclohexyle, prop-2-ényle, méthylprop-2-ényle, prop-2-inyle, 1,1-diméthylbut-2-inyle, 3-chloro-prop-2-ényle, 3,3-dichloro-prop-2-ényle, 3,3-dichloro-1,1-diméthylprop-2-ényle, oxétan-3-yle, thiétan-3-yle, 1-oxydo-thiétan-3-yle, 1,1-dioxydo-thiétan-3-yle, isoxazol-3-ylméthyle, 1,2,4-triazol-3-ylméthyle, 3-méthyloxétan-3-ylméthyle, benzyle, 2,6-difluorophénylméthyle, 3-fluorophénylméthyle, 2-fluorophénylméthyle, 4-fluorophénylméthyle, 2,5-difluorophénylméthyle, 1-phényléthyle, 4-chlorophényléthyle, 2-trifluorométhylphényléthyle, 1-pyridin-2-yléthyle, pyridin-2-ylméthyle, (6-chloro-pyridin-3-yl)méthyle, 5-fluoropyridin-2-ylméthyle, pyrimidin-2-ylméthyle, méthoxy, 2-éthoxyéthyle, 2-(méthylsulfanyl)éthyle, 1-méthyl-2-(éthylsulfanyl)éthyle, 2-méthyl-1-(méthylsulfanyl)propan-2-yle, méthoxycarbonyle, méthoxycarbonylméthyle, NH₂, *N*-éthylamino, *N*-allylamino, *N*,*N*-diméthylamino, *N,N*-diéthylamino ; ou
Q représente un phényle, un naphtyle, une pyridazine, une pyrazine, une pyrimidine, une triazine, une pyridine, un pyrazole, un thiazole, un isothiazole, un oxazole, un isoxazole, un triazole, un imidazole, un furane, un thiophène, un pyrrole, un oxadiazole, un thiadiazole substitué avec 0, 1, 2 ou 3 substituants V, les V représentant indépendamment les uns des autres fluor, chlore, brome, iode, cyano, nitro, méthyle, éthyle, difluorométhyle, trichlorométhyle, chlorodifluorométhyle, dichlorofluorométhyle, trifluorométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-tert-butyle, heptafluoro-n-propyle, heptafluoro-isopropyle, nonafluoro-n-butyle, cyclopropyle, cyclobutyle, méthoxy, éthoxy, n-propoxy, 1-méthyléthoxy, fluorométhoxy, difluorométhoxy, chloro-difluorométhoxy, dichloro-fluorométhoxy, trifluorométhoxy, 2,2,2-trifluoroéthoxy, 2-chloro-2,2-difluoroéthoxy, pentafluoroéthoxy, *N-*méthoxyiminométhyle, 1-(*N*-méthoxyimino)-éthyle, méthylsulfanyle, méthylsulfonyle, méthylsulfinyle, trifluorométhylsulfonyle, trifluorométhylsulfinyle, trifluorométhylsulfanyle, *N,N*-diméthylamino ;
lorsque T est T23 ou T24, un des radicaux Z¹, Z² ou Z³ étant substitué avec au moins 3 atomes d'halogène.

6. Composés selon l'une quelconque des revendications 1 à 5, et selon l'une quelconque des formules générales (Iaa) à (Ibi) suivantes :

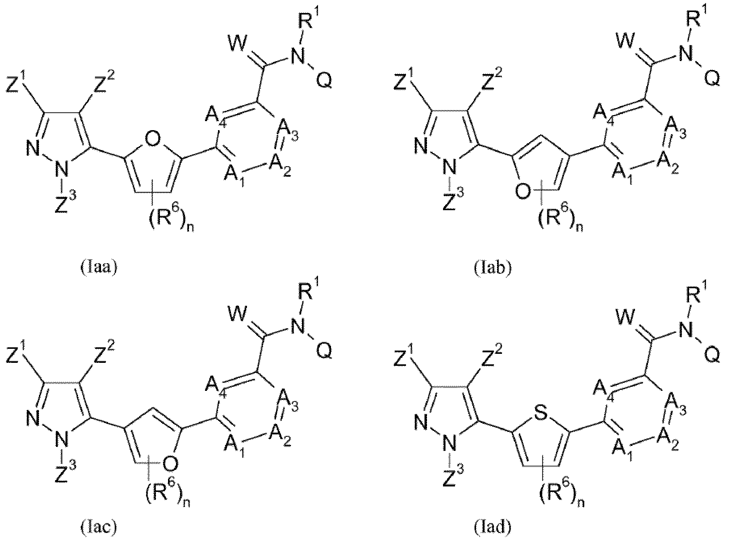

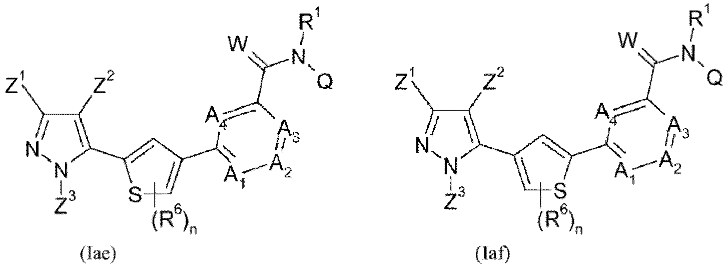

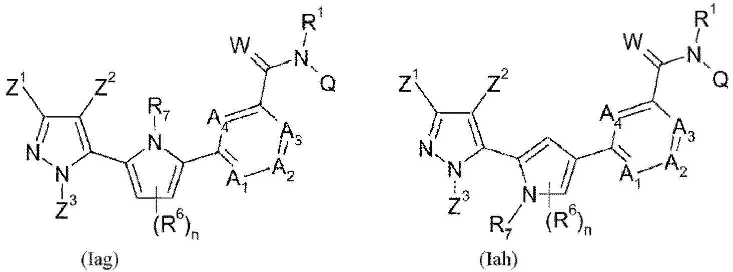

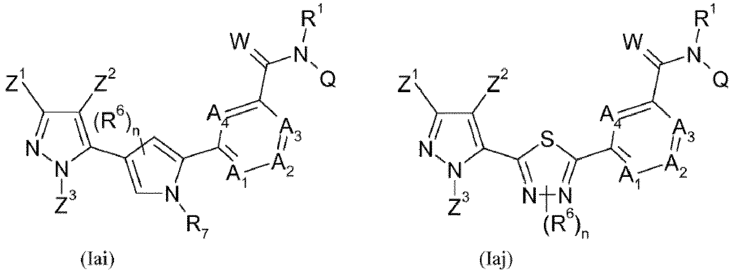

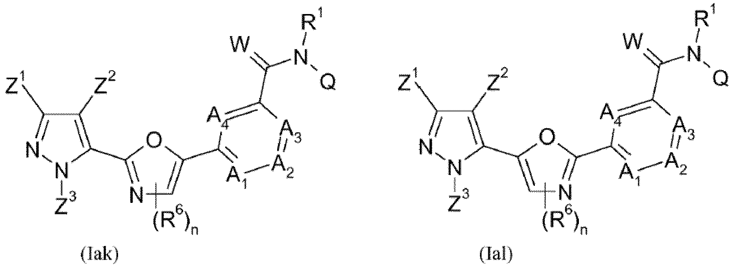

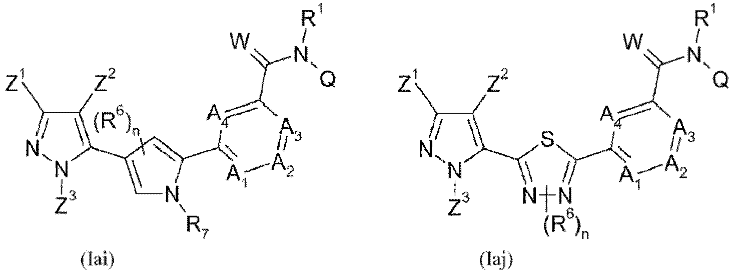

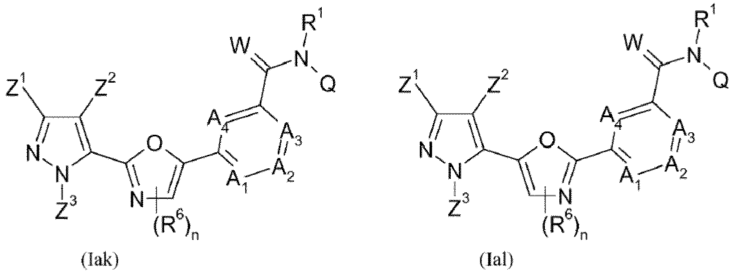

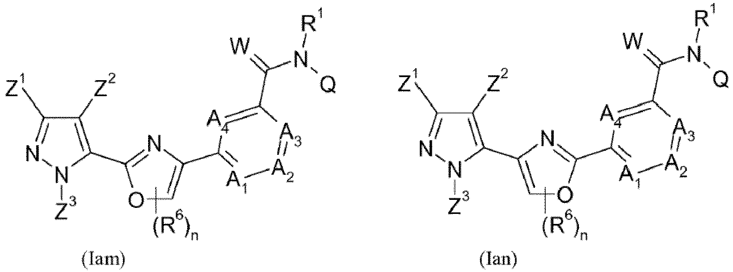

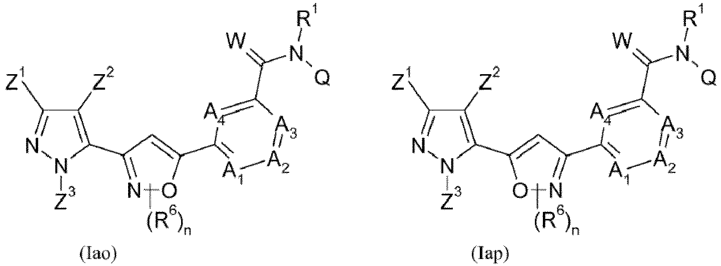

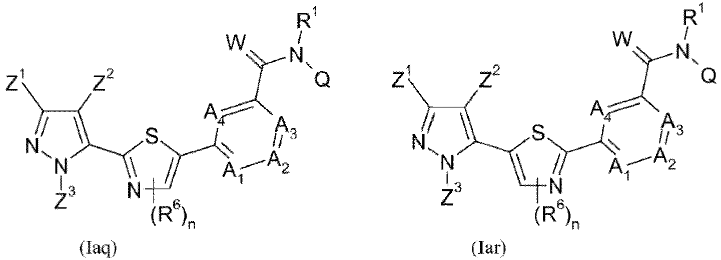

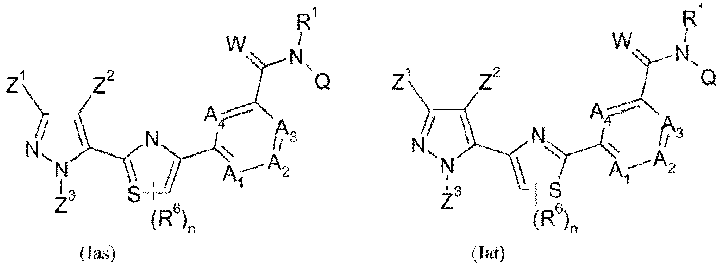

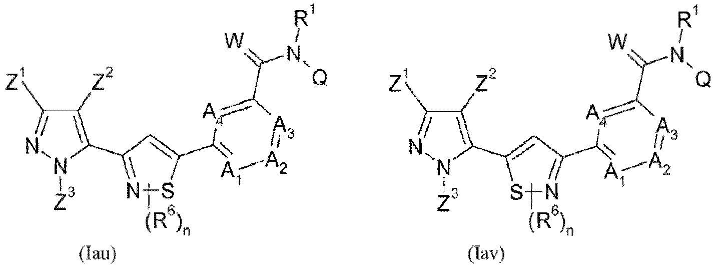

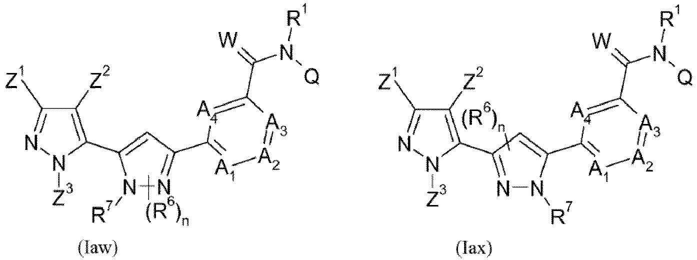

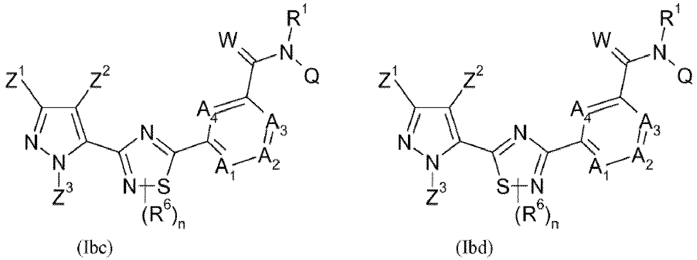

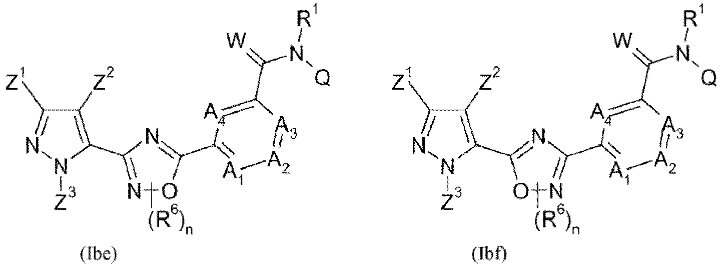

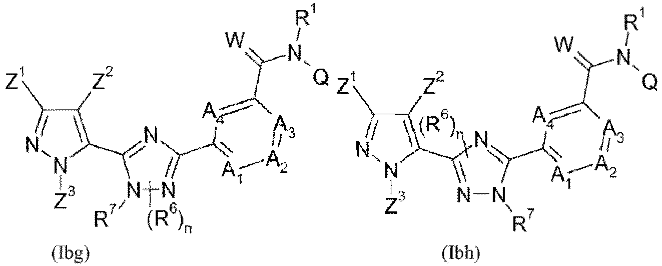

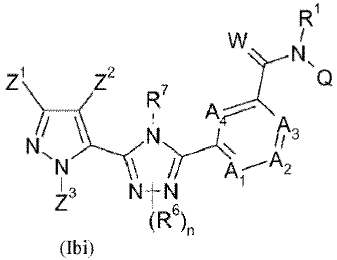

7. Composés selon la revendication 6, dans lesquels Z¹ représente CF₂CF₃, Z² représente CF₃, Z³ représente CH₃, les radicaux R¹ et R⁶ représentent l'hydrogène, A¹ et A⁴ représentent CH, et A² représente CH ou N, A³ représente C-Cl, W représente l'oxygène et Q représente 1-cyano-cyclopropyle ou cyclopropyle.

8. Utilisation de composés de formule générale (I) selon l'une quelconque des revendications 1 à 5, ainsi que des composés selon la revendication 6 ou 7 pour lutter contre des insectes, des araignées et des nématodes, le traitement chirurgical, thérapeutique et diagnostique du corps humain ou animal étant exclu.

9. Compositions pharmaceutiques, contenant au moins un composé selon l'une quelconque des revendications 1 à 7.

10. Composés selon l'une quelconque des revendications 1 à 7, destinés à une utilisation en tant que médicament.

11. Utilisation de composés selon l'une quelconque des revendications 1 à 7 pour la fabrication de compositions pharmaceutiques pour lutter contre des parasites sur des animaux.

12. Procédé de fabrication d'agents phytoprotecteurs contenant des composés selon l'une quelconque des revendications 1 à 7, ainsi que des extendeurs et/ou substances tensioactives usuels.

13. Procédé de lutte contre des nuisibles, **caractérisé en ce qu'**un composé selon l'une quelconque des revendications 1 à 7 est laissé agir sur les nuisibles et/ou leur habitat, le traitement chirurgical, thérapeutique et diagnostique du corps humain ou animal étant exclu.

14. Utilisation de composés selon l'une quelconque des revendications 1 à 7 pour la protection du matériel reproductif de plantes.
